# EUROPEAN PATENT APPLICATION

(11) **EP 4 382 519 A1**
(43) Date of publication of application: **12.06.2024**
(21) Application number: 22860571.3
(22) Date of filing: 25.08.2022
(51) Int. Cl.: C07D 401/02, C07D 401/14, A61K 31/506, A61P 35/00, A61P 31/12, A61P 29/00, A61P 37/00

(54) **CYCLIN K DEGRADATION AGENT**

(30) Priority: 27.08.2021 CN 202110994191
(71) Applicant: Adlai Nortye Biopharma Co., Ltd., Yuhang District Hangzhou Zhejiang 311121 (CN)
(72) Inventor: XU, Beidi, Hangzhou, Zhejiang 311121 (CN); QIU, Qingchong, Hangzhou, Zhejiang 311121 (CN); YU, Zhiyong, Hangzhou, Zhejiang 311121 (CN); HE, Nanhai, Hangzhou, Zhejiang 311121 (CN); JI, Bin, Hangzhou, Zhejiang 311121 (CN); ZHANG, Ling, Hangzhou, Zhejiang 311121 (CN); WANG, Xiaohan, Hangzhou, Zhejiang 311121 (CN); XIA, Xiangyu, Hangzhou, Zhejiang 311121 (CN); SUN, Zhao, Hangzhou, Zhejiang 311121 (CN)
(74) Representative: Michalski Hüttermann & Partner Patentanwälte mbB
(86) International application number: PCT/CN2022/114693
(87) International publication number: WO 2023/025225

(57) **Abstract**

The invention provides a compound of formula (I) and pharmaceutical compositions thereof. The compound of formula (I) of the present invention can be used as a degradation agent of cyclin K (CyclinK protein), and can be used to prevent or treat diseases related to CyclinK protein.

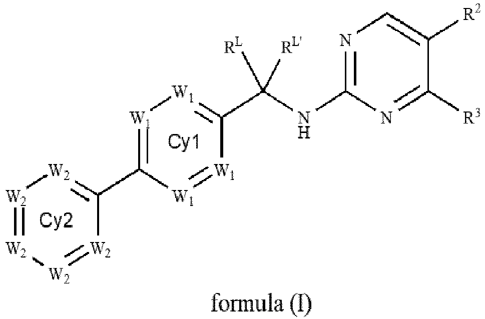

## Description

### Technical field

The present invention relates to a compound that degrades cyclin K, and methods of using the same to treat/prevent cyclin K-related disorders.

### Background

Tumor is the second largest killer threatening human health. About 10 million people die from tumors around the world every year. The abnormal expression of cellular proteins is considered to be an important factor leading to the occurrence and development of tumors, so most drugs target these abnormally expressed proteins. Compared with traditional inhibitor-based drug development, drug-induced protein degradation is a new strategy for targeting these tumor-associated proteins. According to the mechanism of action, protein degron can be divided into three categories, namely proteolysis targeting chimera (PROTAC), monovalent degron and molecular glue degron (Burslem, G. M. & Crews, C. M. Chem. Rev. 117, 11269-11301 (2017).). In recent years, technology based on protein degradation has been successfully used in the development of anti-tumor drugs. It is expected that at least 15 protein degradation drugs will enter clinical practice by the end of 2021.

PROTAC is currently the most widely used protein degradation technology, usually consisting of a protein targeting binding region, an E3 ubiquitin ligase recruitment region and a linker. PROTAC molecules bind to target proteins and recruit E3 ligases, which ubiquitinate the target proteins and ultimately lead to the degradation of the target proteins. Due to the large molecular weight of PROTACs (usually between 700-1200), their membrane permeability and oral bioavailability are poor. The molecular weight of monovalent degron and molecular glue is much smaller than PROTAC, which is more consistent with Lipinski's five principles (den Besten, W. etal. Nat Chem Biol 16, 1157-1158 (2020)). Monovalent degron induces degradation by binding to the protein and changing its conformation or other changes. Molecular glue ultimately leads to the degradation of the target protein by inducing the interaction between Culin-RING E3 ligase and the target protein. Molecular glue-mediated degradation of target proteins can be independent of the ligand pocket of the target protein, such as thalidomide analogs (Simonetta, K.R. etal. Nat Commun 10, 1402 (2019)) and arylsulfonamide analogs (Baek, K. etal. Nat Chem Biol 16, 2-3 (2020)) that have been reported so far and so on were developed according to this type of mechanism. Therefore, molecular glues can bring new hope to targets that were previously difficult to drug due to the lack of suitable ligand pockets.

CCNK is cell cycle protein K, also known as Cyclin K. It is the most important cyclin of cyclin-dependent kinase 12/13 (CDK12/13) (Pawel Lukasik,eral.Int J Mol Sci. 2021 Mar; 22(6) ): 2935). It can participate in the regulation of multiple biological processes such as transcription, post-transcriptional modification, cell cycle, and cell proliferation by forming a complex with CDK12/13. Early studies have shown that CDK12/13 forms a complex with Cyclin K and phosphorylates the C-terminal domain of RNA polymerase II to regulate its activity, thereby regulating the expression of DNA damage repair genes, such as BRCA1, ATR, FANC1, etc. (Malgorzata Krajewska et al. Nat Commun. 2019 Apr 15;10(1):1757.). CDK12/13 is considered a potential tumor treatment target (Cells 2020, 9 (6), 1483;). By designing a degron targeting Cyclin K, it affects the formation of the complex between CDK12/13 and Cyclin K protein, which provides a new idea for inhibiting the functions of CDK12/13. In 2020, Benjamin L. Ebert et al. reported (Nature 2020, 585, 293-297.) that the CDK inhibitor CR8 induces the degradation of Cyclin K protein through the mechanism of a molecular glue degrader. However, there are currently few molecular glue-based degradation agents for Cyclin K and they are unsatisfactory. It is urgent to find compounds with good degradation activity for Cyclin K.

### Contents of the invention

The invention provides a class of compounds with good Cyclin K degradation activity, and the use of the compound and its pharmaceutically acceptable salts, prodrugs, isotope derivatives, isomers, solvents or metabolites thereof, and pharmaceutical compositions containing the compound in the treatment or prevention of cyclin K related diseases.

In one aspect, the present invention provides a compound having the structure of formula (I) or a pharmaceutically acceptable salt, prodrug, isotope derivative, isomer, solvate or metabolite thereof: 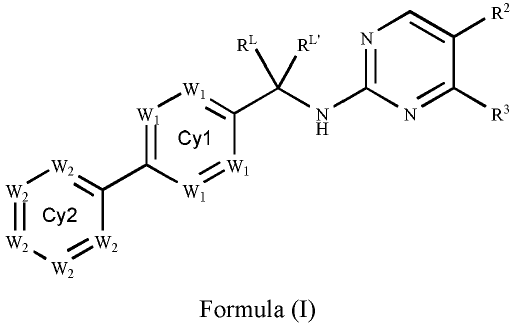
wherein, Cy¹ and Cy² each independently represent a 6-membered aromatic ring or a 6-membered heteroaromatic ring;
wherein, R^{L} and R^{L'} each independently represent hydrogen, C₁-C₆ alkyl or C₃-C₆ cycloalkyl, and R^{L} and R^{L'} can form a 3-6 membered ring together with the carbon atoms connected to them;
wherein, Wi is each independently selected from CR⁰ or N;
wherein, R⁰ represents hydrogen, halogen, nitro, cyano, -R^{a}, -OR^{a}, -SR^{a}, -NR^{a}R^{b}, -C(O)R^{a}, -C(O)OR^{a}, -C(O)NR^{a}R^{b}, -S(O)₂R^{a}, -NR^{a} C(O)R^{b}, -S(O)₂NR^{a}, -S(O)R^{a}, -P(O)R^{a}R^{b};
wherein, W₂ is each independently selected from CR¹ or N;
wherein, R¹ each independently represents hydrogen, halogen, nitro, cyano, -R^{a}, -OR^{a}, -SR^{a}, -NR^{a}R^{b}, -C(O)R^{a}, -C(O)OR^{a}, -C(O)NR^{a}R^{b}, - NR^{a} C(O)R^{b}, -S(O)₂R^{a}, -S(O)R^{a}, -S(O)₂NR^{a}R^{b}, -P(O)R^{a}R^{b}, and C₁-C₆ alkyl, (C₂-C₆) alkenyl, (C₂-C₆) alkynyl optionally substituted by 0, 1, 2, or 3 substituents selected from OR^{a}, SR^{a}, NR^{a}R^{b}, NR^{a} C(O)R^{b}, C(O)R^{a}, C(O)OR^{a}, C(O)NR^{a}R^{b}, S(O)₂R^{a}, S(O)R^{a}, S(O)₂NR^{a}R^{b}, P(O)R^{a}R^{b};
wherein, R² represents halogen, -R^{a}, -OR^{a}, -SR^{a}, nitro, cyano, -NR^{a}R^{b}, -NR^{a} C(O)R^{b}, -C(O)R^{a}, -C(O)OR^{a}, -C(O )NR^{a}R^{b}, -S(O)₂R^{a}, -S(O)R^{a}, -S(O)₂NR^{a}R^{b}, -P(O)R^{a}R^{b}, (C₂-C₆) alkenyl, (C₂-C₆) alkynyl;
wherein, R³ represents C₁-C₆ alkyl, C₁-C₆ alkenyl, C₁-C₆ alkynyl, C₃-C₁₀ cycloalkyl, 3-10 membered heterocycloalkyl, C₆-C₁₀ membered aryl, 5-10 membered heteroaryl, -NR^{M}R^{N}, -NHR^{M}, -OR^{M};
when R³ represents C₁-C₆ alkyl, C₁-C₆ alkenyl, C₁-C₆ alkynyl, C₃-C₁₀ cycloalkyl, 3-10 membered heterocycloalkyl, it may optionally be substituted by 0, 1, 2, 3 of the following substituents: oxo, nitro, halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, -(C₀-C₆ alkylene)P(O)R^{a}R^{b};
when R³ represents a C₆-C₁₀-membered aryl or a 5-10-membered heteroaryl, it may optionally be substituted by 0, 1, 2, or 3 of the following substituents: nitro, halogen, cyano, - R^{a}, - (C₀-C₆ alkylene)OR^{a}, -(C₀-C₆ alkylene)SRa, -(C₀-C₆ alkylene)NR^{a}R^{b}, - (C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, -(C₀-C₆ alkylene)P(O)R^{a}R^{b};
when R³ represents -NR^{M}R^{N}, -NHR^{M}, -OR^{M}, R^{M} and R^{N} each independently represent C₁-C₆ alkyl, -(C₀-C₆ alkylene)(C₃-C₁₀ cycloalkyl), -(C₀-C₆ alkylene) (3-10 membered heterocycloalkyl), -(C₀-C₆alkylene) (C₆-C₁₀ membered aryl), -(C₀-C₆ alkylene) (5-10 membered heteroaryl);
wherein, R^{M} and R^{N} can optionally be substituted by 0, 1, 2, or 3 of the following substituents: oxo, nitro, halogen, cyano, -R^{a}, -(C₀-C₆ alkylene)OR^{a}, -(C₀-C₆ alkylene)SR^{a}, -(C₀-C₆ alkylene)NR^{a}R^{b}, - (C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, -(C₀-C₆ alkylene)P(O)R^{a}R^{b};
wherein, R^{a} and R^{b} each independently represent hydrogen, C₁-C₆ alkyl or C₃-C₈ cycloalkyl, which may optionally be substituted by 0, 1, 2 or 3 halogen atoms;
provided that the compound of formula (I) does not include

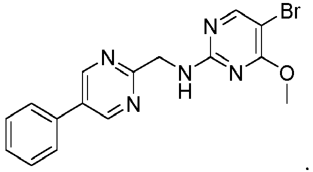

In another embodiment, R² is halo, trifluoromethyl or cyano.

In another embodiment, Cy1 is selected from 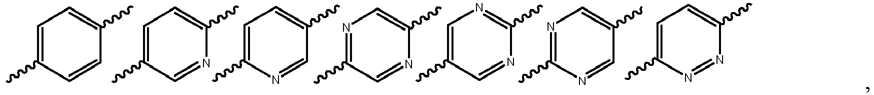 preferably Cy1 is selected from 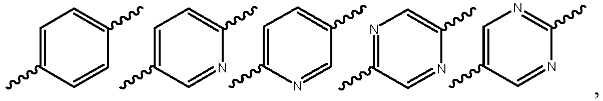 wherein the wavy line indicates that Cy1 is connected to the site in formula (I); wherein, the Cy1 is optionally substituted by 0, 1, 2 or 3 R⁰; preferably the Cy1 is substituted by 0 R⁰.

In another embodiment, Cy2 is selected from 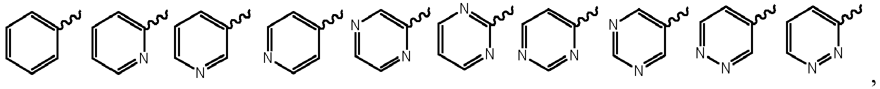 preferably Cy2 is selected from 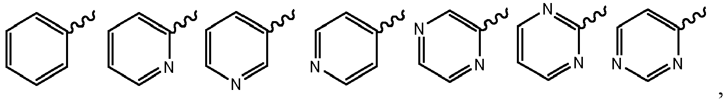 wherein the wavy line indicates that Cy2 is connected to the site in formula (I); wherein, the Cy2 is optionally substituted by 0, 1, 2 or 3 R¹; preferably, the Cy2 is substituted by 0 R¹; preferably, Cy2 is substituted by 1 or 2 R¹.

In yet another embodiment, R⁰ is hydrogen, halogen, -R^{a}, -OR^{a}, -SR^{a}.

In another embodiment, R¹ each independently represents hydrogen, halogen, -R^{a}, -OR^{a}, -NR^{a}R^{b}, -C(O)R^{a}, -C(O)OR^{a}, -C(O)NR^{a}R^{b}, -NRaC( O)R^{b}, -S(O)₂R^{a}, -S(O)R^{a}, -P(O)R^{a}R^{b}, -(C₂-C₆)alkenyl, -(C₂-C₆)alkynyl, -(C₁-C₆ alkylene)OR^{a}, -(C₁-C₆ alkylene)NR^{a}R^{b}, -(C₁-C₆ alkylene)NR^{a}C(O)R^{b}, -(C₁-C₆ alkylene)C(O)R^{a}, -(C₁-C₆ alkylene)C(O)OR^{a}, -(C₁-C₆ alkylene)C(O)NR^{a}R^{b}, -(C₁-C₆ alkylene)S(O)₂R^{a}, -(C₁-C₆ alkylene)S(O)R^{a}, -(C₁-C₆ alkylene)P(O)R^{a}R^{b}.

In yet another embodiment, R³ is C₁-C₆ alkyl, C₁-C₆ alkenyl, C₁-C₆ alkynyl, C₃-C₁₀ cycloalkyl, 3-10 membered heterocycloalkyl, which may optionally be substituted by 0 , 1, 2, 3 of the following substituents: nitro, halogen, cyano, -R^{a}, -(C₀-C₆ alkylene)OR^{a}, -(C₀-C₆ alkylene)SR^{a}, -(C₀-C₆ alkylene) NR^{a}R^{b}, - NR^{a} C(O)R^{b}, -C(O)R^{a}, -C(O)OR^{a}, -C(O)NR^{a}R^{b}, -S(O)₂R^{a}, -S(O)R^{a}, - S(O)₂NR^{a}R^{b}, -P(O)R^{a}R^{b}; preferably, R³ may be optionally substituted by 0, 1, 2, or 3 of the following substituents: halogen, -R^{a}, -(C₀-C₆ alkylene) OR^{a} or -(C₀-C₆ alkylene)SR^{a}, -(C₀-C₆ alkylene)NR^{a}R^{b}.

In another embodiment, R³ is a C₆-C₁₀-membered aryl or a 5-10-membered heteroaryl, which may be optionally substituted by 0, 1, 2, or 3 of the following substituents: nitro, halogen, cyano , -R^{a}, -(C₀-C₆ alkylene)OR^{a}, -(C₀-C₆ alkylene)SR^{a}, -(C₀-C₆ alkylene)NR^{a}R^{b}, -NR^{a}C(O)R^{b}, -C(O)R^{a}, -C(O)OR^{a}, -C(O)NR^{a}R^{b}, -S(O)₂R^{a}, -S(O)R^{a}, -S(O)₂NR^{a}R^{b}, -P(O)R^{a}R^{b}; preferably, the R³ may be optionally substituted by 0, 1, 2, 3 of the following substituents: halogen, -R^{a}, -(C₀-C₆ alkylene)OR^{a}, -(C₀-C₆ alkylene)SR^{a}, -(C₀-C₆ alkylene)NR^{a}R^{b}, -NR^{a} C(O)R^{b}, -C(O)R^{a}, -C(O)OR^{a}, -C(O)NR^{a}R^{b}, -S(O)₂R^{a}, -S(O)R^{a}, -S (O)₂NR^{a}R^{b} or -P(O)R^{a}R^{b}.

In yet another embodiment, R³ is -NR^{M}R^{N}, -NHR^{M}, -OR^{M}, and R^{M} and R^{N} each independently represent C₁-C₆ alkyl, -(C₀-C₆ alkylene) (C₃-C₁₀ cycloalkyl), - (C₀-C₆ alkylene)(3-10 membered heterocycloalkyl), -(C₀-C₆ alkylene)(C₆-C₁₀ aryl), -(C₀-C₆ alkylene) (5-10 heteroaryl); wherein, R^{M} and R^{N} may be optionally substituted by 0, 1, 2, or 3 of the following substituents: oxo, nitro, halogen, cyano, -R^{a}, -OR^{a}, -SR^{a}, - NR^{a}R^{b}, - NR^{a}C(O)R^{b}, -C(O)R^{a}, -C(O)OR^{a}, -C(O)NR^{a}R^{b}, -S(O)₂R^{a}, -S(O)R^{a}, -S(O)₂NR^{a}R^{b}, -P(O)R^{a}R^{b}; more preferably, the R^{M} and R^{N} may optionally be substituted by 0, 1, 2, 3 of the following substituents: oxo, -R^{a}, -OR^{a}, -SR^{a}, -NR^{a}R^{b}, - NR^{a}C(O)R^{b}, -C(O)R^{a}, -C(O)OR^{a}, -C(O)NR^{a}R^{b}, -S(O)₂R^{a}, -S(O)R^{a}, -S(O)₂NR^{a}R^{b} or -P(O)R^{a}R^{b}.

In one embodiment, R^{a} and R^{b} each independently represent hydrogen, C₁-C₃ alkyl or C₃-C₆ cycloalkyl, which may optionally be substituted by 0, 1, 2 or 3 halogen atoms.

In one embodiment, R^{a} and R^{b} each independently represent hydrogen, C₁-C₃ alkyl, which may optionally be substituted by 0, 1, 2, or 3 halogen atoms. Preferably, the compound described in any of the above embodiments is selected from the following structures:

| | | | |
|---|---|---|---|
| 1 | 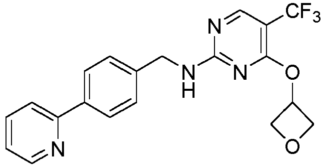 | 2 | 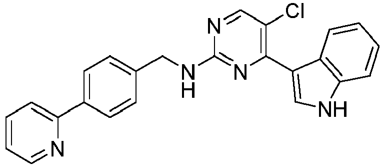 |
| 3 | 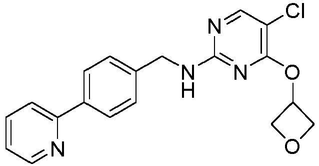 | 4 | 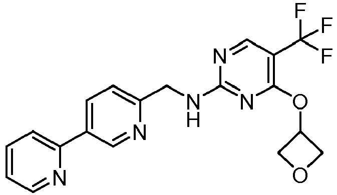 |
| 5 | 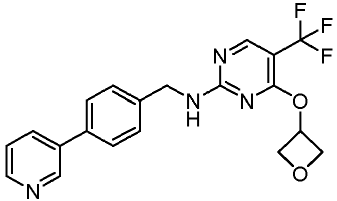 | 6 | 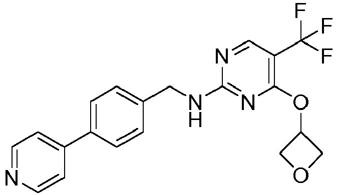 |
| 7 | 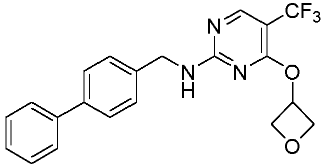 | 8 | 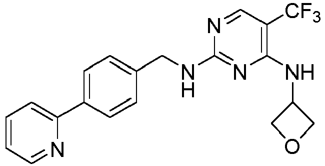 |
| 9 | 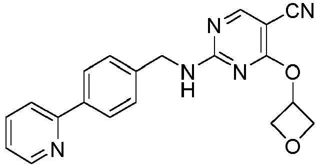 | 10 | 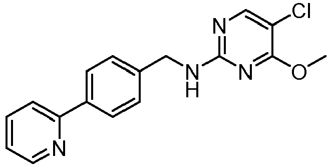 |
| 11 | 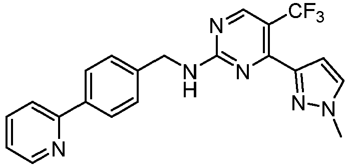 | 12 | 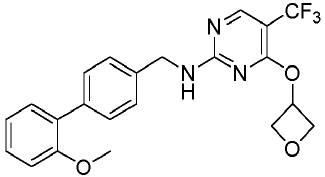 |
| 13 | 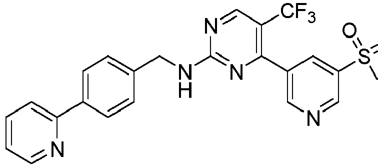 | 14 | 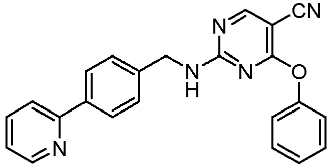 |
| 15 | 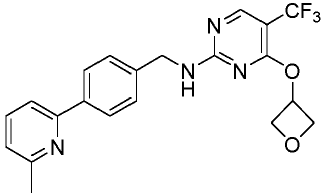 | 16 | 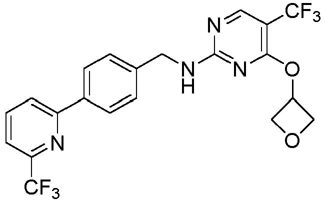 |
| 17 | 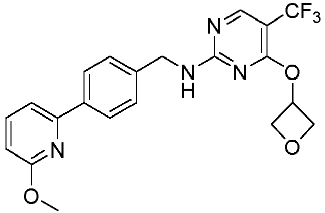 | 18 | 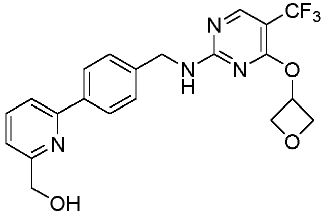 |
| 19 | 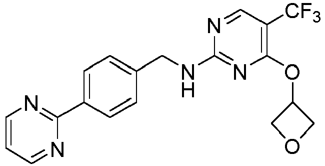 | 20 | 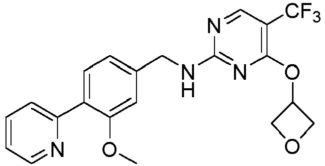 |
| 21 | 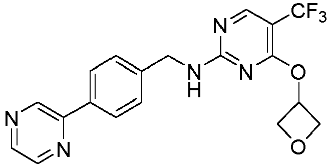 | 22 | 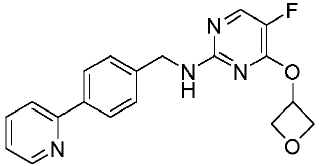 |
| 23 | 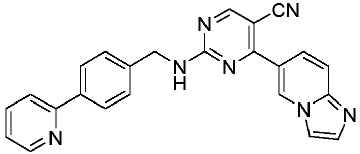 | 24 | 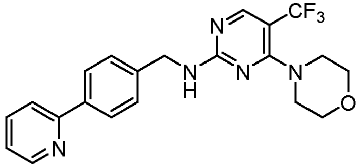 |
| 25 | 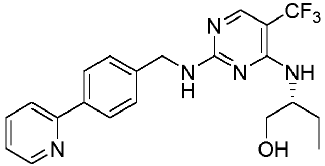 | 26 | 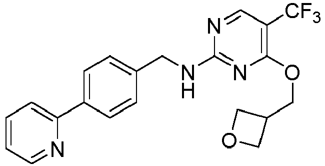 |
| 27 | 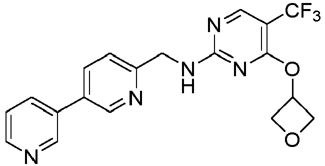 | 28 | 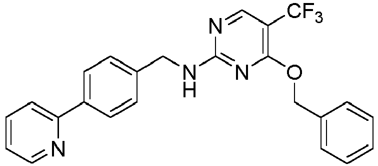 |
| 29 | 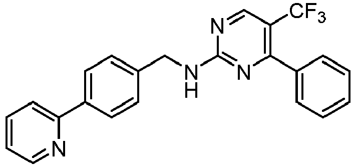 | 30 | 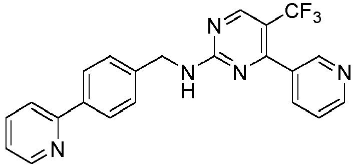 |
| 31 | 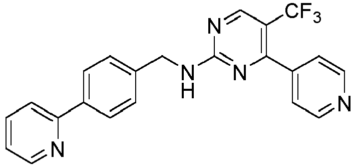 | 32 | 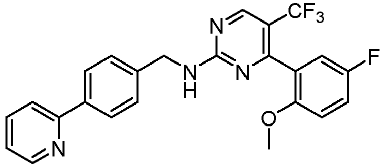 |
| 33 | 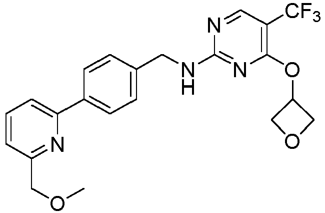 | 34 | 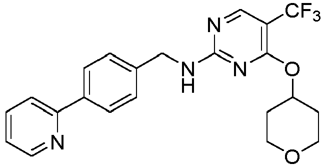 |
| 35 | 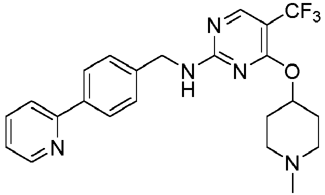 | 36 | 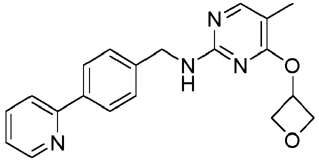 |
| 37 | 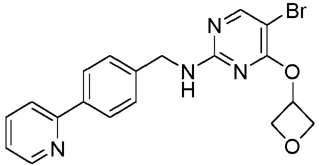 | 38 | 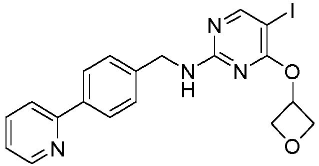 |
| 39 | 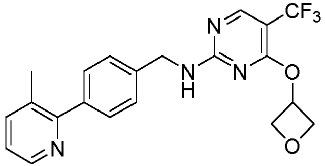 | 40 | 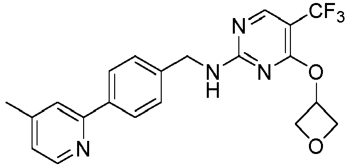 |
| 41 | 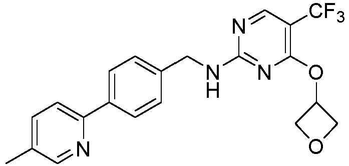 | 42 | 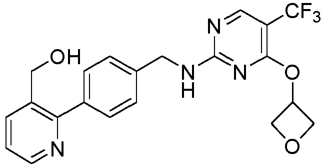 |
| 43 | 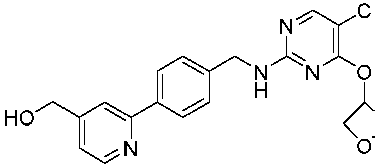 | 44 | 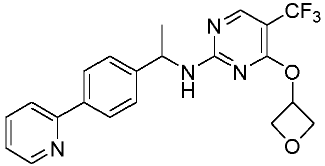 |
| 45 | 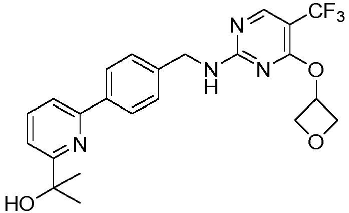 | 46 | 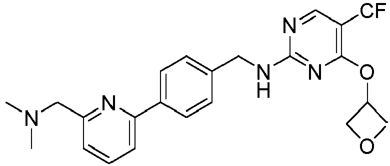 |
| 47 | 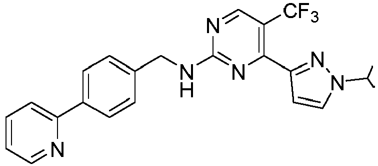 | 48 | 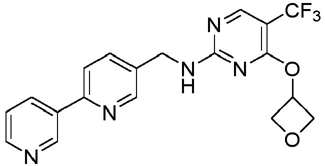 |
| 49 | 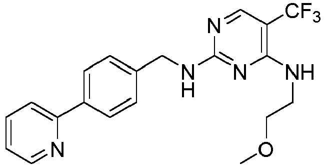 | 50 | 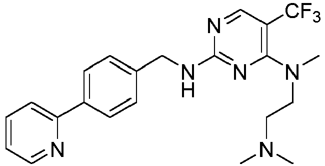 |
| 51 | 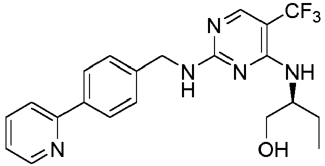 | 52 | 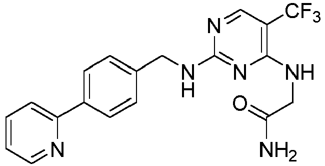 |
| 53 | 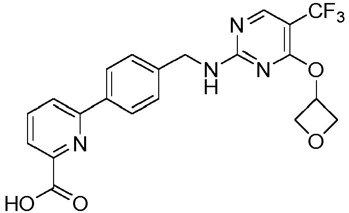 | 54 | 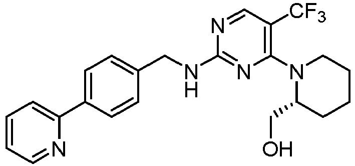 |
| 55 | 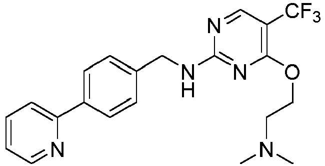 | 56 | 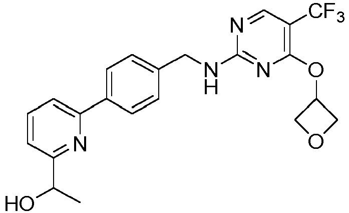 |
| 57 | 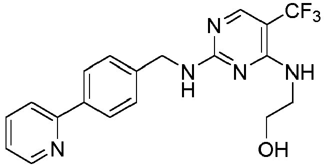 | 58 | 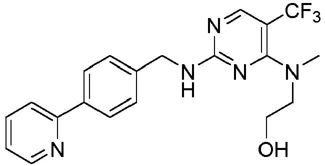 |
| 59 | 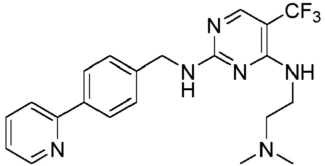 | 60 | 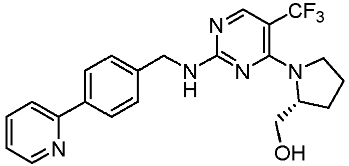 |
| 61 | 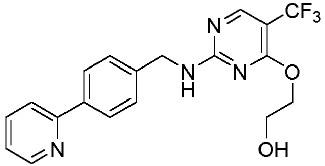 | 62 | 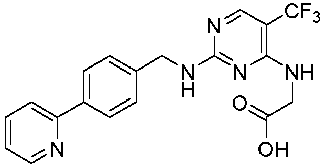 |
| 63 | 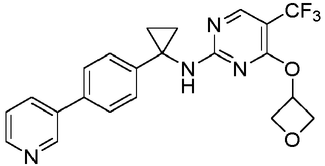 | 64 | 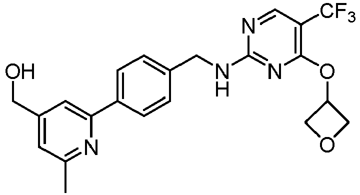 |
| 65 | 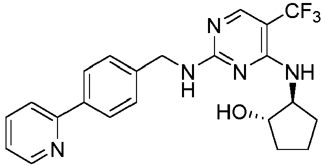 | 66 | 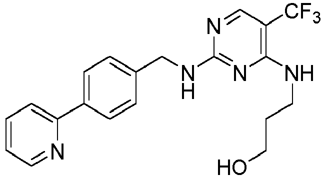 |
| 67 | 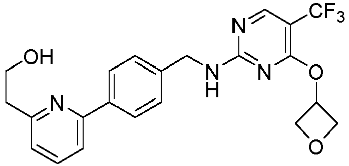 | 68 | 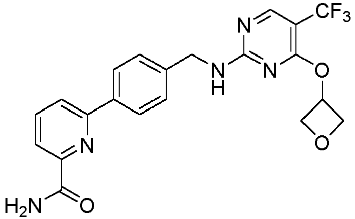 |
| 69 | 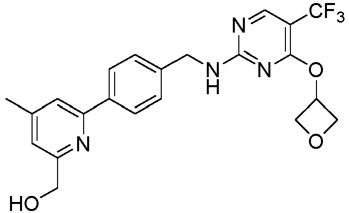 | 70 | 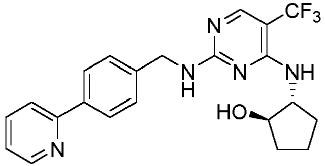 |
| 71 | 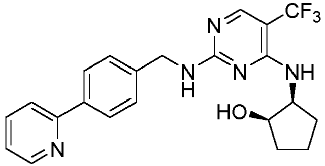 | 72 | 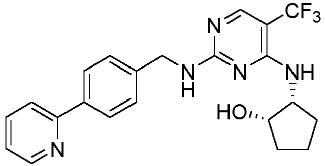 |
| 73 | 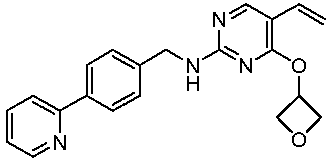 | 74 | 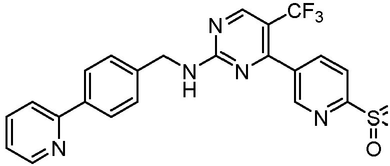 |
| 75 | 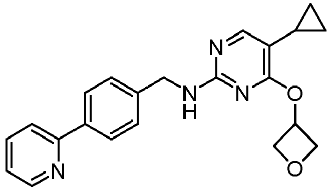 | 76 | 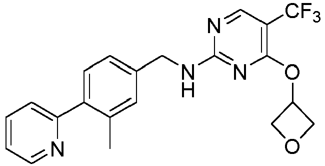 |
| 77 | 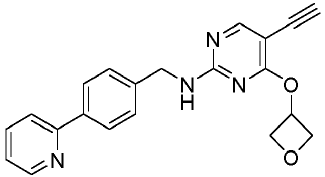 | 78 | 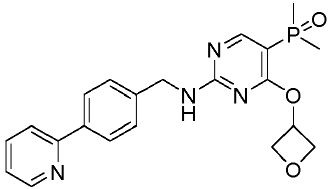 |
| 79 | 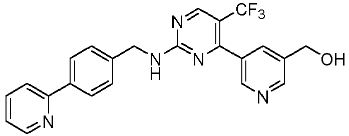 | 80 | 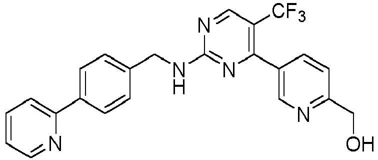 |
| 81 | 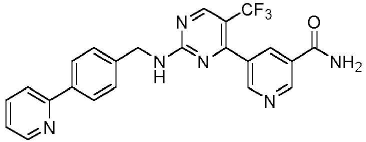 | 82 | 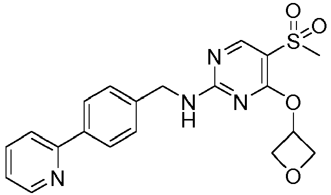 |
| 83 | 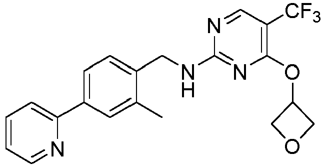 | 84 | 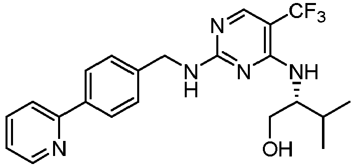 |
| 85 | 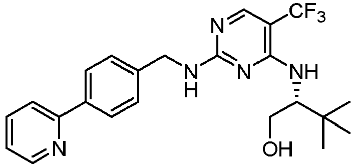 | 86 | 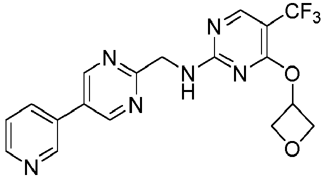 |
| 87 | 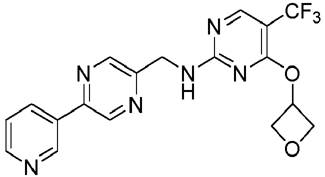 | 88 | 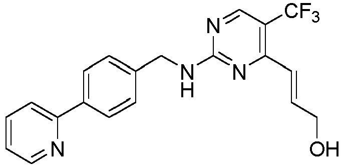 |
| 89 | 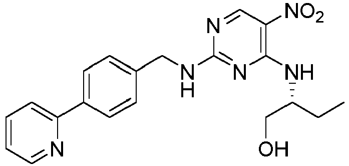 | 90 | 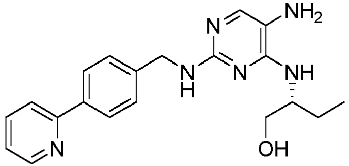 |
| 91 | 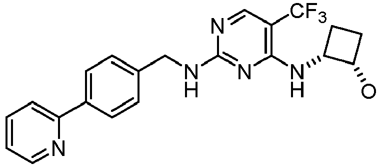 | 92 | 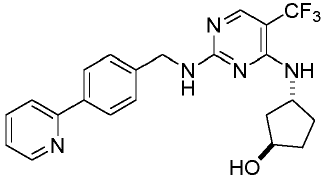 |
| 93 | 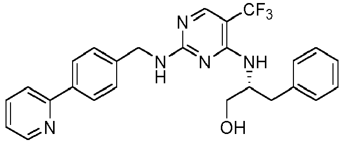 | 94 | 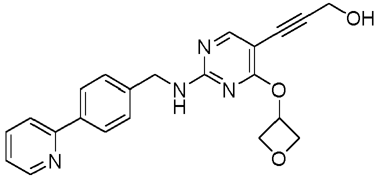 |
| 95 | 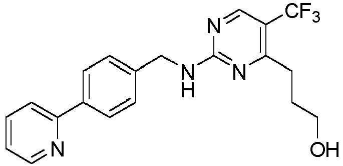 | 96 | 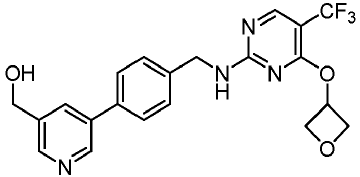 |
| 97 | 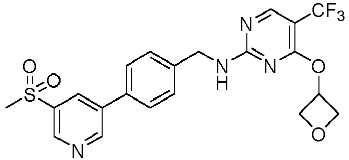 | 98 | 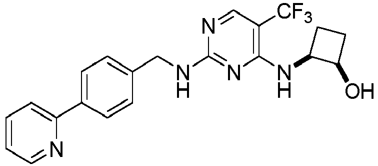 |
| 99 | 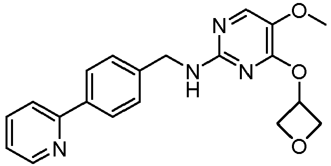 | 10 0 | 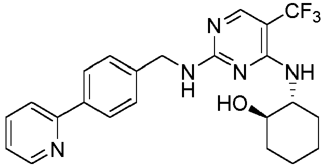 |
| 10 1 | 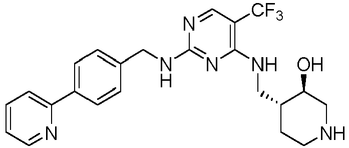 | 10 2 | 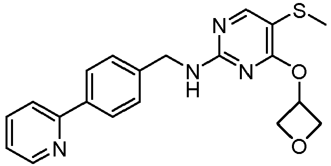 |
| 10 3 | 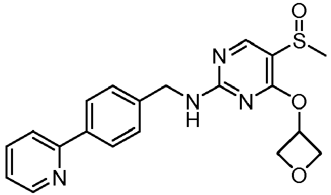 | 10 4 | 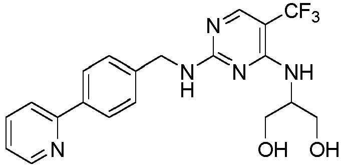 |
| 10 5 | 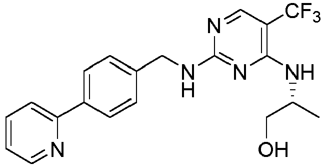 | 10 6 | 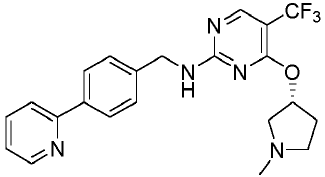 |
| 10 7 | 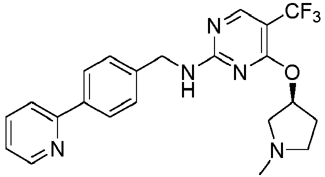 | 10 8 | 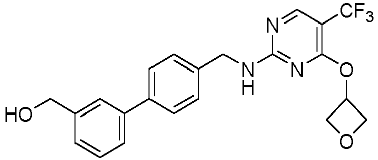 |
| 10 9 | 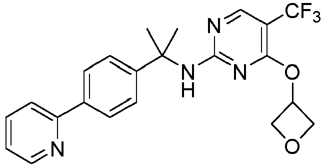 | 11 0 | 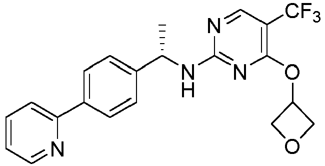 |
| 11 1 | 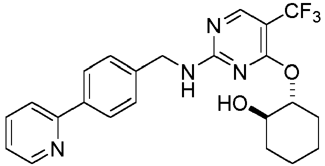 | 11 2 | 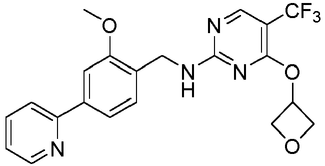 |
| 11 3 | 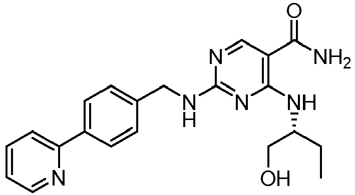 | 11 4 | 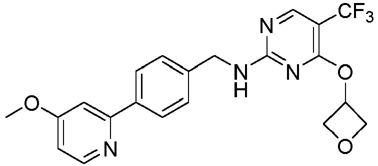 |
| 11 5 | 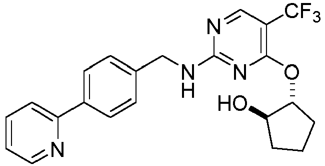 | 11 6 | 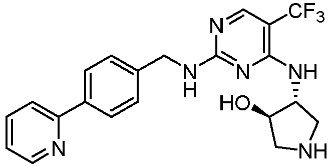 |
| 11 7 | 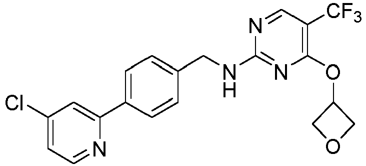 | 11 8 | 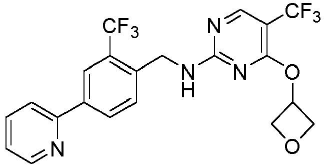 |
| 11 9 | 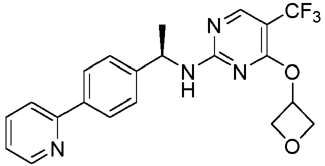 | 12 0 | 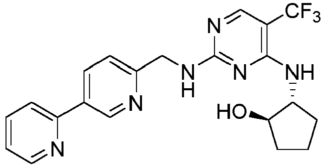 |
| 12 1 | 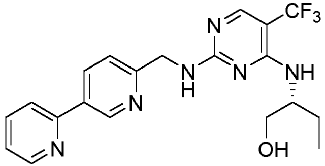 | 12 2 | 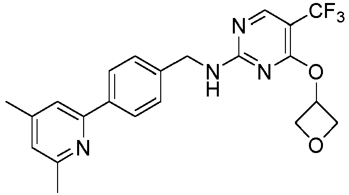 |
| 12 3 | 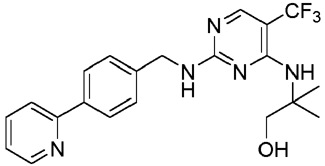 | 12 4 | 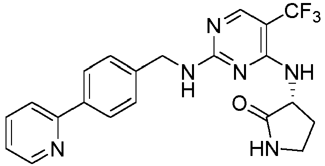 |
| 12 5 | 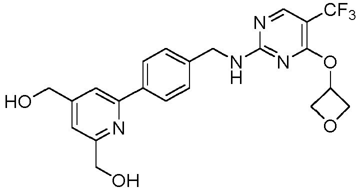 | 12 6 | 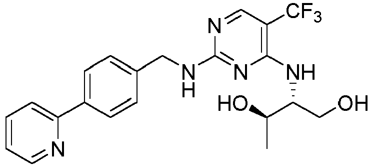 |
| 12 7 | 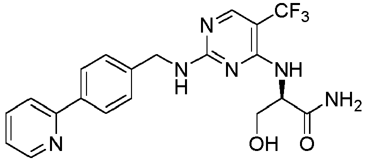 | 12 8 | 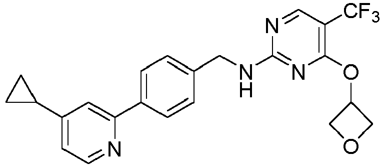 |
| 12 9 | 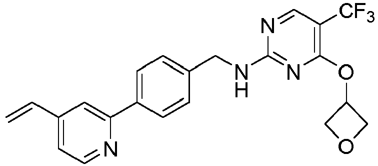 | 13 0 | 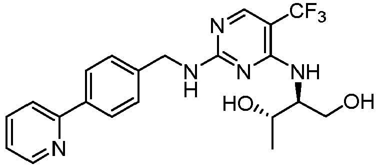 |
| 13 1 | 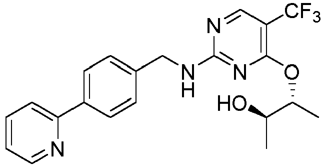 | 13 2 | 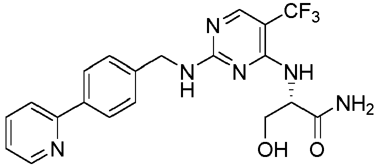 |
| 13 3 | 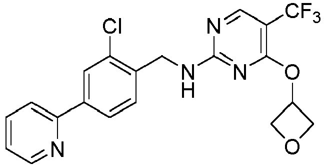 | 13 4 | 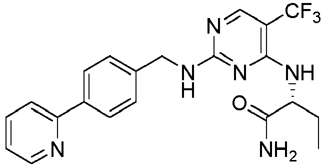 |
| 13 5 | 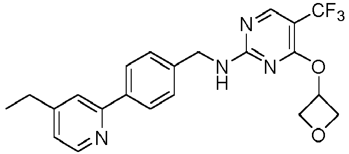 | 13 6 | 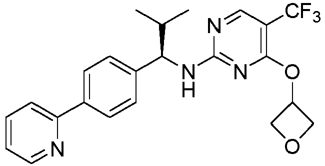 |
| 13 7 | 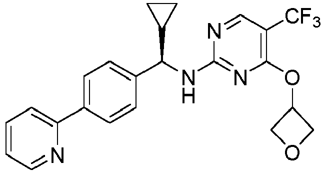 | 13 8 | 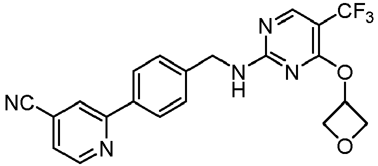 |
| 13 9 | 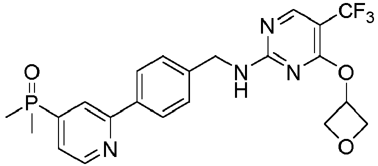 | 14 0 | 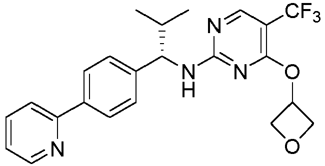 |
| 14 1 | 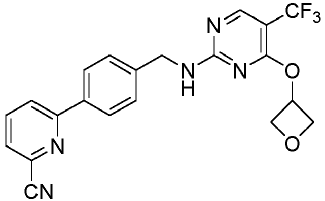 | 14 2 | 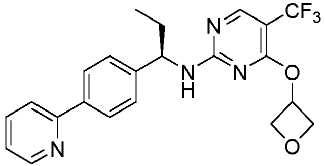 |
| 14 3 | 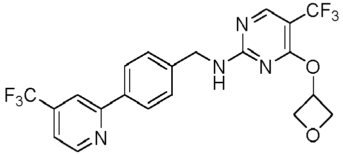 | 14 4 | 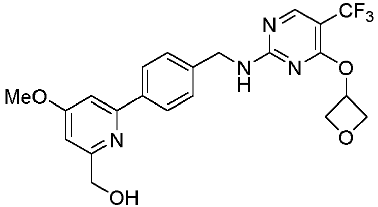 |
| 14 5 | 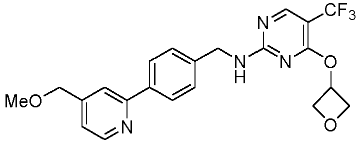 | 14 6 | 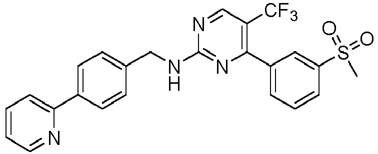 |
| 14 7 | 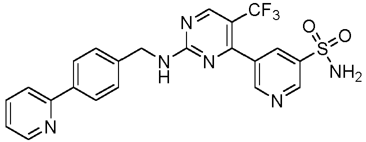 | 14 8 | 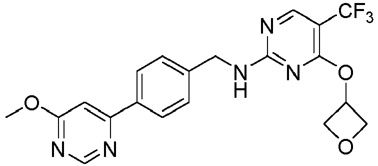 |
| 14 9 | 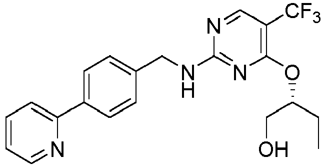 | 15 0 | 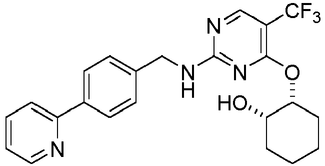 |
| 15 1 | 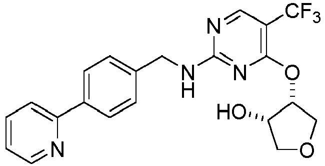 | 15 2 | 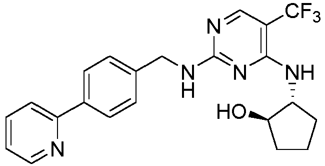 |
| 15 3 | 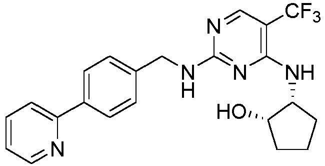 | 15 4 | 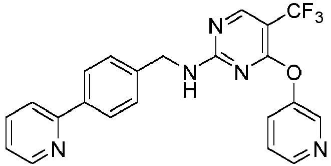 |
| 15 5 | 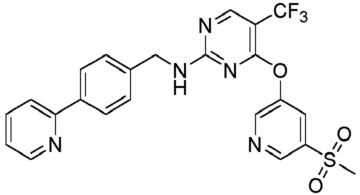 | 15 6 | 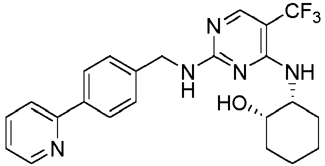 |
| 15 7 | 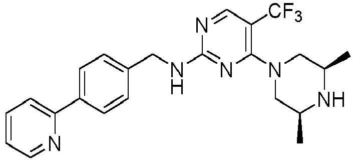 | 15 8 | 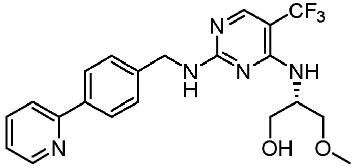 |
| 15 9 | 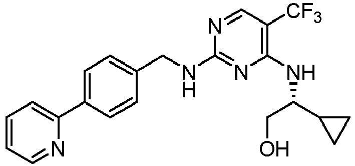 | 16 0 | 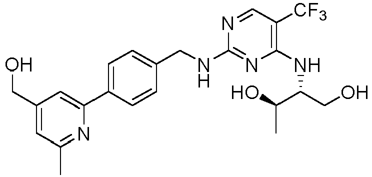 |
| 16 1 | 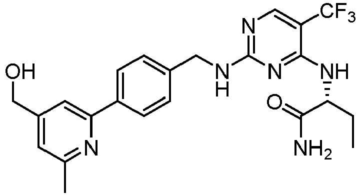 | 16 2 | 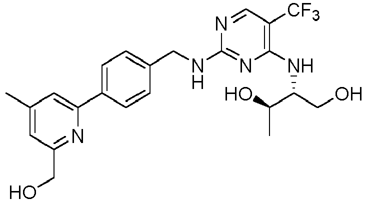 |
| 16 3 | 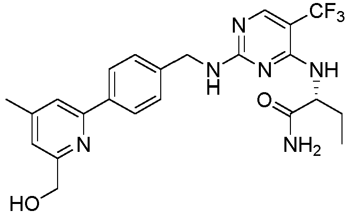 | 16 4 | 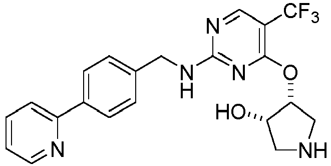 |
| 16 5 | 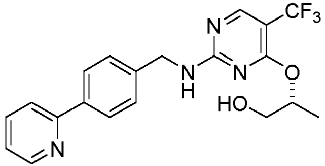 | 16 6 | 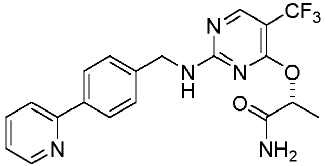 |
| 16 7 | 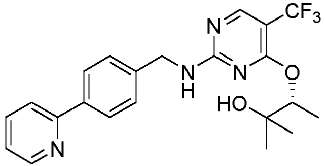 | 16 8 | 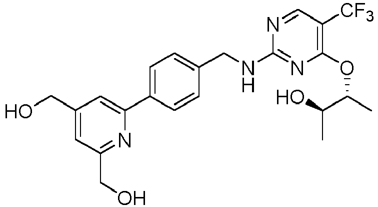 |
| 16 9 | 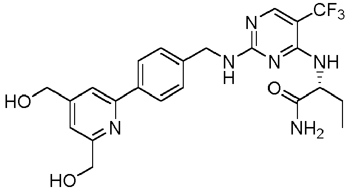 | 17 0 | 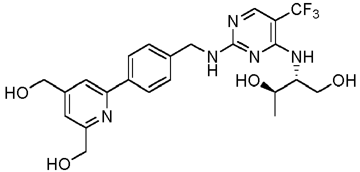 |
| 17 1 | 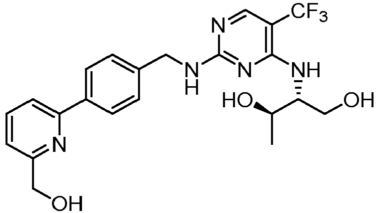 | 17 2 | 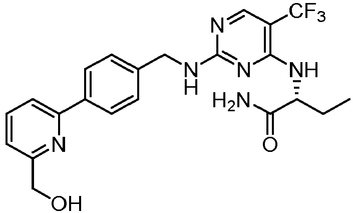 |
| 17 3 | 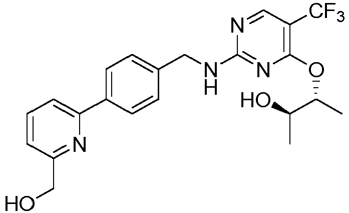 | 17 4 | 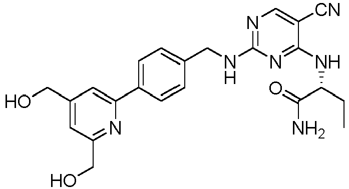 |
| 17 5 | 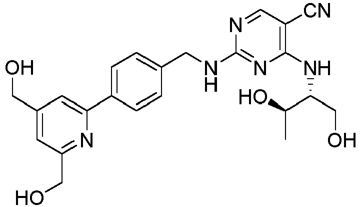 | 17 6 | 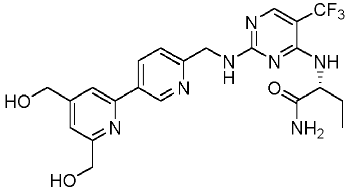 |
| 17 7 | 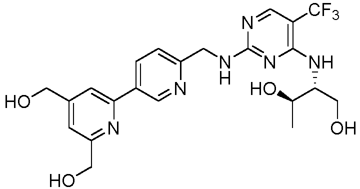 | 17 8 | 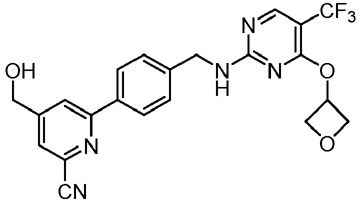 |
| 17 9 | 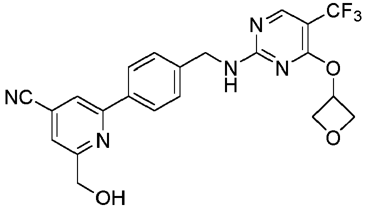 | 18 0 | 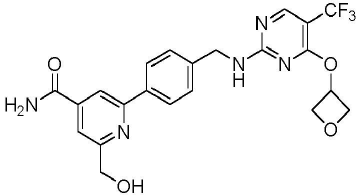 |
| 18 1 | 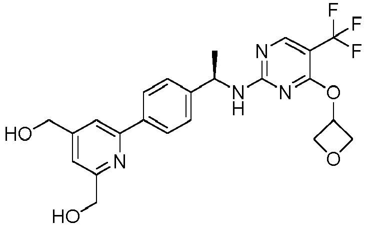 | 18 2 | 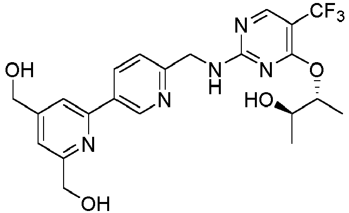 |
| 18 3 | 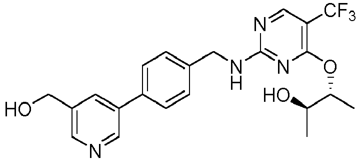 | 18 4 | 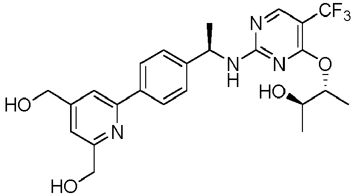 |
| 18 5 | 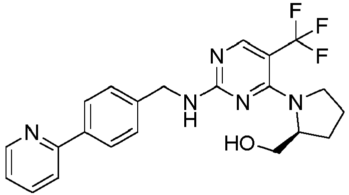 | 18 6 | 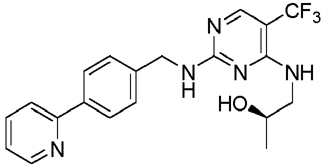 |
| 18 7 | 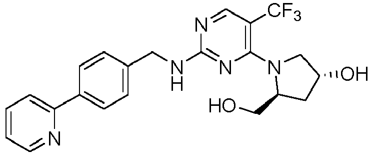 | 18 8 | 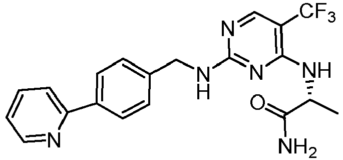 |
| 18 9 | 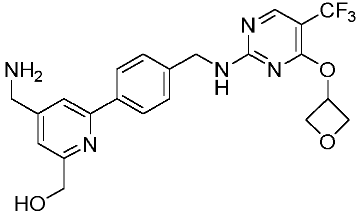 | 19 0 | 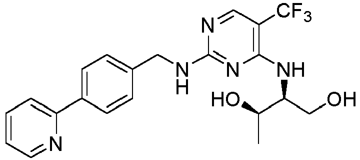 |
| 19 1 | 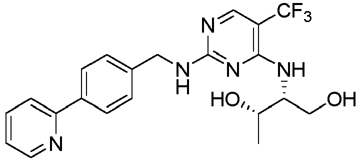 | 19 2 | 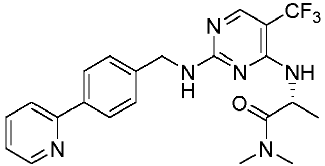 |
| 19 3 | 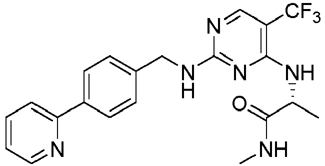 | 19 4 | 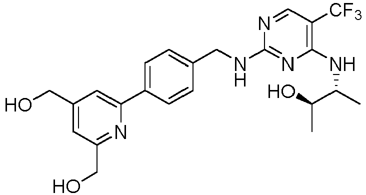 |
| 19 5 | 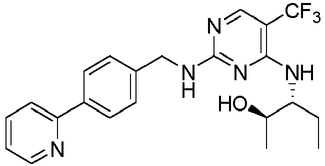 | 19 6 | 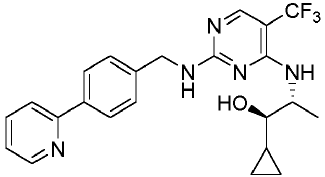 |
| 19 7 | 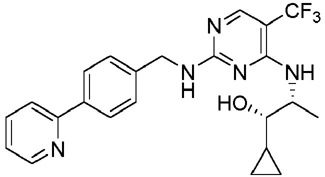 | 19 8 | 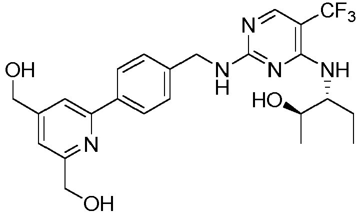 |
| 19 9 | 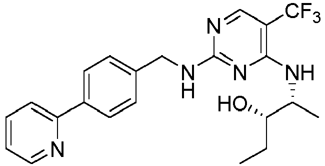 | 20 0 | 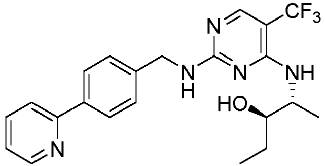 |
| 20 1 | 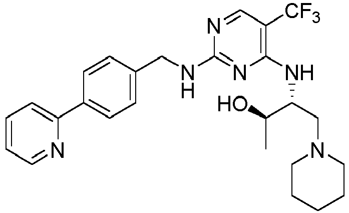 | 20 2 | 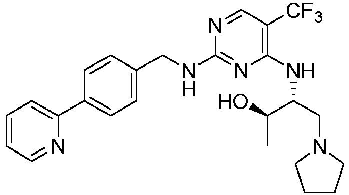 |
| 20 3 | 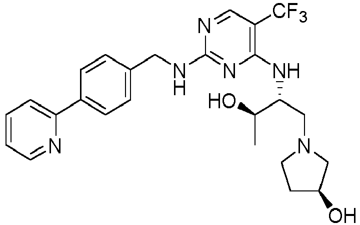 | 20 4 | 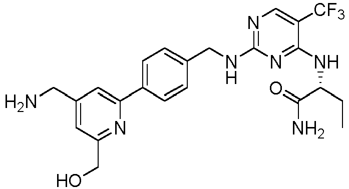 |
| 20 5 | 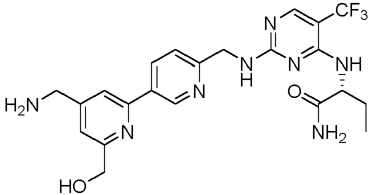 | 20 6 | 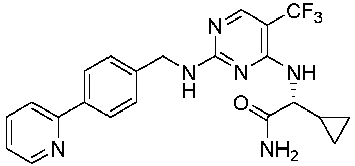 |
| 20 7 | 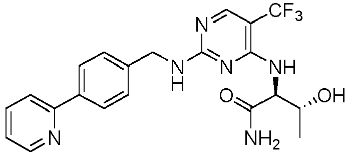 | 20 8 | 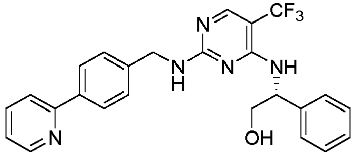 |
| 20 9 | 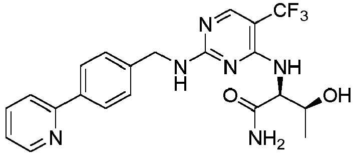 | 21 0 | 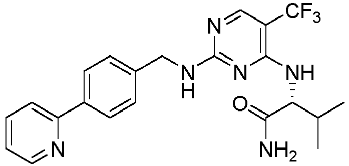 |
| 21 1 | 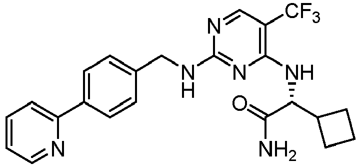 | 21 2 | 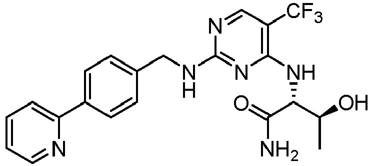 |
| 21 3 | 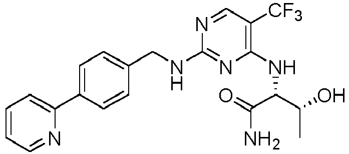 | 21 4 | 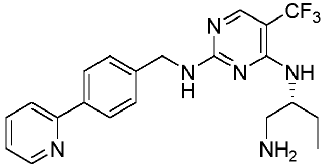 |
| 21 5 | 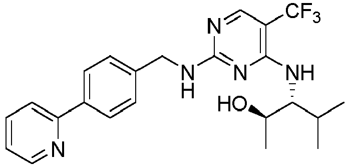 | 21 6 | 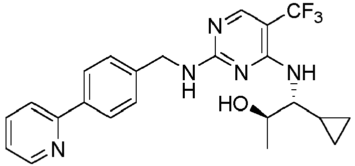 |
| 21 7 | 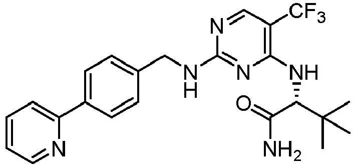 | 21 8 | 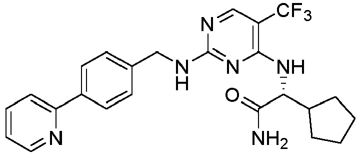 |
| 21 9 | 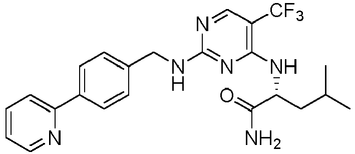 | 22 0 | 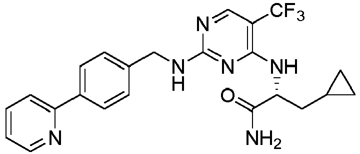 |
| 22 1 | 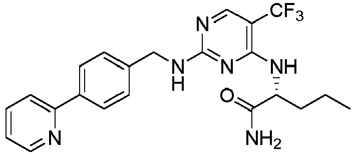 | 22 2 | 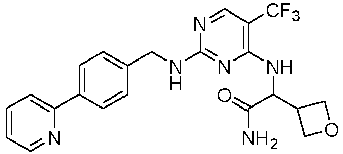 |
| 22 3 | 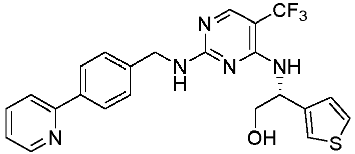 | 22 4 | 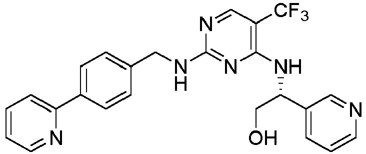 |

Another embodiment of the present invention provides a pharmaceutical composition comprising a compound according to the present invention or a pharmaceutically acceptable salt, prodrug, isotopic derivative, isomer, solvate or a pharmaceutically acceptable salt thereof. metabolites, and optionally a pharmaceutically acceptable carrier.

Another embodiment of the present invention provides use of the compound described in the present invention or a pharmaceutically acceptable salt, prodrug, isotope derivative, isomer, solvate or metabolite thereof, or the pharmaceutical composition described in the present invention in the preparation of a medicament for preventing or treating diseases or disorders related to CyclinK protein. In particular, the disease or disorder is selected from tumors, cancers, viral infections, inflammation-related diseases and autoimmune diseases.

Another embodiment of the present invention provides a method for treating diseases or disorders related to Cyclin K protein, which comprises administering the compound of the present invention or a pharmaceutically acceptable salt or prodrug, isotope derivative, isomer, solvate or metabolite thereof, or the pharmaceutical composition of the present invention to a mammal in need thereof.

### Description of the drawings

Figure 1: Inducing effect of compound 1 of the present invention on the degradation of Cyclin K.
Figure 2: Inducing effect of multiple compounds of the present invention on the degradation of Cyclin K

### Embodiments

The compound of the present invention or its prodrug can have excellent Cyclin K degradation activity in a living body, and can be used as a preventive or therapeutic drug for cancer, a cancer proliferation inhibitor, or a cancer metastasis inhibitor. The compounds of the present invention exhibit Cyclin K degradation activity, and the compounds of the present invention are expected to perform well in terms of efficacy expression, pharmacokinetics (eg, absorption, distribution, metabolism, excretion), solubility (eg, water solubility), and interaction with other pharmaceutical products (for example, inhibition of drug metabolizing enzymes) and stability (for example, chemical stability, stability against enzymes), thus the compound can be used as a drug.

The compounds of the present invention are expected to have low toxicity (e.g., acute toxicity, chronic toxicity, genotoxicity, reproductive toxicity, cardiotoxicity, carcinogenicity, central nervous system toxicity) and can be used to administer to mammals (e.g., mice, rats, hamsters, rabbits, cats, dogs, cattle, sheep, monkeys, humans).

The compounds of the present invention can be used as preventive or therapeutic agents for pathologies or diseases caused by Cyclin K. Furthermore, the compounds of the present invention may be superior in selectivity for degrading Cyclin K in the Cyclin protein subfamily and are expected to have low toxicity.

The compounds of the present invention are expected to be useful in the prevention or treatment of, for example, cancer [e.g., colorectal cancer (e.g., colon cancer, rectal cancer, anal cancer, familial colorectal cancer, hereditary nonpolyposis colorectal cancer, gastrointestinal interstitial cancer tumor), lung cancer (e.g., non-small cell lung cancer, small cell lung cancer, malignant mesothelioma), mesothelioma, pancreatic cancer (e.g., pancreatic duct cancer, pancreatic endocrine tumor), pharyngeal cancer, laryngeal cancer, esophagus carcinoma, gastric cancer (e.g., papillary adenocarcinoma, mucinous adenocarcinoma, adenosquamous carcinoma), duodenal cancer, small bowel cancer, breast cancer (e.g., invasive ductal carcinoma, ductal carcinoma in situ, inflammatory breast cancer), Ovarian cancer (e.g., ovarian epithelial cancer, extragonadal germ cell tumor, ovarian germ cell tumor, ovarian neoplasm of low malignant potential), testicular cancer, prostate cancer (e.g., hormone-dependent prostate cancer, hormone-independent prostate cancer, castration resistant prostate cancer), liver cancer (e.g., hepatoma, primary liver cancer, extrahepatic cholangiocarcinoma), thyroid cancer (e.g., medullary thyroid carcinoma), renal cancer (e.g., renal cell carcinoma (e.g., clear cell type) renal cell carcinoma), transitional cell carcinoma in the renal pelvis and ureter), uterine cancer (e.g., cervical cancer, uterine corpus cancer, uterine sarcoma), pregnancy choriocarcinoma, brain tumors (e.g., medulloblastoma, neuroblastoma stromal tumor, pineal astrocytoma, fibrous astrocytoma, diffuse astrocytoma, anaplastic astrocytoma, pituitary adenoma), neuroblastoma, retinoblastoma, skin cancer (For example, basal sarcoma, malignant melanoma (melanoma)), sarcoma (eg, rhabdomyosarcoma, leiomyosarcoma, soft tissue sarcoma, spindle cell sarcoma, osteosarcoma), malignant bone tumor, bladder cancer, blood cancer (eg, multiple Myeloma, leukemia (e.g., acute myeloid leukemia, acute lymphoblastic leukemia), malignant lymphoma, Hodgkin's disease, chronic myeloproliferative disorder), cancer of unknown primary], inhibiting the spread of cancer, inhibiting metastasis, promoting apoptosis, or preventing or treating precancerous lesions (e.g., myelodysplastic syndrome). Furthermore, it is contemplated that the compounds of the present invention may be useful in the prevention or treatment of scleroderma, cirrhosis, idiopathic pulmonary fibrosis, inflammatory bowel disease or muscular dystrophy.

The compounds of the present invention may be administered to mammals, preferably humans, as such or as pharmaceuticals mixed with a pharmaceutically acceptable carrier. Suitable routes of administration include, but are not limited to, oral, intravenous, rectal, aerosol, parenteral, ocular, pulmonary, transdermal, vaginal, and ear canal administration, nasal administration and topical administration. In addition, by way of example only, parenteral administration includes intramuscular injection, subcutaneous injection, intravenous injection, intramedullary injection, ventricular injection, intraperitoneal injection, intralymphatic injection, and intranasal injection. Medicaments containing compounds of the present invention (sometimes simply referred to as "medicines of the present invention") are described in detail below. Examples of dosage forms of the medicament of the present invention include oral preparations, such as tablets (e.g., sugar-coated tablets, film-coated tablets, sublingual tablets, buccal tablets, oral quick-dissolving tablets), pills, granules, powders, capsules (e.g., soft capsules, microcapsules), syrups, emulsions, suspensions, films (for example, orally disintegrating films, oral mucosa films), etc. In addition, examples of dosage forms of the medicine of the present invention include parenteral pharmaceuticals such as injections, drips, transdermal agents (for example, iontophoresis transdermal preparations), suppositories, ointments, nasal preparations, pulmonary preparations, and eye drops.

The compounds of the invention can be used concurrently with other drugs. In particular, the compounds of the present invention may be used together with drugs such as hormonal therapeutic, chemotherapeutic agents, immunotherapeutic agents, drugs that inhibit the action of cell growth factors or cell growth factor receptors. Hereinafter, drugs that can be used in combination with the compounds of the present invention are simply referred to as concomitant drugs.

Examples of the "hormone therapeutic" used include fosbestrol, diethylstilbestrol, chlorotrianisene, medroxyprogesterone acetate, megestrol acetate, chlormadinone acetate, cyproterone acetate, danazol, allylestrenol, gestrinone, mepartricin, raloxifene, ormeloxifene, levormeloxifene, anti-estrogen (e.g., tamoxifen citrate and toremifene citrate), contraceptive pills, mepitiostane, testrolactone, aminoglutethimide, LH-RH agonists (e.g., goserelin acetate, buserelin, and leuprorelin acetate), droloxifene, epitiostanol, ethinyl estradiol sulfonate, aromatase inhibitors (e.g., fadrozole hydrochloride, anastrozole, letrozole, exemestane, vorozole, and formestane), anti-androgens (e.g. , flutamide, bicalutamide, nilutamide, and enzalutamide), 5α-reductase inhibitors (e.g., finasteride, epristeride, and dutasteride), adrenal corticosteroid agents (e.g., dexamethasone, predonisolone, betamethasone, and triamcinolone), androgen synthesis inhibitors (e.g., abiraterone), retinoids and agents delaying retinoid metabolism (e.g., liarozole), thyroid hormones, and DDS (drug delivery system) preparations thereof.

As "chemotherapeutic agents" alkylating agents, antimetabolites, anticancer antibiotics and plant-derived anticancer agents can be used.

Examples of the "alkylating agent" used include nitrogen mustard, nitrogen mustard-N-oxide hydrochloride, chlorambucil, cyclophosphamide, ifosfamide, thiotepa, carboquone, improsulfan tosylate, busulfan, nimustine hydrochloride, mitobronitol, melphalan, dacarbazine, ranimustine, estramustine sodium phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, Ribomustin, temozolomide, treosulfan, trofosfamide, zinostatin stimalamer, adozelesin, cystemustine, bizelesin, and DDS preparations thereof.

Examples of the "antimetabolite" used include mercaptopurine, 6-mercaptopurine riboside, thioinosine, methotrexate, pemetrexed, enocitabine, cytarabine, cytarabine ocfosfate, ancitabine hydrochloride, 5-FU drugs (e.g., fluorouracil, tegafur, UFT, doxifluridine, carmofur, galocitabine, emitefur, and capecitabine), aminopterin, nelarabine, leucovorin calcium, Tabloid, butocin, calcium folinate, calcium levofolinate, cladribine, emitefur, fludarabine, gemcitabine, hydroxycarbamide, pentostatin, piritrexim, idoxuridine, mitoguazone, tiazofurin, ambamustine, bendamustine, and DDS preparations thereof.

Examples of the "anticancer antibiotic" used include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarkomycin, carzinophilin, mitotane, zorubicin hydrochloride, mitoxantrone hydrochloride, idarubicin hydrochloride, and DDS preparations (e.g., PEG liposomal doxorubicin) thereof.

Examples of the "plant-derived anticancer agent" used include etoposide, etoposide phosphate, vinblastine sulfate, vincristine sulfate, vindesine sulfate, teniposide, paclitaxel, docetaxel, cabazitaxel, vinorelbine, and DDS preparations thereof.

Examples of the "immunotherapeutic" used include picibanil, Krestin, schizophyllan, lentinan, ubenimex, interferon, interleukin, macrophage colony-stimulating factor, granulocyte colony-stimulating factor, erythropoietin, lymphotoxin, BCG vaccines, Corynebacterium parvum, levamisole, polysaccharide K, procodazol, anti-CTLA4 antibodies (e.g., ipilimumab and tremelimumab), anti-PD-1 antibodies (e.g., nivolumab and pembrolizumab), and anti-PD-L1 antibodies.

The "cell growth factor" in "drugs that inhibit the action of cell growth factors and their receptors" can be any substance that can promote cell proliferation. It is usually a peptide with a molecular weight of no more than 20,000 and can bind to receptors. expresses activity at low concentrations, and specifically can be used
(1) EGF (epidermal growth factor) or a substance with substantially the same activity as EGF (for example, TGFα);
(2) Insulin or a substance with substantially the same activity as insulin (for example, insulin, IGF (insulin-like growth factor)-1, IGF-2);
(3) FGF (fibroblast growth factor) or a substance with substantially the same activity as FGF (for example, acidic FGF, basic FGF, KGF (keratinocyte growth factor), FGF-10);
(4) Other cell growth factors (for example, CSF (clone stimulating factor), EPO (erythropoietin), IL-2 (interleukin-2), NGF (nerve growth factor), PDGF (platelet growth factor), TGFβ (Transforming growth factor beta), HGF (hepatocyte growth factor), VEGF (vascular endothelial growth factor), regulatory proteins, angiopoietin).

"Cell growth factor receptor" can be any receptor capable of binding to any of the above-mentioned cell growth factors, and specifically the following can be used: EGF receptor, heregulin receptor (eg, HER3), insulin receptor, IGF receptor-1, IGF receptor-2, FGF receptor-1 or FGF receptor-2, VEGF receptor, angiopoietin receptor (eg, Tie2), PDGF receptor, etc.

As "drugs that inhibit the action of cell growth factors and their receptors", for example, the following can be used: EGF inhibitors, TGFα inhibitors, regulatory protein inhibitors, insulin inhibitors, IGF inhibitors, FGF inhibitors, KGF inhibitors, CSF Inhibitors, EPO inhibitors, IL-2 inhibitors, NGF inhibitors, PDGF inhibitors, TGFβ inhibitors, HGF inhibitors, VEGF inhibitors, angiopoietin inhibitors, EGF receptor inhibitors, HER2 inhibitors, HER4 Inhibitors, insulin receptor inhibitors, IGF-1 receptor inhibitors, IGF-2 receptor inhibitors, FGF receptor-1 inhibitors, FGF receptor-2 inhibitors, FGF receptor-3 inhibitors, FGF Receptor-4 inhibitor, VEGF receptor inhibitor, Tie-2 inhibitor, PDGF receptor inhibitor, Abl inhibitor, Raf inhibitor, FLT3 inhibitor, c-Kit inhibitor, Src inhibitor, PKC inhibitor , Smo inhibitor, ALK inhibitor, ROR1 inhibitor, Trk inhibitor, Ret inhibitor, mTOR inhibitor, Aurora inhibitor, PLK inhibitor, MEK (MEK1/2) inhibitor, MET inhibitor, CDK inhibitor, Akt inhibitors, ERK inhibitors, PI3K inhibitors, etc. More specifically, the following can be used: anti-VEGF antibodies (eg, Bevacizumab, Ramucirumab), anti-HER2 antibodies (eg, Trastuzumab, Pertuzumab), (Pertuzumab), anti-EGFR antibodies (e.g., Cetuximab, Panitumumab, Matuzumab, Nimotuzumab), anti-HGF antibodies , Imatinib, Erlotini b, Gefitinib, Sorafenib, Sunitinib, Dasatinib, Lapatinib, Vatalanib, Ibrutinib, Bosutinib, Cabozantinib, Crizotinib, Arela Alectinib, Vismodegib, Axitinib, Motesanib, Nilotinib, 6-[4-(4-ethylpiperazine) -1-ylmethyl)phenyl]-N-[1(R)-phenylethyl]-7H-pyrrolo[2,3-D]pyrimidin-4-amine (AEE-788), Vanderta Vandetanib, Temsirolimus, Everolimus, Enzastaurin, Tozasertib, 2-[N-[3-[4-[5-[N-(3-Fluorophenyl)carbamoylmethyl]-1H-pyrazol-3-ylamino]quinazo lin-7-yloxy]propyl]-N-ethylamino]ethyl ester (AZD-1152 ), 4-[9-chloro-7-(2,6-difluorophenyl)-5H-pyrimido[5,4-D] [2]benzazepin-2-ylamino]ben zoic acid, N -[2-Methoxy-5-[(E)-2-(2,4,6-trimethoxyphenyl)vinylsulfonylmethyl]phenyl]glycine sodium salt (ON-1910Na), Volasertib , Selumetinib, Trametinib, N-[2(R),3-dihydroxypropoxy]-3,4-difluoro-2-(2-fluoro-4- Iodophenylamino) benzamide (PD-0325901), Bosutinib, Regorafenib, Afatinib, Idelalisib, Ceritinib, Dabrafenib, etc..

In addition to the above drugs, asparaginase, acetoglucuronide, procarbazine hydrochloride, protoporphyrin-cobalt complex salt, mercury hematoporphyrin-sodium, topoisomerase I inhibitors ( For example, irinotecan, topotecan, Indotecan, Indimitecan), topoisomerase II inhibitors (e.g., sobuzoxane), differentiation-inducing agents (e.g., retinoids, vitamin D), other angiogenic Inhibitors (e.g., fumagillin, shark extract, COX-2 inhibitors), alpha-blockers (e.g., tamsulosin hydrochloride), bisphosphonates (e.g., pamidronate, zoledronate salt), thalidomide, lenalidomide, pomalidomide, 5-azacytidine, decitabine, proteasome inhibitors (e.g., Bortezomib, carfilzomib, ixazomib, NEDD8 inhibitors (e.g., pevonedistat), UAE inhibitors, PARP inhibitors (e.g., olaparib, niraparib, velipa Veliparib, Rucaparib), anti-tumor antibodies, such as anti-CD20 antibodies (such as rituximab, Obinutuzumab), anti-CCR4 antibodies (such as Mogamulizumab), etc., antibody-drug complexes (For example, trastuzumab emtansine, brentuximabvedotin, etc. can be used as concomitant drugs.

Hereinafter, the compound of the present invention and a concomitant drug used in combination are referred to as the "combination drug of the present invention". As a method of administering the compound of the present invention and the concomitant drug, the following methods may be mentioned: (1) Simultaneously formulating the compound of the present invention and the concomitant drug to prepare a single preparation for administration. (2) Prepare the compound of the present invention and the concomitant drug separately to prepare two preparations, and administer them simultaneously through the same administration route. (3) Prepare the compound of the present invention and the concomitant drug separately to obtain two preparations, which are administered via the same administration route and at staggered times. (4) Prepare the compound of the present invention and the concomitant drug separately to obtain two preparations, and administer them simultaneously through different administration routes. (5) Prepare the compound of the present invention and the concomitant drug separately to obtain two preparations, which are administered by different administration routes and at staggered times (for example, the compound of the present invention and the concomitant drug are administered in this order or in the reverse order), etc.

The compounds of the invention or the combination agents of the invention may further be used in combination with non-pharmacological therapies. Specifically, the compounds of the present invention or the combination agents of the present invention can be used in combination with non-pharmacological therapies, for example, (1) surgery, (2) pressurized chemotherapy, (3) gene therapy, (4) hyperthermia, ( 5) cold therapy, (6) laser cauterization, (7) radiotherapy.

The compound of the present invention or the combination drug of the present invention is used, for example, before or after the surgery or the like or before or after treatment involving two or three of these therapies in combination to produce effects such as prevention of devel opment of resistance, prolonged disease-free survival, inhibition of cancer metastasis or recurrence, and life prolon gation.

In addition, the compounds of the present invention or the combination agents of the present invention can be combined with the following supportive therapies: (i) administration of multiple antibiotics (for example, β-lactams such as Pansporin, etc., macrolides (such as clarithromycin, etc.) to treat infectious diseases, (ii) intravenous administration of nutritional solutions, amino acid preparations, and general vitamin preparations to improve malnutrition, (iii) administration of morphine to relieve pain, (iv) ) administration of drugs that can improve side effects such as nausea, vomiting, anorexia, diarrhea, leukopenia, thrombocytopenia, decreased hemoglobin concentration, alopecia, liver disease, kidney disease, DIC, fever, etc. (v) administration of drugs that inhibits the resistance of cancer to multiple drugs.

### Definition of Terms

The following definitions are provided for a better understanding of the invention.

In this context, when reference is made to a "compound" having a particular structural formula, this generally also encompasses its stereoisomers, diastereoisomers, enantiomers, racemic mixtures and isotopic derivatives, as well as alternative forms thereof include pharmaceutically acceptable salts, solvates, hydrates, and the like. It is well known to those skilled in the art that salts, solvates, and hydrates of a compound are alternative forms of the compound, and they can all be converted into the compound under certain conditions. Therefore, special attention should be paid to when mentioning these compounds in this article. When referring to a compound, it generally also includes its pharmaceutically acceptable salts, and further includes its solvates and hydrates.

Similarly, reference herein to a compound generally includes its prodrugs, metabolites and nitrogen oxides.

The pharmaceutically acceptable salts or pharmaceutically acceptable salts of the present invention can be formed using inorganic acids or organic acids. The "pharmaceutically acceptable salts" refer to such salts: within the scope of reasonable medical judgment, it is suitable for use in contact with tissues of humans and lower animals without undue toxicity, irritation, allergic reactions, etc., and can be described as a reasonable benefit/risk ratio. The salts may be prepared in situ during the final isolation and purification of the compounds of the invention, or separately by reacting the free base or free acid with a suitable reagent, as summarized below. For example, the free base can be reacted with a suitable acid. In addition, when the compound of the present invention carries an acidic moiety, suitable pharmaceutically acceptable salts thereof may include metal salts, such as alkali metal salts (such as sodium or potassium salts) and alkaline earth metal salts (such as calcium salts or magnesium salts). Examples of pharmaceutically acceptable nontoxic acid addition salts are salts formed by amino groups with inorganic acids (e.g., hydrochloric acid, hydrobromic acid, phosphoric acid, sulfuric acid, and perchloric acid) or organic acids (e.g., acetic acid, oxalic acid, maleic acid, tartaric acid, citric acid, succinic acid or malonic acid) or by using other methods in the art such as ion exchange. Other pharmaceutically acceptable salts include adipate, sodium alginate, ascorbate, aspartate, benzenesulfonate, benzoate, bisulfate, borate, butyrate, camphorate, camphorsulfonate, citrate, cyclopentane propionate, digluconate, lauryl sulfate, ethanesulfonate, formate, fumarate, glucoheptonate, glycerophosphate, gluconate, enanthate, hexanoate, hydroiodide, 2-hydroxyethanesulfonate, lactobiate, lactate, laurate, lauryl sulfate, malate, maleate, malonate, methanesulfonate, 2-naphthalenesulfonate, nicotinate, nitrate, oleate, oxalate, palmitate, pamateate, pectate, persulfate, 3-phenylpropionate, phosphate, bitter salt, pivalate, propionate, stearate, succinate, sulfate, tartrate, thiocyanate, tosylate, undecanoate, valerate, etc. Representative alkali metal or alkaline earth metal salts include sodium salts, lithium salts, potassium salts, calcium salts, magnesium salts, and the like. Other pharmaceutically acceptable salts include, where appropriate, nontoxic ammonium salts, quaternary ammonium salts, and ammonium cations formed with counterions, e.g., halides, hydroxides, carboxylates, sulfates, phosphates, nitrates, lower alkylsulfonates and arylsulfonates.

The pharmaceutically acceptable salts of the present invention can be prepared by conventional methods, for example by dissolving the compound of the present invention in a water-miscible organic solvent (such as acetone, methanol, ethanol and acetonitrile), adding thereto an excess of organic acid or an aqueous inorganic acid solution to precipitate the salt from the resulting mixture, remove the solvent and remaining free acid **therefrom,** and then isolate the precipitated salt.

The precursors or metabolites described in the present invention can be precursors or metabolites known in the art, as long as the precursors or metabolites are converted into compounds through in vivo metabolism. For example, "prodrugs" refer to those prodrugs of the compounds of the present invention which, within the scope of reasonable medical judgment, are suitable for contact with tissues of humans and lower animals without undue toxicity, irritation, allergic reactions, etc., and demonstrate a reasonable benefit/risk ratio and are effective for its intended use. The term "prodrug" refers to a compound that is rapidly converted in vivo to produce the parent compound of the formula above, for example by metabolism in the body, or N-demethylation of a compound of the invention.

"Solvate" as used herein means a physical association of a compound of the invention with one or more solvent molecules, whether organic or inorganic. This physical association includes hydrogen bonding. In certain circumstances, such as when one or more solvent molecules are incorporated into the crystal lattice of a crystalline solid, solvates will be able to be isolated. The solvent molecules in a solvate may exist in regular and/or disordered arrangements. Solvates may contain stoichiometric or non-stoichiometric amounts of solvent molecules. "Solvate" encompasses both solution phase and isolable solvates. Exemplary solvates include, but are not limited to, hydrates, ethanolates, methoxides, and isopropoxides. Solvation methods are well known in the art.

The "stereoisomerism" mentioned in the present invention is divided into conformational isomerism and configurational isomerism. Configurational isomerism can also be divided into cis-trans isomerism and optical isomerism (ie, optical isomerism). Conformational isomerism refers to a stereoisomerism phenomenon in which organic molecules with a certain configuration are arranged differently in space due to the rotation or distortion of carbon and carbon single bonds. Common ones include the structures of alkanes and cycloalkanes, such as the chair conformation and boat conformation that appear in the cyclohexane structure. "Stereoisomers" means when a compound of the present invention contains one or more asymmetric centers and is thus available as racemates and racemic mixtures, single enantiomers, diastereomeric mixtures and single diastereomer. The compounds of the present invention may have asymmetric centers, each asymmetric center will produce two optical isomers, and the scope of the present invention includes all possible optical isomers and diastereomeric mixtures and pure or partially pure compound. The compounds described in this invention may exist as tautomers having different points of attachment of hydrogens through the displacement of one or more double bonds. For example, a ketone and its enol form are keto-enol tautomers. Each tautomer and mixtures thereof are included in the compounds of the invention. All enantiomers, diastereomers, racemates, mesomers, cis-trans isomers, tautomers, geometric isomers and epimers of the compounds of formula (I) and mixtures thereof are all included in the scope of the present invention.

"Isotopic derivatives" of the present invention refer to molecules in which compounds in this patent are isotopically labeled. The isotopes commonly used as isotope labels are: hydrogen isotopes: ²H and ³H; carbon isotopes: ¹¹C, ¹³C and ¹⁴C; chlorine isotopes: ³⁵Cl and ³⁷Cl; fluorine isotopes: ¹⁸F; iodine isotopes: ¹²³I and ¹²⁵I; nitrogen isotopes: ¹³N and ¹⁵N; oxygen isotopes: ¹⁵O, ¹⁷O and ¹⁸O and sulfur isotope ³⁵S. These isotopically labeled compounds can be used to study the distribution of pharmaceutical molecules in tissues. In particular, deuterium ²H and carbon ¹³C are more widely used because they are easy to label and detect. Substitution of certain heavy isotopes, such as deuterium (²H), can enhance metabolic stability, extend half-life, thereby reducing dosage and providing therapeutic advantages. Isotopically labeled compounds generally start from labeled starting materials and are synthesized using known synthetic techniques as for non-isotopically labeled compounds.

Other features of the invention will become apparent in the course of describing the exemplary embodiments of the invention which are given to illustrate the invention and are not intended to be limiting thereof. The following examples were prepared, separated and characterized using the methods disclosed in the invention

Unless otherwise stated, the terms used in this application (including the specification and claims) are defined as follows. It must be noted that, in the specification and the appended claims, the singular forms "a," "a" and "an" include plural referents unless the context clearly dictates otherwise. If not otherwise stated, conventional methods of mass spectrometry, nuclear magnetic resonance, HPLC, protein chemistry, biochemistry, recombinant DNA technology, and pharmacology were used. In this application, the use of "or" or "and" means "and/or" unless stated otherwise.

In the description and claims, a given chemical formula or name shall cover all stereo and optical isomers as well as the racemates in which the above-mentioned isomers are present. Unless otherwise indicated, all chiral (enantiomeric and diastereomeric) and racemic forms are within the scope of the invention. Many geometric isomers of C=C double bonds, C=N double bonds, ring systems, etc. may also exist in the compounds, and all the above stable isomers are encompassed by the present invention. This invention describes the cis- and trans- (or E- and Z-) geometric isomers of the compounds of the invention, and they can be separated into mixtures of isomers or separate isomeric forms. The compounds of the present invention can be isolated in optically active or racemic form. All methods for preparing the compounds of the invention and the intermediates prepared therein are considered to be part of the invention. When preparing enantiomeric or diastereomeric products, they can be separated by conventional methods, for example by chromatography or fractional crystallization. Depending on the process conditions, the final product of the invention is obtained in free (neutral) or salt form. Both free forms and salts of these end products are within the scope of this invention. If desired, one form of the compound can be converted into another form. The free base or acid can be converted into a salt; the salt can be converted into the free compound or another salt; and mixtures of isomeric compounds of the invention can be separated into individual isomers. The compounds of the present invention, their free forms and salts, may exist in a variety of tautomeric forms in which hydrogen atoms are transposed to other parts of the molecule and thereby the chemical bonds between the atoms of the molecule are rearranged. It is to be understood that all tautomeric forms which may exist are included in the present invention.

Unless otherwise defined, the definitions of the substituents in the present invention are independent and not related to each other. For example, for R^{a} (or R^{b}) in the substituent, it is independent in the definition of different substituents. Specifically, when one definition is chosen for R^{a} (or R^{b}) in one substituent, it does not mean that R^{a} (or R^{b}) has the same definition in other substituents. More specifically, for example (not exhaustive) for NR^{a}R^{b}, when the definition of R^{a} (or R^{b}) is selected from hydrogen, it does not mean that in -C(O)-NR^{a}R^{b}, R^{a} (or R^{b}) must be hydrogen. On the other hand, when there is more than one R^{a} (or R^{b}) in a certain substituent, these R^{a} (or R^{b}) are also independent. For example, in the substituent -(CR^{a}R^{b})m-O-(CR^{a}R^{b})n-, when m+n is greater than or equal to 2, the m+n R^{a} (or R^{b}) are independent, and they can have the same or different meanings.

Unless otherwise defined, when a substituent is designated as "optionally substituted," the substituent is selected from, for example, substituents such as alkyl, cycloalkyl, aryl, heterocyclyl, halogen, hydroxyl, alkoxy, oxo, alkanoyl, aryloxy, alkanoyloxy, amino, alkylamino, arylamino, arylalkylamino, disubstituted amine groups (where the two amino substituents are selected from alkyl, aryl or arylalkyl), alkanoylamino, arolylamino, aralkanoylamino, substituted alkanoylamino, substituted arylamino, substituted aralkanoylamino, thio, alkylthio group, arylthio group, arylalkylthio group, arylthiocarbonyl group, arylalkylthiocarbonyl group, alkylsulfonyl group, arylsulfonyl group, arylalkylsulfonyl group, sulfonamido group such as -SO₂NH₂, substituted sulfonylamino, nitro, cyano, carboxyl, carbamoyl such as -CONH₂, substituted carbamoyl (such as -CONH alkyl, -CONH aryl, -CONH arylalkyl where there are two substituents selected from alkyl, aryl or arylalkyl on the nitrogen, alkoxycarbonyl, aryl, substituted aryl, guanidine, heterocyclyl such as indolyl, imidazolyl, furyl, Thienyl, thiazolyl, pyrrolidinyl, pyridyl, pyrimidinyl, pyrrolidinyl, piperidyl, morpholinyl, piperazinyl, homopiperazinyl, etc. and substituted heterocyclyl.

The term "alkyl" or "alkylene" as used herein is intended to include both branched and straight chain saturated aliphatic hydrocarbon groups having the specified number of carbon atoms. For example, "C₁-C₆ alkyl" means an alkyl group having 1 to 6 carbon atoms. Examples of alkyl groups include, but are not limited to, methyl (Me), ethyl (Et), n-propyl, isopropyl, n-butyl, isobutyl, tert-butyl, n-pentyl, isopentyl, neopentyl. Preferred alkyl groups of the present invention include C₁-C₆ alkyl or C₁-C₄ alkyl.

The term "alkenyl" refers to a straight or branched hydrocarbon group containing one or more double bonds and usually having a length of 2 to 20 carbon atoms. For example, "C₂-C₆ alkenyl" contains two to six carbon atoms. Alkenyl groups include, but are not limited to, vinyl, propenyl, butenyl, 1-methyl-2-buten-1-yl, and the like. Preferred alkenyl groups of the present invention include C₂-C₆ alkenyl groups.

The term "alkynyl" refers to a straight or branched hydrocarbon radical containing one or more triple bonds and usually having a length of 2 to 20 carbon atoms. For example, "C₂-C₆ alkynyl" contains two to six carbon atoms. Representative alkynyl groups include, but are not limited to, for example, ethynyl, 1-propynyl, 1-butynyl, and the like. Preferred alkynyl groups of the present invention include C₂-C₆ alkynyl groups. The term "alkoxy" or "alkyloxy" refers to -O-alkyl. "C₁-C₆ alkoxy" (or alkyloxy) is intended to include C₁, C₂, C₃, C₄, C₅, C₆ alkoxy. Examples of alkoxy include, but are not limited to, methoxy, ethoxy, propoxy (eg, n-propoxy and isopropoxy), and tert-butoxy. Similarly, "alkylthio" or "thioalkoxy" means a sulfur-bridged alkyl group as defined above having the specified number of carbon atoms; for example, methyl-S- and ethyl-S-. Preferred alkoxy groups in the present invention include C₁-C₆ alkoxy groups or C₁-C₄ alkoxy groups.

The term "carbonyl" refers to an organic functional group consisting of two atoms, carbon and oxygen, linked by a double bond (C=O).

The term "aryl", alone or as part of a larger moiety such as "aralkyl", "aralkoxy" or "aryloxyalkyl", refers to a monocyclic ring having a total of 5 to 12 ring members , a bicyclic or tricyclic ring system, wherein at least one ring in the system is aromatic and wherein each ring in the system contains 3 to 7 ring members. In certain embodiments of the present invention, "aryl" refers to an aromatic ring system including, but not limited to, phenyl, biphenyl, indanyl, 1-naphthyl, 2-naphthyl, and tetralin. The term "aralkyl" or "arylalkyl" refers to an alkyl residue attached to an aryl ring. Non-limiting examples include benzyl, phenethyl, and the like. The fused aryl group can be attached to another group at a suitable position on the cycloalkyl ring or aromatic ring. A dashed line drawn from a ring system shows that the bond can be attached to any suitable ring atom.

The term "cycloalkyl" refers to a monocyclic or bicyclic cyclic alkyl group. Monocyclic cyclic alkyl refers to unbranched or branched cyclic alkyl, including but not limited to cyclopropyl, cyclobutyl, cyclopentyl, cyclohexyl, norbornyl, 1-methylcyclopropyl and 2-methylcyclopropyl. Bicyclic cyclic alkyl groups include bridged, spiro or fused ring cycloalkyl groups. Preferred cycloalkyl groups of the present invention include C₃-C₆ cycloalkyl groups.

The term "cycloalkenyl" refers to a monocyclic or bicyclic cyclic alkenyl group. Monocyclic cyclic alkenyl refers to unbranched or branched cyclic alkenyl, including but not limited to cyclopropenyl, cyclobutenyl, cyclopentenyl, cyclohexenyl and norbornenyl, 1-methylcyclopropenyl and 2-methylcyclopropenyl. Bicyclic cyclic alkenyl groups include bridged, spiro or fused ring cyclic alkenyl groups. Preferred cycloalkenyl groups of the present invention include C₃-C₆ cycloalkenyl groups.

"Halo" or "halogen" includes fluorine, chlorine, bromine and iodine. "Haloalkyl" is intended to include branched and straight chain saturated aliphatic hydrocarbon groups having the specified number of carbon atoms substituted with one or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl and heptachloropropyl. Examples of haloalkyl groups also include "fluoroalkyl groups" which are intended to include branched and straight chain saturated aliphatic hydrocarbon groups having the specified number of carbon atoms substituted with one or more fluorine atoms.

"Haloalkoxy" or "haloalkyloxy" means a haloalkyl group as defined above having the specified number of carbon atoms linked via an oxygen bridge. For example, "C₁-C₆ haloalkoxy" is intended to include C₁, C₂, C₃, C₄, C₅, C₆ haloalkoxy. Examples of haloalkoxy include, but are not limited to, trifluoromethoxy, 2,2,2-trifluoroethoxy, and pentafluoroethoxy. Similarly, "haloalkylthio" or "thiohaloalkoxy" means a sulfur-bridged haloalkyl group as defined above having the specified number of carbon atoms; for example, trifluoromethyl-S- and pentafluoroethyl - S -.

In this disclosure, the expression Cₓ₁-Cₓ₂ is used when referring to some substituent groups, which means that the number of carbon atoms in the substituent group may be x1 to x2. For example, C₀-C₈ means that the group contains 0, 1, 2, 3, 4, 5, 6, 7 or 8 carbon atoms, and C₁-C₈ means that the group contains 1, 2, 3, 4, 5 , 6, 7 or 8 carbon atoms, C₂-C₈ means that the group contains 2, 3, 4, 5, 6, 7 or 8 carbon atoms, C₃-C₈ means that the group contains 3, 4, 5 , 6, 7 or 8 carbon atoms, C₄-C₈ means that the group contains 4, 5, 6, 7 or 8 carbon atoms, C₀-C₆ means that the group contains 0, 1, 2, 3, 4 , 5 or 6 carbon atoms, C₁-C₆ means that the group contains 1, 2, 3, 4, 5 or 6 carbon atoms, C₂-C₆ means that the group contains 2, 3, 4, 5 or 6 carbon atoms, C₃-C₆ means that the group contains 3, 4, 5 or 6 carbon atoms.

In this disclosure, the expression "x1-x2 membered ring" is used when referring to cyclic groups (such as aryl, heteroaryl, cycloalkyl, and heterocycloalkyl), which means that the ring atom number of the group can be x1 to x2. For example, the 3-12-membered cyclic group can be a 3, 4, 5, 6, 7, 8, 9, 10, 11 or 12-membered ring, and the number of ring atoms can be 3, 4, 5, 6, 7, 8, 9, 10, 11 or 12; 3-6 membered ring means that the cyclic group can be a 3, 4, 5 or 6 membered ring, and the number of ring atoms can be 3, 4, 5 or 6; 3-8 membered ring means that the cyclic group can be 3, 4, 5, 6, 7 or 8 membered ring, and the number of ring atoms can be 3, 4, 5, 6, 7 or 8; 3-9 membered ring means that the cyclic group can be a 3, 4, 5, 6, 7, 8 or 9-membered ring, and the number of ring atoms can be 3, 4, 5, 6, 7, 8 or 9; 4-7 membered ring means that the cyclic group can be a 4, 5, 6 or 7-membered ring, and the number of ring atoms can be 4, 5, 6 or 7; 5-8-membered ring means that the cyclic group can be 5, 6, 7 or 8-membered ring, and the number of ring atoms can be 5, 6, 7 or 8; 5-12-membered ring means that the cyclic group can be 5, 6, 7, 8, 9, 10, 11 or 12-membered ring, and the number of ring atoms can be 5, 6, 7, 8, 9, 10, 11 or 12; 6-12-membered ring means that the cyclic group can be 6, 7, 8, 9, 10, 11 or 12-membered ring, and the number of ring atoms may be 6, 7, 8, 9, 10, 11 or 12. The ring atoms may be carbon atoms or heteroatoms, such as heteroatoms selected from N, O and S. When the ring is a heterocycle, the heterocycle may contain 1, 2, 3, 4, 5, 6, 7, 8, 9, 10 or more ring heteroatoms, for example heteroatoms selected from N, O and S.

In the context of the present invention, one or more halogens may be each independently selected from fluorine, chlorine, bromine and iodine.

The term "heteroaryl" means a stable 5-membered, 6-membered, or 7-membered aromatic monocyclic ring or aromatic bicyclic ring or a 7-membered, 8-membered, 9-membered, 10-membered, 11-membered, or 12-membered aromatic polycyclic heterocyclic ring, which is fully unsaturated, partially unsaturated, and it contains carbon atoms and 1, 2, 3 or 4 heteroatoms independently selected from N, O and S; and includes any of the following polycyclic groups, wherein any heterocyclic ring as defined above is fused to a benzene ring. Nitrogen and sulfur heteroatoms may optionally be oxidized. Nitrogen atoms are substituted or unsubstituted (ie, N or NR, where R is H or another substituent, if defined). Heterocycles can be attached to their pendant groups at any heteroatom or carbon atom that results in a stable structure. If the resulting compound is stable, the heterocyclyl groups described herein may be substituted on the carbon or nitrogen atom. The nitrogen in the heterocycle may optionally be quaternized. Preferably, when the total number of S and O atoms in the heterocycle exceeds 1, then these heteroatoms are not adjacent to each other. Preferably, the total number of S and O atoms in the heterocycle is not greater than 1. When the term "heterocycle" is used, it is intended to include heteroaryl groups. Examples of aryl hetero groups include, but are not limited to, acridinyl, azetidinyl, azecinyl, benzimidazolyl, benzofuryl, benzothiofuranyl, benzothienyl, benzoxazolyl, benzoxazolinyl, benzothiazolyl, benzotriazolyl, benzotetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolyl, 2H,6H-1,5,2-dithiazinyl, dihydrofuro[2, 3-b] tetrahydrofuryl, furanyl, furanyl, imidazolidinyl, imidazolinyl, imidazolyl, 1H-indazolyl, imidazopyridyl, pseudoindolenyl, indolenyl, Indolinyl, indolyl, 3H-indolyl, isatinoyl, isobenzofuranyl, isochromanyl, isoindazolyl, isoindolyl, isoindolyl, isoquinolinyl, isothiazolyl, isothiazopyridyl, isoxazolyl, isoxazolopyridyl, methylenedioxyphenyl, morpholinyl, diazanaphthyl, octahydroisoquinoline base, oxadiazolyl, 1,2,3-oxadiazolyl, 1,2,4-oxadiazolyl, 1,2,5-oxadiazolyl, 1,3,4-oxadiazolyl , oxazolidinyl, oxazolyl, oxazolopyridyl, oxazolidinyl, naphthyridyl, oxindolyl, pyrimidinyl, phenanthridinyl, phenanthrolinyl, phenazinyl , phenothiazinyl, phenoxathiyl, phenoxazinyl, phthalazinyl, piperazinyl, piperidinyl, piperidinonyl, 4-piperidinonyl, piperonyl, pteridinyl, purinyl, Pyranyl, pyrazinyl, pyrazolidinyl, pyrazolinyl, pyrazopyridyl, pyrazolyl, pyridazinyl, pyridoxazolyl, pyridimidazolyl, pyridothiazolyl, pyridyl , pyrimidinyl, pyrrolidinyl, pyrrolinyl, 2-pyrrolidonyl, 2H-pyrrolyl, pyrrolyl, quinazolinyl, quinolinyl, 4H-quinolinyl, quinoxalinyl, quinuclidinyl, tetrazolyl, tetrahydrofuryl, tetrahydroisoquinolyl, tetrahydroquinolyl, 6H-1,2,5-thiadiazinyl, 1,2,3-thiadiazolyl, 1,2,4- Thiadiazolyl, 1,2,5-thiadiazolyl, 1,3,4-thiadiazolyl, thianthrenyl, thiazolyl, thienyl, thiazolopyridyl, thienothiazolyl, thienoxanyl Azolyl, thienoimidazolyl, thienyl, triazinyl, 1,2,3-triazolyl, 1,2,4-triazolyl, 1,2,5-triazolyl, 1,3,4 -Triazolyl and xanthyl, quinolyl, isoquinolyl, phthalazinyl, quinazolinyl, indolyl, isoindolyl, indolyl, 1H-indazolyl, benzo Imidazolyl, 1,2,3,4-tetrahydroquinolyl, 1,2,3,4-tetrahydroisoquinolyl, 5,6,7,8-tetrahydro-quinolyl, 2,3-dihydro-benzofuryl, chromanyl, 1,2,3,4-tetrahydro-quinoxalinyl and 1,2,3,4-tetrahydro-quinazolinyl. The term "heteroaryl" may also include a biaryl structure formed by the above-defined "aryl" and a monocyclic "heteroaryl", such as but not limited to "-phenylbipyridyl-", "- "phenylbipyrimidinyl", "-pyridylbiphenyl", "-pyridylbipyrimidinyl-", "-pyrimidinylbiphenyl-"; wherein the present invention also includes fused rings containing, for example, the above-mentioned heterocyclic rings and spirocyclic compounds.

As used herein, the term "heterocycloalkyl" or "heterocycle" refers to a monocyclic heterocycloalkyl system, or to a bicyclic heterocycloalkyl system, and also includes spiroheterocycles or bridged heterocycloalkyl. Monocyclic heterocycloalkyl refers to a saturated or unsaturated but non-aromatic cyclic alkyl system containing at least one heteroatom selected from O, N, S, and P. Bicyclic heterocycloalkyl system refers to a ring system formed by heterocycloalkyl group fused with a phenyl group, a cycloalkyl group, a cycloalkenyl group, a heterocycloalkyl group, or a heteroaryl group. Preferably, the "heterocycloalkyl" or "heterocycle" contains at least one or two heteroatoms selected from O, N, and S.

The term "bridged cycloalkyl" as used herein refers to polycyclic compounds sharing two or more carbon atoms. It can be divided into two-ring bridged cyclic hydrocarbons and polycyclic bridged cyclic hydrocarbons. The former is composed of two alicyclic rings sharing more than two carbon atoms; the latter is a bridged cyclic hydrocarbon composed of more than three rings.

The term "spirocycloalkyl" as used herein refers to polycyclic hydrocarbons that share one carbon atom (called a spiro atom) between the single rings.

The term "bridged cycloheteroyl" as used herein refers to a polycyclic compound sharing two or more carbon atoms, and the ring contains at least one atom selected from O, N, and S. It can be divided into two-ring bridged heterocycles and polycyclic bridged heterocycles.

The term "heterospirocyclyl" used herein refers to polycyclic hydrocarbons that share one carbon atom (called a spiro atom) between single rings, and the ring contains at least one heteroatom selected from O, N, and S.

The term "substituted" as used herein means that at least one hydrogen atom is replaced by a non-hydrogen group, provided that normal valency is maintained and that the substitution results in a stable compound. As used herein, a cyclic double bond is a double bond formed between two adjacent ring atoms (eg, C=C, C=N, or N=N).

In the case where nitrogen atoms (e.g. amines) are present on the compounds of the invention, these nitrogen atoms can be converted into N-oxides by treatment with oxidizing agents (e.g. mCPBA and/or hydrogen peroxide) to obtain other compounds of the invention.. Therefore, the nitrogen atoms shown and claimed are deemed to encompass the nitrogen atoms shown and their N-oxides to obtain the derivatives of the invention.

When any variable occurs more than once in any composition or formula of a compound, its definition on each occurrence is independent of its definition on every other occurrence. Thus, for example, if a group is shown substituted with 0-3 R, then the group may be optionally substituted with up to three R groups, with R on each occurrence being independently selected from the definition of R. Furthermore, combinations of substituents and/or variables are permitted only if such combinations result in stable compounds.

The term "patient" as used herein refers to an organism to be treated by the methods of the present invention. Such organisms preferably include, but are not limited to, mammals (eg, rodents, apes/monkeys, horses, cattle, pigs, dogs, cats, etc.) and most preferably refer to humans.

As used herein, the term "effective amount" means an amount of a drug or agent (i.e., a compound of the invention) that will elicit the biological or medical response in a tissue, system, animal, or human, for example, that is sought by a researcher or clinician. Furthermore, the term "therapeutically effective amount" refers to an amount that results in improved treatment, cure, prevention or alleviation of a disease, disorder, or side effect, or a reduction in the rate of progression of the disease or disorder, as compared with the corresponding subject that does not receive such amount. An effective amount may be administered in one or more dosing, administrations or dosages and is not intended to be limited to a particular formulation or route of administration. The term also includes within its scope an amount effective to enhance normal physiological functions.

The term "treating" is used herein in its broadest sense and encompasses therapeutic and/or prophylactic treatment of a subject. In particular, "treating" includes any treatment that results in the alleviation, suppression, elimination and amelioration and/or prevention of a condition, disease, disorder, etc., such as alleviation, reduction, regulation, amelioration, elimination, prevention, prevention or amelioration of symptom. The therapeutic treatment includes alleviating, inhibiting or ameliorating the symptoms or condition of the disease; inhibiting the occurrence of complications; improving the underlying metabolic syndrome; inhibiting the occurrence of the disease or symptoms, such as controlling the development of the disease or condition; alleviating the disease or symptoms; Reduction of disease or symptoms; reduction of complications caused by disease or symptoms, or treatment of symptoms caused by disease or disorders. Preventative treatment includes prior treatment to prevent, block or delay, slow the onset or progression of a disease or disorder or reduce the severity of a disease or disorder.

Likewise, "therapeutic agent" also includes agents or reagents that have therapeutic and/or prophylactic treatment in a subject.

The term "pharmaceutical" or "pharmaceutically acceptable" is used herein to refer to those compounds, materials, composition, and/or dosage forms which are, within the scope of sound medical judgment, suitable for use in contact with the tissues of human beings and animals without excessive toxicity, irritation, allergic response, and/or other problems or complication, commensurate with a reasonable benefit/risk ratio.

The phrase "pharmaceutically acceptable carrier" as used herein means a pharmaceutical substance, composition or vehicle such as a liquid or solid filler, diluent, excipient, manufacturing aid (e.g., lubricant, talc, stearin magnesium stearate, calcium stearate or zinc stearate or stearic acid) or solvent encapsulated substances which involve carrying or transporting the subject compound from one organ or part of the body to another. Each carrier must be "acceptable" in the sense of being compatible with the other ingredients of the formulation and not deleterious to the patient.

The term "pharmaceutical composition" means a composition comprising a compound of the invention and at least one other pharmaceutically acceptable carrier. "Pharmaceutically acceptable carrier" refers to a medium generally accepted in the art for delivering a biologically active agent to an animal, particularly a mammal, including (i.e.) an adjuvant, excipient or vehicle, such as a diluent, preservatives, fillers, flow regulators, disintegrants, wetting agents, emulsifiers, suspending agents, sweeteners, flavoring agents, aromatics, antibacterial agents, antifungal agents, lubricants and dispersants, This depends on the mode of administration and the nature of the dosage form.

### Specific pharmaceutical and medical terms

The term "acceptable," as used herein, means that a formulation component or active ingredient does not have undue deleterious effects on the health of the general target of treatment.

The term "cancer", as used herein, refers to an abnormal growth of cells that is uncontrollable and, under certain conditions, capable of metastasis (spread). This type of cancer includes, but is not limited to, solid tumors (such as bladder, bowel, brain, chest, uterus, heart, kidney, lung, lymphoid tissue (lymphoma), ovaries, pancreas or other endocrine organs (such as thyroid), prostate , skin (melanoma) or blood tumors (such as non-leukemic leukemia).

The term "coadministration" or similar terms, as used herein, refers to the administration of several selected therapeutic agents to a patient, in the same or different modes of administration and at the same or different times.

The term "enhance" or "potentiate," as used herein, means that the desired outcome is increased or prolonged, either in potency or duration. Thus, in terms of enhancing the therapeutic effect of a drug, the term "enhance" refers to the ability of a drug to increase or prolong its potency or duration in the system. As used in this article, "synergistic value " refers to the ability to maximize the enhancement of another therapeutic drug in an ideal system.

The term "immunological disease" refers to a disease or condition resulting from an adverse or harmful response to endogenous or exogenous antigens. The result is usually dysfunction of cells, or damage to organs or tissues that may produce immune symptoms.

The terms "kit" and "product packaging" are synonymous.

The terms "object," "subject," or "patient" include mammals and non-mammals. Mammals include, but are not limited to, mammals: humans, non-human primates such as orangutans, apes, and monkeys; agricultural animals such as cattle, horses, goats, sheep, and pigs; domestic animals such as rabbits and dogs; experimental animals including rodents, such as rats, mice and guinea pigs. Non-mammals include, but are not limited to, birds, fish, etc. In a preferred embodiment, the selected mammal is a human.

As used herein, a compound or pharmaceutical composition, when administered, can ameliorate a disease, symptom or condition, especially its severity, delay its onset, slow down its progression, or reduce its duration. Circumstances that may be attributed to or related to the administration, whether fixed or temporary, continuous or intermittent.

### Specific embodiments

The present invention can be better understood with reference to the following specific examples, which are only used to illustrate but not limit the invention.

### Raw material

When no preparation route is mentioned, the relevant intermediates are commercially available (eg from Sigma Aldrich, Alfa).

### General process

Commercially available reagents were used without further purification. ¹H-NMR spectra were recorded on a Bruker instrument at 500 MHz. Chemical shift values are expressed in parts per million, or δ values. The following abbreviations are used for the multiplicity of NMR signals: s = singlet, brs = broadt, d = doublet, t = triplet, m = multiplet. Coupling constants are listed as J values, measured in Hz. NMR and mass spectrometry results were corrected for background peaks. Chromatography refers to column chromatography using 100 mesh silica gel and completed under nitrogen pressure (flash chromatography). TLC used to monitor the reaction refers to TLC performed using a specific mobile phase and silica gel F254 from Merck as stationary phase.

### LC-MS experiments were measured under the following conditions:

Instrument: Thermo U3000, ALLtech ELSD, MSQ, UV detector combined ELSD and MSD (efflux ratio 4:1). Column: Waters X-Bridge C-18, 3.5 µm, 4.6x50 mm; column temperature: 30°C. Gradient [time (min)/solvent B in A (%)]: 0.00/5.0, 1.40/95, 2.80/95, 2.82/5, 3.00/5. (Solvent A = 0.01% trifluoroacetic acid in water; Solvent B = 0.01% trifluoroacetic acid in acetonitrile). UV detection: 214/254/280/300 nm; DAD detection: 210-350 nm; flow rate: 2 mL/min; MS: ESI, 100-1500 m/z

Preparative HPLC typically uses an alkaline method (gradient of acetonitrile and water containing 10 mM ammonium bicarbonate); Thermo U3000 AFC-3000 is used; column: Globalsil C-18 12 nm, 250 x 20 mm, 10 µm, or equivalent ; flow rate: 20 mL/min for separation.

### Example 1:

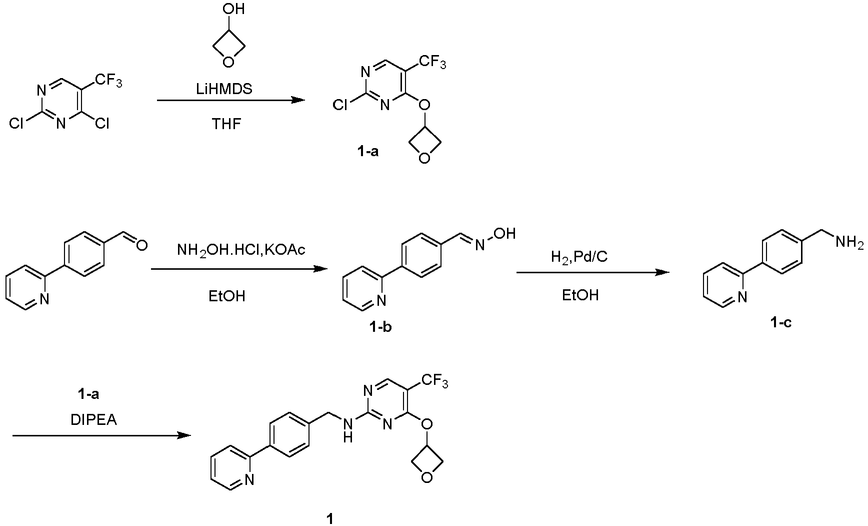

3-oxetanol (1.26 g, 17.0 mmol) and 2,4-dichloro-5-trifluoromethylpyrimidine (3.50 g, 16.1 mmol) were dissolved in tetrahydrofuran (50 mL), and lithium bis(trimethylsilyl)amide (1 M in THF, 17.7 mL) was slowly added dropwised at 0°C. After the mixture was stirred at 0°C for 3 hours, the reaction system was added with 50 mL of ammonium chloride solution, and was extracted twice with 100 mL of ethyl acetate. The combined organic phase was washed three times with water and once with saturated brine, dried over anhydrous sodium sulfate, filtered and concentrated. The residue was purified by column chromatography to obtain compound **1-a** (1.10 g, 4.33 mmol), a white solid, with a yield of 26.9%.

4-(2-pyridyl)benzaldehyde (10.0 g, 54.6 mmol) was dissolved in a mixture of ethanol (25 mL) and water (10 mL), hydroxylamine hydrochloride (4.17 g, 60.0 mmol) and sodium acetate trihydrate ( 8.17 g, 60.0 mmol) was added. The mixture was stirred at room temperature for 12 hours. The reaction solution was concentrated, and the residue was washed twice with 2 mL of water and once with 2 mL of ethanol, filtered and dried to obtain compound **1-b** (10.5 g, 53.0 mmol), a white solid, with a yield of 97.0%. MS (ESI): m/z 199.4 (M+H)⁺.

Compound **1-b** (10.5 g, 53.0 mmol) was dissolved in ethanol (210 mL), and palladium on carbon (10% w/w, 1.05 g) was added. The mixture was stirred at room temperature under a hydrogen atmosphere for 12 hours. The reaction solution was filtered through diatomaceous earth and concentrated. The residue was purified by column chromatography to obtain **1-c** (6.10 g, 33.1 mmol), a yellow oily liquid, with a yield of 62.5%. MS (ESI): m/z 185.5 (M+H)⁺.

Compound **1-c** (30.0 mg, 163 µmol) and compound **1-a** (41.5 mg, 163 µmol) were dissolved in N,N-dimethylformamide (2 mL), and diisopropylethylamine (63.1 mg, 488 µmol) was added. The mixture was stirred at 80°C for 2 hours. After the reaction solution was cooled to room temperature, compound **1** (10.0 mg, 24.8 µmol) was obtained as a white solid with a yield of 15.3%. ¹H NMR (500 MHz, DMSO-d₆) δ 8.71 - 8.38 (m, 3H), 8.09 - 8.01 (m, 2H), 7.93 (t, J = 7.0 Hz, 1H), 7.86 (td, J = 7.7, 1.9 Hz, 1H), 7.41 (d, J = 8.0 Hz, 2H), 7.36 - 7.31 (m, 1H), 5.67 - 5.52 (m, 1H), 4.92 - 4.70 (m, 2H), 4.62 - 4.41 (m, 4H); MS (ESI): m/z 403.4 (M+H)⁺.

### Example 2:

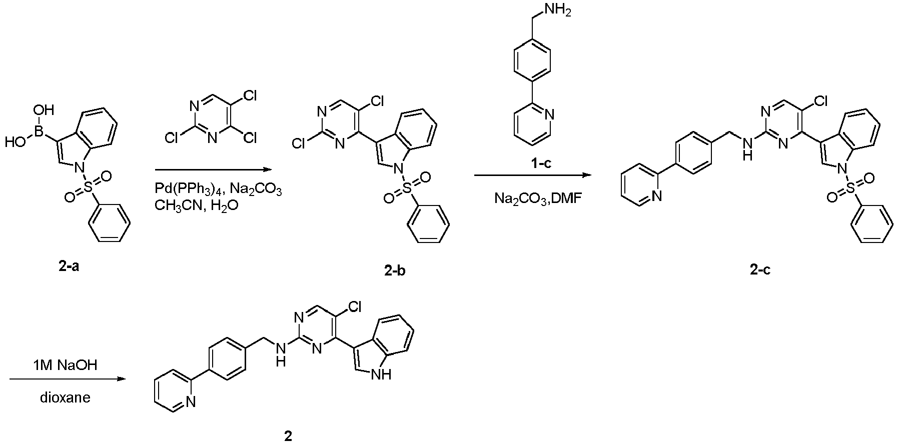

Compound **2-a** (200 mg, 664 µmol) and 2,4,5-trichloropyrimidine (122 mg, 664 µmol) were dissolved in a mixture of acetonitrile (3 mL) and water (3 mL), and sodium carbonate (141 mg, 1.33 mmol) and tetrakis triphenylphosphine palladium (38.2 mg, 33.1 µmol) were added. The mixture was stirred under nitrogen atmosphere at 90°C for 16 hours. The reaction solution was cooled t to room temperature, added with 30 mL of ethyl acetate, filtered through diatomaceous earth, and the filtrate was washed three times with water and once with saturated brine, dried over anhydrous sodium sulfate, filtered and concentrated. The residue was purified by column chromatography to obtain compound **2-b** (150 mg, 371 µmol), a white solid, with a yield of 55.9%.

Compound **2-b** (50.0 mg, 124 µmol) and compound **1-c** (27.3 mg, 148 µmol) were dissolved in N,N-dimethylformamide (2 mL), and sodium carbonate (32.8 mg, 309 µmol) was added . The mixture was stirred at 80°C for 8 hours. The reaction solution was cooled to room temperature, added with 30 mL of ethyl acetate, washed three times with water and once with saturated brine, dried over anhydrous sodium sulfate, filtered and concentrated. The residue was purified by column chromatography to obtain compound **2-c** (50.0 mg, 90.6 µmol), a light yellow solid, with a yield of 73.2%.

Compound **2-c** (50.0 mg, 90.6 µmol) was dissolved in 1,4-dioxane (2 mL), and sodium hydroxide solution (1 N, 0.5 mL) was added. The mixture was stirred at 40°C for 4 hours. The reaction solution was cooled to room temperature, adjusted to pH 7 with 1N hydrochloric acid, and concentrated to dryness. The residue was purified by preparative liquid chromatography to obtain compound **2** (6.0 mg, 14.6 µmol) as a white solid, yield 16.1%. ¹H NMR (500 MHz, DMSO-d₆) δ 11.79 (s, 1H), 8.64 - 8.60 (m, 1H), 8.41 (s, 1H), 8.27 (s, 1H), 8.19 - 8.01 (m, 3H), 7.96 - 7.88 (m, 2H), 7.86 - 7.80 (m, 1H), 7.51 - 7.39 (m, 3H), 7.33 - 7.27 (m, 1H), 7.27 - 6.77 (m, 2H), 4.65 (s, 2H); MS (ESI): m/z 412.3 (M+H)⁺.

### Example 3:

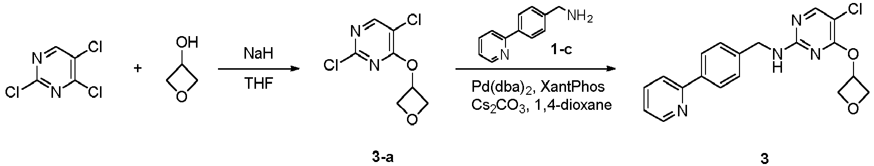

3-oxetanol (242 mg, 3.27 mmol) was dissolved in tetrahydrofuran (10 mL), and sodium hydride (60% w/w, 131 mg, 3.27 mmol) was slowly added at 0°C. After the mixture was stirred at 0°C for 1 hour, a solution of 2,4,5-trichloropyrimidine (500 mg, 2.73 mmol) in tetrahydrofuran (5 mL) was slowly added dropwise to the reaction system, and then it was raised to room temperature and stirred for 1 hour. The reaction system was added with 15 mL of ammonium chloride solution, and was extracted twice with 50 mL of ethyl acetate. The combined organic phase was washed three times with water and once with saturated brine, dried over anhydrous sodium sulfate, filtered and concentrated. The residue was purified by column chromatography to obtain compound **3-a** (600 mg, 2.71 mmol), a white solid, with a yield of 99.6%.

Compound **3-a** (50.0 mg, 226 µmol) and compound **1-c** (41.7 mg, 226 µmol) were dissolved in 1,4-dioxane (3 mL), and Pd(dba)₂ (20.7 mg, 22.6 µmol), Xantphos (13.1 mg, 22.6 µmol), cesium carbonate (147 mg, 452 µmol) were added. After the mixture was stirred at 100° C for 16 hours under a nitrogen atmosphere, the reaction solution was cooled to room temperature and concentrated. The residue was purified by preparative liquid chromatography to obtain compound 3 (13.0 mg, 35.2 µmol) as a white solid, yield 15.6%. ¹H NMR (500 MHz, Chloroform-d) δ 8.76 - 8.67 (m, 1H), 8.08 (s, 1H), 8.01 - 7.96 (m, 2H), 7.80 - 7.70 (m, 2H), 7.41 (d, J = 7.9 Hz, 2H), 7.30 - 7.28 (m, 1H), 7.26 - 7.23 (m, 1H), 5.61 - 5.51 (m, 1H), 4.96 - 4.82 (m, 2H), 4.79 - 4.70 (m, 2H), 4.66 - 4.56 (m, 2H); MS (ESI): m/z 369.4 (M+H)⁺.

### Example 4:

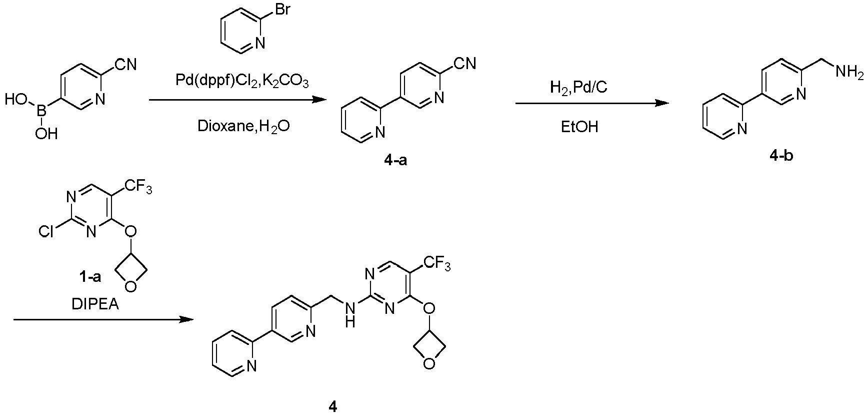

(6-cyanopyridin-3-yl)boronic acid (1.00 g, 6.76 mmol) and 2-bromopyridine (1.07 g, 6.76 mmol) were dissolved in a mixture of dioxane (10 mL) and water (1 mL) , potassium carbonate (1.87 g, 13.5 mmol) and 1,1'-bis(diphenylphosphino)ferrocene palladium(II) dichloride (495 mg, 676 µmol) were added. The mixture was stirred at 95°C for 6 hours under a nitrogen atmosphere. The reaction solution was cooled to room temperature, 50 mL of ethyl acetate was added, and filtered through diatomaceous earth. The filtrate was washed three times with water and once with saturated brine, dried over anhydrous sodium sulfate, filtered and concentrated. The residue was purified by column chromatography to obtain compound **4-a** (760 mg, 4.19 mmol), a white solid, with a yield of 62.0%.

Compound **4-a** (760 mg, 4.19 mmol) was dissolved in ethanol (10 mL), and palladium on carbon (10% w/w, 76.0 mg) was added. The mixture was stirred at room temperature under a hydrogen atmosphere for 12 hours. The reaction solution was filtered through diatomaceous earth and concentrated. The residue was purified by column chromatography to obtain compound **4-b** (300 mg, 1.62 mmol), a yellow oily liquid, with a yield of 38.6%.

Compound **4-b** (36.4 mg, 196 µmol) and compound **1-a** (50.0 mg, 196 µmol) were dissolved in N,N-dimethylformamide (2 mL), and diisopropylethylamine (76.2 mg, 589 µmol) was added. The mixture was stirred at 80°C for 2 hours. After the reaction solution was cooled to room temperature, compound **4** (20.0 mg, 49.6 µmol) was obtained as a white solid with a yield of 25.2%. ¹H NMR (500 MHz, DMSO-d₆) δ 9.24 - 9.17 (m, 1H), 8.73 - 8.36 (m, 4H), 8.06 - 8.00 (m, 1H), 7.94 - 7.90 (m, 1H), 7.43 - 7.37 (m, 2H), 5.69 - 5.40 (m, 1H), 4.94 - 4.32 (m, 6H); MS (ESI): m/z 404.4 (M+H)⁺.

### Example 5:

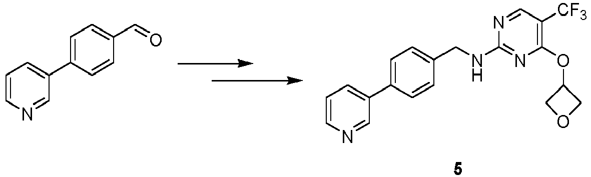

Compound **5** was obtained by referring to the synthesis of compound **1** starting from 4-(3-pyridyl)benzaldehyde. ¹H NMR (500 MHz, DMSO-*d*₆) δ 8.91 - 8.84 (m, 1H), 8.74 - 8.37 (m, 3H), 8.09 - 8.03 (m, 1H), 7.75 - 7.66 (m, 2H), 7.50 - 7.39 (m, 3H), 5.68 - 5.53 (m, 1H), 4.92 - 4.72 (m, 2H), 4.61 - 4.43 (m, 4H); MS (ESI): m/z 403.4 (M+H)⁺.

### Example 6:

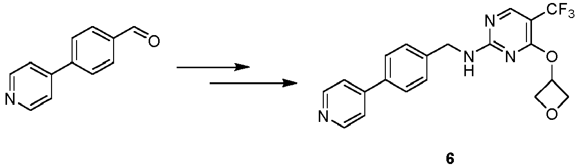

Compound **6** was obtained by referring to the synthesis of compound **1** starting from 4-(4-pyridyl)benzaldehyde. ¹H NMR (500 MHz, DMSO-*d*₆) δ 8.73 - 8.39 (m, 4H), 7.81 - 7.74 (m, 2H), 7.72 - 7.67 (m, 2H), 7.47 - 7.42 (m, 2H), 5.67 - 5.51 (m, 1H), 4.92 - 4.70 (m, 2H), 4.62 - 4.41 (m, 4H); MS (ESI): m/z 403.4 (M+H)⁺.

### Example 7:

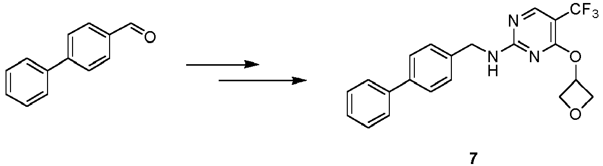

Compound 7 was obtained by referring to the synthesis of compound **1** starting from 4-biphenylcarbaldehyde. 1H NMR (500 MHz, DMSO-d6) δ 8.72 - 8.37 (m, 2H), 7.69 - 7.58 (m, 4H), 7.49 - 7.42 (m, 2H), 7.41 - 7.33 (m, 3H), 5.67 - 5.53 (m, 1H), 4.91 - 4.71 (m, 2H), 4.61 - 4.42 (m, 4H); MS (ESI): m/z 402.4 (M+H)+.

### Example 8:

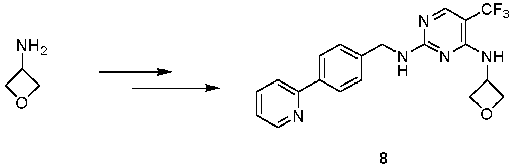

Compound **8** was obtained by referring to the synthesis of compound **1** starting from 3-oxetanine. 1H NMR (500 MHz, Chloroform-d) δ 8.74 - 8.68 (m, 1H), 8.23 - 8.06 (m, 1H), 8.03 - 7.95 (m, 2H), 7.80 - 7.71 (m, 2H), 7.48 - 7.37 (m, 2H), 7.31 - 7.29 (m, 1H), 7.27 - 7.23 (m, 1H), 5.60 - 5.34 (m, 1H), 5.19 - 5.02 (m, 1H), 5.00 - 4.82 (m, 2H), 4.71 - 4.51 (m, 4H); MS (ESI): m/z 402.3 (M+H)+.

### Example 9:

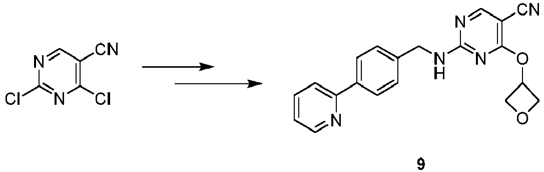

Compound **9** was obtained by referring to the synthesis of compound **1** starting from 2,4-dichloro-5-cyanopyrimidine. 1H NMR (500 MHz, Chloroform-d) δ 8.73 (d, J = 4.6 Hz, 1H), 8.48 - 8.29 (m, 1H), 8.02 (dd, J = 8.3, 2.1 Hz, 2H), 7.87 - 7.79 ( m, 1H), 7.79 - 7.72 (m, 1H), 7.48 - 7.37 (m, 2H), 7.32 - 7.29 (m, 1H), 6.34 - 5.72 (m, 1H), 5.66 - 5.56 (m, 1H), 4.99 - 4.57 (m, 6H); MS (ESI): m/z 360.4 (M+H)+.

### Example 10:

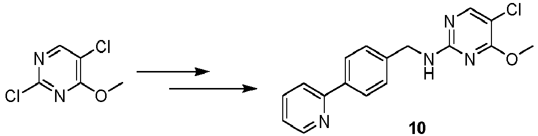

Starting from 2,5-dichloro-4-methoxypyrimidine, compound 10 was obtained by referring to the synthesis of compound 2. ¹H NMR (500 MHz, DMSO-*d*₆) δ 8.69 - 8.63 (m, 1H), 8.10 (s, 1H), 8.06 - 8.01 (m, 2H), 7.95 - 7.91 (m, 1H), 7.91 - 7.84 (m, 1H), 7.48 - 7.32 (m, 4H), 4.52 (d, *J* = 6.3 Hz, 2H), 3.90 (s, 3H); MS (ESI): m/z 327.3 (M+H)⁺.

### Example 11:

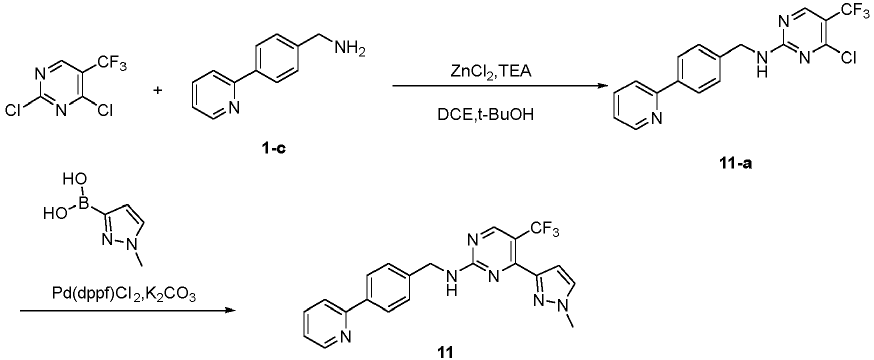

2,4-dichloro-5-trifluoromethylpyrimidine (300 mg, 1.38 mmol), triethylamine (251 mg, 2.49 mmol), and zinc chloride (565 mg, 4.15 mmol) were dissolved in a mixture of tert-butanol ( 4 mL) and 1,2-dichloroethane (4 mL). The mixture was stirred in an ice bath for 30 minutes, compound 1-c (254 mg, 1.38 mmol) was added, and then stirred at 50°C for 16 hours. The mixture was cooled to room temperature, 20 mL of water was added, the pH was adjusted to 4 with 1N hydrochloric acid, and extracted three times with 20 mL of dichloromethane. Combine the organic phases, wash three times with water and once with saturated brine, dry over anhydrous sodium sulfate, filter and concentrate. The residue was purified by column chromatography to obtain compound **11-a** (210 mg, 575 µmol), a white solid, with a yield of 41.64%.

Compound **11-a** (30 mg, 82.3 µmol) and (1-methyl-1H-pyrazol-3-yl)boronic acid (20.7 mg, 164 µmol) were dissolved in a mixture of 1,4-dioxane (3 mL) and water (0.3 mL), potassium carbonate (34.1 mg, 246 µmol) and 1,1'-bis(diphenylphosphino)ferrocene palladium(II) dichloride (3.0 mg, 4.11 µmol) were added. The mixture was stirred at 95°C for 3 hours under nitrogen atmosphere. The reaction solution was cooled t to room temperature, added with 20 mL of ethyl acetate, filtered through diatomaceous earth, and the filtrate was washed three times with water and once with saturated brine, dried over anhydrous sodium sulfate, filtered and concentrated. The residue was purified by preparative liquid chromatography to obtain compound **11** (10.0 mg, 24.4 µmol), a white solid, with a yield of 29.6%. ¹H NMR (500 MHz, Chloroform-*d*) δ 8.81 - 8.52 (m, 2H), 8.06 - 7.89 (m, 2H), 7.81 - 7.69 (m, 2H), 7.52 - 7.44 (m, 2H), 7.41 (s, 1H), 7.25 - 7.23 (m, 1H), 6.78 (s, 1H), 6.16 - 5.75 (m, 1H), 4.78 (d, *J=* 5.4 Hz, 2H), 4.10 - 3.95 (m, 3H); MS (ESI): m/z 411.4 (M+H)⁺.

### Example 12:

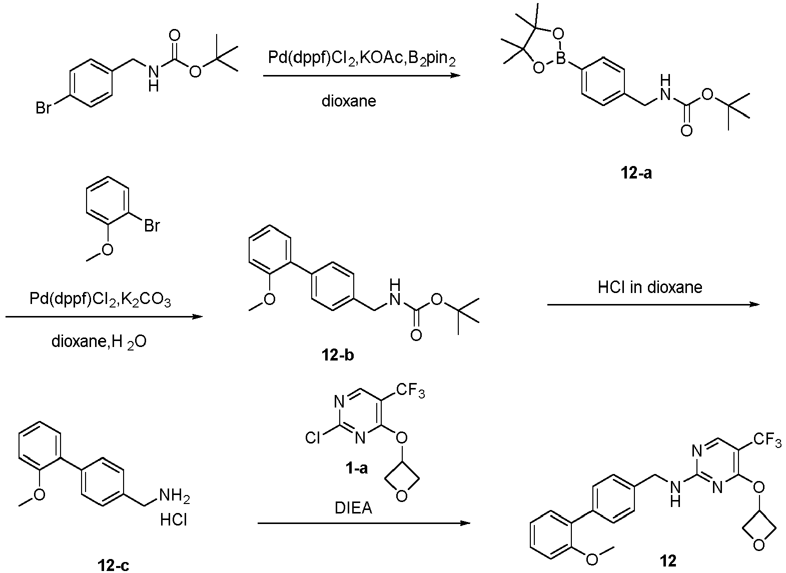

Dissolve N-BOC-4-bromobenzylamine (3.00 g, 10.5 mmol) and pinacol bisborate (2.93 g, 11.5 mmol) in 1,4-dioxane (45 mL), add 1, 1'-bis(diphenylphosphino)ferrocene palladium(II) dichloride (383 mg, 524 µmol) and potassium acetate (3.09 g, 31.5 mmol). The mixture was stirred at 95°C for 16 hours under nitrogen atmosphere. The reaction solution was cooled to room temperature, 50 mL of ethyl acetate was added, filtered through diatomaceous earth, and the filtrate was concentrated. The residue was purified by column chromatography to obtain compound **12-a** (3.30 g, 9.90 mmol), a colorless oily liquid, with a yield of 94.5%.

Compound **12-a** (100 mg, 300 µmol) and 2-bromoanisole (56.1 mg, 300 µmol) were dissolved in a mixture of 1,4-dioxane (3 mL) and water (0.3 mL), and potassium carbonate (124 mg, 900 µmol) and 1,1'-bis(diphenylphosphino)ferrocenepalladium(II) dichloride (11.0 mg, 15.0 µmol) were added. The mixture was stirred at 95°C for 3 hours under nitrogen atmosphere. The reaction solution was cooled t to room temperature, added with 20 mL of ethyl acetate, filtered through diatomaceous earth, and the filtrate was washed three times with water and once with saturated brine, dried over anhydrous sodium sulfate, filtered and concentrated. The residue was purified by column chromatography to obtain compound **12-b** (80.0 mg, 255 µmol), a colorless oily liquid, with a yield of 85.1%.

Compound **12-b** (80 mg, 255 µmol) was dissolved in dichloromethane (2 mL), and hydrogen chloride dioxane solution (4N, 1 mL) was added. The mixture was stirred at room temperature for 2 hours. The reaction solution was directly concentrated to obtain compound **12-c** (63.8 mg, 255 µmol), hydrochloride, as a white solid, with a yield of 100%.

Compound **12-c** (29.4 mg, 118 µmol) and compound **1-a** (30 mg, 118 µmol) were dissolved in N,N-dimethylformamide (2 mL), and diisopropylethylamine (45.7 mg, 354 µmol) was added. The mixture was stirred at 80°C for 2 hours. The reaction solution was cooled to room temperature and purified by preparative liquid chromatography to obtain compound **12** (15.0 mg, 34.8 µmol), a white solid, with a yield of 29.5%. ¹H NMR (400 MHz, DMSO-*d*₆) δ 8.70 - 8.38 (m, 2H), 7.44 - 7.30 (m, 5H), 7.29 - 7.20 (m, 1H), 7.15 - 6.94 (m, 2H), 5.66 - 5.56 (m, 1H), 4.91 - 4.73 (m, 2H), 4.67 - 4.35 (m, 4H), 3.88 - 3.61 (m, 3H); MS (ESI): m/z 432.4 (M+H)⁺.

### Example 13:

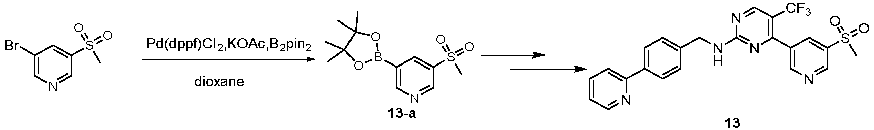

Starting from 3-bromo-5-(methylsulfonyl)pyridine, compound **13-a** was obtained by referring to the synthesis of compound **12-a.** Starting from **13-a,** compound 13 was obtained by referring to the synthesis of compound **11.** ¹H NMR (500 MHz, DMSO-*d*₆) δ 9.27 - 9.18 (m, 1H), 9.05 - 8.88 (m, 2H), 8.82 - 8.72 (m, 1H), 8.68 - 8.61 (m, 1H), 8.53 - 8.39 (m, 1H), 8.07 - 8.00 (m, 2H), 7.98 - 7.82 (m, 2H), 7.50 - 7.41 (m, 2H), 7.39 - 7.28 (m, 1H), 4.72 - 4.56 (m, 2H), 3.40 - 3.37 (m, 3H); MS (ESI): m/z 486.3 (M+H)⁺.

### Example 14:

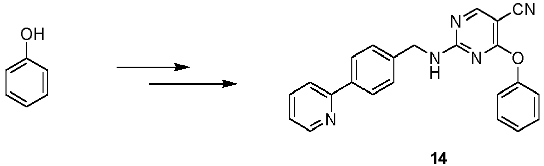

Starting from phenol, compound **14** was obtained by referring to the synthesis of compound **9.** ¹H NMR (400 MHz, DMSO-*d*₆) δ 9.05 - 8.72 (m, 1H), 8.72 - 8.61 (m, 2H), 8.05 - 7.85 (m, 4H), 7.55 - 7.44 (m, 2H), 7.41 - 7.27 (m, 4H), 7.26 - 7.21 (m, 1H), 6.99 - 6.95 (m, 1H), 4.63 - 4.06 (m, 2H); MS (ESI): m/z 380.4 (M+H)⁺.

### Example 15:

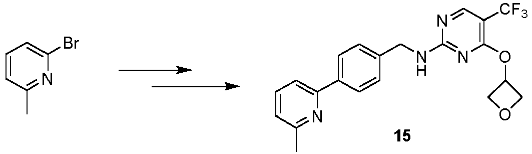

Starting from 2-bromo-6-methylpyridine, compound 15 was obtained by referring to the synthesis of compound 12. ¹H NMR (400 MHz, DMSO-*d*₆) δ 8.74 - 8.39 (m, 2H), 8.09 - 7.98 (m, 2H), 7.78 - 7.69 (m, 2H), 7.40 (d, *J=* 8.0 Hz, 2H), 7.20 (dd, *J* = 6.8, 1.5 Hz, 1H), 5.68 - 5.50 (m, 1H), 4.93 - 4.68 (m, 2H), 4.61 - 4.39 (m, 4H), 2.53 (s, 3H); MS (ESI): m/z 417.4 (M+H)⁺.

### Example 16:

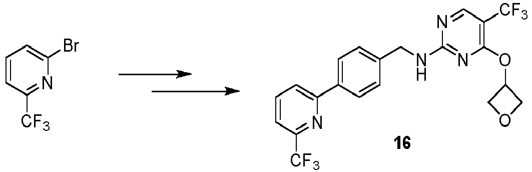

Starting from 2-bromo-6-trifluoromethylpyridine, compound **16** was obtained by referring to the synthesis of compound **12.** ¹H NMR (400 MHz, DMSO-*d*₆) δ 8.76 - 8.45 (m, 1H), 8.44 - 8.39 (m, 1H), 8.30 - 8.23 (m, 1H), 8.20 - 8.14 (m, 1H), 8.13 - 8.06 (m, 2H), 7.85 (d, *J=* 7.6 Hz, 1H), 7.50 - 7.43 (m, 2H), 5.69 - 5.49 (m, 1H), 4.93 - 4.68 (m, 2H), 4.64 - 4.40 (m, 4H).; MS (ESI): m/z 471.1 (M+H)⁺.

### Example 17:

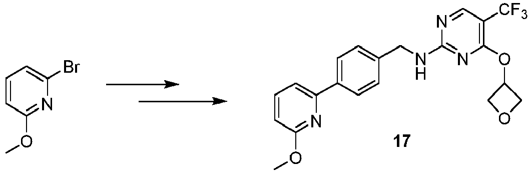

Starting from 2-bromo-6-methoxypyridine, compound **17** was obtained by referring to the synthesis of compound **12.** ¹H NMR (400 MHz, DMSO-*d*₆) δ 8.75 - 8.37 (m, 2H), 8.11 - 8.00 (m, 2H), 7.81 - 7.73 (m, 1H), 7.57 - 7.50 (m, 1H), 7.44 - 7.37 (m, 2H), 6.80 - 6.73 (m, 1H), 5.68 - 5.50 (m, 1H), 4.92 - 4.68 (m, 2H), 4.61 - 4.40 (m, 4H), 3.99 - 3.92 (m, 3H); MS (ESI): m/z 433.4 (M+H)⁺.

### Example 18:

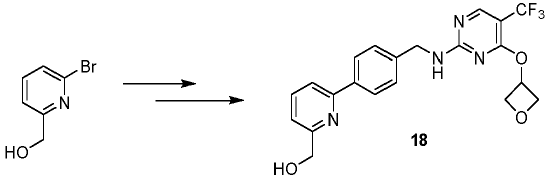

Starting from 2-bromo-6-hydroxymethylpyridine, compound **18** was obtained by referring to the synthesis of compound **12.** ¹H NMR (400 MHz, DMSO-*d*₆) δ 8.74 - 8.39 (m, 2H), 8.08 - 8.00 (m, 2H), 7.90 - 7.84 (m, 1H), 7.81 - 7.76 (m, 1H), 7.44 - 7.38 (m, 3H), 5.68 - 5.50 (m, 1H), 5.49 - 5.43 (m, 1H), 4.91 - 4.69 (m, 2H), 4.65 - 4.61 (m, 2H), 4.61 - 4.41 (m, 4H); MS (ESI): m/z 433.4 (M+H)⁺.

### Example 19:

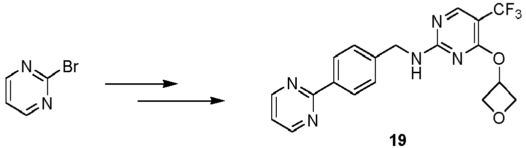

Starting from 2-bromopyrimidine, compound **19** was obtained by referring to the synthesis of compound **12.** ¹H NMR (400 MHz, DMSO-d₆) δ 8.92 - 8.87 (m, 2H), 8.75 - 8.44 (m, 1H), 8.44 - 8.40 (m, 1H), 8.39 - 8.32 (m, 2H), 7.47 - 7.42 (m, 3H), 5.68 - 5.49 (m, 1H), 4.92 - 4.66 (m, 2H), 4.64 - 4.39 (m, 4H); MS (ESI): m/z 403.9 (M+H)⁺.

### Example 20:

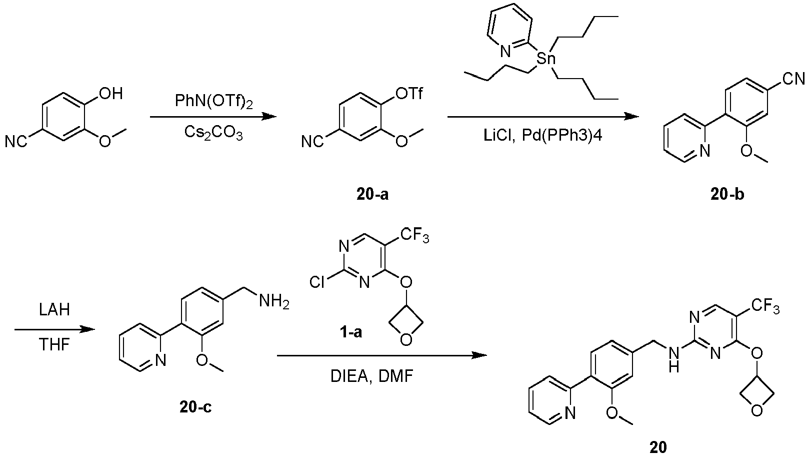

4-hydroxy-3-methoxybenzonitrile (2.00 g, 13.4 mmol) was dissolved in acetonitrile (30 mL) and N,N-dimethylformamide (5 mL), N-phenylbis(trisfluoromethanesulfonyl)imide (5.74 g, 14.8 mmol) and cesium carbonate (6.55 g, 20.1 mmol) were added. The mixture was stirred at room temperature for 16 hours. The reaction solution was added with 50 mL of ethyl acetate, washed three times with 2N sodium hydroxide, three times with water, and once with saturated brine, dried over anhydrous sodium sulfate, filtered, and concentrated to obtain compound **20-a** (3.40 g, 12.1 mmol), brown oily compound, yield 90.2%.

Compound **20-a** (1.00 g, 3.56 mmol) and (2-pyridyl)tributylstannane (1.44 g, 3.91 mmol) were dissolved in N,N-dimethylformamide (20 mL), and lithium chloride ( 196 mg, 4.62 mmol) and tetrakis triphenylphosphine palladium (411 mg, 356 µmol) were added. The mixture was stirred at 95°C for 5 hours under nitrogen atmosphere. The reaction solution was cooled to room temperature, added with 50 mL of ethyl acetate and 100 mL of 1N potassium fluoride solution, and stirred for 1 hour. The mixture was filtered through diatomaceous earth, and the organic phase was washed three times with water and once with saturated brine, dried over anhydrous sodium sulfate, filtered, and concentrated. The residue was purified by column chromatography to obtain compound **20-b** (450 mg, 2.14 mmol), a white solid, with a yield of 60.2%.

Lithium aluminum hydride (158 mg, 4.16 mmol) and tetrahydrofuran (10 mL) were heated to 70°C, and a solution of compound **20-b** (350 mg, 1.66 mmol) in tetrahydrofuran (5 mL) was slowly added dropwise within 10 minutes. The mixture was stirred at 70°C for a further 10 minutes. The reaction solution was cooled to room temperature, added with 0.16 mL of water and 0.16 mL of 10% sodium hydroxide solution, and stirred for 30 minutes. The mixture was filtered through diatomaceous earth, 20 mL of ethyl acetate was added to the filtrate, washed three times with water and once with saturated brine, dried over anhydrous sodium sulfate, filtered, and concentrated to obtain **20-c** (320 mg, 1.49 mmol), a light yellow oily compound, with a yield of 90.1%.

Compound **20-c** (25.2 mg, 118 µmol) and compound **1-a** (30.0 mg, 118 µmol) were dissolved in N,N-dimethylformamide (2 mL), and diisopropylethylamine (45.7 mg, 354 µmol) was added. The mixture was stirred at 80°C for 2 hours. After the reaction solution was cooled to room temperature, compound **20** (8.5 mg, 19.7 µmol) was obtained as a white solid with a yield of 16.7%. ¹H NMR (400 MHz, DMSO-*d*₆) δ 8.73 - 8.39 (m, 3H), 7.87 - 7.75 (m, 2H), 7.74 - 7.65 (m, 1H), 7.35 - 7.26 (m, 1H), 7.12 (s, 1H), 7.03 - 6.95 (m, 1H), 5.69 - 5.54 (m, 1H), 4.93 - 4.74 (m, 2H), 4.62 - 4.42 (m, 4H), 3.90 - 3.77 (m, 3H); MS (ESI): m/z 433.4 (M+H)⁺.

### Example 21:

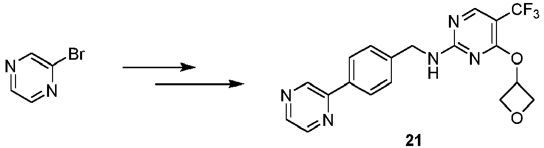

Starting from 2-bromopyrazine, compound **21** was obtained by referring to the synthesis of compound **12.** ¹H NMR (500 MHz, DMSO-*d*₆) δ 9.28 - 9.21 (m, 1H), 8.75 - 8.38 (m, 4H), 8.16 - 8.07 (m, 2H), 7.51 - 7.44 (m, 2H), 5.71 - 5.49 (m, 1H), 4.95 - 4.42 (m, 6H); MS (ESI): m/z 404.2 (M+H)⁺.

### Example 22:

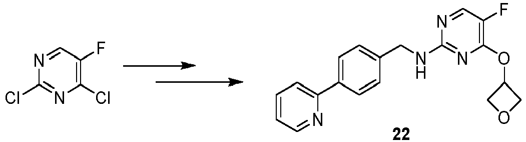

Starting from 2,4-dichloro-5-fluoropyrimidine, compound **22** was obtained by referring to the synthesis of compound **3.** ¹H NMR (500 MHz, DMSO-*d*₆) δ 8.70 - 8.62 (m, 1H), 8.17 (d, *J* = 3.2 Hz, 1H), 8.04 (d, *J* = 8.3 Hz, 2H), 7.93 (dd, *J* = 8.0, 1.2 Hz, 1H), 7.87 (td, *J=* 7.7, 1.9 Hz, 1H), 7.81 (s, 2H), 7.41 (d, *J=* 8.2 Hz, 2H), 5.65 - 5.45 (m, 1H), 4.91 - 4.66 (m, 2H), 4.64 - 4.34 (m, 4H); MS (ESI): m/z 353.3 (M+H)⁺.

### Example 23:

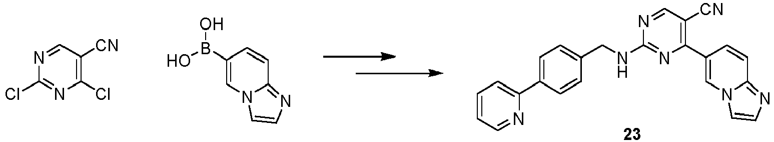

Starting from 2,4-dichloro-5-cyanopyrimidine and imidazo[1,2-a]pyridine-6-boronic acid, compound **23** was obtained by referring to the synthesis of compound **11.** ¹H NMR (500 MHz, DMSO-*d*₆) δ 9.23 (s, 1H), 9.10 - 8.99 (m, 1H), 8.90 - 8.79 (m, 1H), 8.65 (t, *J* = 5.2 Hz, 1H), 8.22 - 8.14 (m, 1H), 8.06 (dd, *J* = 8.2, 4.2 Hz, 2H), 7.98 - 7.84 (m, 2H), 7.83 - 7.59 (m, 3H), 7.58 - 7.40 (m, 2H), 7.40 - 7.29 (m, 1H), 4.79 - 4.66 (m, 2H); MS (ESI): m/z 404.2 (M+H)⁺.

### Example 24:

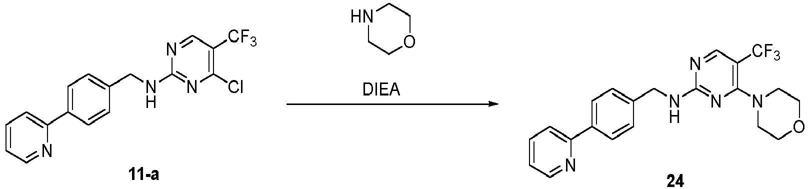

Compound **11-a** (30.0 mg, 82.2 µmol) and morpholine (14.3 mg, 164 µmol) were dissolved in N,N-dimethylformamide (2 mL), and diisopropylethylamine (45.7 mg, 353 µmol) was added. The mixture was stirred at 80°C for 2 hours. After the reaction solution was cooled to room temperature, compound **24** (10 mg, 24.07 µmol) was obtained as a white solid with a yield of 29.27%. ¹H NMR (500 MHz, DMSO-*d*₆) δ 8.68 - 8.60 (m, 1H), 8.32 - 8.23 (m, 1H), 8.23 - 8.00 (m, 3H), 7.95 - 7.90 (m, 1H), 7.89 - 7.84 (m, 1H), 7.46 - 7.37 (m, 2H), 7.36 - 7.30 (m, 1H), 4.62 - 4.45 (m, 2H), 3.70 - 3.53 (m, 4H), 3.52 - 3.41 (m, 4H); MS (ESI): m/z 416.4 (M+H)⁺.

### Example 25:

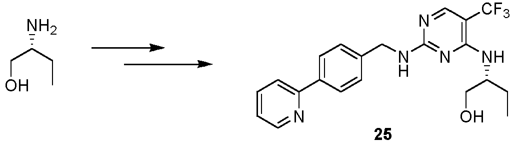

Starting from (R)-2-aminobutanol, compound **25** was obtained by referring to the synthesis of compound **24.** ¹H NMR (500 MHz, DMSO-*d*₆) δ 8.68 - 8.62 (m, 1H), 8.08 - 7.76 (m, 6H), 7.45 - 7.37 (m, 2H), 7.37 - 7.30 (m, 1H), 5.94 - 5.81 (m, 1H), 4.82 - 4.71 (m, 1H), 4.61 - 4.41 (m, 2H), 4.27 - 4.04 (m, 1H), 3.54 - 3.35 (m, 2H), 1.68 - 1.40 (m, 2H), 0.91 - 0.65 (m, 3H); MS (ESI): m/z 418.4 (M+H)⁺.

### Example 26:

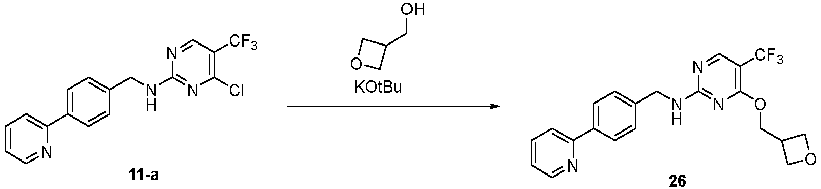

Compound **11-a** (30.0 mg, 82.3 µmol) and oxetan-3-ylmethanol (10.9 mg, 123 µmol) were dissolved in tetrahydrofuran (4 mL), and potassium tert-butoxide (18.5 mg, 164 µmol) was added. The mixture was stirred at 80°C for 16 hours. After the reaction solution was cooled to room temperature, 10 mL of ammonium chloride was added, and the mixture was extracted with 20 mL of ethyl acetate. The organic phase was washed three times with water and once with saturated brine, dried over anhydrous sodium sulfate, filtered and concentrated. The residue was purified by preparative liquid chromatography to obtain compound **26** (15.0 mg, 36.0 µmol) as a white solid, yield 43.8%. ¹H NMR (500 MHz, DMSO-*d*₆) δ 8.70 - 8.45 (m, 2H), 8.39 - 8.32 (m, 1H), 8.08 - 8.01 (m, 2H), 7.95 - 7.90 (m, 1H), 7.90 - 7.84 (m, 1H), 7.48 - 7.39 (m, 2H), 7.36 - 7.31 (m, 1H), 4.70 - 4.64 (m, 1H), 4.61 - 4.32 (m, 7H), 3.30 - 3.23 (m, 1H); MS (ESI): m/z 417.3 (M+H)⁺.

### Example 27:

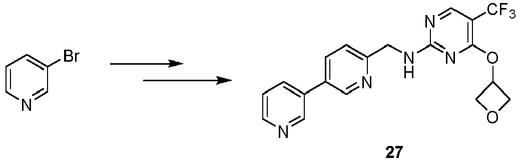

Starting from 3-bromopyridine, compound **27** was obtained by referring to the synthesis of compound **4.** ¹H NMR (500 MHz, DMSO-*d*₆) δ 8.99 - 8.86 (m, 2H), 8.72 - 8.38 (m, 3H), 8.19 - 8.07 (m, 2H), 7.59 - 7.48 (m, 1H), 7.47 - 7.37 (m, 1H), 5.73 - 5.39 (m, 1H), 4.95 - 4.54 (m, 5H), 4.41 - 4.35 (m, 1H); MS (ESI): m/z 404.3 (M+H)⁺.

### Example 28:

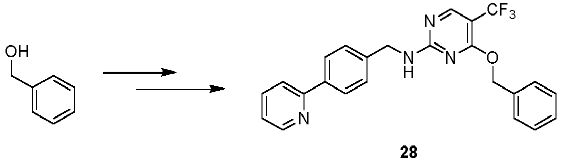

Starting from benzyl alcohol, compound **28** was obtained by referring to the synthesis of compound **26.** ¹H NMR (500 MHz, DMSO-*d*₆) δ 8.68 - 8.48 (m, 2H), 8.42 - 8.34 (m, 1H), 8.06 - 8.01 (m, 2H), 7.95 - 7.89 (m, 1H), 7.90 - 7.84 (m, 1H), 7.49 - 7.25 (m, 8H), 5.52 - 5.43 (m, 2H), 4.66 - 4.55 (m, 2H); MS (ESI): m/z 437.5(M+H)⁺.

### Example 29:

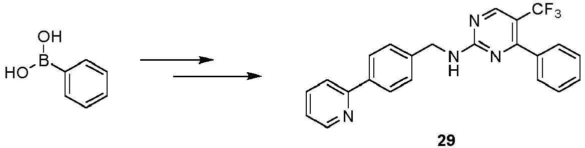

Compound **29** was obtained by referring to the synthesis of compound **11** starting from phenylboronic acid. 1H NMR (500 MHz, DMSO-d6) δ 8.79 - 8.57 (m, 3H), 8.10 - 7.98 (m, 2H), 7.97 - 7.91 (m, 1H), 7.90 - 7.81 (m, 1H), 7.55 - 7.40 (m, 7H), 7.38 - 7.28 (m, 1H), 4.71 - 4.57 (m, 2H); MS (ESI): m/z 407.2 (M+H)+.

### Example 30:

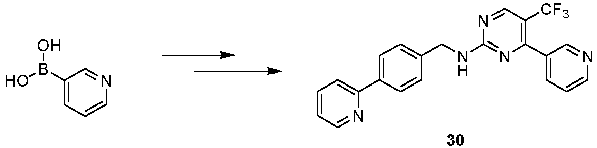

Compound **30** was obtained by referring to the synthesis of compound **11** starting from pyridin-3-ylboronic acid. 1H NMR (500 MHz, DMSO-d6) δ 8.86 - 8.78 (m, 1H), 8.78 - 8.57 (m, 4H), 8.08 - 8.02 (m, 2H), 7.96 - 7.84 (m, 3H), 7.58 - 7.51 (m, 1H), 7.49 - 7.40 (m, 2H), 7.37 - 7.30 (m, 1H), 4.69 - 4.60 (m, 2H); MS (ESI): m/z 408.4 (M+H)+.

### Example 31:

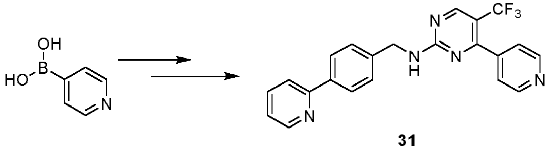

Compound **31** was obtained by referring to the synthesis of compound **11** starting from pyridin-4-ylboronic acid. ¹H NMR (500 MHz, DMSO-*d*₆) δ 8.94 - 8.80 (m, 1H), 8.80 - 8.68 (m, 3H), 8.68 - 8.59 (m, 1H), 8.10 - 8.00 (m, 2H), 7.97 - 7.91 (m, 1H), 7.91 - 7.81 (m, 1H), 7.54 - 7.38 (m, 4H), 7.38 - 7.28 (m, 1H), 4.72 - 4.55 (m, 2H); MS (ESI): m/z 408.3 (M+H)⁺.

### Example 32:

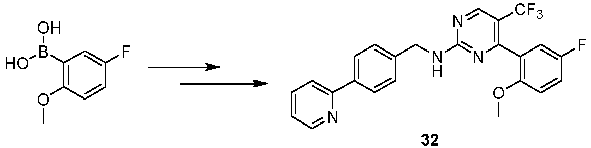

Compound **32** was obtained by referring to the synthesis of compound **11** starting from (5-fluoro-2-methoxyphenyl)boronic acid. 1H NMR (500 MHz, DMSO-d6) δ 8.78 - 8.57 (m, 3H), 8.11 - 7.98 (m, 2H), 7.98 - 7.91 (m, 1H), 7.91 - 7.82 (m, 1H), 7.51 - 7.37 (m, 2H), 7.37 - 7.22 (m, 2H), 7.21 - 7.01 (m, 2H), 4.76 - 4.46 (m, 2H), 3.74 - 3.61 (m, 3H); MS (ESI): m/z 455.3 (M+H)+.

### Example 33:

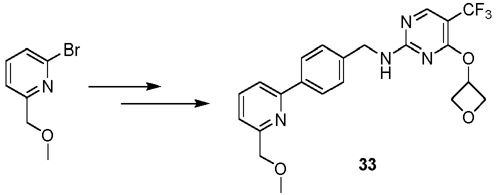

Compound **33** was obtained by referring to the synthesis of compound **12** starting from 2-bromo-6-(methoxymethyl)pyridine. 1H NMR (500 MHz, DMSO-d6) δ 8.72 - 8.38 (m, 2H), 8.09 - 7.81 (m, 4H), 7.46 - 7.34 (m, 3H), 5.70 - 5.52 (m, 1H), 4.92 - 4.71 (m, 2H), 4.61 - 4.42 (m, 6H), 3.41 (s, 3H); MS (ESI): m/z 447.3 (M+H)+.

### Example 34:

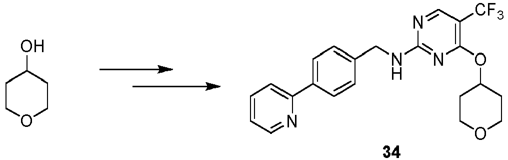

Compound **34** was obtained by referring to the synthesis of compound **26** starting from tetrahydropyran-4-ol. 1H NMR (500 MHz, DMSO-d6) δ 8.68 - 8.63 (m, 1H), 8.63 - 8.40 (m, 1H), 8.39 - 8.32 (m, 1H), 8.08 - 8.00 (m, 2H), 7.95 - 7.90 (m, 1H), 7.90 - 7.83 (m, 1H), 7.46 - 7.38 (m, 2H), 7.37 - 7.29 (m, 1H), 5.45 - 5.17 (m, 1H), 4.65 - 4.46 (m, 2H), 3.85 - 3.64 (m, 2H), 3.59 - 3.39 (m, 2H), 2.05 - 1.73 (m, 2H), 1.71 - 1.43 (m, 2H); MS (ESI): m/z 431.3 (M+H)+.

### Example 35:

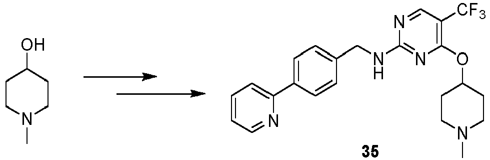

Starting from N-methyl-4-piperidinol, compound **35** was obtained by referring to the synthesis of compound **26.** 1H NMR (500 MHz, DMSO-d6) δ 8.68 - 8.63 (m, 1H), 8.62 - 8.36 (m, 1H), 8.37 - 8.30 (m, 1H), 8.06 - 8.00 (m, 2H), 7.95 - 7.89 (m, 1H), 7.90 - 7.83 (m, 1H), 7.47 - 7.38 (m, 2H), 7.37 - 7.30 (m, 1H), 5.25 - 4.99 (m, 1H), 4.62 - 4.47 (m, 2H), 2.46 - 2.21 (m, 2H), 2.19 - 2.10 (m, 4H), 1.97 - 1.51 (m, 4H); MS (ESI): m/z 444.4 (M+H)+.

### Example 36:

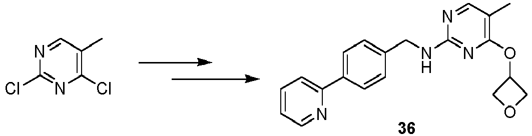

Starting from 2,4-dichloro-5-methylpyrimidine, compound **36** was obtained by referring to the synthesis of compound **3.** ¹H NMR (500 MHz, DMSO-*d*₆) δ 8.70 - 8.61 (m, 1H), 8.03 (d, *J* = 8.0 Hz, 2H), 7.93 (d, *J* = 9.0 Hz, 2H), 7.86 (td, *J* = 7.7, 1.8 Hz, 1H), 7.64 - 7.45 (m, 1H), 7.40 (d, *J=* 8.0 Hz, 2H), 7.33 (dd, *J=* 7.4, 4.8 Hz, 1H), 5.61 - 5.36 (m, 1H), 4.95 - 4.62 (m, 2H), 4.61 - 4.32 (m, 4H), 1.96 (s, 3H); MS (ESI): m/z 349.3 (M+H)⁺.

### Example 37:

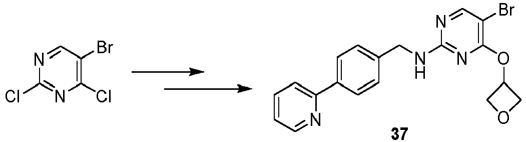

Starting from 2,4-dichloro-5-bromopyrimidine, compound **37** was obtained by referring to the synthesis of compound **1.** ¹H NMR (500 MHz, DMSO-*d*₆) δ 8.73 - 8.58 (m, 1H), 8.22 (s, 1H), 8.19 - 7.81 (m, 5H), 7.39 (d, *J* = 8.0 Hz, 2H), 7.37 - 7.29 (m, 1H), 5.65 - 5.37 (m, 1H), 4.98 - 4.65 (m, 2H), 4.64 - 4.35 (m, 4H); MS (ESI): m/z 413.2 (M+H)⁺.

### Example 38:

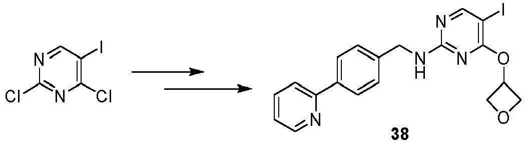

Starting from 2,4-dichloro-5-iodopyrimidine, compound **38** was obtained by referring to the synthesis of compound **1.** ¹H NMR (500 MHz, DMSO-*d*₆) δ 8.64 (d, *J* = 4.8 Hz, 1H), 8.29 (s, 1H), 8.16 - 7.96 (m, 3H), 7.92 (d, *J* = 8.0 Hz, 1H), 7.88 - 7.83 (m, 1H), 7.39 (d, *J* = 8.0 Hz, 2H), 7.33 (dd, *J* = 7.3, 4.9 Hz, 1H), 5.61 - 5.36 (m, 1H), 4.96 - 4.64 (m, 2H), 4.61 - 4.37 (m, 4H); MS (ESI): m/z 461.2 (M+H)⁺.

### Example 39:

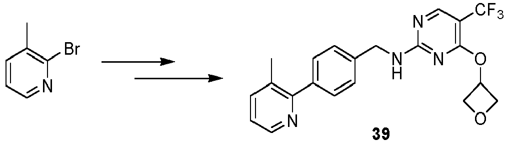

Starting from 2-bromo-3-methylpyridine, compound 39 was obtained by referring to the synthesis of compound 12. ¹H NMR (500 MHz, DMSO-*d*₆) δ 8.74 - 8.34 (m, 3H), 7.76 - 7.49 (m, 3H), 7.46 - 7.24 (m, 3H), 5.71 - 5.52 (m, 1H), 4.93 - 4.68 (m, 2H), 4.66 - 4.42 (m, 4H), 2.34 - 2.26 (m, 3H); MS (ESI): m/z 417.4 (M+H)⁺.

### Example 40:

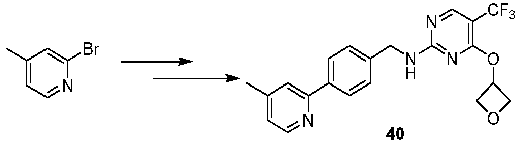

Starting from 2-bromo-4-methylpyridine, compound 40 was obtained by referring to the synthesis of compound 12. ¹H NMR (500 MHz, DMSO-*d*₆) δ 8.73 - 8.36 (m, 3H), 8.13 - 7.73 (m, 3H), 7.47 - 7.14 (m, 3H), 5.69 - 5.51 (m, 1H), 4.93 - 4.69 (m, 2H), 4.62 - 4.40 (m, 4H), 2.38 (s, 3H); MS (ESI): m/z 417.4 (M+H)⁺.

### Example 41:

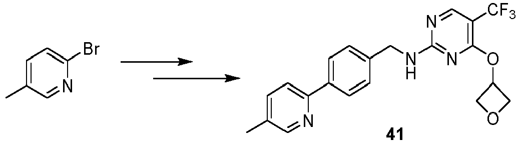

Starting from 2-bromo-5-methylpyridine, compound 41 was obtained by referring to the synthesis of compound 12. ¹H NMR (500 MHz, DMSO-*d*₆) δ 8.69 - 8.37 (m, 3H), 8.06 - 7.81 (m, 3H), 7.74 - 7.38 (m, 3H), 5.70 - 5.52 (m, 1H), 4.92 - 4.71 (m, 2H), 4.62 - 4.49 (m, 3H), 4.46 - 4.43 (m, 1H), 2.33 (s, 3H); MS (ESI): m/z 417.6 (M+H)⁺.

### Example 42:

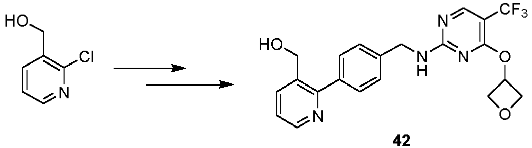

Compound **42** was obtained by referring to the synthesis of compound **12** starting from 2-chloro-3-pyridinemethanol. 1H NMR (500 MHz, DMSO-d6) δ 8.72 - 8.34 (m, 3H), 7.98 - 7.93 (m, 1H), 7.57 - 7.52 (m, 2H), 7.43 - 7.37 (m, 3H), 5.71 - 5.53 (m, 1H), 5.44 - 5.32 (m, 1H), 4.94 - 4.70 (m, 2H), 4.67 - 4.42 (m, 6H); MS (ESI): m/z 433.4 (M+H)+.

### Example 43:

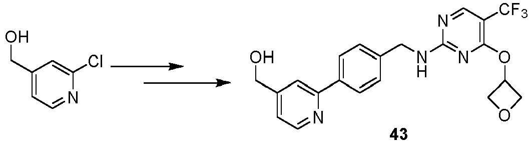

Compound **43** was obtained following the synthesis of compound **12** starting from (2-chloro-4-pyridyl)methanol. 1H NMR (500 MHz, DMSO-d6) δ 8.74 - 8.35 (m, 3H), 8.11 - 7.83 (m, 3H), 7.48 - 7.26 (m, 3H), 5.71 - 5.52 (m, 1H), 5.49 - 5.43 (m, 1H), 4.95 - 4.70 (m, 2H), 4.63 - 4.42 (m, 6H); MS (ESI): m/z 433.4 (M+H)+.

### Example 44:

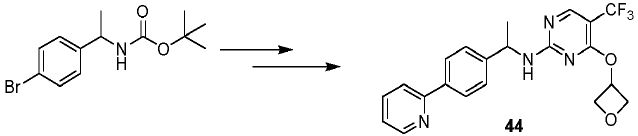

Compound **44** was obtained by referring to the synthesis of compound **12** starting from (1-(4-bromophenyl)ethyl)carbamic acid tert-butyl ester. 1H NMR (500 MHz, DMSO-d6) δ 8.75 - 8.35 (m, 3H), 8.12 - 8.00 (m, 2H), 7.98 - 7.83 (m, 2H), 7.57 - 7.28 (m, 3H), 5.77 - 5.46 (m, 1H), 5.30 - 4.18 (m, 5H), 1.55 - 1.44 (m, 3H); MS (ESI): m/z 417.4 (M+H)+.

### Example 45:

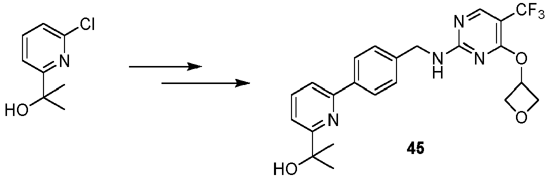

Starting from 2-(6-chloropyridin-2-yl)propan-2-ol, compound **45** was obtained by referring to the synthesis of compound **12.** ¹H NMR (500 MHz, DMSO-*d*₆) δ 8.72 - 8.39 (m, 2H), 8.11 - 8.01 (m, 2H), 7.87 - 7.72 (m, 2H), 7.62 - 7.39 (m, 3H), 5.68 - 5.52 (m, 1H), 5.24 (s, 1H), 4.92 - 4.72 (m, 2H), 4.61 - 4.44 (m, 4H), 1.51 (s, 6H); MS (ESI): m/z 461.4 (M+H)⁺.

### Example 46:

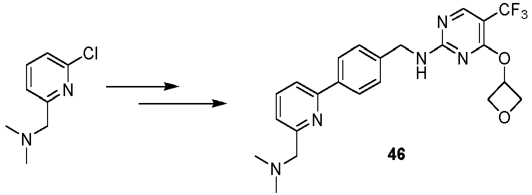

Compound **46** was obtained by referring to the synthesis of compound **12** starting from 1-(6-chloropyridin-2-yl)-N,N-dimethylmethylamine. 1H NMR (500 MHz, DMSO-d6) δ 8.73 - 8.37 (m, 2H), 8.11 - 7.75 (m, 4H), 7.46 - 7.34 (m, 3H), 5.72 - 5.51 (m, 1H), 4.98 - 4.67 (m, 2H), 4.63 - 4.38 (m, 4H), 3.58 (s, 2H), 2.23 (s, 6H); MS (ESI): m/z 460.4 (M+H)+.

### Example 47:

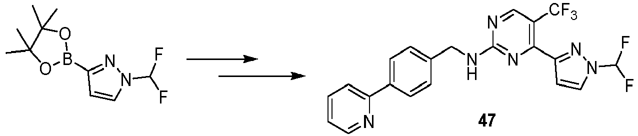

Compound **47** was obtained by referring to the synthesis of compound **11** starting from 1-(difluoromethyl)-1H-pyrazole-3-boronic acid pinacol ester. 1H NMR (500 MHz, DMSO-d6) δ 8.84 - 8.68 (m, 2H), 8.68 - 8.62 (m, 1H), 8.42 - 8.33 (m, 1H), 8.05 (d, J = 8.0 Hz, 2H), 8.03 - 7.76 (m, 3H), 7.52 - 7.42 (m, 2H), 7.38 - 7.29 (m, 1H), 6.96 - 6.88 (m, 1H), 4.70 - 4.64 (m, 2H); MS (ESI): m/z 447.4 (M+H)+.

### Example 48:

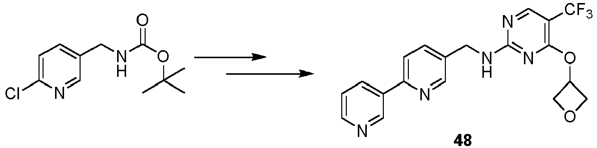

Compound **48** was obtained by referring to the synthesis of compound **12** starting from tert-butyl ((6-chloropyridin-3-yl)methyl)carbamate. 1H NMR (500 MHz, DMSO-d6) δ 9.27 - 9.22 (m, 1H), 8.72 - 8.37 (m, 5H), 8.06 - 7.99 (m, 1H), 7.86 - 7.80 (m, 1H), 7.55 - 7.48 (m, 1H), 5.70 - 5.53 (m, 1H), 4.93 - 4.74 (m, 2H), 4.63 - 4.45 (m, 4H); MS (ESI): m/z 404.3 (M+H)+.

### Example 49:

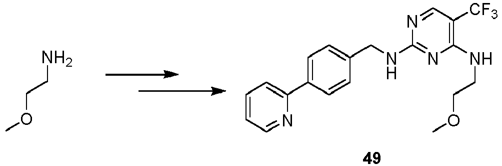

Starting from 2-methoxyethylamine, compound **49** was obtained by referring to the synthesis of compound **24.** ¹H NMR (500 MHz, DMSO-*d*₆) δ 8.71 - 8.56 (m, 1H), 8.07 - 7.79 (m, 6H), 7.46 - 7.26 (m, 3H), 6.81 - 6.51 (m, 1H), 4.63 - 4.43 (m, 2H), 3.62 - 3.38 (m, 3H), 3.29 - 3.24 (m, 2H), 3.17 - 3.07 (m, 2H); MS (ESI): m/z 404.3 (M+H)⁺.

### Example 50:

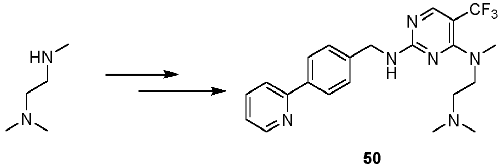

Starting from N,N,N'-trimethylethylenediamine, compound **50** was obtained by referring to the synthesis of compound **24.** ¹H NMR (500 MHz, DMSO-*d*₆) δ 8.64 (d, *J* = 5.0 Hz, 1H), 8.24 - 8.17 (m, 1H), 8.08 - 7.95 (m, 3H), 7.94 - 7.88 (m, 1H), 7.88 - 7.83 (m, 1H), 7.38 (d, *J* = 7.9 Hz, 2H), 7.32 (dd, *J* = 7.3, 4.9 Hz, 1H), 4.53 (d, *J* = 6.4 Hz, 2H), 3.64 - 3.49 (m, 2H), 3.01 (s, 3H), 2.41 - 2.28 (m, 2H), 2.20 - 2.01 (m, 6H); MS (ESI): m/z 431.5 (M+H)⁺.

### Example 51:

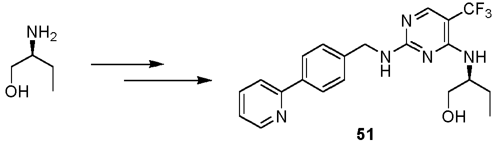

Starting from (S)-2-aminobutanol, compound **51** was obtained by referring to the synthesis of compound **24.** ¹H NMR (500 MHz, DMSO-*d*₆) δ 8.66 - 8.60 (m, 1H), 8.10 - 7.99 (m, 3H), 7.99 - 7.81 (m, 3H), 7.45 - 7.37 (m, 2H), 7.35 - 7.29 (m, 1H), 5.94 - 5.78 (m, 1H), 4.84 - 4.67 (m, 1H), 4.62 - 4.35 (m, 2H), 4.23 - 4.02 (m, 1H), 3.56 - 3.35 (m, 2H), 1.61 - 1.37 (m, 2H), 0.88 - 0.67 (m, 3H); MS (ESI): m/z 418.4 (M+H)⁺.

### Example 52:

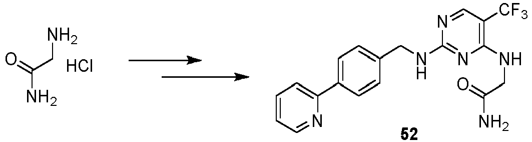

Compound **52** was obtained by referring to the synthesis of compound **24** starting from glycinamide hydrochloride. ¹H NMR (500 MHz, DMSO-*d*₆) δ 8.74 - 8.57 (m, 1H), 8.12 - 7.94 (m, 4H), 7.94 - 7.82 (m, 2H), 7.51 - 7.29 (m, 4H), 7.17 - 7.04 (m, 1H), 6.93 - 6.63 (m, 1H), 4.59 - 4.45 (m, 2H), 3.98 - 3.86 (m, 2H); MS (ESI): m/z 403.2 (M+H)⁺.

### Example 53:

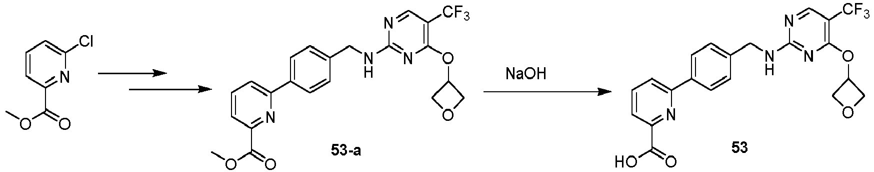

Compound **53-a** was obtained by referring to the synthesis of compound **12** starting from 6-chloropyridine-2-carboxylic acid methyl ester.

Compound **53-a** (30.0 mg, 65.2 µmol) was dissolved in a mixed solvent of tetrahydrofuran (2 mL) and methanol (1 mL), and 1N sodium hydroxide solution (1 mL) was added. The mixture was stirred at room temperature for 6 hours. Add 1N hydrochloric acid solution to the reaction solution to pH=7, and concentrate to dryness. The residue was purified by preparative liquid chromatography to obtain compound **53** (10.1 mg, 22.4 µmol), a white solid, with a yield of 34.4%. ¹H NMR (500 MHz, DMSO-*d*₆) δ 8.70 - 8.37 (m, 2H), 8.21 - 8.02 (m, 3H), 8.01 - 7.88 (m, 2H), 7.50 - 7.40 (m, 2H), 5.72 - 5.49 (m, 1H), 4.93 - 4.70 (m, 2H), 4.64 - 4.38 (m, 4H); MS (ESI): m/z 447.4 (M+H)⁺.

### Example 54:

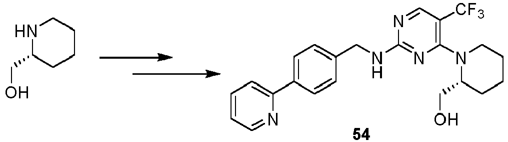

Compound **54** was obtained by referring to the synthesis of compound **24** starting from (R)-2-piperidinemethanol. ¹H NMR (500 MHz, DMSO-*d*₆) δ 8.73 - 8.57 (m, 1H), 8.26 - 8.14 (m, 1H), 8.12 - 7.98 (m, 3H), 7.98 - 7.80 (m, 2H), 7.49 - 7.28 (m, 3H), 4.68 - 4.60 (m, 1H), 4.60 - 4.46 (m, 2H), 4.36 - 4.24 (m, 1H), 3.73 - 3.43 (m, 3H), 3.12 - 2.99 (m, 1H), 1.87 - 1.74 (m, 1H), 1.64 - 1.35 (m, 5H); MS (ESI): m/z 444.5 (M+H)⁺.

### Example 55:

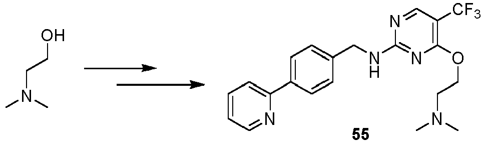

Compound **55** was obtained by referring to the synthesis of compound **26** starting from N,N-dimethylethanolamine. ¹H NMR (500 MHz, DMSO-*d*₆) δ 8.66 - 8.62 (m, 1H), 8.61 - 8.37 (m, 1H), 8.36 - 8.29 (m, 1H), 8.08 - 7.99 (m, 2H), 7.96 - 7.83 (m, 2H), 7.46 - 7.29 (m, 3H), 4.66 - 4.51 (m, 2H), 4.51 - 4.36 (m, 2H), 2.65 - 2.51 (m, 2H), 2.24 - 2.06 (m, 6H); MS (ESI): m/z 418.5 (M+H)⁺.

### Example 56:

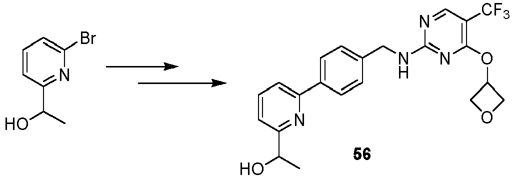

Compound **56** was obtained by referring to the synthesis of compound **12** starting from 1-(6-bromopyridin-2-yl)ethan-1-ol. ¹H NMR (500 MHz, DMSO-*d*₆) δ 8.72 - 8.33 (m, 2H), 8.16 - 7.95 (m, 2H), 7.89 - 7.68 (m, 2H), 7.49 - 7.37 (m, 3H), 5.65 - 5.52 (m, 1H), 5.39 - 5.31 (m, 1H), 4.96 - 4.80 (m, 1H), 4.79 - 4.69 (m, 2H), 4.61 - 4.41 (m, 4H), 1.48 - 1.36 (m, 3H); MS (ESI): m/z 447.5 (M+H)⁺.

### Example 57:

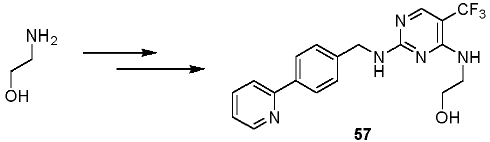

Compound **57** was obtained by referring to the synthesis of compound **24** starting from ethanolamine. ¹H NMR (500 MHz, DMSO-*d*₆) δ 8.67 - 8.60 (m, 1H), 8.05 - 7.76 (m, 6H), 7.50 - 7.28 (m, 3H), 6.67 - 6.43 (m, 1H), 4.77 - 4.60 (m, 1H), 4.59 - 4.45 (m, 2H), 3.49 - 3.37 (m, 4H); MS (ESI): m/z 390.4 (M+H)⁺.

### Example 58:

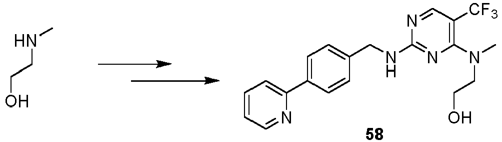

Compound **58** was obtained by referring to the synthesis of compound **24** starting from 2-(methylamino)ethanol. ¹H NMR (500 MHz, DMSO-*d*₆) δ 8.72 - 8.59 (m, 1H), 8.27 - 8.12 (m, 1H), 8.10 - 7.95 (m, 3H), 7.95 - 7.81 (m, 2H), 7.45 - 7.36 (m, 2H), 7.36 - 7.27 (m, 1H), 4.65 - 4.44 (m, 2H), 3.66 - 3.47 (m, 2H), 3.03 - 2.98 (m, 2H), 2.35 - 2.09 (m, 3H); MS (ESI): m/z 404.2 (M+H)⁺.

### Example 59:

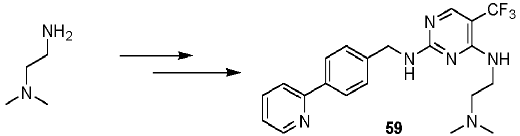

Compound **59** was obtained by referring to the synthesis of compound **24** starting from N,N-dimethylethylenediamine. ¹H NMR (500 MHz, DMSO-*d*₆) δ 8.65 - 8.59 (m, 1H), 8.27 - 7.96 (m, 5H), 7.93 - 7.80 (m, 2H), 7.42 - 7.30 (m, 3H), 4.82 - 4.35 (m, 4H), 3.72 - 3.55 (m, 2H), 3.55 - 3.49 (m, 3H), 3.07 - 3.04 (m, 3H); MS (ESI): m/z 417.7 (M+H)⁺.

### Example 60:

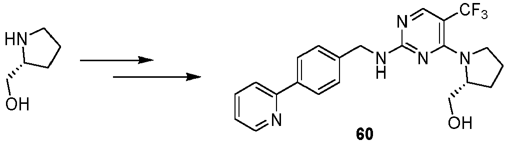

Starting from D-prolinol, compound **60** was obtained by referring to the synthesis of compound **24.** ¹H NMR (500 MHz, DMSO-*d*₆) δ 8.66 - 8.62 (m, 1H), 8.21 - 8.13 (m, 1H), 8.04 - 7.99 (m, 2H), 7.97 - 7.80 (m, 3H), 7.46 - 7.28 (m, 3H), 4.64 - 4.58 (m, 1H), 4.57 - 4.41 (m, 3H), 3.62 - 3.32 (m, 4H), 1.98 - 1.73 (m, 4H); MS (ESI): m/z 430.5 (M+H)⁺.

### Example 61:

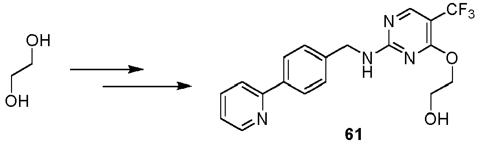

Starting from ethylene glycol, compound **61** was obtained by referring to the synthesis of compound **26.** ¹H NMR (500 MHz, DMSO-*d*₆) δ 8.70 - 8.26 (m, 3H), 8.07 - 7.99 (m, 2H), 7.95 - 7.82 (m, 2H), 7.48 - 7.37 (m, 2H), 7.36 - 7.29 (m, 1H), 4.87 - 4.73 (m, 1H), 4.65 - 4.50 (m, 2H), 4.45 - 4.32 (m, 2H), 3.79 - 3.58 (m, 2H); MS (ESI): m/z 391.5 (M+H)⁺.

### Example 62:

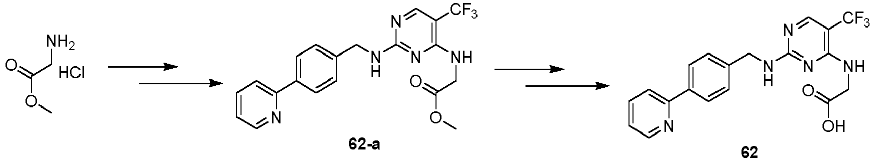

Starting from glycine methyl ester hydrochloride, compound **62-a** was obtained with reference to the synthesis of compound **24.**

Starting from compound **62-a,** compound **62** was obtained by referring to the synthesis of compound **53.** ¹H NMR (500 MHz, DMSO-*d*₆) δ 8.66 - 8.60 (m, 1H), 8.17 - 7.74 (m, 6H), 7.46 - 7.29 (m, 3H), 7.16 - 6.78 (m, 1H), 4.55 - 4.44 (m, 2H), 4.03 - 3.78 (m, 2H); MS (ESI): m/z 404.2 (M+H)⁺.

### Example 63:

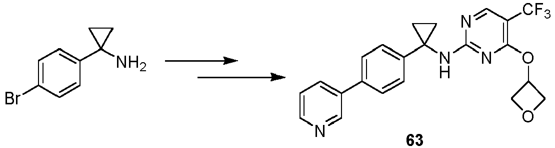

Starting from 1-(4-bromophenyl)cyclopropylamine, compound **63** was obtained by referring to the synthesis of compound **12.** ¹H NMR (500 MHz, DMSO-*d*₆) δ 9.01 - 8.67 (m, 2H), 8.65 - 8.37 (m, 2H), 8.06 - 7.98 (m, 1H), 7.73 - 7.53 (m, 2H), 7.52 - 7.20 (m, 3H), 5.69 - 5.27 (m, 1H), 4.70 - 4.27 (m, 4H), 1.40 - 1.22 (m, 4H); MS (ESI): m/z 429.5 (M+H)⁺.

### Example 64:

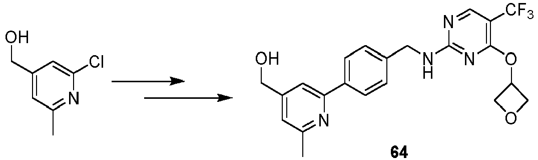

Starting from (2-chloro-6-methylpyridin-4-yl)methanol, compound **64** was obtained following the synthesis of compound **12.** ¹H NMR (500 MHz, DMSO-*d*₆) δ 8.67 - 8.36 (m, 2H), 8.08 - 7.92 (m, 2H), 7.68 - 7.60 (m, 1H), 7.46 - 7.35 (m, 2H), 7.13 (s, 1H), 5.71 - 5.49 (m, 1H), 5.47 - 5.34 (m, 1H), 4.90 - 4.70 (m, 2H), 4.62 - 4.40 (m, 6H), 2.51 - 2.50 (m, 3H); MS (ESI): m/z 447.5 (M+H)⁺.

### Example 65:

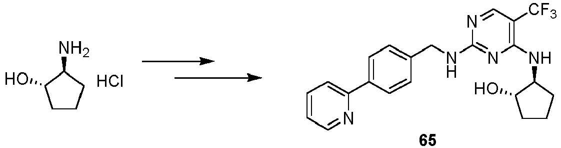

Starting from (1S,2S)-2-aminocyclopentanol hydrochloride, compound **65** was obtained by referring to the synthesis of compound **24.** ¹H NMR (500 MHz, DMSO-*d*₆) δ 8.67 - 8.61 (m, 1H), 8.07 - 8.00 (m, 3H), 7.99 - 7.76 (m, 3H), 7.51 - 7.29 (m, 3H), 6.33 - 6.04 (m, 1H), 4.81 - 4.64 (m, 1H), 4.64 - 4.37 (m, 2H), 4.31 - 3.94 (m, 2H), 1.93 - 1.36 (m, 6H); MS (ESI): m/z 430.5 (M+H)⁺.

### Example 66:

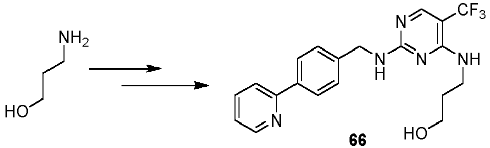

Starting from 3-aminopropanol, compound **66** was obtained by referring to the synthesis of compound **24.** ¹H NMR (500 MHz, DMSO-*d*₆) δ 8.67 - 8.61 (m, 1H), 8.05 - 7.73 (m, 6H), 7.47 - 7.14 (m, 3H), 6.92 - 6.62 (m, 1H), 4.61 - 4.45 (m, 3H), 3.49 - 3.36 (m, 4H), 1.72 - 1.55 (m, 2H); MS (ESI): m/z 404.2 (M+H)⁺.

### Example 67:

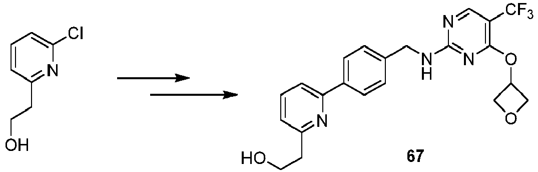

Starting from 2-(6-chloropyridin-2-yl)ethan-1-ol, compound **67** was obtained by referring to the synthesis of compound **12.** ¹H NMR (500 MHz, DMSO-*d*₆) δ 8.71 - 8.36 (m, 2H), 8.08 - 7.96 (m, 2H), 7.81 - 7.69 (m, 2H), 7.45 - 7.37 (m, 2H), 7.24 - 7.19 (m, 1H), 5.66 - 5.51 (m, 1H), 4.90 - 4.70 (m, 2H), 4.65 (s, 1H), 4.59 - 4.40 (m, 4H), 3.86 - 3.77 (m, 2H), 2.95 - 2.90 (m, 2H); MS (ESI): m/z 447.3 (M+H)⁺.

### Example 68:

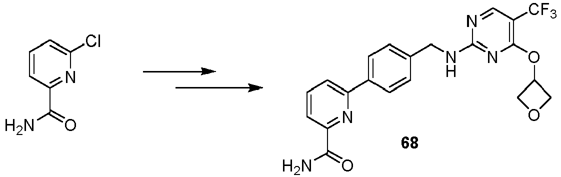

Starting from 6-chloropyridinamide, compound **68** was obtained by referring to the synthesis of compound **12.** ¹H NMR (500 MHz, DMSO-*d*₆) δ 8.71 - 8.38 (m, 2H), 8.29 - 8.10 (m, 4H), 8.07 - 7.90 (m, 2H), 7.69 (s, 1H), 7.45 - 7.38 (m, 2H), 5.69 - 5.49 (m, 1H), 4.91 - 4.70 (m, 2H), 4.61 - 4.42 (m, 4H); MS (ESI): m/z 446.3 (M+H)⁺.

### Example 69:

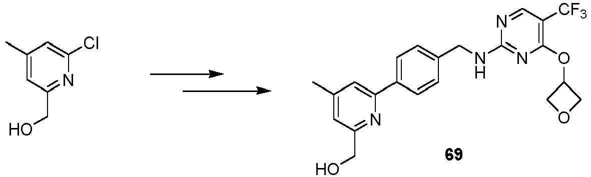

Starting from (6-chloro-4-methylpyridin-2-yl)methanol, compound **69** was obtained following the synthesis of compound **12.** ¹H NMR (500 MHz, DMSO-*d*₆) δ 8.71 - 8.36 (m, 2H), 8.11 - 7.94 (m, 2H), 7.72 - 7.55 (m, 1H), 7.51 - 7.33 (m, 2H), 7.31 - 7.17 (m, 1H), 5.68 - 5.49 (m, 1H), 5.43 - 5.28 (m, 1H), 4.93 - 4.66 (m, 2H), 4.61 - 4.40 (m, 6H), 2.41 - 2.36 (m, 3H); MS (ESI): m/z 447.4 (M+H)⁺.

### Example 70:

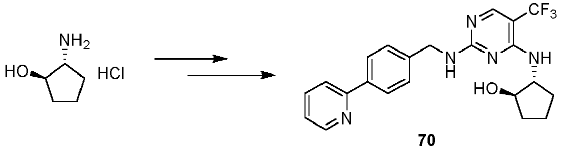

Starting from (1R,2R)-2-aminocyclopentanol hydrochloride, compound **70** was obtained by referring to the synthesis of compound **24.** ¹H NMR (500 MHz, DMSO-*d*₆) δ 8.70 - 8.61 (m, 1H), 8.09 - 8.00 (m, 3H), 7.99 - 7.75 (m, 3H), 7.54 - 7.27 (m, 3H), 6.24 - 6.12 (m, 1H), 4.78 - 4.65 (m, 1H), 4.62 - 4.40 (m, 2H), 4.28 - 3.96 (m, 2H), 1.94 - 1.25 (m, 6H); MS (ESI): m/z 430.5 (M+H)⁺.

### Example 71:

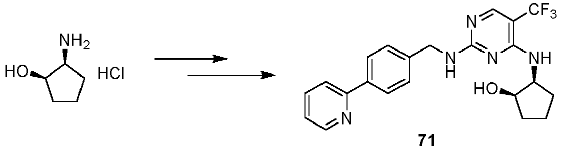

Starting from (1R,2S)-2-aminocyclopentanol hydrochloride, compound **71** was obtained by referring to the synthesis of compound **24.** ¹H NMR (500 MHz, DMSO-*d*₆) δ 8.67 - 8.62 (m, 1H), 8.12 - 7.99 (m, 4H), 7.97 - 7.81 (m, 2H), 7.44 - 7.37 (m, 2H), 7.36 - 7.29 (m, 1H), 6.01 - 5.91 (m, 1H), 5.26 - 5.12 (m, 1H), 4.62 - 4.39 (m, 2H), 4.23 - 3.91 (m, 2H), 1.90 - 1.32 (m, 6H); MS (ESI): m/z 430.5 (M+H)⁺.

### Example 72:

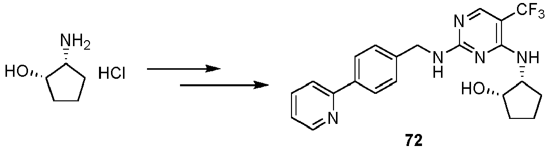

Starting from (1S,2R)-2-aminocyclopentanol hydrochloride, compound **72** was obtained by referring to the synthesis of compound **24.** ¹H NMR (500 MHz, DMSO-*d*₆) δ 8.75 - 8.57 (m, 1H), 8.14 - 7.94 (m, 4H), 7.92 - 7.83 (m, 2H), 7.46 - 7.27 (m, 3H), 6.06 - 5.91 (m, 1H), 5.31 - 5.12 (m, 1H), 4.66 - 4.40 (m, 2H), 4.20 - 3.88 (m, 2H), 1.86 - 1.27 (m, 6H); MS (ESI): m/z 430.5 (M+H)⁺.

### Example 73:

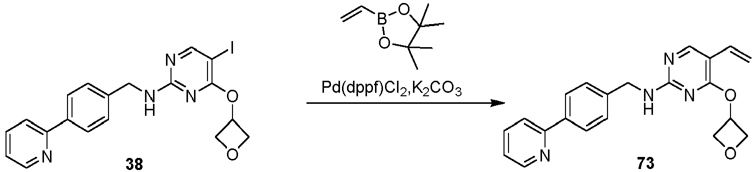

Compound **38** (30.0 mg, 65.2 µmol) and vinylboronic acid pinacol ester (50.2 mg, 326 µmol) were dissolved in a mixture of 1,4-dioxane (3 mL) and water (0.3 mL), and potassium carbonate (18.0 mg, 130 µmol) and 1,1'-bis(diphenylphosphino)ferrocenepalladium(II) dichloride (4.8 mg, 6.52 µmol) were added. The mixture was stirred at 95°C for 3 hours under nitrogen atmosphere. The reaction solution was cooled t to room temperature, added with 20 mL of ethyl acetate, filtered through diatomaceous earth, and the filtrate was washed three times with water and once with saturated brine, dried over anhydrous sodium sulfate, filtered and concentrated. The residue was purified by preparative liquid chromatography to obtain compound **73** (10.0 mg, 27.8 µmol), a white solid, with a yield of 42.6%. ¹H NMR (500 MHz, DMSO-*d*₆) δ 8.63 (d, *J* = 4.8 Hz, 1H), 8.26 (s, 1H), 8.15 - 7.95 (m, 3H), 7.91 (d, *J* = 8.0 Hz, 1H), 7.88 - 7.82 (m, 1H), 7.39 (d, *J* = 8.0 Hz, 2H), 7.32 (dd, *J* = 7.4, 4.9 Hz, 1H), 6.60 - 6.49 (m, 1H), 5.78 - 5.66 (m, 1H), 5.65 - 5.37 (m, 1H), 5.10 (d, *J* = 11.5 Hz, 1H), 4.91 - 4.65 (m, 2H), 4.63 - 4.38 (m, 4H); MS (ESI): m/z 361.1 (M+H)⁺.

### Example 74:

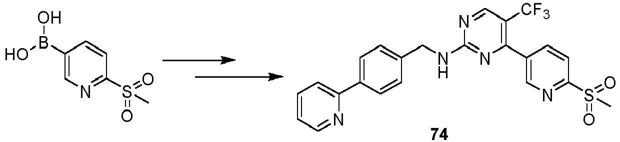

Starting from (6-(methylsulfonyl)pyridin-3-yl)boronic acid, compound **74** was obtained by referring to the synthesis of compound **11.** ¹H NMR (500 MHz, DMSO-*d*₆) δ 8.93 - 8.84 (m, 2H), 8.83 - 8.76 (m, 1H), 8.68 - 8.61 (m, 1H), 8.32 - 8.22 (m, 1H), 8.22 - 8.16 (m, 1H), 8.08 - 8.01 (m, 2H), 7.96 - 7.90 (m, 1H), 7.90 - 7.82 (m, 1H), 7.50 - 7.40 (m, 2H), 7.37 - 7.28 (m, 1H), 4.70 - 4.59 (m, 2H), 3.37 - 3.34 (m, 3H); MS (ESI): m/z 486.4 (M+H)⁺.

### Example 75:

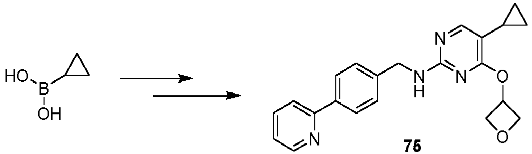

Starting from cyclopropylboronic acid, compound **75** was obtained by referring to the synthesis of compound **73.** ¹H NMR (500 MHz, DMSO-*d*₆) δ 8.63 (d, *J* = 4.5 Hz, 1H), 8.01 (d, *J* = 7.9 Hz, 2H), 7.91 (d, *J* = 7.9 Hz, 1H), 7.88 - 7.82 (m, 1H), 7.76 (s, 1H), 7.61 - 7.35 (m, 3H), 7.31 (dd, *J* = 7.4, 4.8 Hz, 1H), 5.61 - 5.35 (m, 1H), 4.89 - 4.62 (m, 2H), 4.57 - 4.37 (m, 4H), 1.73 - 1.66 (m, 1H), 0.79 - 0.73 (m, 2H), 0.60 - 0.55 (m, 2H); MS (ESI): m/z 375.4 (M+H)⁺.

### Example 76:

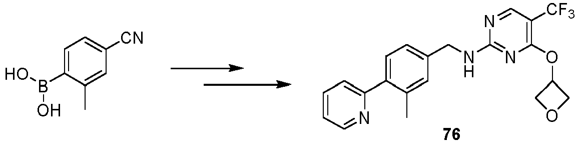

Starting from 2-methyl-4-cyanobenzeneboronic acid, compound **76** was obtained by referring to the synthesis of compound **4.** ¹H NMR (500 MHz, DMSO-*d*₆) δ 8.69 - 8.35 (m, 3H), 7.90 - 7.82 (m, 1H), 7.51 - 7.45 (m, 1H), 7.38 - 7.30 (m, 2H), 7.26 - 7.18 (m, 2H), 5.68 - 5.55 (m, 1H), 4.91 - 4.74 (m, 2H), 4.59 - 4.44 (m, 4H), 2.33 - 2.27 (m, 3H); MS (ESI): m/z 417.4 (M+H)⁺.

### Example 77:

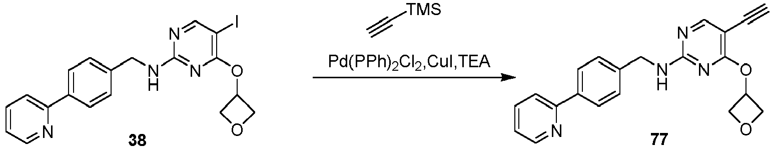

Compound **38** (25.0 mg, 54.3 µmol) and trimethylsilyl acetylene (16.0 mg, 163 µmol) were dissolved in tetrahydrofuran (3 mL), triethylamine (27.5 mg, 271 µmol) and copper iodide (1.0 mg were added , 5.43 µmol) and diphenylphosphine palladium dichloride (3.8 mg, 5.43 µmol). The mixture was stirred at 50°C for 3 hours under nitrogen atmosphere. Cool the reaction solution to room temperature, add 3 mL of 1M tetrabutylammonium fluoride tetrahydrofuran solution, and stir for 1 hour. Add 20 mL of ethyl acetate to the mixture, filter through diatomaceous earth, wash the filtrate three times with water and once with saturated brine, dry over anhydrous sodium sulfate, filter and concentrate. The residue was purified by preparative liquid chromatography to obtain compound 77 (5.0 mg, 14.0 µmol), a white solid, with a yield of 25.7%. ¹H NMR (500 MHz, DMSO-*d*₆) δ 8.64 (d, *J* = 4.8 Hz, 1H), 8.38 - 8.27 (m, 1H), 8.26 - 8.21 (m, 1H), 8.06 - 8.01 (m, 2H), 7.94 - 7.90 (m, 1H), 7.88 - 7.83 (m, 1H), 7.42 - 7.36 (m, 2H), 7.35 - 7.29 (m, 1H), 5.60 - 5.41 (m, 1H), 4.91 - 4.68 (m, 2H), 4.62 - 4.40 (m, 4H), 4.36 - 4.29 (m, 1H); MS (ESI): m/z 359.4 (M+H)⁺.

### Example 78:

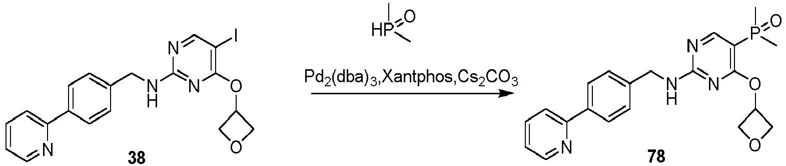

Compound **38** (30.0 mg, 65.2 µmol) and dimethylphosphine oxide (6.4 mg, 81.5 µmol) were dissolved in 1,4-dioxane (3 mL), and cesium carbonate (42.5 mg, 130 µmol), Pd2 (dba)3 (6.0 mg, 6.52 µmol) and Xantphos (7.5 mg, 13.0 µmol). The mixture was stirred at 95°C for 3 hours under nitrogen atmosphere. The reaction solution was cooled t to room temperature, added with 20 mL of ethyl acetate, filtered through diatomaceous earth, and the filtrate was washed three times with water and once with saturated brine, dried over anhydrous sodium sulfate, filtered and concentrated. The residue was purified by preparative liquid chromatography to obtain compound **78** (8.0 mg, 19.5 µmol), a white solid, with a yield of 29.9%. ¹H NMR (500 MHz, DMSO-*d*₆) δ 8.64 (d, *J* = 4.9 Hz, 1H), 8.49 - 8.15 (m, 2H), 8.10 - 8.00 (m, 2H), 7.99 - 7.82 (m, 2H), 7.41 (d, *J* = 8.0 Hz, 2H), 7.32 (dd, *J* = 7.4, 4.8 Hz, 1H), 5.66 - 5.49 (m, 1H), 4.90 - 4.68 (m, 2H), 4.62 - 4.43 (m, 4H), 1.67 - 1.58 (m, 6H); MS (ESI): m/z 411.3 (M+H)⁺.

### Example 79:

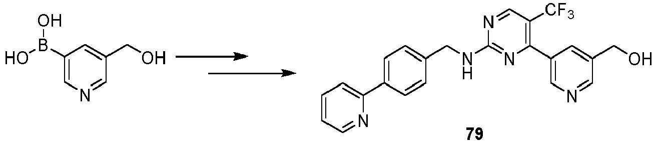

Compound 79 was obtained by referring to the synthesis of compound 11 starting from 5-(hydroxymethyl)pyridine-3-boronic acid. ¹H NMR (500 MHz, DMSO-*d*₆) δ 8.84 - 8.78 (m, 1H), 8.77 - 8.71 (m, 1H), 8.70 - 8.60 (m, 2H), 8.58 - 8.49 (m, 1H), 8.09 - 8.01 (m, 2H), 7.96 - 7.91 (m, 1H), 7.90 - 7.79 (m, 2H), 7.50 - 7.41 (m, 2H), 7.37 - 7.31 (m, 1H), 5.50 - 5.41 (m, 1H), 4.71 - 4.57 (m, 4H); MS (ESI): m/z 437.7 (M+H)⁺.

### Example 80:

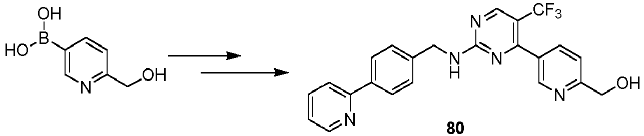

Compound 80 was obtained by referring to the synthesis of compound 11 starting from 6-(hydroxymethyl)pyridine-3-boronic acid. ¹H NMR (500 MHz, DMSO-*d*₆) δ 8.81 - 8.76 (m, 1H), 8.75 - 8.69 (m, 1H), 8.69 - 8.62 (m, 1H), 8.60 - 8.52 (m, 1H), 8.06 - 8.02 (m, 2H), 7.99 - 7.83 (m, 3H), 7.62 - 7.56 (m, 1H), 7.49 - 7.40 (m, 2H), 7.36 - 7.30 (m, 1H), 5.55 - 5.47 (m, 1H), 4.67 - 4.60 (m, 4H); MS (ESI): m/z 437.7 (M+H)⁺.

### Example 81:

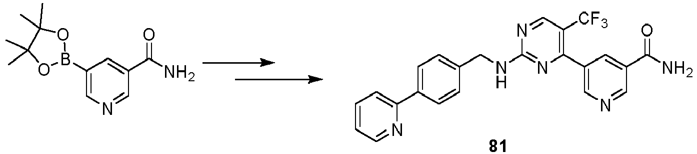

Compound 81 was obtained by referring to the synthesis of compound 11 starting from 5-carbamoylpyridine-3-boronic acid pinacol ester. ¹H NMR (500 MHz, DMSO-*d*₆) δ 9.19 - 9.11 (m, 1H), 8.92 - 8.72 (m, 3H), 8.68 - 8.61 (m, 1H), 8.38 - 8.30 (m, 1H), 8.29 - 8.19 (m, 1H), 8.07 - 8.01 (m, 2H), 7.95 - 7.89 (m, 1H), 7.87 (d, *J* = 7.0 Hz, 1H), 7.78 - 7.66 (m, 1H), 7.49 - 7.40 (m, 2H), 7.37 - 7.29 (m, 1H), 4.70 - 4.59 (m, 2H); MS (ESI): m/z 451.4 (M+H)⁺.

### Example 82:

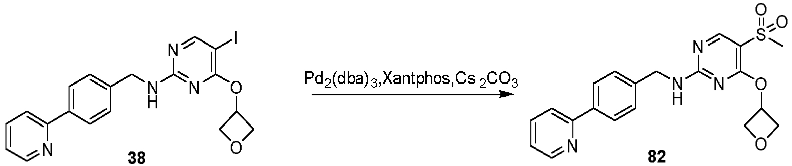

Compound **38** (30.0 mg, 65.2 µmol) and N,N'-dimethylethylenediamine (1.92 mg, 21.7 µmol) were dissolved in 1,4-dioxane (3 mL), and sodium methanesulfonate (6.4 mg, 81.5 µmol) and copper trifluoromethanesulfonate (3.9 mg, 10.8 µmol) were added. The mixture was stirred at 115°C for 3 hours under nitrogen atmosphere. The reaction solution was cooled t to room temperature, added with 20 mL of ethyl acetate, filtered through diatomaceous earth, and the filtrate was washed three times with water and once with saturated brine, dried over anhydrous sodium sulfate, filtered and concentrated. The residue was purified by preparative liquid chromatography to obtain compound **82** (15.0 mg, 36.4 µmol), a white solid, with a yield of 33.5%. ¹H NMR (500 MHz, DMSO-*d*₆) δ 8.94 - 8.61 (m, 2H), 8.45 - 8.38 (m, 1H), 8.09 - 8.01 (m, 2H), 7.96 - 7.90 (m, 1H), 7.89 - 7.83 (m, 1H), 7.44 - 7.39 (m, 2H), 7.36 - 7.29 (m, 1H), 5.70 - 5.56 (m, 1H), 4.90 - 4.71 (m, 2H), 4.66 - 4.48 (m, 4H), 3.23 - 3.18 (m, 3H); MS (ESI): m/z 413.4 (M+H)⁺.

### Example 83:

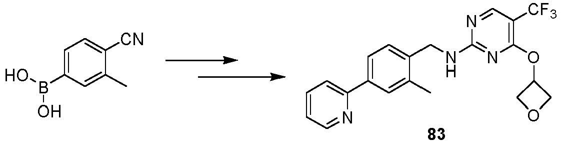

Compound 83 was obtained by referring to the synthesis of compound 4 starting from 4-cyano-3-methylphenylboronic acid. ¹H NMR (500 MHz, DMSO-*d*₆) δ 8.64 (d, *J=* 4.5 Hz, 1H), 8.60 - 8.31 (m, 2H), 7.98 - 7.89 (m, 2H), 7.89 - 7.82 (m, 2H), 7.36 - 7.20 (m, 2H), 5.68 - 5.49 (m, 1H), 4.93 - 4.34 (m, 6H), 2.44 - 2.37 (m, 3H); MS (ESI): m/z 417.4 (M+H)⁺.

### Example 84:

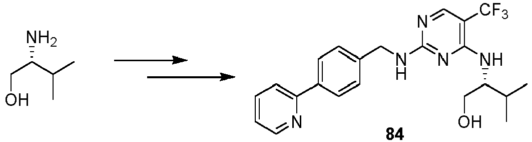

Compound 84 was obtained by referring to the synthesis of compound 24 starting from D-valinol. ¹H NMR (500 MHz, DMSO-*d*₆) δ 8.67 - 8.61 (m, 1H), 8.12 - 7.72 (m, 6H), 7.45 - 7.36 (m, 2H), 7.35 - 7.27 (m, 1H), 5.81 - 5.66 (m, 1H), 4.76 - 4.62 (m, 1H), 4.60 - 4.38 (m, 2H), 4.17 - 3.95 (m, 1H), 3.64 - 3.36 (m, 2H), 2.01 - 1.80 (m, 1H), 0.95 - 0.67 (m, 6H); MS (ESI): m/z 432.5 (M+H)⁺.

### Example 85:

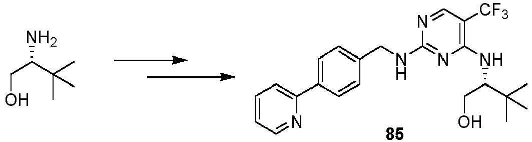

Compound 85 was obtained by referring to the synthesis of compound 24 starting from D-tert-leucinol. ¹H NMR (500 MHz, DMSO-*d*₆) δ 8.69 - 8.62 (m, 1H), 8.13 - 7.77 (m, 6H), 7.40 (d, *J* = 7.8 Hz, 2H), 7.34 - 7.30 (m, 1H), 5.65 - 5.53 (m, 1H), 4.72 - 4.60 (m, 1H), 4.60 - 4.36 (m, 2H), 4.32 - 4.10 (m, 1H), 3.70 - 3.44 (m, 2H), 0.96 - 0.70 (m, 9H); MS (ESI): m/z 446.5 (M+H)⁺.

### Example 86:

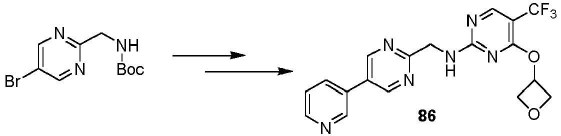

Compound **86** was obtained by referring to the synthesis of compound **48** starting from tert-butyl (5-bromopyrimidin-2-yl)methylcarbamate. ¹H NMR (500 MHz, DMSO-*d*₆) δ 9.21 - 9.12 (m, 2H), 9.05 - 8.98 (m, 1H), 8.70 - 8.32 (m, 3H), 8.26 - 8.18 (m, 1H), 7.58 - 7.51 (m, 1H), 5.69 - 5.29 (m, 1H), 4.95 - 4.51 (m, 5H), 4.33 - 4.30 (m, 1H); MS (ESI): m/z 405.6 (M+H)⁺.

### Example 87:

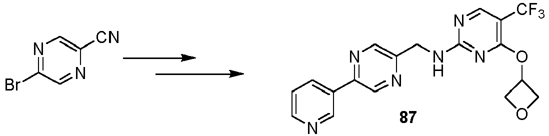

Compound 87 was obtained by referring to the synthesis of compound 48 starting from 2-bromo-5-cyanopyrazine. ¹H NMR (500 MHz, DMSO-*d*₆) δ 9.33 - 9.22 (m, 2H), 8.74 - 8.39 (m, 5H), 7.58 - 7.53 (m, 1H), 5.68 - 5.43 (m, 1H), 4.91 - 4.57 (m, 5H), 4.40 - 4.38 (m, 1H); MS (ESI): m/z 405.4 (M+H)⁺.

### Example 88:

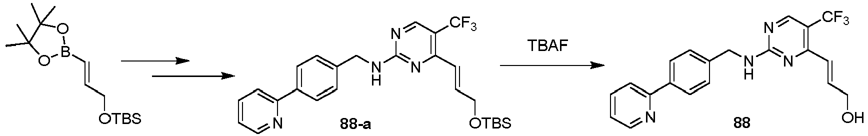

Starting from (E)-3-(tert-butyldimethylsiloxy)propen-1-yl-boronic acid pinacol ester, compound **88-a** was obtained by referring to the synthesis of compound **11.**

Compound **88-a** (55.0 mg, 110 µmol) was dissolved in tetrahydrofuran (3 mL), and tetrabutylammonium fluoride (1 M, 0.33 mL) was added. The mixture was stirred at room temperature for 2 hours. The reaction solution was added with 20 mL of ethyl acetate, washed three times with water and once with saturated brine, dried over anhydrous sodium sulfate, filtered and concentrated. The residue was purified by preparative liquid chromatography to obtain compound **88** (15.0 mg, 38.8 µmol), a white solid, with a yield of 35.3%. ¹H NMR (500 MHz, DMSO-*d*₆) δ 8.63 (d, *J* = 4.8 Hz, 1H), 8.57 - 8.50 (m, 1H), 8.49 - 8.41 (m, 1H), 8.02 (d, *J* = 7.9 Hz, 2H), 7.91 (d, *J* = 8.0 Hz, 1H), 7.87 - 7.83 (m, 1H), 7.47 - 7.39 (m, 2H), 7.36 - 7.29 (m, 2H), 6.76 - 6.67 (m, 1H), 4.67 - 4.57 (m, 2H), 4.28 - 4.17 (m, 2H); MS (ESI): m/z 387.3 (M+H)⁺.

### Example 89:

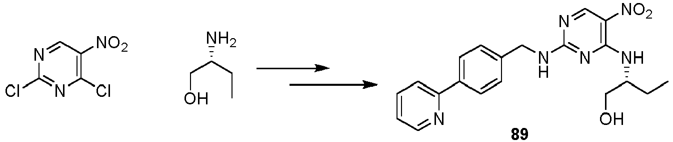

Compound **89** was obtained by referring to the synthesis of compound **1** starting from 2,4-dichloro-5-nitropyrimidine and (R)-2-aminobutanol. ¹H NMR (500 MHz, DMSO-*d*₆) δ 8.96 - 8.58 (m, 3H), 8.55 - 8.38 (m, 1H), 8.04 (d, *J=* 8.1 Hz, 2H), 7.93 (d, *J* = 7.9 Hz, 1H), 7.90 - 7.84 (m, 1H), 7.46 - 7.38 (m, 2H), 7.37 - 7.30 (m, 1H), 4.95 - 4.86 (m, 1H), 4.73 - 4.49 (m, 2H), 4.24 - 4.10 (m, 1H), 3.60 - 3.40 (m, 2H), 1.73 - 1.43 (m, 2H), 0.93 - 0.74 (m, 3H); MS (ESI): m/z 395.5 (M+H)⁺.

### Example 90:

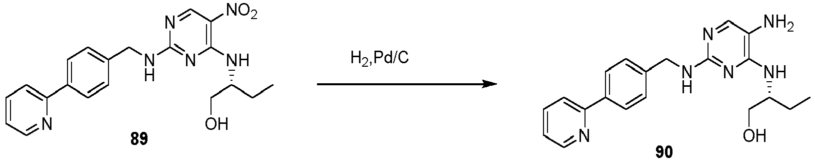

Compound **89** (50 mg, 127 µmol) was dissolved in ethanol (5 mL) and palladium on carbon (10% w/w, 10 mg) was added. The mixture was stirred at room temperature under a hydrogen atmosphere for 12 hours. The reaction solution was filtered through diatomaceous earth and concentrated. The residue was purified by preparative liquid chromatography to obtain compound **90** (12.0 mg, 32.9 µmol), a white solid, with a yield of 26.0%. ¹H NMR (500 MHz, DMSO-*d*₆) δ 8.63 (d, *J=* 4.8 Hz, 1H), 7.98 (d, *J* = 8.0 Hz, 2H), 7.90 (d, *J=* 8.0 Hz, 1H), 7.84 (td, *J=* 7.7, 1.8 Hz, 1H), 7.40 (d, *J=* 8.0 Hz, 2H), 7.31 (dd, *J* = 7.3, 4.9 Hz, 1H), 7.24 (s, 1H), 6.30 (t, *J* = 6.4 Hz, 1H), 5.71 (d, *J* = 8.1 Hz, 1H), 4.71 - 4.52 (m, 1H), 4.45 - 4.35 (m, 2H), 3.96 (td, *J* = 7.9, 3.6 Hz, 1H), 3.74 (s, 2H), 3.46 (dd, *J* = 10.7, 4.7 Hz, 1H), 3.40 - 3.36 (m, 1H), 1.67 - 1.58 (m, 1H), 1.48 - 1.39 (m, 1H), 0.83 (t, *J=* 7.4 Hz, 3H); MS (ESI): m/z 365.5 (M+H)⁺.

### Example 91:

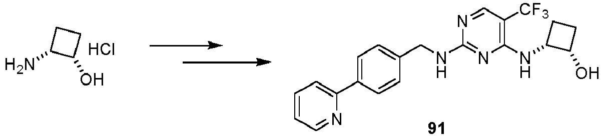

Compound **91** was obtained by referring to the synthesis of compound **24** starting from cis-2-aminocyclobutan-1-ol hydrochloride. ¹H NMR (500 MHz, DMSO-*d*₆) δ 8.69 - 8.61 (m, 1H), 8.14 - 7.97 (m, 4H), 7.95 - 7.90 (m, 1H), 7.88 - 7.83 (m, 1H), 7.47 - 7.36 (m, 2H), 7.36 - 7.30 (m, 1H), 6.28 - 6.20 (m, 1H), 5.67 - 5.55 (m, 1H), 4.58 - 4.28 (m, 4H), 2.13 - 1.97 (m, 2H), 1.84 - 1.69 (m, 2H); MS (ESI): m/z 416.4 (M+H)⁺.

### Example 92:

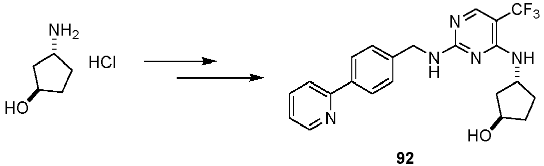

Compound **92** was obtained by referring to the synthesis of compound **24** starting from cis-3-aminocyclopentanol hydrochloride. ¹H NMR (500 MHz, DMSO-*d*₆) δ 8.67 - 8.62 (m, 1H), 8.07 - 7.99 (m, 3H), 7.98 - 7.78 (m, 3H), 7.49 - 7.37 (m, 2H), 7.36 - 7.30 (m, 1H), 6.29 - 6.21 (m, 1H), 4.84 - 4.62 (m, 1H), 4.57 - 4.44 (m, 3H), 4.24 - 4.13 (m, 1H), 1.99 - 1.77 (m, 3H), 1.77 - 1.67 (m, 1H), 1.52 - 1.38 (m, 2H); MS (ESI): m/z 430.4 (M+H)⁺.

### Example 93:

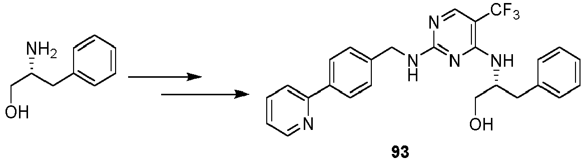

Compound **93** was obtained by referring to the synthesis of compound **24** starting from D-phenylalaninol. ¹H NMR (500 MHz, DMSO-*d*₆) δ 8.69 - 8.63 (m, 1H), 8.10 - 7.99 (m, 3H), 7.99 - 7.75 (m, 3H), 7.47 - 7.35 (m, 2H), 7.34 - 7.04 (m, 6H), 6.04 - 5.93 (m, 1H), 4.98 - 4.89 (m, 1H), 4.62 - 4.31 (m, 3H), 3.48 - 3.35 (m, 2H), 2.91 - 2.77 (m, 2H); MS (ESI): m/z 480.4 (M+H)⁺.

### Example 94:

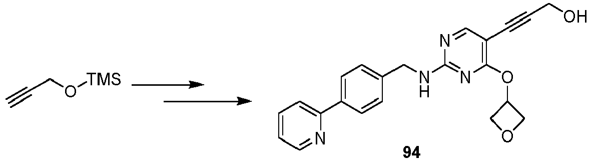

Compound **94** was obtained by referring to the synthesis of compound **77** starting from 3-trimethylsiloxy-1-propyne. ¹H NMR (500 MHz, DMSO-*d*₆) δ 8.64 (d, *J* = 4.8 Hz, 1H), 8.27 - 7.97 (m, 4H), 7.92 (d, *J* = 8.1 Hz, 1H), 7.85 (td, *J* = 7.6, 1.7 Hz, 1H), 7.39 (d, *J* = 8.0 Hz, 2H), 7.32 (dd, *J* = 7.3, 4.9 Hz, 1H), 5.54 - 5.45 (m, 1H), 5.25 (s, 1H), 4.87 - 4.70 (m, 2H), 4.58 - 4.43 (m, 4H), 4.28 (d, *J* = 4.5 Hz, 2H); MS (ESI): m/z 389.5 (M+H)⁺.

### Example 95:

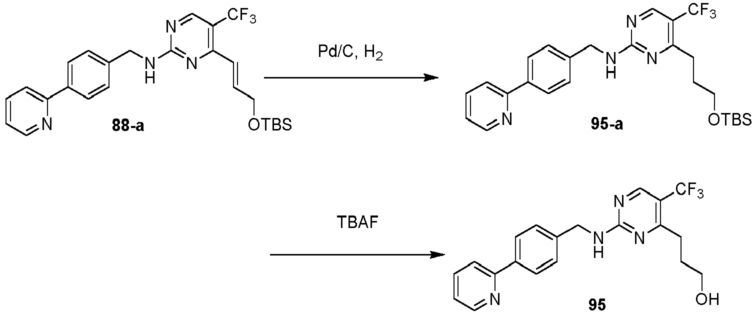

Compound **88-a** (55.0 mg, 110 µmol) was dissolved in ethanol (5 mL) and palladium on carbon (10% w/w, 10.0 mg) was added. The mixture was stirred at room temperature under a hydrogen atmosphere for 12 hours. The reaction solution was filtered through diatomaceous earth and concentrated to obtain compound **95-a** (50.0 mg, 99.4 µmol), a white solid, with a yield of 90.5%.

Starting from **95-a,** compound 95 was obtained by referring to the synthesis of compound **88.** ¹H NMR (500 MHz, DMSO-*d*₆) δ 8.63 (d, *J=* 4.8 Hz, 1H), 8.56 - 8.41 (m, 2H), 8.02 (d, *J* = 7.9 Hz, 2H), 7.91 (d, *J* = 8.0 Hz, 1H), 7.85 (t, *J* = 7.7 Hz, 1H), 7.44 - 7.40 (m, 7.9 Hz, 2H), 7.32 (dd, *J* = 7.3, 4.8 Hz, 1H), 4.60 (d, *J* = 6.4 Hz, 2H), 4.55 - 4.47 (m, 1H),3.48 - 3.41 (m, 2H), 2.70 (t, *J=* 7.8 Hz, 2H), 1.80 (d, *J* = 7.6 Hz, 2H); MS (ESI): m/z 388.9 (M+H)⁺.

### Example 96:

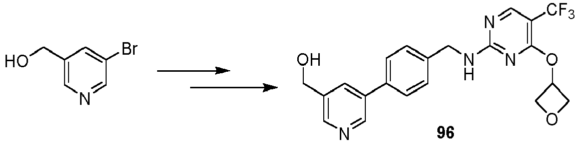

Compound **96** was obtained by referring to the synthesis of compound **12** starting from 3-bromopyridine-5-methanol. ¹H NMR (500 MHz, DMSO-*d*₆) δ 8.78 - 8.72 (m, 1H), 8.72 - 8.35 (m, 3H), 7.95 (d, *J* = 7.4 Hz, 1H), 7.70 - 7.65 (m, 2H), 7.42 (d, *J* = 7.9 Hz, 2H), 5.64 - 5.55 (m, 1H), 5.41 - 5.30 (m, 1H), 4.89 - 4.72 (m, 2H), 4.61 - 4.42 (m, 6H); MS (ESI): m/z 433.4 (M+H)⁺.

### Example 97:

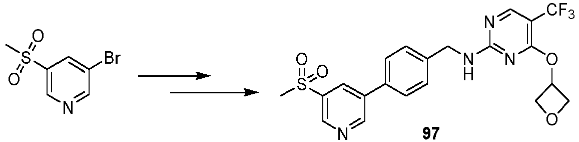

Compound **97** was obtained by referring to the synthesis of compound **12** starting from 3-bromo-5-(methylsulfonyl)pyridine. ¹H NMR (500 MHz, DMSO-*d*₆) δ 9.25 - 9.19 (m, 1H), 9.08 - 9.00 (m, 1H), 8.75 - 8.37 (m, 3H), 7.89 - 7.78 (m, 2H), 7.52 - 7.44 (m, 2H), 5.68 - 5.51 (m, 1H), 4.91 - 4.72 (m, 2H), 4.63 - 4.42 (m, 4H), 3.39 (s, 3H); MS (ESI): m/z 481.3 (M+H)⁺.

### Example 98:

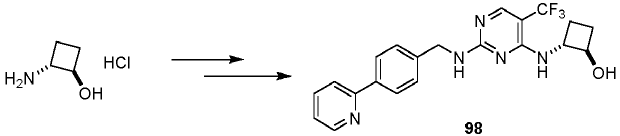

Compound **98** was obtained by referring to the synthesis of compound **24** starting from trans-2-aminocyclobutanol hydrochloride. ¹H NMR (500 MHz, DMSO-*d*₆) δ 8.63 (d, *J* = 4.8 Hz, 1H), 8.02 (d, *J* = 7.8 Hz, 3H), 7.98 - 7.77 (m, 3H), 7.51 - 7.36 (m, 2H), 7.36 - 7.29 (m, 1H), 6.79 - 6.69 (m, 1H), 5.22 - 5.12 (m, 1H), 4.60 - 4.31 (m, 3H), 4.22 - 4.11 (m, 1H), 2.03 - 1.87 (m, 2H), 1.49 - 1.30 (m, 2H); MS (ESI): m/z 416.5 (M+H)⁺.

### Example 99:

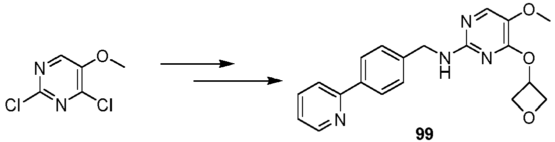

Starting from 2,4-dichloro-5-methoxypyrimidine, compound 99 was obtained by referring to the synthesis of compound 3. ¹H NMR (500 MHz, DMSO-*d*₆) δ 8.66 - 8.62 (m, 1H), 8.01 (d, *J=* 8.1 Hz, 2H), 7.97 - 7.82 (m, 3H), 7.45 - 7.28 (m, 4H), 5.54 - 5.41 (m, 1H), 4.75 (s, 2H), 4.49 (t, *J=* 6.4 Hz, 2H), 4.42 (d, *J=* 6.3 Hz, 2H), 3.70 (s, 3H); MS (ESI): m/z 365.4 (M+H)⁺.

### Example 100:

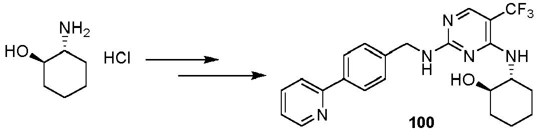

Compound **100** was obtained by referring to the synthesis of compound **24** starting from (1R,2R)-2-aminocyclohexanol hydrochloride. ¹H NMR (500 MHz, DMSO-*d*₆) δ 8.63 (d, *J* = 4.8 Hz, 1H), 8.08 - 7.93 (m, 4H), 7.92 - 7.89 (m, 1H), 7.87 - 7.83 (m, 1H), 7.43 - 7.37 (m, 2H), 7.34 - 7.30 (m, 1H), 5.88 (d, *J* = 6.3 Hz, 1H), 4.65 - 4.59 (m, 1H), 4.57 - 4.38 (m, 2H), 3.91 - 3.59 (m, 1H), 3.50 - 3.36 (m, 1H), 2.07 - 1.95 (m, 1H), 1.95 - 1.77 (m, 2H), 1.64 - 1.57 (m, 1H), 1.50 - 1.42 (m, 1H), 1.19 - 1.11 (m, 2H), 1.08 - 0.95 (m, 1H).; MS (ESI): m/z 444.5 (M+H)⁺.

### Example 101:

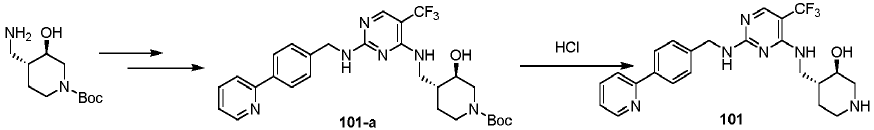

Starting from ((3R,4R)-4-(aminomethyl)-3-hydroxypiperidine-1-carboxylic acid tert-butyl ester, compound **101-a** was obtained by referring to the synthesis of compound **24.**

Compound **101-a** (50.0 mg, 89.5 µmol) was dissolved in dichloromethane (3 mL), and dioxane hydrochloride (4 M, 0.50 mL) was added. The mixture was stirred at room temperature for 2 hours. The reaction solution was concentrated, and the residue was purified by preparative liquid chromatography to obtain compound **101** (15.0 mg, 32.7 µmol), a white solid, with a yield of 36.5%. ¹H NMR (500 MHz, DMSO-*d*₆) δ 8.63 (d, *J* = 4.9 Hz, 1H), 8.05 - 7.89 (m, 5H), 7.88 - 7.83 (m, 1H), 7.47 - 7.36 (m, 2H), 7.35 - 7.29 (m, 1H), 7.01 - 6.83 (m, 1H), 5.23 - 5.01 (m, 1H), 4.56 - 4.49 (m, 2H), 3.64 - 3.36 (m, 3H), 2.99 - 2.92 (m, 1H), 2.90 - 2.71 (m, 1H), 2.38 - 2.18 (m, 2H), 1.59 - 1.40 (m, 2H), 1.12 - 0.95 (m, 1H); MS (ESI): m/z 459.4 (M+H)⁺.

### Example 102:

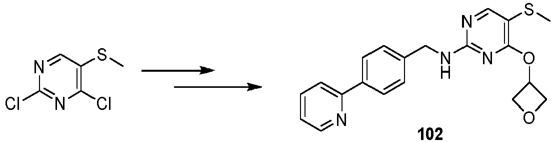

Compound **102** was obtained by referring to the synthesis of compound **3** starting from 2,4-dichloro-5-methylthiopyrimidine. ¹H NMR (500 MHz, DMSO-*d*₆) δ 8.67 - 8.61 (m, 1H), 8.11 (s, 1H), 8.06 - 7.82 (m, 5H), 7.39 (d, *J* = 8.0 Hz, 2H), 7.32 (dd, *J* = 7.3, 4.9 Hz, 1H), 5.65 - 5.37 (m, 1H), 4.93 - 4.63 (m, 2H), 4.60 - 4.38 (m, 4H), 2.27 (s, 3H); MS (ESI): m/z 381.4 (M+H)⁺.

### Example 103:

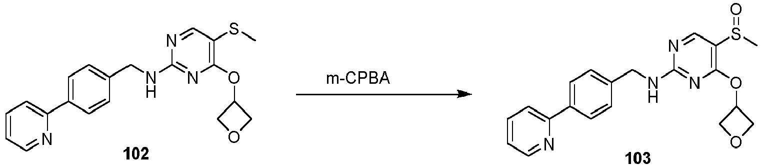

Compound **102** (30.0 mg, 78.8 µmol) was dissolved in dichloromethane (5 mL), and m-chloroperoxybenzoic acid (13.6 mg, 78.8 µmol) was added. The mixture was stirred at room temperature for 2 hours. The reaction solution was added with 20 mL of ethyl acetate, washed once with saturated sodium bicarbonate, once with water, once with saturated brine, dried and concentrated. The residue was purified by preparative liquid chromatography to obtain compound **103** (12 mg, 30.2 µmol), a white solid, with a yield of 38.4%. ¹H NMR (500 MHz, DMSO-*d*₆) δ 8.64 (d, *J* = 4.8 Hz, 1H), 8.53 - 8.19 (m, 2H), 8.07 - 8.00 (m, 2H), 7.95 - 7.90 (m, 1H), 7.89 - 7.83 (m, 1H), 7.44 - 7.39 (m, 2H), 7.35 - 7.30 (m, 1H), 5.66 - 5.49 (m, 1H), 4.88 - 4.69 (m, 2H), 4.62 - 4.44 (m, 4H), 2.84 - 2.79 (m, 3H); MS (ESI): m/z 397.3 (M+H)⁺.

### Example 104:

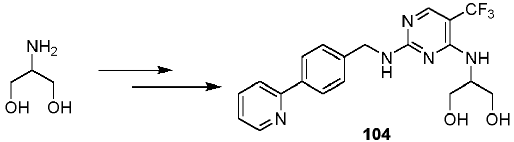

Starting from serinol, compound **104** was obtained by referring to the synthesis of compound **24.** ¹H NMR (500 MHz, DMSO-*d*₆) δ 8.70 - 8.61 (m, 1H), 8.13 - 7.80 (m, 6H), 7.53 - 7.30 (m, 3H), 5.86 - 5.72 (m, 1H), 4.87 - 4.72 (m, 2H), 4.58 - 4.47 (m, 2H), 4.24 - 4.12 (m, 1H), 3.65 - 3.54 (m, 2H), 3.55 - 3.41 (m, 2H); MS (ESI): m/z 420.5 (M+H)⁺.

### Example 105:

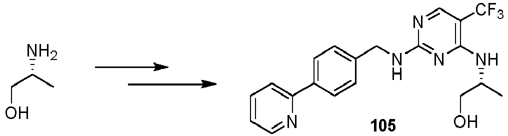

Starting from (R)-1-amino-2-propanol, compound **105** was obtained by referring to the synthesis of compound **24.** ¹H NMR (500 MHz, DMSO-*d*₆) δ 8.69 - 8.57 (m, 1H), 8.10 - 7.76 (m, 6H), 7.49 - 7.23 (m, 3H), 6.00 - 5.89 (m, 1H), 4.88 - 4.76 (m, 1H), 4.60 - 4.43 (m, 2H), 4.31 - 4.16 (m, 1H), 3.50 - 3.35 (m, 2H), 1.18 - 1.00 (m, 3H); MS (ESI): m/z 404.2 (M+H)⁺.

### Example 106:

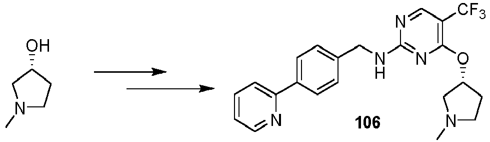

Compound **106** was obtained by referring to the synthesis of compound **26** starting from (R)-1-methyl-3-pyrrolidinol. ¹H NMR (500 MHz, DMSO-*d*₆) δ 8.67 - 8.30 (m, 3H), 8.12 - 7.83 (m, 4H), 7.49 - 7.24 (m, 3H), 5.51 - 5.25 (m, 1H), 4.68 - 4.46 (m, 2H), 2.66 - 2.55 (m, 2H), 2.40 - 2.34 (m, 1H), 2.31 - 1.99 (m, 5H), 1.83 - 1.65 (m, 1H); MS (ESI): m/z 430.4 (M+H)⁺.

### Example 107:

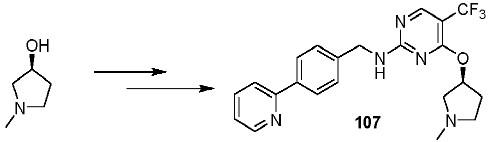

Starting from (S)-1-methyl-3-pyrrolidinol, compound **107** was obtained by referring to the synthesis of compound **26.** ¹H NMR (500 MHz, DMSO-*d*₆) δ 8.67 - 8.61 (m, 1H), 8.61 - 8.30 (m, 2H), 8.07 - 8.00 (m, 2H), 7.94 - 7.81 (m, 2H), 7.47 - 7.27 (m, 3H), 5.50 - 5.24 (m, 1H), 4.70 - 4.46 (m, 2H), 2.68 - 2.54 (m, 2H), 2.44 - 2.10 (m, 6H), 1.86 - 1.64 (m, 1H); MS (ESI): m/z 430.4 (M+H)⁺.

### Example 108:

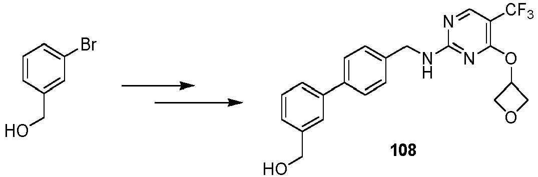

Compound **108** was obtained by referring to the synthesis of compound **12** starting from 3-bromobenzyl alcohol. ¹H NMR (500 MHz, DMSO-*d*₆) δ 8.72 - 8.33 (m, 2H), 7.65 - 7.56 (m, 3H), 7.52 - 7.47 (m, 1H), 7.42 - 7.36 (m, 3H), 7.32 - 7.28 (m, 1H), 5.66 - 5.55 (m, 1H), 5.26 - 5.19 (m, 1H), 4.91 - 4.72 (m, 2H), 4.59 - 4.43 (m, 6H); MS (ESI): m/z 432.3 (M+H)⁺.

### Example 109:

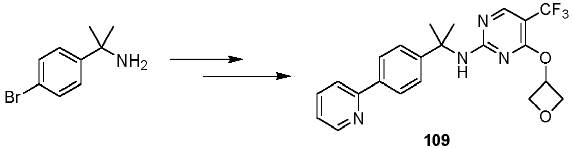

Compound **109** was obtained by referring to the synthesis of compound **12** starting from 1-(4-bromophenyl)-1-methylethylamine. ¹H NMR (500 MHz, DMSO-*d*₆) δ 8.79 - 8.35 (m, 3H), 8.12 - 7.83 (m, 4H), 7.57 - 7.23 (m, 3H), 5.04 - 4.02 (m, 5H), 1.77 - 1.60 (m, 6H); MS (ESI): m/z 431.4 (M+H)⁺.

### Example 110:

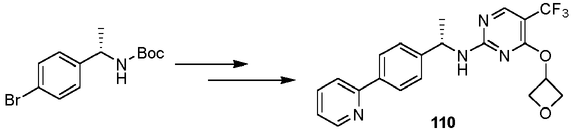

Compound **110** was obtained by referring to the synthesis of compound **12** starting from [(S)-1-(4-bromophenyl)ethyl]carbamic acid tert-butyl ester. ¹H NMR (500 MHz, DMSO-*d*₆) δ 8.72 - 8.32 (m, 3H), 8.12 - 7.80 (m, 4H), 7.60 - 7.24 (m, 3H), 5.71 - 5.43 (m, 1H), 5.06 - 4.87 (m, 2H), 4.64 - 4.18 (m, 3H), 1.48 (d, *J* = 6.9 Hz, 3H); MS (ESI): m/z 417.4 (M+H)⁺.

### Example 111:

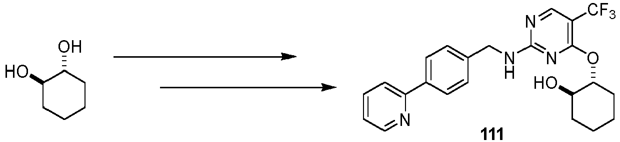

Starting from trans-1,2-cyclohexanediol, compound **111** was obtained by referring to the synthesis of compound **26.** ¹H NMR (500 MHz, DMSO-*d*₆) δ 8.67 - 8.61 (m, 1H), 8.57 - 8.27 (m, 2H), 8.06 - 7.99 (m, 2H), 7.94 - 7.89 (m, 1H), 7.89 - 7.82 (m, 1H), 7.47 - 7.38 (m, 2H), 7.36 - 7.29 (m, 1H), 5.13 - 4.91 (m, 1H), 4.83 - 4.72 (m, 1H), 4.63 - 4.42 (m, 2H), 3.57 - 3.50 (m, 1H), 2.05 - 1.92 (m, 1H), 1.88 - 1.54 (m, 3H), 1.49 - 1.24 (m, 4H); MS (ESI): m/z 445.4 (M+H)⁺.

### Example 112:

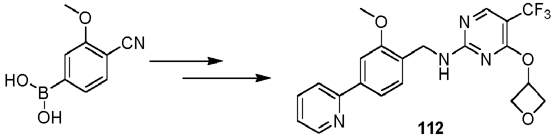

Compound **112** was obtained by referring to the synthesis of compound **4** starting from 4-cyano-3-methoxyphenylboronic acid. ¹H NMR (500 MHz, DMSO-*d*₆) δ 8.70 - 8.64 (m, 1H), 8.56 - 8.23 (m, 2H), 8.02 - 7.94 (m, 1H), 7.92 - 7.84 (m, 1H), 7.78 - 7.69 (m, 1H), 7.66 - 7.57 (m, 1H), 7.39 - 7.32 (m, 1H), 7.28 - 7.17 (m, 1H), 5.69 - 5.45 (m, 1H), 4.96 - 4.39 (m, 6H), 4.03 - 3.88 (m, 3H); MS (ESI): m/z 433.3 (M+H)⁺.

### Example 113:

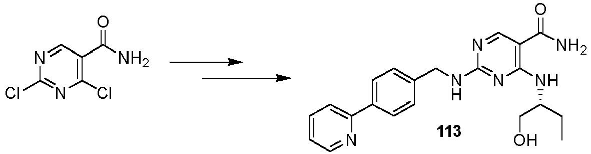

Compound **113** was obtained by referring to the synthesis of compound **89** starting from 2,4-dichloropyrimidine-5-carboxamide. ¹H NMR (500 MHz, DMSO-*d*₆) δ 9.08 - 8.87 (m, 1H), 8.64 (d, *J* = 4.9 Hz, 1H), 8.36 (s, 1H), 8.01 (d, *J* = 7.9 Hz, 2H), 7.92 (d, *J* = 7.9 Hz, 1H), 7.89 - 7.83 (m, 1H), 7.79 - 7.63 (m, 1H), 7.61 - 7.47 (m, 1H), 7.47 - 7.36 (m, 2H), 7.36 - 7.29 (m, 1H), 4.79 - 4.65 (m, 1H), 4.60 - 4.41 (m, 2H), 4.07 - 3.93 (m, 1H), 3.53 - 3.43 (m, 1H), 3.42 - 3.35 (m, 1H), 3.30 - 3.29 (m, 1H), 2.05 - 1.95 (m, 1H), 1.68 - 1.51 (m, 1H), 1.50 - 1.25 (m, 3H); MS (ESI): m/z 393.5 (M+H)⁺.

### Example 114:

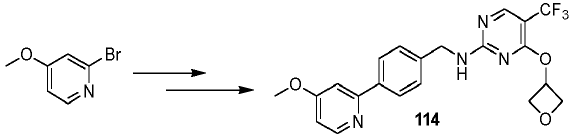

Starting from 2-bromo-4-methoxypyridine, compound **114** was obtained by referring to the synthesis of compound **12.** ¹H NMR (500 MHz, DMSO-*d*₆) δ 8.70 - 8.37 (m, 3H), 8.08 - 7.99 (m, 2H), 7.48 - 7.42 (m, 1H), 7.41 - 7.36 (m, 2H), 6.95 - 6.90 (m, 1H), 5.69 - 5.49 (m, 1H), 4.90 - 4.69 (m, 2H), 4.59 - 4.40 (m, 4H), 3.89 (s, 3H); MS (ESI): m/z 433.4 (M+H)⁺.

### Example 115:

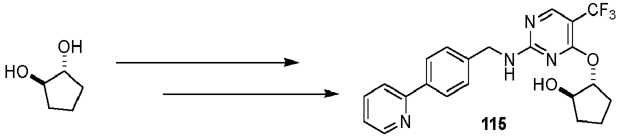

Compound **115** was obtained by referring to the synthesis of compound **26** starting from trans-1,2-cyclopentanediol. ¹H NMR (500 MHz, DMSO-*d*₆) δ 8.69 - 8.61 (m, 1H), 8.61 - 8.36 (m, 1H), 8.36 - 8.28 (m, 1H), 8.08 - 7.98 (m, 2H), 7.97 - 7.82 (m, 2H), 7.50 - 7.38 (m, 2H), 7.38 - 7.28 (m, 1H), 5.24 - 5.12 (m, 1H), 4.99 - 4.88 (m, 1H), 4.65 - 4.47 (m, 2H), 4.11 - 3.99 (m, 1H), 2.13 - 1.92 (m, 1H), 1.91 - 1.75 (m, 1H), 1.75 - 1.41 (m, 4H); MS (ESI): m/z 431.3 (M+H)⁺.

### Example 116:

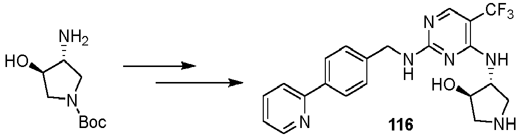

Compound **116** was obtained by referring to the synthesis of compound **101** starting from trans-1,2-cyclopentanediol. ¹H NMR (500 MHz, DMSO-*d*₆) δ 8.67 - 8.61 (m, 1H), 8.13 - 7.77 (m, 6H), 7.50 - 7.35 (m, 2H), 7.35 - 7.29 (m, 1H), 6.52 - 6.24 (m, 1H), 5.32 - 4.79 (m, 1H), 4.65 - 4.43 (m, 2H), 4.31 - 4.02 (m, 2H), 3.20 - 2.94 (m, 3H), 2.59 - 2.52 (m, 1H); MS (ESI): m/z 431.4 (M+H)⁺.

### Example 117:

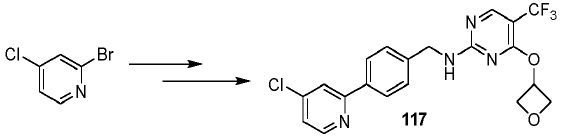

Compound **117** was obtained by referring to the synthesis of compound **12** starting from 2-bromo-4-chloropyridine. ¹H NMR (500 MHz, DMSO-*d*₆) δ 8.73 - 8.37 (m, 3H), 8.13 - 8.04 (m, 3H), 7.49 - 7.46 (m, 1H), 7.43 - 7.38 (m, 2H), 5.67 - 5.50 (m, 1H), 4.93 - 4.69 (m, 2H), 4.65 - 4.38 (m, 4H); MS (ESI): m/z 437.1 (M+H)⁺.

### Example 118:

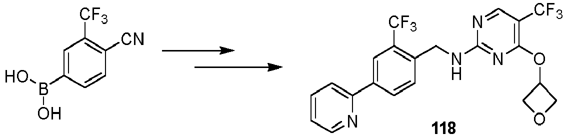

Compound **118** was obtained by referring to the synthesis of compound **4** starting from 4-cyano-3-trifluoromethylbenzeneboronic acid. ¹H NMR (500 MHz, DMSO-*d*₆) δ 8.79 - 8.39 (m, 4H), 8.39 - 8.27 (m, 1H), 8.11 - 8.02 (m, 1H), 7.99 - 7.90 (m, 1H), 7.61 - 7.50 (m, 1H), 7.46 - 7.37 (m, 1H), 5.73 - 5.37 (m, 1H), 4.97 - 4.28 (m, 6H); MS (ESI): m/z 471.1 (M+H)⁺.

### Example 119:

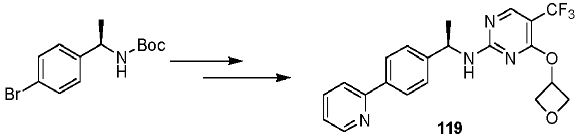

Compound **119** was obtained by referring to the synthesis of compound **12** starting from [(R)-1-(4-bromophenyl)ethyl]carbamic acid tert-butyl ester. ¹H NMR (500 MHz, DMSO-*d*₆) δ 8.79 - 8.27 (m, 3H), 8.14 - 7.80 (m, 4H), 7.57 - 7.29 (m, 3H), 5.73 - 5.39 (m, 1H), 5.10 - 4.84 (m, 2H), 4.65 - 4.47 (m, 2H), 4.33 - 4.15 (m, 1H), 1.53 - 1.43 (m, 3H); MS (ESI): m/z 417.4 (M+H)⁺.

### Example 120:

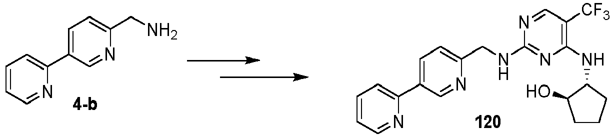

Starting from compound **4-b,** compound **120** was obtained by referring to the synthesis of compound **70.** ¹H NMR (500 MHz, DMSO-*d*₆) δ 9.20 - 9.15 (m, 1H), 8.72 - 8.67 (m, 1H), 8.41 - 8.35 (m, 1H), 8.03 (s, 1H), 8.02 - 7.99 (m, 1H), 7.98 - 7.81 (m, 2H), 7.43 - 7.37 (m, 2H), 6.25 - 6.15 (m, 1H), 4.75 - 4.69 (m, 1H), 4.67 - 4.53 (m, 2H), 4.24 - 3.88 (m, 2H), 2.11 - 1.95 (m, 1H), 1.77 - 1.61 (m, 2H), 1.51 - 1.43 (m, 2H), 1.39 - 1.29 (m, 1H); MS (ESI): m/z 431.4 (M+H)⁺.

### Example 121:

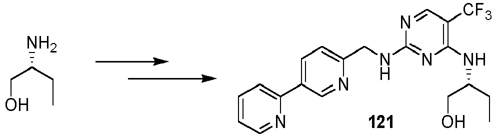

Starting from (R)-2-aminobutanol, compound **121** was obtained by referring to the synthesis of compound **120.** ¹H NMR (500 MHz, DMSO-*d*₆) δ 9.18 - 9.15 (m, 1H), 8.70 - 8.66 (m, 1H), 8.39 - 8.35 (m, 1H), 8.08 - 7.73 (m, 4H), 7.41 - 7.34 (m, 2H), 5.91 - 5.85 (m, 1H), 4.80 - 4.49 (m, 3H), 4.24 - 3.88 (m, 1H), 3.57 - 3.35 (m, 2H), 1.71 - 1.33 (m, 2H), 0.86 - 0.54 (m, 3H); MS (ESI): m/z 419.4 (M+H)⁺.

### Example 122:

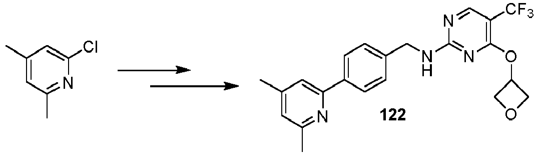

Compound **122** was obtained by referring to the synthesis of compound **12** starting from 2-chloro-4,6-lutidine. ¹H NMR (500 MHz, DMSO-*d*₆) δ 8.69 - 8.37 (m, 2H), 8.06 - 7.97 (m, 2H), 7.60 - 7.53 (m, 1H), 7.43 - 7.35 (m, 2H), 7.02 (s, 1H), 5.67 - 5.51 (m, 1H), 4.90 - 4.69 (m, 2H), 4.61 - 4.40 (m, 4H), 2.47 (s, 3H), 2.32 (s, 3H).; MS (ESI): m/z 431.5 (M+H)⁺.

### Example 123:

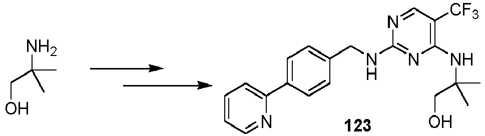

Compound **123** was obtained by referring to the synthesis of compound **24** starting from 2-amino-2-methyl-1-propanol. ¹H NMR (500 MHz, DMSO-*d*₆) δ 8.63 (d, *J=* 4.8 Hz, 1H), 8.12 - 7.96 (m, 4H), 7.93 - 7.89 (m, 1H), 7.87 - 7.83 (m, 1H), 7.42 - 7.36 (m, 2H), 7.35 - 7.29 (m, 1H), 5.74 - 5.56 (m, 1H), 5.33 - 5.24 (m, 1H), 4.54 (d, *J =* 6.3 Hz, 2H), 3.30 - 3.25 (m, 2H), 1.28 - 1.13 (m, 6H); MS (ESI): m/z 418.5 (M+H)⁺.

### Example 124:

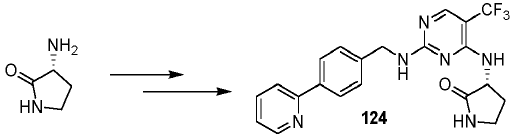

Starting from (R)-3-amino-2-pyrrolidone, compound **124** was obtained by referring to the synthesis of compound **24.** ¹H NMR (500 MHz, DMSO-*d*₆) δ 8.63 (d, *J* = 4.8 Hz, 1H), 8.11 - 8.09 (m, 1H), 8.01 (d, *J* = 7.9 Hz, 2H), 7.99 - 7.74 (m, 4H), 7.39 (d, *J=* 7.8 Hz, 2H), 7.35 - 7.27 (m, 1H), 6.83 - 6.62 (m, 1H), 4.83 - 4.36 (m, 3H), 3.11 - 2.93 (m, 2H), 2.11 - 1.95 (m, 2H); MS (ESI): m/z 429.4 (M+H)⁺.

### Example 125:

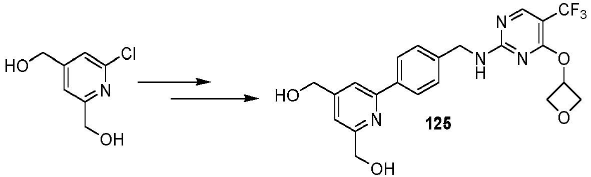

Compound **125** was obtained by referring to the synthesis of compound **12** starting from 2-chloro-4,6-dihydroxymethylpyridine. ¹H NMR (500 MHz, DMSO-*d*₆) δ 8.70 - 8.36 (m, 2H), 8.04 - 7.97 (m, 2H), 7.70 - 7.66 (m, 1H), 7.41 - 7.37 (m, 3H), 5.68 - 5.50 (m, 1H), 5.46 - 5.43 (m, 1H), 5.43 - 5.38 (m, 1H), 4.93 - 4.66 (m, 2H), 4.64 - 4.54 (m, 6H), 4.53 - 4.39 (m, 3H); MS (ESI): m/z 463.6 (M+H)⁺.

### Example 126:

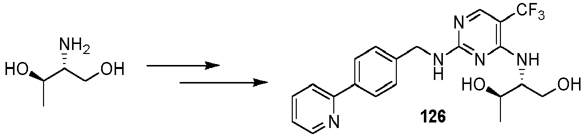

Compound **126** was obtained by referring to the synthesis of compound **24** starting from L-threoninol. ¹H NMR (500 MHz, DMSO-*d*₆) δ 8.75 - 8.54 (m, 1H), 8.20 - 7.72 (m, 6H), 7.55 - 7.28 (m, 3H), 5.81 - 5.63 (m, 1H), 5.13 - 4.44 (m, 4H), 4.17 - 3.95 (m, 2H), 3.55 - 3.43 (m, 2H), 1.09 - 0.92 (m, 3H); MS (ESI): m/z 434.4 (M+H)⁺.

### Example 127:

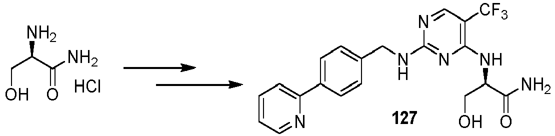

Starting from D-serinamide hydrochloride, compound **127** was obtained with reference to the synthesis of compound **24.** ¹H NMR (500 MHz, DMSO-*d*₆) δ 8.67 - 8.63 (m, 1H), 8.10 - 7.99 (m, 4H), 7.94 - 7.90 (m, 1H), 7.89 - 7.84 (m, 1H), 7.56 - 7.41 (m, 3H), 7.35 - 7.31 (m, 1H), 7.28 - 7.20 (m, 1H), 6.41 - 6.09 (m, 1H), 5.04 - 4.97 (m, 1H), 4.59 - 4.47 (m, 3H), 3.83 - 3.61 (m, 2H); MS (ESI): m/z 433.3 (M+H)⁺.

### Example 128:

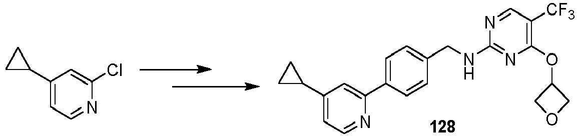

Compound **128** was obtained by referring to the synthesis of compound **12** starting from 2-chloro-4-cyclopropylpyridine. ¹H NMR (500 MHz, DMSO-*d*₆) δ 8.75 - 8.36 (m, 3H), 8.09 - 7.97 (m, 2H), 7.64 - 7.57 (m, 1H), 7.42 - 7.34 (m, 2H), 7.03 - 6.97 (m, 1H), 5.68 - 5.48 (m, 1H), 4.93 - 4.37 (m, 6H), 2.04 - 1.95 (m, 1H), 1.10 - 1.02 (m, 2H), 0.92 - 0.86 (m, 2H); MS (ESI): m/z 443.5 (M+H)⁺.

### Example 129:

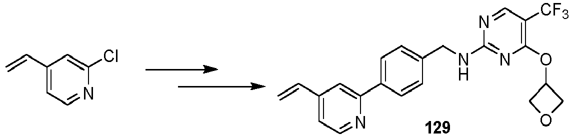

Compound **129** was obtained by referring to the synthesis of compound **12** starting from 2-chloro-4-vinylpyridine. ¹H NMR (500 MHz, DMSO-*d*₆) δ 8.75 - 8.36 (m, 3H), 8.16 - 8.04 (m, 2H), 8.04 - 7.93 (m, 1H), 7.48 - 7.37 (m, 3H), 6.90 - 6.73 (m, 1H), 6.32 - 6.18 (m, 1H), 5.67 - 5.50 (m, 2H), 4.94 - 4.67 (m, 2H), 4.62 - 4.41 (m, 4H); MS (ESI): m/z 429.4 (M+H)⁺.

### Example 130:

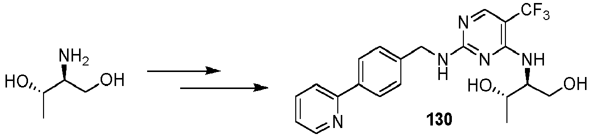

Starting from D-threoninol, compound **130** was obtained by referring to the synthesis of compound **24.** ¹H NMR (500 MHz, DMSO-*d*₆) δ 9.01 - 8.67 (m, 2H), 8.10 - 7.99 (m, 4H), 7.65 - 7.41 (m, 3H), 6.88 - 6.56 (m, 1H), 4.72 - 4.60 (m, 2H), 4.18 - 3.96 (m, 2H), 3.57 - 3.47 (m, 2H), 1.08 - 0.93 (m, 3H); MS (ESI): m/z 434.4 (M+H)⁺.

### Example 131:

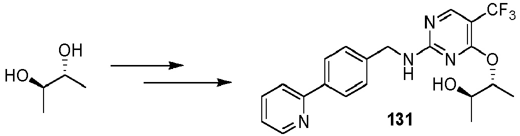

Starting from (R,R)-2,3-butanediol, compound **131** was obtained by referring to the synthesis of compound **26.** ¹H NMR (500 MHz, DMSO-*d*₆) δ 8.72 - 8.66 (m, 1H), 8.59 - 8.35 (m, 1H), 8.35 - 8.29 (m, 1H), 8.08 - 7.97 (m, 4H), 7.54 - 7.41 (m, 3H), 5.27 - 5.12 (m, 1H), 4.62 - 4.48 (m, 2H), 3.79 - 3.74 (m, 2H), 1.23 - 1.20 (m, 1H), 1.12 - 1.04 (m, 3H), 1.03 - 0.95 (m, 2H); MS (ESI): m/z 419.4 (M+H)⁺.

### Example 132:

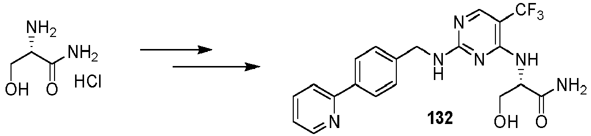

Compound **132** was obtained by referring to the synthesis of compound **24** starting from L-serinamide hydrochloride. ¹H NMR (500 MHz, DMSO-*d*₆) δ 8.82 - 8.68 (m, 2H), 8.40 - 8.30 (m, 1H), 8.10 - 7.98 (m, 4H), 7.66 - 7.49 (m, 3H), 7.46 - 7.35 (m, 2H), 4.69 - 4.58 (m, 3H), 3.82 - 3.78 (m, 2H), 3.74 - 3.70 (m, 2H); MS (ESI): m/z 433.4 (M+H)⁺.

### Example 133:

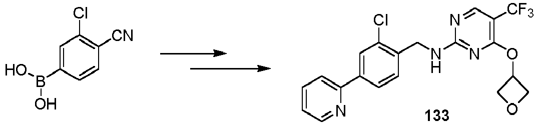

Compound **133** was obtained by referring to the synthesis of compound **4** starting from 4-cyano-3-chlorophenylboronic acid. ¹H NMR (500 MHz, DMSO-*d*₆) δ 8.68 - 8.66 (m, 1H), 8.45 - 8.39 (m, 1H), 8.22 - 8.14 (m, 1H), 8.06 - 7.97 (m, 2H), 7.95 - 7.87 (m, 1H), 7.43 - 7.35 (m, 2H), 5.74 - 5.44 (m, 1H), 4.96 - 4.80 (m, 1H), 4.72 - 4.32 (m, 6H); MS (ESI): m/z 437.5 (M+H)⁺.

### Example 134:

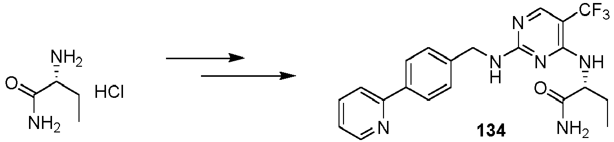

Compound **134** was obtained by referring to the synthesis of compound **24** starting from R-2-aminobutanamide hydrochloride. ¹H NMR (500 MHz, DMSO-*d*₆) δ 8.67 - 8.63 (m, 1H), 8.13 - 7.84 (m, 6H), 7.82 - 7.50 (m, 1H), 7.49 - 7.24 (m, 4H), 6.59 - 6.12 (m, 1H), 4.67 - 4.16 (m, 3H), 2.02 - 1.60 (m, 2H), 0.87 - 0.67 (m, 3H); MS (ESI): m/z 431.4 (M+H)⁺.

### Example 135:

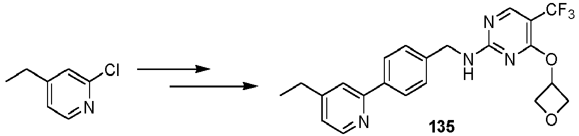

Compound **135** was obtained by referring to the synthesis of compound **12** starting from 2-chloro-4-ethylpyridine. ¹H NMR (500 MHz, DMSO-*d*₆) δ 8.75 - 8.36 (m, 3H), 8.09 - 7.97 (m, 2H), 7.64 - 7.57 (m, 1H), 7.42 - 7.34 (m, 2H), 7.03 - 6.97 (m, 1H), 5.68 - 5.48 (m, 1H), 4.93 - 4.37 (m, 6H), 2.04 - 1.95 (m, 1H), 1.10 - 1.02 (m, 2H), 0.92 - 0.86 (m, 2H); MS (ESI): m/z 431.5 (M+H)⁺.

### Example 136:

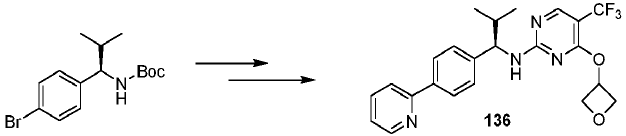

Compound **136** was obtained by referring to the synthesis of compound **12** starting from [(R)-1-(4-bromophenyl)-2-methylpropyl]carbamic acid tert-butyl ester. ¹H NMR (500 MHz, DMSO-*d*₆) δ 8.72 - 8.66 (m, 1H), 8.66 - 8.62 (m, 1H), 8.37 - 8.33 (m, 1H), 8.07 - 7.99 (m, 2H), 7.96 - 7.90 (m, 1H), 7.89 - 7.83 (m, 1H), 7.54 - 7.45 (m, 2H), 7.36 - 7.29 (m, 1H), 5.69 - 5.58 (m, 1H), 5.05 - 4.33 (m, 5H), 2.17 - 2.07 (m, 1H), 1.04 - 0.96 (m, 3H), 0.79 - 0.71 (m, 3H); MS (ESI): m/z 445.4 (M+H)⁺.

### Example 137:

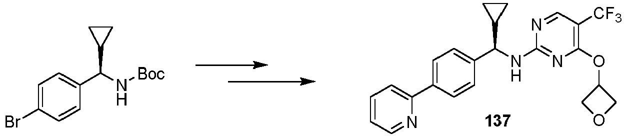

Compound **137** was obtained by referring to the synthesis of compound **12** starting from [(R)-(4-bromophenyl)-cyclopropylmethyl]carbamic acid tert-butyl ester. ¹H NMR (500 MHz, DMSO-*d*₆) δ 8.88 - 8.56 (m, 2H), 8.39 - 8.29 (m, 1H), 8.09 - 7.98 (m, 2H), 7.92 (t, *J* = 7.8 Hz, 1H), 7.86 (td, *J* = 7.7, 1.9 Hz, 1H), 7.59 - 7.50 (m, 2H), 7.38 - 7.29 (m, 1H), 5.70 - 5.45 (m, 1H), 5.35 - 4.16 (m, 5H), 2.04 - 1.92 (m, 1H), 0.61 - 0.33 (m, 4H); MS (ESI): m/z 443.4 (M+H)⁺.

### Example 138:

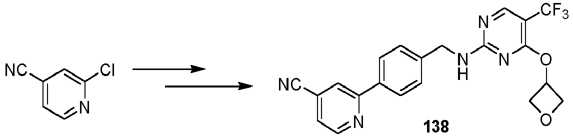

Compound **138** was obtained by referring to the synthesis of compound **12** starting from 2-chloro-4-cyanopyridine. ¹H NMR (500 MHz, DMSO-*d*₆) δ 8.88 (d, *J* = 5.0 Hz, 1H), 8.72 - 8.36 (m, 3H), 8.18 - 8.07 (m, 2H), 7.78 (dd, *J* = 5.0, 1.4 Hz, 1H), 7.43 (dd, *J* = 8.5, 2.4 Hz, 2H), 5.69 - 5.47 (m, 1H), 4.90 - 4.38 (m, 6H); MS (ESI): m/z 428.5 (M+H)⁺.

### Example 139:

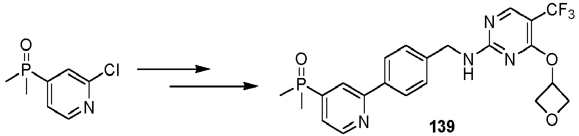

Compound **139** was obtained by referring to the synthesis of compound **12** starting from 2-chloro-4-dimethoxyphosphinylpyridine. ¹H NMR (500 MHz, DMSO-*d*₆) δ 8.80 (t, *J =* 4.4 Hz, 1H), 8.71 - 8.38 (m, 2H), 8.25 - 8.16 (m, 1H), 8.16 - 8.06 (m, 2H), 7.72 - 7.65 (m, 1H), 7.43 (d, *J=* 8.1 Hz, 2H), 5.68 - 5.51 (m, 1H), 4.91 - 4.39 (m, 6H), 1.74 (d, *J=* 13.6 Hz, 6H); MS (ESI): m/z 479.4 (M+H)⁺.

### Example 140:

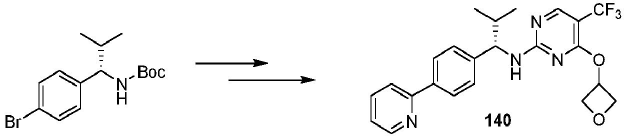

Compound **140** was obtained by referring to the synthesis of compound **12** starting from [(S)-1-(4-bromophenyl)-2-methylpropyl]carbamic acid tert-butyl ester. ¹H NMR (500 MHz, DMSO-*d*₆) δ 8.73 - 8.66 (m, 1H), 8.66 - 8.62 (m, 1H), 8.37 - 8.33 (m, 1H), 8.07 - 7.98 (m, 2H), 7.96 - 7.90 (m, 1H), 7.89 - 7.83 (m, 1H), 7.54 - 7.44 (m, 2H), 7.36 - 7.29 (m, 1H), 5.69 - 5.58 (m, 1H), 5.05 - 4.33 (m, 5H), 2.17 - 2.07 (m, 1H), 1.04 - 0.96 (m, 3H), 0.79 - 0.71 (m, 3H); MS (ESI): m/z 445.4 (M+H)⁺.

### Example 141:

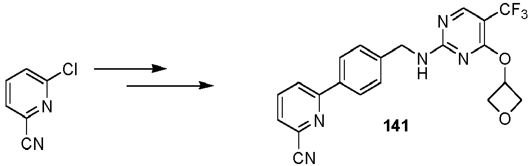

Compound **141** was obtained by referring to the synthesis of compound **12** starting from 6-chloro-2-cyanopyridine. ¹H NMR (500 MHz, DMSO-*d*₆) δ 8.72 - 8.39 (m, 2H), 8.34 - 8.25 (m, 1H), 8.17 - 8.04 (m, 3H), 7.98 (d, *J=* 7.7 Hz, 1H), 7.47 (d, *J* = 8.0 Hz, 2H), 5.70 - 5.51 (m, 1H), 4.95 - 4.40 (m, 6H); MS (ESI): m/z 428.4 (M+H)⁺.

### Example 142:

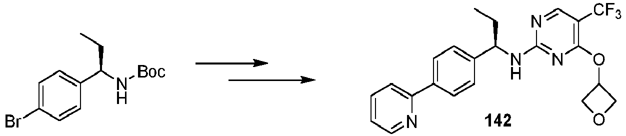

Compound **142** was obtained by referring to the synthesis of compound **12** starting from [(R)-1-(4-bromophenyl)propyl]carbamic acid tert-butyl ester. ¹H NMR (500 MHz, DMSO-*d*₆) δ 8.73 - 8.30 (m, 3H), 8.12 - 8.00 (m, 2H), 7.97 - 7.91 (m, 1H), 7.91 - 7.82 (m, 1H), 7.56 - 7.45 (m, 2H), 7.40 - 7.29 (m, 1H), 5.75 - 5.52 (m, 1H), 5.04 - 4.25 (m, 5H), 1.93 - 1.74 (m, 2H), 0.98 - 0.89 (m, 3H); MS (ESI): m/z 431.4 (M+H)⁺.

### Example 143:

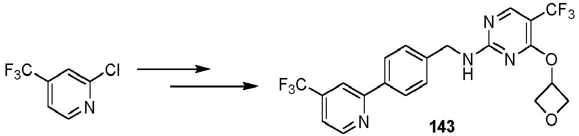

Compound **143** was obtained by referring to the synthesis of compound **12** starting from 2-chloro-4-trifluoromethylpyridine. ¹H NMR (500 MHz, DMSO-*d*₆) δ 8.93 (d, *J* = 5.1 Hz, 1H), 8.74 - 8.37 (m, 2H), 8.31 - 8.23 (m, 1H), 8.22 - 8.13 (m, 2H), 7.71 (d, *J* = 5.1 Hz, 1H), 7.46 (d, *J* = 8.0 Hz, 2H), 5.72 - 5.50 (m, 1H), 4.96 - 4.40 (m, 6H); MS (ESI): m/z 471.4 (M+H)⁺.

### Example 144:

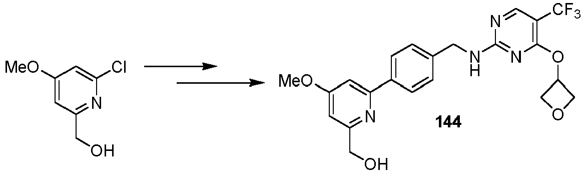

Compound **144** was obtained by referring to the synthesis of compound **12** starting from 6-chloro-4-methoxypyridine-2-methanol. ¹H NMR (500 MHz, DMSO-*d*₆) δ 8.74 - 8.38 (m, 2H), 8.09 - 7.98 (m, 2H), 7.39 (d, *J* = 8.1 Hz, 2H), 7.35 - 7.29 (m, 1H), 6.99 (s, 1H), 5.69 - 5.52 (m, 1H), 5.43 (t, *J=* 5.9 Hz, 1H), 4.97 - 4.40 (m, 8H), 3.91 (s, 3H); MS (ESI): m/z 463.4 (M+H)⁺.

### Example 145:

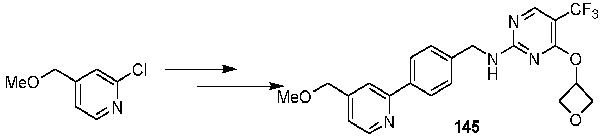

Compound **145** was obtained by referring to the synthesis of compound **12** starting from 6-chloro-4-methoxypyridine-2-methanol. ¹H NMR (500 MHz, DMSO-*d*₆) δ 8.76 - 8.38 (m, 3H), 8.11 - 8.01 (m, 2H), 7.87 - 7.82 (m, 1H), 7.42 (d, *J* = 8.1 Hz, 2H), 7.28 (d, *J* = 4.8 Hz, 1H), 5.74 - 5.53 (m, 1H), 4.91 - 4.40 (m, 8H), 3.38 (s, 3H); MS (ESI): m/z 447.4 (M+H)⁺.

### Example 146:

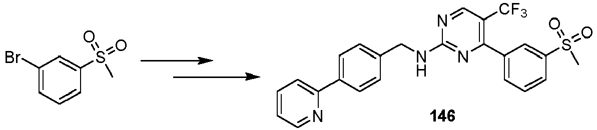

Starting from 3-bromophenylmethylsulfone, compound **146** was obtained by referring to the synthesis of compound **13.** ¹H NMR (500 MHz, DMSO-*d*₆) δ 8.89 - 8.83 (m, 1H), 8.77 - 8.75 (m, 1H), 8.66 (d, *J=* 4.8 Hz, 1H), 8.10 - 8.05 (m, 4H), 7.98 - 7.77 (m, 4H), 7.46 (dd, *J=* 8.4, 2.5 Hz, 2H), 7.34 (t, *J=* 6.1 Hz, 1H), 4.69 - 4.61 (m, 2H), 3.28 (d, *J=* 5.0 Hz, 3H); MS (ESI): m/z 485.4 (M+H)⁺.

### Example 147:

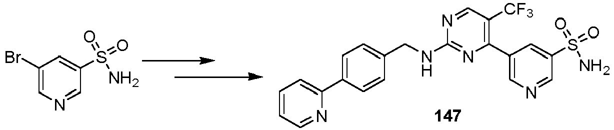

Starting from 5-bromopyridine-3-sulfonamide, compound **147** was obtained with reference to the synthesis of compound **13.** ¹H NMR (500 MHz, DMSO-*d*₆) δ 9.12 (t, *J* = 2.2 Hz, 1H), 8.98 - 8.77 (m, 3H), 8.66 (s, 1H), 8.36 - 8.29 (m, 1H), 8.05 (dd, *J* = 8.1, 4.9 Hz, 2H), 8.00 - 7.69 (m, 4H), 7.46 (t, *J* = 8.4 Hz, 2H), 7.35 (t, *J* = 5.9 Hz, 1H), 4.70 - 4.62 (m, 2H); MS (ESI): m/z 487.4 (M+H)⁺.

### Example 148:

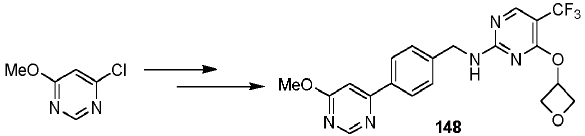

Compound **148** was obtained by referring to the synthesis of compound **12** starting from 4-chloro-6-methoxypyrimidine. ¹H NMR (500 MHz, DMSO-*d*₆) δ 8.93 - 8.38 (m, 3H), 8.17 - 8.12 (m, 2H), 7.53 - 7.41 (m, 3H), 5.67 - 5.53 (m, 1H), 4.92 - 4.68 (m, 2H), 4.64 - 4.40 (m, 4H), 3.98 (s, 3H); MS (ESI): m/z 434.4 (M+H)⁺.

### Example 149:

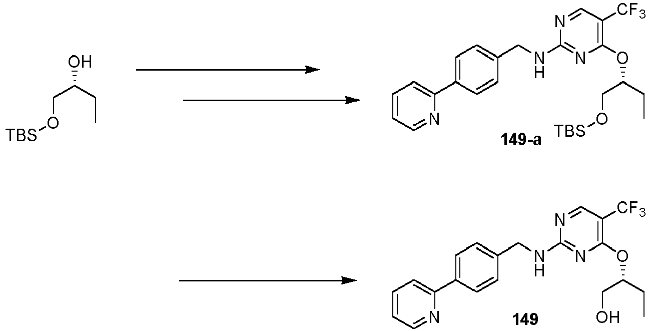

Starting from R-1-tert-butyldimethylsiloxy-2-butanol, compound **149-a** was obtained by referring to the synthesis of compound **26.** Starting from **149-a,** compound **149** was obtained by referring to the synthesis of compound **88**. ¹H NMR (500 MHz, DMSO-*d*₆) δ 8.68 - 8.62 (m, 1H), 8.62 - 8.38 (m, 1H), 8.37 - 8.28 (m, 1H), 8.08 - 8.00 (m, 2H), 7.95 - 7.89 (m, 1H), 7.91 - 7.82 (m, 1H), 7.48 - 7.38 (m, 2H), 7.36 - 7.30 (m, 1H), 5.29 - 3.44 (m, 6H), 1.80 - 1.29 (m, 2H), 0.96 - 0.72 (m, 3H); MS (ESI): m/z 419.4 (M+H)⁺.

### Example 150:

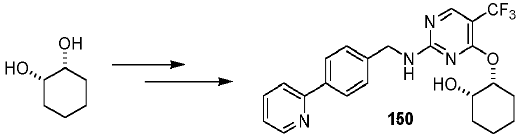

Starting from cis-1,2-cyclohexanediol, compound **150** was obtained by referring to the synthesis of compound **26.** ¹H NMR (500 MHz, DMSO-*d*₆) δ 8.71 - 8.62 (m, 1H), 8.54 - 8.29 (m, 2H), 8.07 - 8.01 (m, 2H), 7.96 - 7.89 (m, 1H), 7.89 - 7.83 (m, 1H), 7.50 - 7.37 (m, 2H), 7.37 - 7.29 (m, 1H), 5.48 - 5.21 (m, 1H), 4.71 - 4.39 (m, 3H), 3.77 - 3.65 (m, 1H), 1.94 - 1.21 (m, 8H); MS (ESI): m/z 445.5 (M+H)⁺.

### Example 151:

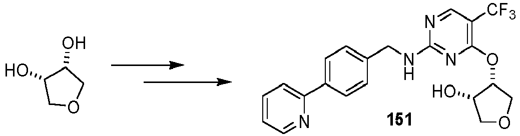

Compound **151** was obtained by referring to the synthesis of compound **26** starting from cis-tetrahydrofuran-3,4-diol. ¹H NMR (500 MHz, DMSO-*d*₆) δ 8.67 (d, *J* = 4.9 Hz, 1H), 8.62 - 8.33 (m, 2H), 8.09 - 8.01 (m, 2H), 8.00 - 7.90 (m, 2H), 7.52 - 7.42 (m, 2H), 7.42 - 7.35 (m, 1H), 5.54 - 5.32 (m, 1H), 4.69 - 4.28 (m, 3H), 4.13 - 3.94 (m, 1H), 3.93 - 3.84 (m, 1H), 3.75 - 3.63 (m, 1H), 3.57 - 3.50 (m, 2H); MS (ESI): m/z 433.4 (M+H)⁺.

### Example 152:

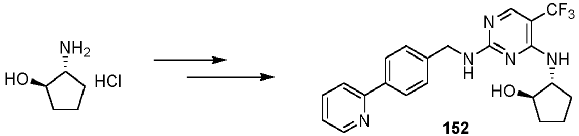

Compound **152** was obtained by referring to the synthesis of compound **24** starting from (1R,2R)-2-aminocyclopent-1-ol hydrochloride. ¹H NMR (500 MHz, DMSO-*d*₆) δ 8.65 (d, *J* = 4.8 Hz, 1H), 8.10 - 8.02 (m, 3H), 8.00 - 7.81 (m, 3H), 7.50 - 7.39 (m, 2H), 7.38 - 7.32 (m, 1H), 6.18 (d, *J* = 7.0 Hz, 1H), 4.75 - 4.70 (m, 1H), 4.61 - 4.46 (m, 2H), 4.22 - 4.02 (m, 2H), 2.02 - 1.82 (m, 2H), 1.69 - 1.40 (m, 4H); MS (ESI): m/z 430.5 (M+H)⁺.

### Example 153:

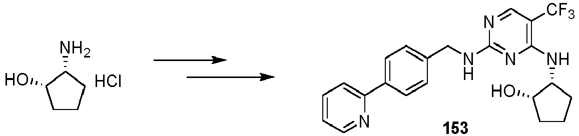

Compound **153** was obtained by referring to the synthesis of compound **24** starting from (1S,2R)-2-aminocyclopent-1-ol hydrochloride. ¹H NMR (500 MHz, DMSO-*d*₆) δ 8.65 (d, *J* = 4.8 Hz, 1H), 8.15 - 7.98 (m, 4H), 7.93 (d, *J* = 7.9 Hz, 1H), 7.89 - 7.85 (m, 1H), 7.42 (d, *J* = 7.8 Hz, 2H), 7.37 - 7.31 (m, 1H), 6.02 - 5.93 (m, 1H), 5.27 - 5.15 (m, 1H), 4.60 - 4.44 (m, 2H), 4.21 - 3.94 (m, 2H), 1.88 - 1.76 (m, 2H), 1.73 - 1.52 (m, 2H), 1.47 - 1.31 (m, 2H); MS (ESI): m/z 430.5 (M+H)⁺.

### Example 154:

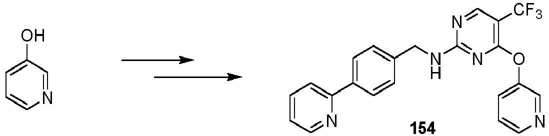

Starting from 3-hydroxypyridine, compound **154** was obtained by referring to the synthesis of compound **24.** ¹H NMR (500 MHz, DMSO-*d*₆) δ 8.84 - 8.51 (m, 5H), 8.04 - 7.89 (m, 4H), 7.86 - 7.68 (m, 1H), 7.62 - 7.53 (m, 1H), 7.49 - 7.37 (m, 2H), 7.00 (d, *J* = 8.0 Hz, 1H), 4.59 - 4.13 (m, 2H); MS (ESI): m/z 424.4 (M+H)⁺.

### Example 155:

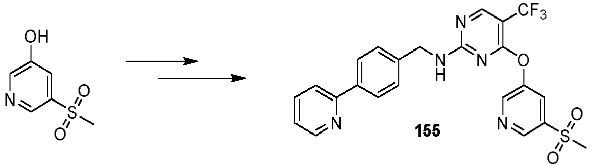

Compound **155** was obtained by referring to the synthesis of compound **24** starting from 5-hydroxypyridine-3-methylsulfone. ¹H NMR (500 MHz, DMSO-*d*₆) δ 9.09 - 9.03 (m, 1H), 9.00 - 8.61 (m, 3H), 8.57 (d, *J* = 6.7 Hz, 1H), 8.42 - 8.35 (m, 1H), 8.01 (d, *J* = 8.0 Hz, 1H), 7.94 - 7.86 (m, 3H), 7.45 - 7.32 (m, 2H), 6.98 (d, *J* = 7.9 Hz, 1H), 4.58 - 4.16 (m, 2H), 3.42 - 3.38 (m, 3H); MS (ESI): m/z 502.4 (M+H)⁺.

### Example 156:

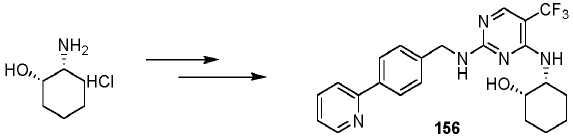

Starting from (1S,2R)-2-aminocyclohexan-1-ol hydrochloride, compound **156** was obtained by referring to the synthesis of compound **24.** ¹H NMR (500 MHz, DMSO-*d*₆) δ 8.65 (d, *J* = 4.8 Hz, 1H), 8.13 - 7.99 (m, 4H), 7.92 (d, *J* = 7.9 Hz, 1H), 7.87 (t, *J* = 7.6 Hz, 1H), 7.41 (d, *J* = 7.9 Hz, 2H), 7.35 - 7.31 (m, 1H), 5.68 (d, *J* = 7.6 Hz, 1H), 5.08 - 4.91 (m, 1H), 4.55 - 4.40 (m, 2H), 4.08 - 3.68 (m, 2H), 1.79 - 1.58 (m, 2H), 1.51 - 1.46 (m, 4H), 1.38 - 1.30 (m, 2H); MS (ESI): m/z 444.6 (M+H)⁺.

### Example 157:

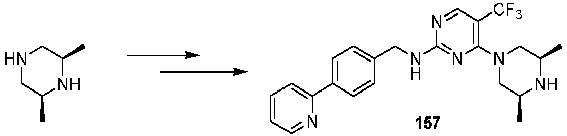

Starting from cis-2,6-dimethylpiperazine, compound **157** was obtained by referring to the synthesis of compound **24.** ¹H NMR (500 MHz, DMSO-*d*₆) δ 8.69 - 8.60 (m, 1H), 8.33 - 7.72 (m, 6H), 7.51 - 7.17 (m, 3H), 4.65 - 4.41 (m, 2H), 3.96 - 3.72 (m, 2H), 2.80 - 2.57 (m, 2H), 2.44 - 2.35 (m, 2H), 0.99 - 0.89 (m, 6H); MS (ESI): m/z 443.4 (M+H)⁺.

### Example 158:

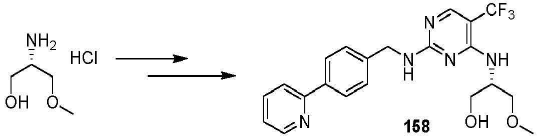

Compound **158** was obtained by referring to the synthesis of compound **24** starting from (S)-2-amino-3-methoxypropan-1-ol hydrochloride. ¹H NMR (500 MHz, DMSO-*d*₆) δ 8.74 - 8.59 (m, 1H), 8.13 - 7.82 (m, 6H), 7.50 - 7.31 (m, 3H), 5.94 - 5.79 (m, 1H), 5.00 - 4.80 (m, 1H), 4.59 - 4.26 (m, 3H), 3.59 - 3.35 (m, 4H), 3.30 - 3.12 (m, 3H); MS (ESI): m/z 434.5 (M+H)⁺.

### Example 159:

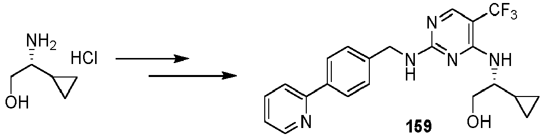

Compound 159 was obtained by referring to the synthesis of compound 24 starting from (R)-2-amino-2-cyclopropethanol hydrochloride. ¹H NMR (500 MHz, DMSO-*d*₆) δ 8.75 - 8.49 (m, 1H), 8.12 - 8.01 (m, 3H), 7.98 - 7.73 (m, 3H), 7.44 - 7.36 (m, 2H), 7.36 - 7.30 (m, 1H), 6.15 - 5.82 (m, 1H), 4.87 - 4.70 (m, 1H), 4.61 - 4.37 (m, 2H), 3.77 - 3.45 (m, 3H), 1.09 - 0.97 (m, 1H), 0.52 - 0.25 (m, 2H), 0.22 - 0.06 (m, 2H); MS (ESI): m/z 430.4 (M+H)⁺.

### Example 160:

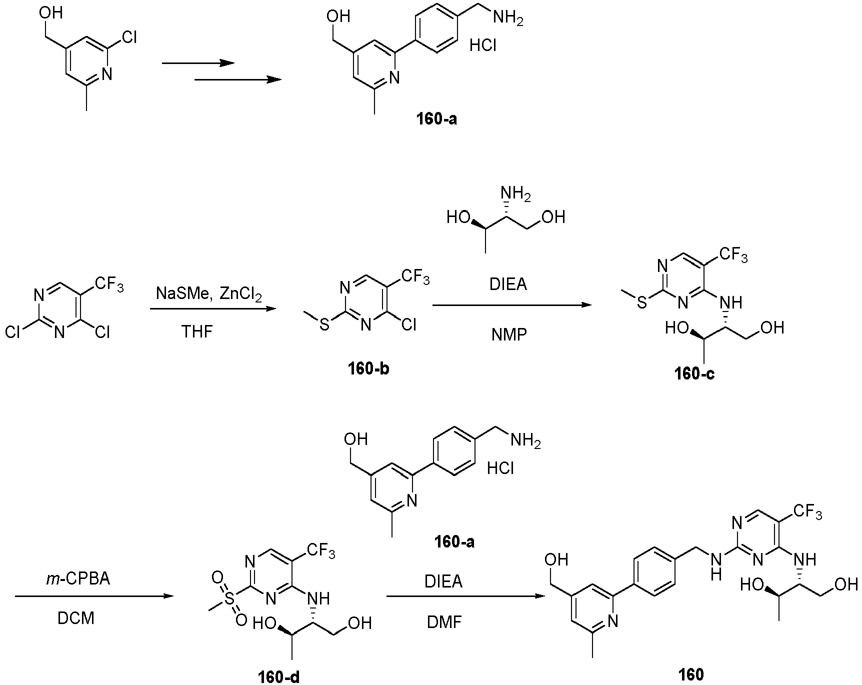

Starting from (2-chloro-6-methylpyridin-4-yl)methanol, compound **160-a** was obtained by referring to the synthesis of compound **12-c.**

2,4-dichloro-5-(trifluoromethyl)pyrimidine (20.00 g, 92.18 mmol) was dissolved in tetrahydrofuran (400 mL), and zinc chloride (16.33 g, 119.8 mmol) was added in portions at 0°C. After the mixture was stirred at 0°C for 0.5 hours, 20% sodium methylmercaptide aqueous solution (48.45 g, 138.3 mmol) was slowly added, and the mixture was continued to stir at room temperature for 5 hours. The reaction system was added with 400 mL water and 400 mL ethyl acetate, and filtered, and the filtrate was separated, the aqueous phase was extracted twice with 200 mL ethyl acetate. The organic phases were combined, washed three times with water, once with saturated brine, and dried with anhydrous sodium sulfate, filtered and concentrated. The residue was purified by column chromatography to obtain compound **160-b** (8.44 g, 36.9 mmol), with a yield of 40.0%.

Compound **160-b** (100 mg, 0.437 mmol) and (2R,3R)-2-aminobutane-1,3-diol (48.3 mg, 0.459 mmol) were dissolved in N-methylpyrrolidone (3 mL), N,N-diisopropylethylamine (170 mg, 1.31 mmol) was added. The mixture was stirred at 90°C for 16 hours. Add 20 mL of water to the reaction system, extract twice with 20 mL of ethyl acetate, wash the combined organic phase three times with water, once with saturated brine, dry over anhydrous sodium sulfate, filter and concentrate. The residue was purified by column chromatography to obtain compound **160-c** (110 mg, 0.37 mmol), with a yield of 84.6%. MS (ESI): m/z 298.4 (M+H)⁺.

Compound **160-b** (100 mg, 0.437 mmol) was dissolved in ethyl acetate (4 mL), and 3-chloroperoxybenzoic acid (192 mg, 1.11 mmol) was added under ice bath. The mixture was stirred at room temperature for 7 hours. Add 20 mL ethyl acetate to the reaction system, wash three times with 5 mL saturated sodium thiosulfate and 5 mL saturated sodium bicarbonate, wash once with saturated brine, dry over anhydrous sodium sulfate, filter, and concentrate to obtain the crude product compound **160-d.** (100 mg, 0.304 mmol) with a yield of 84.6%. MS (ESI): m/z 330.5 (M+H)⁺.

Compounds **160-d** (37.31 mg, 0.113 mmol) and **160-a** (30 mg, 0.113 mmol) were dissolved in N-methylpyrrolidone (3 mL), and N,N-diisopropylethylamine (73.2 mg, 0.567 mmol) was added. The mixture was stirred at 90°C for 3 hours. After the reaction solution was cooled to room temperature, compound **160** (10 mg, 20.9 µmol) was obtained through purification by preparative liquid chromatography with a yield of 18.5%. ¹H NMR (500 MHz, DMSO-*d*₆) δ 8.15 - 7.78 (m, 4H), 7.67 - 7.61 (m, 1H), 7.53 - 7.35 (m, 2H), 7.20 - 7.11 (m, 1H), 5.70 - 5.63 (m, 1H), 5.45 - 5.39 (m, 1H), 5.03 - 4.92 (m, 1H), 4.81 - 4.73 (m, 1H), 4.63 - 4.45 (m, 4H), 4.11 - 4.02 (m, 2H), 3.56 - 3.41 (m, 2H), 2.53 - 2.51 (m, 3H), 1.09 - 0.93 (m, 3H); MS (ESI): m/z 478.5 (M+H)⁺.

### Example 161:

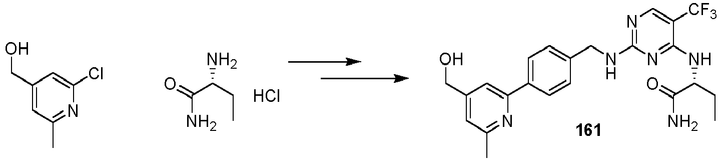

Compound **161** was obtained by referring to the synthesis of compound **160** starting from (2-chloro-6-methylpyridin-4-yl)methanol and R-2-aminobutanamide hydrochloride. ¹H NMR (500 MHz, DMSO-*d*₆) δ 8.16 - 7.98 (m, 3H), 7.87 - 7.59 (m, 2H), 7.46 - 7.42 (m, 1H), 7.40 - 7.29 (m, 1H), 7.27 - 7.14 (m, 1H), 6.30 - 6.21 (m, 1H), 5.48 - 5.32 (m, 1H), 4.62 - 4.46 (m, 4H), 2.53 - 2.51 (m, 3H), 2.04 - 1.90 (m, 1H), 1.90 - 1.73 (m, 1H), 0.85 - 0.69 (m, 3H); MS (ESI): m/z 475.5 (M+H)⁺.

### Example 162:

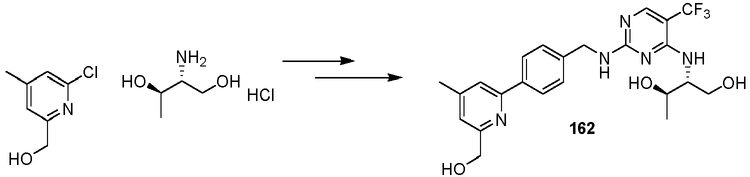

Compound **162** was obtained by referring to the synthesis of compound **160** starting from (6-chloro-4-methylpyridin-2-yl)methanol and L-threoninol hydrochloride. ¹H NMR (500 MHz, DMSO-*d*₆) δ 8.11 - 7.98 (m, 4H), 7.62 (s, 1H), 7.47 - 7.36 (m, 2H), 7.25 (s, 1H), 5.66 (d, *J* = 7.8 Hz, 1H), 5.41 - 5.35 (m, 1H), 5.04 - 4.93 (m, 1H), 4.82 - 4.72 (m, 1H), 4.62 - 4.47 (m, 4H), 4.12 - 4.02 (m, 2H), 3.52 - 3.39 (m, 2H), 2.39 (s, 3H), 1.09 - 0.93 (m, 3H); MS (ESI): m/z 478.4 (M+H)⁺.

### Example 163:

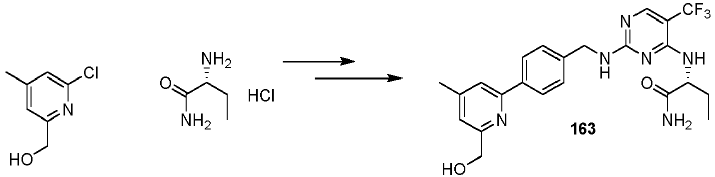

Compound **163** was obtained by referring to the synthesis of compound 160 starting from (6-chloro-4-methylpyridin-2-yl)methanol and R-2-aminobutanamide hydrochloride. ¹H NMR (500 MHz, DMSO-*d*₆) δ 8.24 - 7.97 (m, 4H), 7.70 - 7.59 (m, 2H), 7.47 - 7.37 (m, 2H), 7.36 - 7.28 (m, 1H), 7.25 (s, 1H), 6.34 - 6.21 (m, 1H), 5.43 - 5.36 (m, 1H), 4.62 - 4.46 (m, 5H), 2.40 (s, 3H), 2.06 - 1.90 (m, 1H), 1.88 - 1.65 (m, 1H), 0.84 - 0.69 (m, 3H); MS (ESI): m/z 475.5 (M+H)⁺.

### Example 164:

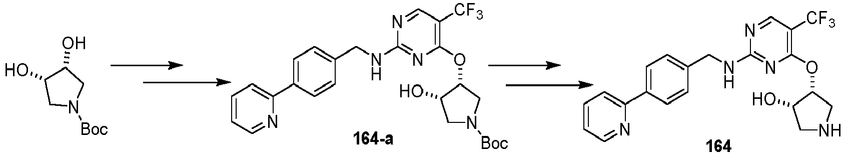

Starting from cis-tetrahydrofuran-3,4-diol, compound **164-a** was obtained by referring to the synthesis of compound **26.** Starting from compound **164-a,** compound **164** was obtained by referring to the synthesis of compound **101.** ¹H NMR (500 MHz, DMSO-*d*₆) δ 8.65 (d, *J* = 4.9 Hz, 1H), 8.58 - 8.27 (m, 2H), 8.07 - 8.00 (m, 2H), 7.92 (d, *J* = 8.0 Hz, 1H), 7.90 - 7.83 (m, 1H), 7.48 - 7.39 (m, 2H), 7.33 (dd, *J* = 7.3, 4.9 Hz, 1H), 5.50 - 5.12 (m, 1H), 4.77 - 4.36 (m, 3H), 4.30 - 4.04 (m, 1H), 3.24 - 2.54 (m, 4H); MS (ESI): m/z 432.5 (M+H)⁺.

### Example 165:

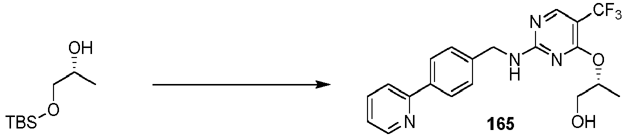

Compound **165** was obtained by referring to the synthesis of compound **149** starting from R-1-tert-butyldimethylsiloxy-2-propanol. ¹H NMR (500 MHz, DMSO-*d*₆) δ 8.68 - 8.63 (m, 1H), 8.63 - 8.40 (m, 1H), 8.38 - 8.28 (m, 1H), 8.07 - 8.01 (m, 2H), 7.95 - 7.84 (m, 2H), 7.48 - 7.39 (m, 2H), 7.36 - 7.31 (m, 1H), 5.38 - 4.21 (m, 4H), 4.19 - 3.41 (m, 2H), 1.28 - 1.03 (m, 3H); MS (ESI): m/z 404.1 (M+H)⁺.

### Example 166:

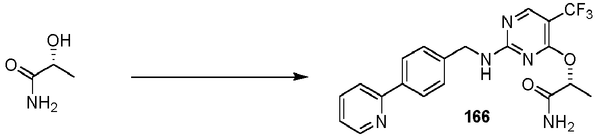

Starting from (R)-2-hydroxypropionamide, compound **166** was obtained by referring to the synthesis of compound **26.** ¹H NMR (500 MHz, DMSO-*d*₆) δ 8.69 - 8.63 (m, 1H), 8.58 - 8.26 (m, 2H), 8.08 - 7.99 (m, 2H), 7.96 - 7.83 (m, 2H), 7.47 - 7.44 (m, 1H), 7.43 - 7.38 (m, 1H), 7.35 - 7.31 (m, 1H), 7.27 - 7.14 (m, 2H), 5.39 - 5.22 (m, 1H), 4.62 - 4.45 (m, 2H), 1.47 - 1.40 (m, 3H); MS (ESI): m/z 418.3 (M+H)⁺.

### Example 167:

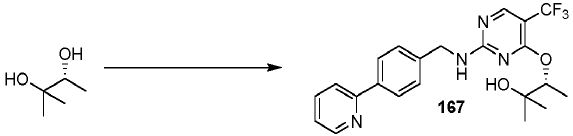

Compound **167** was obtained by referring to the synthesis of compound **26** starting from (R)-2-methylbutane-2,3-diol. ¹H NMR (500 MHz, DMSO-*d*₆) δ 8.69 - 8.62 (m, 1H), 8.60 - 8.28 (m, 2H), 8.08 - 7.99 (m, 2H), 7.97 - 7.90 (m, 1H), 7.90 - 7.82 (m, 1H), 7.49 - 7.38 (m, 2H), 7.37 - 7.29 (m, 1H), 5.14 - 4.99 (m, 1H), 4.64 - 4.46 (m, 3H), 1.27 - 1.05 (m, 9H); MS (ESI): m/z 433.7 (M+H)⁺.

### Example 168:

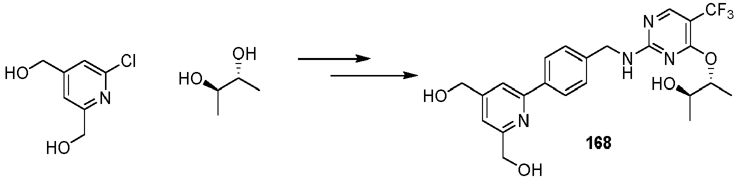

Compound **168** was obtained by referring to the synthesis of compound **160** starting from (6-chloropyridin-2,4-yl)dimethanol and (2R,3R)-butane-2,3-diol. ¹H NMR (500 MHz, DMSO-*d*₆) δ 8.57 - 8.27 (m, 2H), 8.04 - 7.98 (m, 2H), 7.71 - 7.67 (m, 1H), 7.46 - 7.37 (m, 3H), 5.45 (t, *J* = 5.8 Hz, 1H), 5.41 (t, *J* = 5.9 Hz, 1H), 5.32 - 5.12 (m, 1H), 4.83 - 4.73 (m, 1H), 4.68 - 4.45 (m, 6H), 3.84 - 3.73 (m, 1H), 1.24 - 0.98 (m, 6H); MS (ESI): m/z 479.7 (M+H)⁺.

### Example 169:

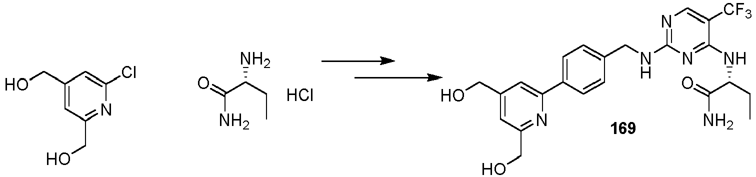

Compound **169** was obtained by referring to the synthesis of compound 160 starting from (6-chloropyridin-2,4-yl)dimethanol and R-2-aminobutanamide hydrochloride. ¹H NMR (500 MHz, DMSO-*d*₆) δ 8.20 - 7.98 (m, 4H), 7.76 - 7.60 (m, 2H), 7.54 - 7.28 (m, 4H), 6.38 - 6.23 (m, 1H), 5.49 - 5.40 (m, 2H), 4.65 - 4.45 (m, 7H), 2.09 - 1.74 (m, 2H), 0.86 - 0.69 (m, 3H); MS (ESI): m/z 491.4 (M+H)⁺.

### Example 170:

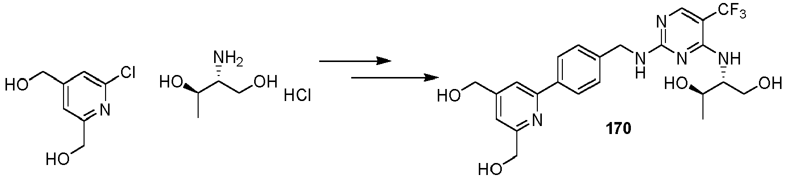

Compound **170** was obtained by referring to the synthesis of compound **160** starting from (6-chloropyridin-2,4-yl)dimethanol and L-threoninol hydrochloride. ¹H NMR (500 MHz, DMSO-*d*₆) δ 8.13 - 7.97 (m, 4H), 7.69 (s, 1H), 7.49 - 7.39 (m, 3H), 5.72 - 5.62 (m, 1H), 5.46 (t, *J* = 5.7 Hz, 1H), 5.41 (t, *J* = 5.8 Hz, 1H), 5.03 - 4.94 (m, 1H), 4.83 - 4.74 (m, 1H), 4.72 - 4.42 (m, 6H), 4.11 - 4.01 (m, 2H), 3.52 - 3.41 (m, 2H), 1.11 - 0.92 (m, 3H); MS (ESI): m/z 494.4 (M+H)⁺.

### Example 171:

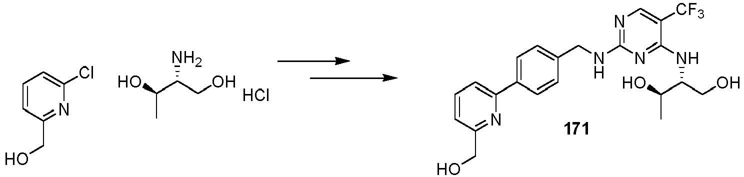

Compound **171** was obtained by referring to the synthesis of compound **160** starting from (6-chloropyridin-2-yl)methanol and L-threoninol hydrochloride. ¹H NMR (500 MHz, DMSO-*d*₆) δ 8.08 - 7.33 (m, 9H), 5.73 - 5.61 (m, 1H), 5.52 - 5.33 (m, 1H), 5.02 - 4.70 (m, 2H), 4.65 - 4.46 (m, 4H), 4.14 - 3.97 (m, 2H), 3.52 - 3.40 (m, 2H), 1.09 - 0.92 (m, 3H); MS (ESI): m/z 464.4 (M+H)⁺.

### Example 172:

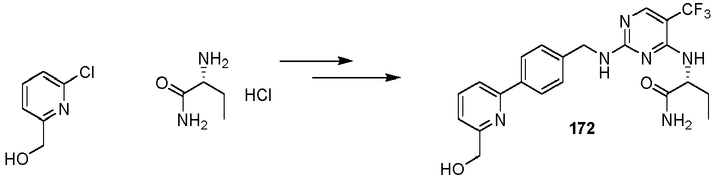

Compound **172** was obtained following the synthesis of compound **160** starting from (6-chloropyridin-2-yl)methanol and R-2-aminobutanamide hydrochloride. ¹H NMR (500 MHz, DMSO-*d*₆) δ 8.14 - 7.99 (m, 4H), 7.90 - 7.77 (m, 2H), 7.67 - 7.60 (m, 1H), 7.47 - 7.28 (m, 4H), 6.30 - 6.22 (m, 1H), 5.45 - 5.37 (m, 1H), 4.64 - 4.61 (m, 2H), 4.60 - 4.47 (m, 3H), 1.91 - 1.64 (m, 2H), 0.82 - 0.69 (m, 3H); MS (ESI): m/z 461.4 (M+H)⁺.

### Example 173:

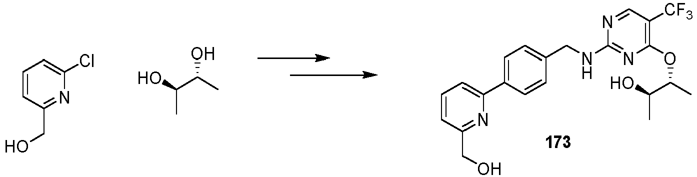

Compound **173** was obtained by referring to the synthesis of compound **160** starting from (6-chloropyridin-2-yl)methanol and (2R,3R)-butane-2,3-diol. ¹H NMR (500 MHz, DMSO-*d*₆) δ 8.59 - 8.28 (m, 2H), 8.07 - 7.73 (m, 4H), 7.49 - 7.38 (m, 3H), 5.45 - 5.39 (m, 1H), 5.28 - 5.14 (m, 1H), 4.83 - 4.74 (m, 1H), 4.65 - 4.49 (m, 4H), 3.90 - 3.68 (m, 1H), 1.24 - 1.00 (m, 6H); MS (ESI): m/z 449.4 (M+H)⁺.

### Example 174:

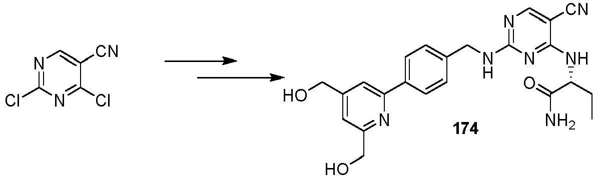

Compound **174** was obtained by referring to the synthesis of compound **169** starting from 2,4-dichloro-5-cyanopyrimidine. ¹H NMR (500 MHz, DMSO-*d*₆) δ 8.51 - 8.10 (m, 2H), 8.00 (d, *J* = 8.2 Hz, 2H), 7.76 (s, 1H), 7.60 - 7.38 (m, 4H), 7.17 (s, 1H), 4.83 - 4.42 (m, 8H), 2.04 - 1.70 (m, 3H), 0.88 - 0.79 (m, 4H); MS (ESI): m/z 448.3 (M+H)⁺.

### Example 175:

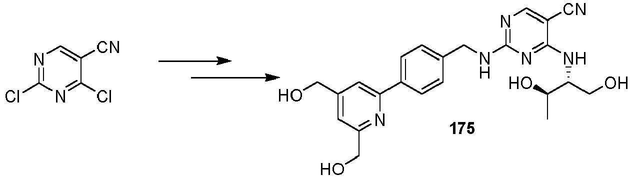

Compound **175** was obtained by referring to the synthesis of compound **170** starting from 2,4-dichloro-5-cyanopyrimidine. ¹H NMR (500 MHz, DMSO-*d*₆) δ 8.34 - 7.97 (m, 4H), 7.69 (s, 1H), 7.57 - 7.36 (m, 3H), 5.53 - 5.39 (m, 2H), 4.90 - 4.54 (m, 7H), 4.53 - 4.43 (m, 1H), 4.17 - 3.80 (m, 2H), 3.54 - 3.41 (m, 2H), 0.96 (d, *J* = 6.7 Hz, 3H); MS (ESI): m/z 451.3 (M+H)⁺.

### Example 176

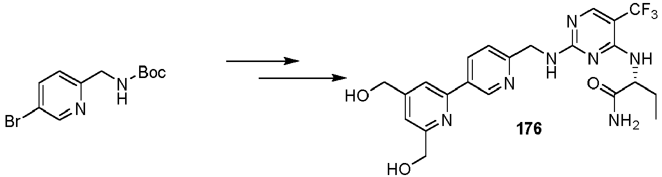

Compound **176** was obtained by referring to the synthesis of compound 169 starting from tert-butyl ((5-bromopyridin-2-yl)methyl)carbamate. ¹H NMR (500 MHz, DMSO-*d*₆) δ 9.19 (s, 1H), 8.45 (d, *J* = 8.6 Hz, 2H), 8.25 (s, 1H), 7.84 - 7.79 (m, 1H), 7.68 - 7.61 (m, 1H), 7.56 - 7.46 (m, 2H), 7.40 - 7.29 (m, 1H), 4.79 - 4.44 (m, 8H), 2.06 - 1.44 (m, 2H), 0.85 - 0.61 (m, 3H); MS (ESI): m/z 492.3 (M+H)⁺.

### Example 177:

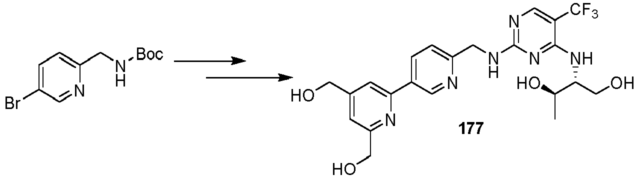

Compound **177** was obtained by referring to the synthesis of compound **170** starting from tert-butyl ((5-bromopyridin-2-yl)methyl)carbamate. ¹H NMR (500 MHz, DMSO-*d*₆) δ 9.22 (d, *J=* 2.2 Hz, 1H), 8.94 - 8.51 (m, 2H), 8.38 (s, 1H), 7.87 (s, 1H), 7.67 (d, *J* = 8.4 Hz, 1H), 7.56 (s, 1H), 6.78 - 6.62 (m, 1H), 4.88 - 4.73 (m, 3H), 4.68 - 4.64 (m, 5H), 3.98 - 3.94 (m, 2H), 3.57 - 3.41 (m, 4H), 0.94 - 0.88 (m, 3H); MS (ESI): m/z 495.2 (M+H)⁺.

### Example 178:

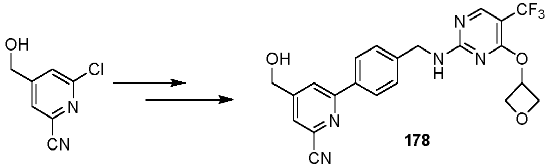

Compound **178** was obtained by referring to the synthesis of compound **12** starting from 6-chloro-4-(hydroxymethyl)pyridinecarbonitrile. ¹H NMR (500 MHz, DMSO-*d*₆) δ 8.72 - 8.40 (m, 2H), 8.19 (d, *J* = 8.7 Hz, 1H), 8.15 - 7.86 (m, 3H), 7.47 (d, *J* = 7.9 Hz, 2H), 5.73 - 5.52 (m, 2H), 4.92 - 4.66 (m, 4H), 4.63 - 4.42 (m, 4H); MS (ESI): m/z 458.3 (M+H)⁺.

### Example 179:

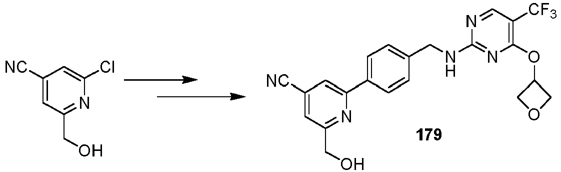

Starting from 2-chloro-6-(hydroxymethyl)isonicotinonitrile, compound **179** was obtained by referring to the synthesis of compound **12.** ¹H NMR (500 MHz, DMSO-*d*₆) δ 8.73 - 8.39 (m, 2H), 8.35 - 8.08 (m, 3H), 7.79 - 7.71 (m, 1H), 7.52 - 7.41 (m, 2H), 5.74 - 5.50 (m, 2H), 4.92 - 4.66 (m, 4H), 4.63 - 4.40 (m, 4H): m/z 458.3 (M+H)⁺.

### Example 180:

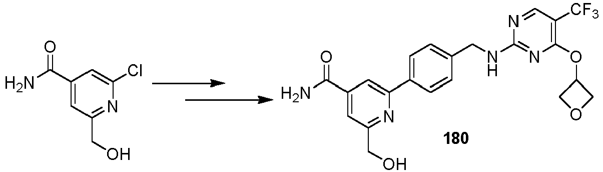

Compound **180** was obtained by referring to the synthesis of compound **12** starting from 2-chloro-6-(hydroxymethyl)isonicotinamide. ¹H NMR (500 MHz, DMSO-*d*₆) δ 8.71 - 8.40 (m, 2H), 8.34 (s, 1H), 8.20 - 8.06 (m, 3H), 7.91 - 7.69 (m, 2H), 7.43 (d, *J* = 8.1 Hz, 2H), 5.66 - 5.53 (m, 2H), 4.90 - 4.66 (m, 4H), 4.61 - 4.41 (m, 4H): m/z 476.3 (M+H)⁺.

### Example 181:

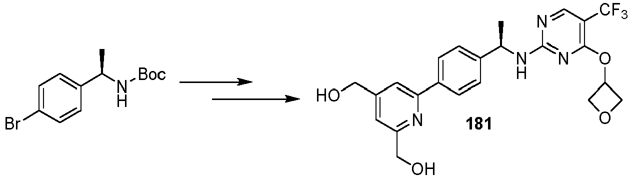

Compound **181** was obtained by referring to the synthesis of compound **125** starting from [(R)-1-(4-bromophenyl)ethyl]carbamic acid tert-butyl ester. ¹H NMR (500 MHz, DMSO-*d*₆) δ 8.73 - 8.33 (m, 2H), 8.06 - 7.64 (m, 3H), 7.52 - 7.38 (m, 3H), 5.71 - 5.33 (m, 3H), 5.26 - 4.18 (m, 9H), 1.55 - 1.44 (m, 3H): m/z 477.3 (M+H)⁺.

### Example 182:

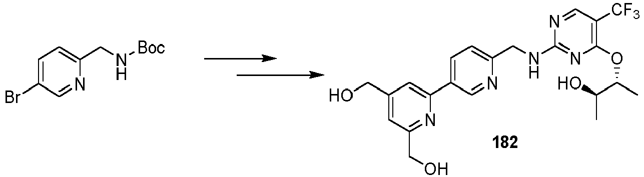

Compound **182** was obtained by referring to the synthesis of compound **168** starting from tert-butyl ((5-bromopyridin-2-yl)methyl)carbamate. ¹H NMR (500 MHz, DMSO-*d*₆) δ 9.17 - 8.32 (m, 3H), 7.80 - 6.96 (m, 4H), 5.60 - 4.56 (m, 8H), 3.84 - 3.59 (m, 1H), 1.28 - 1.24 (m, 3H), 1.01 - 0.83 (m, 3H); MS (ESI): m/z 480.2 (M+H)⁺.

### Example 183:

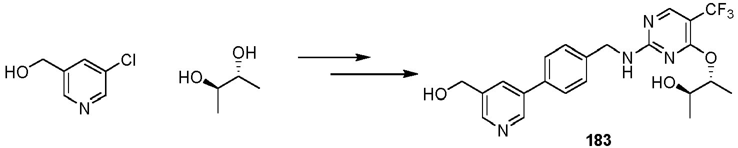

Compound **183** was obtained by referring to the synthesis of compound **160** starting from (5-chloropyridin-3-yl)methanol and (2R,3R)-butane-2,3-diol. ¹H NMR (500 MHz, DMSO-*d*₆) δ 8.78 - 8.72 (m, 1H), 8.59 - 8.28 (m, 3H), 7.95 (s, 1H), 7.70 - 7.65 (m, 2H), 7.49 - 7.41 (m, 2H), 5.40 - 5.35 (m, 1H), 5.29 - 5.13 (m, 1H), 4.83 - 4.74 (m, 1H), 4.66 - 4.46 (m, 4H), 3.82 - 3.72 (m, 1H), 1.24 - 1.09 (m, 3H), 1.09 - 1.00 (m, 3H); MS (ESI): m/z 449.2 (M+H)⁺.

### Example 184:

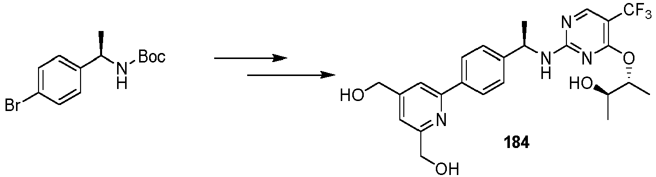

Compound **184** was obtained by referring to the synthesis of compound **168** starting from [(R)-1-(4-bromophenyl)ethyl]carbamic acid tert-butyl ester. ¹H NMR (500 MHz, DMSO-*d*₆) δ 8.61 - 8.33 (m, 1H), 8.28 (s, 1H), 8.03 - 7.94 (m, 2H), 7.67 (s, 1H), 7.54 - 7.46 (m, 2H), 7.40 (s, 1H), 5.52 - 5.34 (m, 2H), 5.30 - 4.98 (m, 2H), 4.86 - 4.72 (m, 1H), 4.67 - 4.57 (m, 4H), 3.83 - 3.74 (m, 1H), 1.54 - 1.45 (m, 3H), 1.10 - 1.02 (m, 3H), 0.97 - 0.87 (m, 3H); MS (ESI): m/z 493.1 (M+H)⁺.

### Example 185:

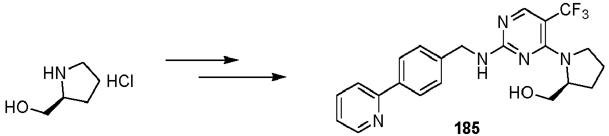

Compound **185** was obtained following the synthesis of compound **24** starting from (S)-pyrrolidin-2-ylmethanol hydrochloride. ¹H NMR (500 MHz, DMSO-*d*₆) δ 8.68 - 8.12 (m, 2H), 8.06 - 7.64 (m, 5H), 7.48 - 7.26 (m, 3H), 4.71 - 4.37 (m, 4H), 3.56 - 3.35 (m, 4H), 2.00 - 1.70 (m, 4H); MS (ESI): m/z 430.3 (M+H)⁺.

### Example 186:

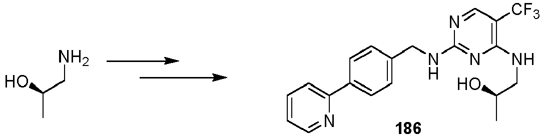

Starting from (R)-1-aminopropan-2-ol, compound **186** was obtained by referring to the synthesis of compound **24.** ¹H NMR (500 MHz, DMSO-*d*₆) δ 8.66 - 8.63 (m, 1H), 8.11 - 7.96 (m, 4H), 7.94 - 7.90 (m, 1H), 7.88 - 7.84 (m, 1H), 7.44 - 7.37 (m, 2H), 7.35 - 7.31 (m, 1H), 6.54 - 6.33 (m, 1H), 4.85 - 4.68 (m, 1H), 4.56 - 4.45 (m, 2H), 3.90 - 3.70 (m, 1H), 3.47 - 3.35 (m, 1H), 3.25 - 3.14 (m, 1H), 1.10 - 0.92 (m, 3H); MS (ESI): m/z 403.8 (M+H)⁺.

### Example 187:

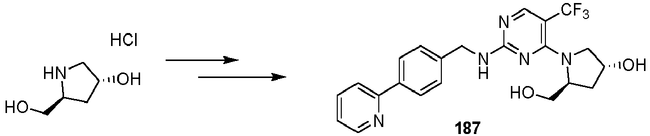

Compound **187** was obtained by referring to the synthesis of compound **24** starting from (3R,5S)-5-(hydroxymethyl)pyrrolidin-3-ol hydrochloride. ¹¹H NMR (500 MHz, DMSO-*d*₆) δ 8.70 - 8.63 (m, 1H), 8.24 - 8.15 (m, 1H), 8.11 - 7.90 (m, 4H), 7.89 - 7.84 (m, 1H), 7.43 (d, *J* = 8.2 Hz, 2H), 7.36 - 7.31 (m, 1H), 4.95 - 4.84 (m, 1H), 4.64 - 4.44 (m, 4H), 4.35 - 4.26 (m, 1H), 3.70 - 3.42 (m, 3H), 3.31 - 3.26 (m, 1H), 2.07 - 1.91 (m, 2H); MS (ESI): m/z 446.2 (M+H)⁺.

### Example 188:

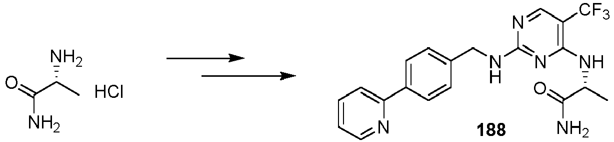

Compound **188** was obtained by referring to the synthesis of compound **24** starting from R-2-aminopropionamide hydrochloride. ¹H NMR (500 MHz, DMSO-*d*₆) δ 8.69 - 8.60 (m, 1H), 8.17 - 7.81 (m, 6H), 7.68 - 7.24 (m, 5H), 6.47 - 6.33 (m, 1H), 4.66 - 4.42 (m, 3H), 1.41 - 1.25 (m, 3H); MS (ESI): m/z 417.2 (M+H)⁺.

### Example 189:

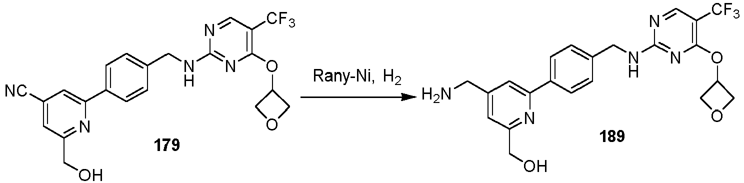

Compound 179 (10 mg, 21.9 µmol) was dissolved in methanol (2 mL), and concentrated ammonia and Raney nickel (1 mg) were added. The mixture was stirred at room temperature under a hydrogen atmosphere for 3 hours. The mixture was filtered through diatomaceous earth and concentrated, and the residue was purified by preparative liquid chromatography to obtain compound **189** (3.0 mg, 6.5 µmol) with a yield of 29.7%. ¹H NMR (500 MHz, DMSO-*d*₆) δ 8.72 - 8.39 (m, 2H), 8.12 - 8.00 (m, 2H), 7.85 - 7.63 (m, 1H), 7.46 - 7.38 (m, 3H), 5.67 - 5.52 (m, 1H), 5.50 - 5.38 (m, 1H), 5.35 - 4.86 (m, 1H), 4.76 - 4.56 (m, 5H), 4.55 - 4.20 (m, 3H), 3.89 - 3.78 (m, 2H): m/z 462.4 (M+H)⁺.

### Example 190:

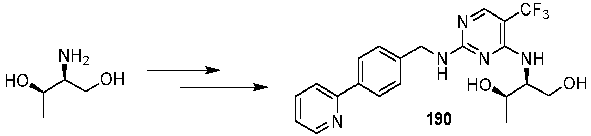

Compound **190** was obtained by referring to the synthesis of compound **24** starting from D-berethreoninol. ¹H NMR (500 MHz, DMSO-*d*₆) δ 8.67 - 8.63 (m, 1H), 8.12 - 8.00 (m, 4H), 7.94 - 7.90 (m, 1H), 7.88 - 7.84 (m, 1H), 7.49 - 7.38 (m, 2H), 7.36 - 7.31 (m, 1H), 5.67 - 5.31 (m, 1H), 5.02 - 4.91 (m, 1H), 4.81 - 4.72 (m, 1H), 4.59 - 4.46 (m, 2H), 4.11 - 4.00 (m, 2H), 3.50 - 3.42 (m, 2H), 1.07 - 0.93 (m, 3H); MS (ESI): m/z 434.4 (M+H)⁺.

### Example 191:

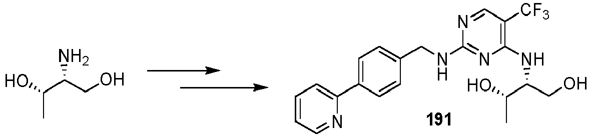

Compound **191** was obtained by referring to the synthesis of compound **24** starting from L-berethreoninol. ¹H NMR (500 MHz, DMSO-*d*₆) δ 8.72 - 8.56 (m, 1H), 8.10 - 7.99 (m, 4H), 7.95 - 7.91 (m, 1H), 7.89 - 7.84 (m, 1H), 7.44 - 7.38 (m, 2H), 7.35 - 7.31 (m, 1H), 5.93 - 5.77 (m, 1H), 4.82 - 4.42 (m, 4H), 4.20 - 4.06 (m, 1H), 3.83 - 3.65 (m, 2H), 3.63 - 3.48 (m, 1H), 1.10 - 0.96 (m, 3H); MS (ESI): m/z 434.4 (M+H)⁺.

### Example 192:

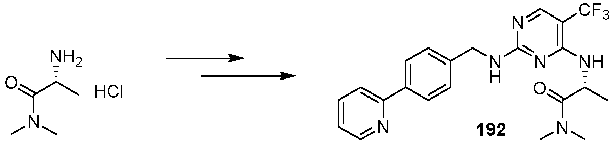

Compound **192** was obtained by referring to the synthesis of compound **24** starting from (R)-2-amino-N,N-dimethylpropionamide hydrochloride. ¹H NMR (500 MHz, DMSO-*d*₆) δ 8.72 - 8.56 (m, 1H), 8.14 - 7.83 (m, 6H), 7.49 - 7.26 (m, 3H), 6.51 - 6.35 (m, 1H), 5.07 - 4.77 (m, 1H), 4.59 - 4.46 (m, 2H), 3.12 - 2.90 (m, 3H), 2.90 - 2.73 (m, 3H), 1.34 - 1.14 (m, 3H); MS (ESI): m/z 445.3 (M+H)⁺.

### Example 192:

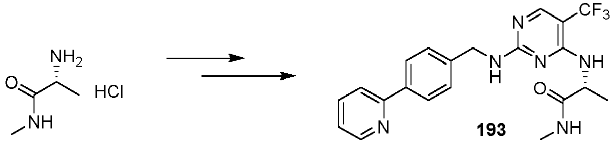

Starting from (R)-2-amino-N-methylpropionamide, compound **193** was obtained by referring to the synthesis of compound **24.** ¹H NMR (500 MHz, DMSO-*d*₆) δ 8.74 - 8.59 (m, 1H), 8.14 - 8.00 (m, 5H), 7.94 - 7.84 (m, 2H), 7.46 - 7.31 (m, 3H), 6.45 - 6.30 (m, 1H), 4.61 - 4.46 (m, 3H), 2.64 - 2.57 (m, 3H), 1.36 - 1.20 (m, 3H); MS (ESI): m/z 431.3 (M+H)⁺.

### Example 194:

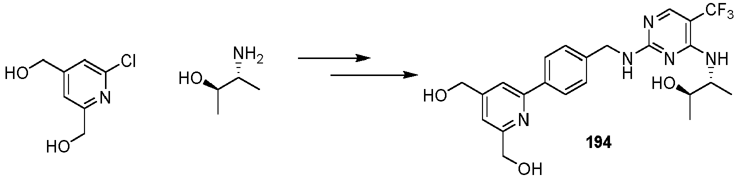

Compound **194** was obtained by referring to the synthesis of compound **160** starting from (6-chloropyridin-2,4-diyl)dimethanol and (2R,3R)-3-aminobutan-2-ol. ¹H NMR (500 MHz, DMSO-*d*₆) δ 8.11 - 7.96 (m, 4H), 7.73 - 7.66 (m, 1H), 7.45 - 7.36 (m, 3H), 5.79 - 5.66 (m, 1H), 5.51 - 5.42 (m, 2H), 5.08 - 4.98 (m, 1H), 4.66 - 4.59 (m, 4H), 4.59 - 4.40 (m, 2H), 4.17 - 4.00 (m, 1H), 3.80 - 3.64 (m, 1H), 1.10 - 0.92 (m, 6H); MS (ESI): m/z 478.4 (M+H)⁺.

### Example 195:

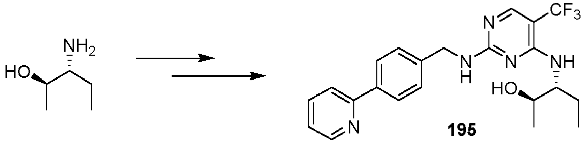

Starting from (2R,3R)-3-aminopentan-2-ol, compound **195** was obtained by referring to the synthesis of compound **24.** ¹H NMR (500 MHz, DMSO-*d*₆) δ 8.67 - 8.63 (m, 1H), 8.10 - 7.99 (m, 4H), 7.91 (d, *J* = 7.9 Hz, 1H), 7.89 - 7.83 (m, 1H), 7.40 (d, *J* = 8.1 Hz, 2H), 7.36 - 7.31 (m, 1H), 5.69 - 5.59 (m, 1H), 4.99 - 4.89 (m, 1H), 4.56 - 4.41 (m, 2H), 4.05 - 3.91 (m, 1H), 3.87 - 3.73 (m, 1H), 1.54 - 1.38 (m, 2H), 0.93 - 0.70 (m, 6H); MS (ESI): m/z 432.3 (M+H)⁺.

### Example 196:

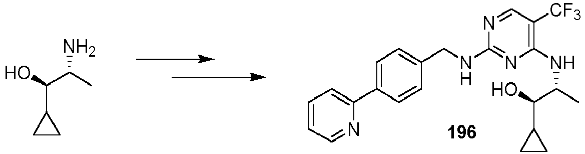

Starting from (1R,2R)-2-amino-1-cyclopropylpropan-1-ol, compound **196** was obtained by referring to the synthesis of compound **24.** ¹H NMR (500 MHz, DMSO-*d*₆) δ 8.72 - 8.61 (m, 1H), 8.14 - 7.83 (m, 6H), 7.48 - 7.28 (m, 3H), 5.81 - 5.70 (m, 1H), 5.19 - 5.02 (m, 1H), 4.60 - 4.45 (m, 2H), 4.35 - 4.20 (m, 1H), 2.98 - 2.85 (m, 1H), 1.22 - 1.07 (m, 3H), 0.87 - 0.65 (m, 1H), 0.44 - 0.04 (m, 4H); MS (ESI): m/z 444.2 (M+H)⁺.

### Example 197:

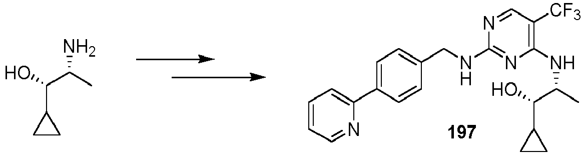

Starting from (1S,2R)-2-amino-1-cyclopropylpropan-1-ol, compound **197** was obtained by referring to the synthesis of compound **24.** ¹H NMR (500 MHz, DMSO-*d*₆) δ 8.72 - 8.59 (m, 1H), 8.12 - 7.83 (m, 6H), 7.55 - 7.30 (m, 3H), 5.82 - 5.71 (m, 1H), 5.19 - 5.00 (m, 1H), 4.70 - 4.46 (m, 2H), 4.37 - 4.18 (m, 1H), 2.97 - 2.85 (m, 1H), 1.20 - 1.03 (m, 3H), 0.90 - 0.67 (m, 1H), 0.47 - 0.08 (m, 4H); MS (ESI): m/z 444.2 (M+H)⁺.

### Example 198:

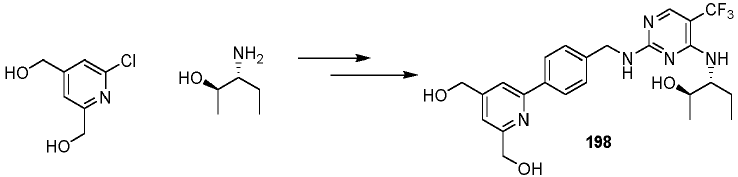

Compound **198** was obtained by referring to the synthesis of compound **160** starting from (6-chloropyridin-2,4-diyl)dimethanol and (2R,3R)-3-aminopentan-2-ol. ¹H NMR (500 MHz, DMSO-*d*₆) δ 8.12 - 7.96 (m, 4H), 7.67 (s, 1H), 7.43 - 7.36 (m, 3H), 5.67 - 5.59 (m, 1H), 5.47 - 5.43 (m, 1H), 5.43 - 5.38 (m, 1H), 5.00 - 4.87 (m, 1H), 4.63 - 4.59 (m, 4H), 4.55 - 4.41 (m, 2H), 4.06 - 3.90 (m, 1H), 3.90 - 3.72 (m, 1H), 1.52 - 1.42 (m, 2H), 0.90 - 0.71 (m, 6H); MS (ESI): m/z 492.4 (M+H)⁺.

### Example 199:

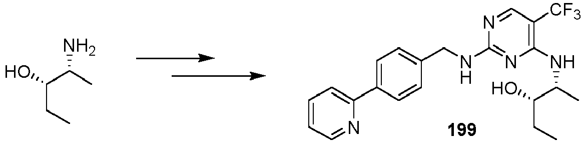

Starting from (2R,3S)-2-aminopentan-3-ol, compound **199** was obtained by referring to the synthesis of compound **24.** ¹H NMR (500 MHz, DMSO-*d*₆) δ 8.76 - 8.60 (m, 1H), 8.13 - 7.97 (m, 4H), 7.93 - 7.89 (m, 1H), 7.88 - 7.83 (m, 1H), 7.47 - 7.28 (m, 3H), 6.03 - 5.88 (m, 1H), 4.98 - 4.78 (m, 1H), 4.62 - 4.46 (m, 2H), 4.31 - 4.10 (m, 1H), 3.52 - 3.36 (m, 1H), 1.45 - 1.21 (m, 2H), 1.12 - 0.70 (m, 6H); MS (ESI): m/z 432.2 (M+H)⁺.

### Example 200:

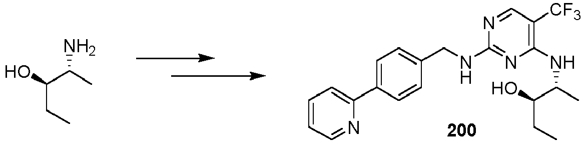

Starting from (2R,3R)-2-aminopentan-3-ol, compound **200** was obtained by referring to the synthesis of compound **24.** ¹H NMR (500 MHz, DMSO-*d*₆) δ 8.69 - 8.62 (m, 1H), 8.14 - 7.98 (m, 4H), 7.96 - 7.89 (m, 1H), 7.89 - 7.82 (m, 1H), 7.46 - 7.36 (m, 2H), 7.36 - 7.30 (m, 1H), 5.74 - 5.62 (m, 1H), 5.15 - 4.99 (m, 1H), 4.60 - 4.42 (m, 2H), 4.27 - 4.09 (m, 1H), 3.45 - 3.35 (m, 1H), 1.43 - 1.13 (m, 3H), 1.11 - 1.00 (m, 2H), 0.92 - 0.65 (m, 3H); MS (ESI): m/z 432.2 (M+H)⁺.

### Example 201:

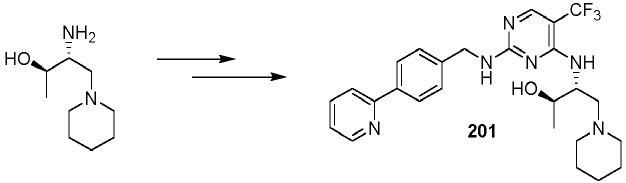

Starting from (2R,3R)-3-amino-4-(piperidin-1-yl)butan-2-ol, compound **201** was obtained by referring to the synthesis of compound **24.** ¹H NMR (500 MHz, DMSO-*d*₆) δ 8.58 (d, *J* = 4.5 Hz, 1H), 8.08 - 7.93 (m, 4H), 7.88 - 7.83 (m, 1H), 7.83 - 7.68 (m, 1H), 7.35 - 7.24 (m, 3H), 5.56 (d, *J* = 8.0 Hz, 1H), 5.18 - 4.92 (m, 1H), 4.54 - 4.37 (m, 2H), 4.12 - 3.86 (m, 2H), 2.34 - 1.98 (m, 6H), 1.31 - 1.21 (m, 4H), 1.21 - 1.16 (m, 2H), 1.03 - 0.94 (m, 1H), 0.86 - 0.82 (m, 2H); MS (ESI): m/z 501.6 (M+H)⁺.

### Example 202:

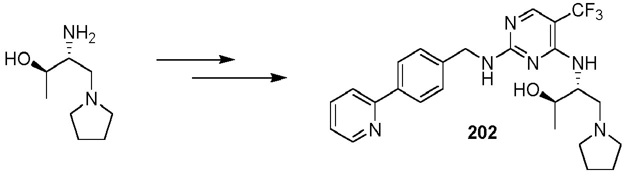

Starting from (2R,3R)-3-amino-4-(pyrrolidin-1-yl)butan-2-ol, compound **202** was obtained by referring to the synthesis of compound **24.** ¹H NMR (500 MHz, DMSO-*d*₆) δ 8.64 (s, 1H), 8.16 - 7.98 (m, 4H), 7.95 - 7.83 (m, 2H), 7.46 - 7.28 (m, 3H), 5.77 - 5.52 (m, 1H), 5.35 - 5.00 (m, 1H), 4.63 - 4.41 (m, 2H), 4.19 - 3.93 (m, 2H), 3.15 (d, J = 9.5 Hz, 2H), 2.32 - 2.19 (m, 4H), 1.71 - 1.51 (m, 4H), 1.34 - 1.27 (m, 3H); MS (ESI): m/z 487.2 (M+H)⁺.

### Example 203:

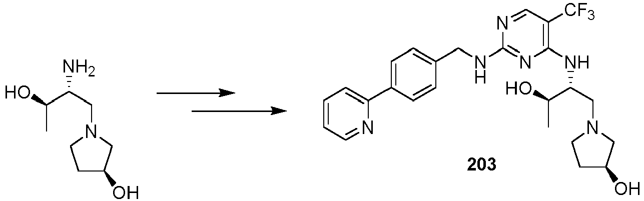

Starting from (2R,3R)-3-amino-4-((R)-3-hydroxypyrrolidin-1-yl)butan-2-ol, compound **203** was obtained by referring to the synthesis of compound **24.** ¹H NMR (500 MHz, DMSO-*d*₆) δ 8.66 - 8.53 (m, 1H), 8.13 - 7.73 (m, 6H), 7.39 - 7.23 (m, 3H), 5.69 - 5.57 (m, 1H), 5.21 - 4.80 (m, 2H), 4.50 - 4.07 (m, 5H), 3.96 - 3.81 (m, 2H), 3.12 - 3.03 (m, 4H), 1.25 - 1.16 (m, 5H); MS (ESI): m/z 503.3 (M+H)⁺.

### Example 204:

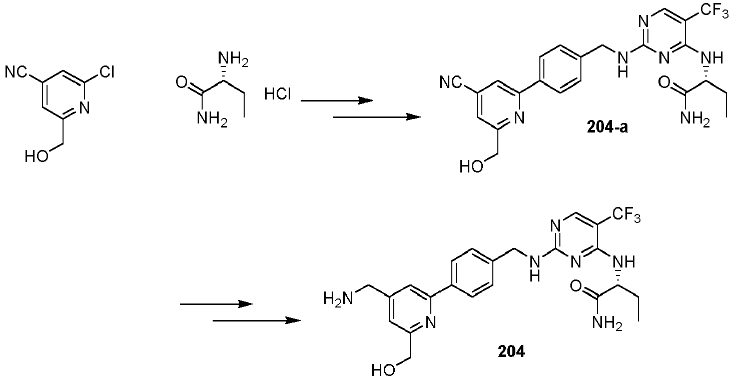

Compound **204-a** was obtained by referring to the synthesis of compound **160** starting from (2-chloro-6-(hydroxymethyl)isonicotinonitrile and R-2-aminobutanamide hydrochloride. Starting from compound **204-a,** compound **204** was obtained by referring to the synthesis of compound **189.** ¹H NMR (500 MHz, DMSO-*d*₆) δ 8.17 - 7.98 (m, 4H), 7.78 (s, 1H), 7.62 (s, 1H), 7.51 - 7.37 (m, 3H), 7.36 - 7.21 (m, 1H), 6.31 - 6.21 (m, 1H), 4.65 - 4.60 (m, 2H), 4.60 - 4.54 (m, 2H), 4.54 - 4.45 (m, 1H), 3.95 - 3.87 (m, 2H), 1.86 - 1.62 (m, 2H), 0.84 - 0.64 (m, 3H); MS (ESI): m/z 490.7 (M+H)⁺.

### Example 205:

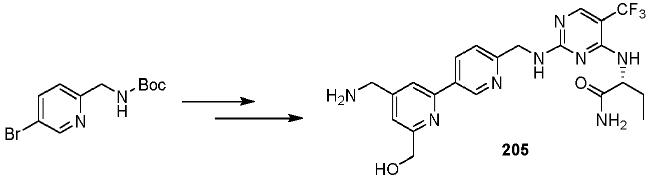

Compound **205** was obtained by referring to the synthesis of compound **204** starting from tert-butyl ((5-bromopyridin-2-yl)methyl)carbamate. ¹H NMR (500 MHz, DMSO-*d*₆) δ 9.21 - 9.12 (m, 1H), 8.39 - 8.31 (m, 1H), 8.12 - 8.09 (m, 1H), 8.09 - 7.77 (m, 2H), 7.68 - 7.58 (m, 1H), 7.46 (s, 1H), 7.41 - 7.38 (m, 1H), 7.31 - 7.22 (m, 1H), 6.32 - 6.21 (m, 1H), 5.45 - 5.38 (m, 1H), 4.74 - 4.53 (m, 5H), 3.84 (s, 2H), 2.06 - 1.95 (m, 2H), 0.82 - 0.57 (m, 3H); MS (ESI): m/z 491.8 (M+H)⁺.

### Example 206:

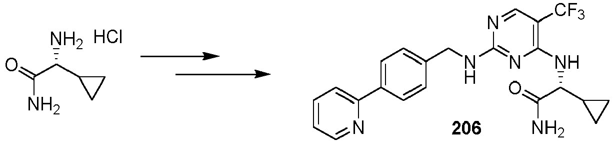

Compound **206** was obtained by referring to the synthesis of compound **24** starting from (R)-2-amino-2-cyclopropylacetamide hydrochloride. ¹H NMR (500 MHz, DMSO-*d*₆) δ 8.69 - 8.61 (m, 1H), 8.16 - 7.78 (m, 6H), 7.63 - 7.24 (m, 5H), 6.28 - 6.19 (m, 1H), 4.66 - 4.43 (m, 2H), 4.28 - 4.17 (m, 1H), 1.22 - 1.13 (m, 1H), 0.53 - 0.16 (m, 4H); MS (ESI): m/z 443.7 (M+H)⁺.

### Example 207:

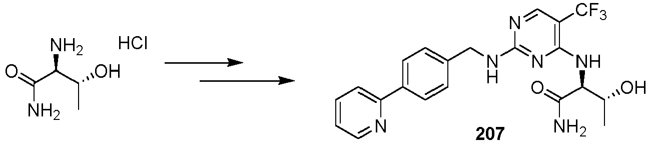

Compound **207** was obtained by referring to the synthesis of compound **24** starting from (2S,3R)-2-amino-3-hydroxybutanamide hydrochloride. ¹H NMR (500 MHz, DMSO-*d*₆) δ 8.72 - 8.60 (m, 1H), 8.18 - 7.80 (m, 6H), 7.52 - 7.13 (m, 5H), 6.17 - 5.97 (m, 1H), 5.28 - 5.12 (m, 1H), 4.62 - 4.40 (m, 3H), 4.28 - 4.10 (m, 1H), 1.10 - 0.94 (m, 3H); MS (ESI): m/z 447.7 (M+H)⁺.

### Example 208:

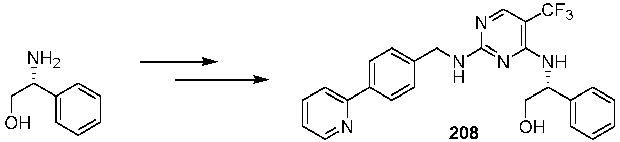

Starting from (R)-2-amino-2-phenylethan-1-ol, compound **208** was obtained by referring to the synthesis of compound **24.** ¹H NMR (500 MHz, DMSO-*d*₆) δ 8.68 - 8.62 (m, 1H), 8.13 - 8.03 (m, 1H), 8.02 - 7.71 (m, 5H), 7.39 - 7.14 (m, 8H), 6.63 - 6.51 (m, 1H), 5.38 - 5.03 (m, 2H), 4.61 - 4.24 (m, 2H), 3.85 - 3.65 (m, 2H); MS (ESI): m/z 466.7 (M+H)⁺.

### Example 209:

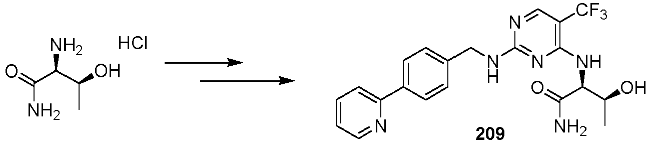

Compound **209** was obtained by referring to the synthesis of compound **24** starting from (2S,3S)-2-amino-3-hydroxybutanamide hydrochloride. ¹H NMR (500 MHz, DMSO-*d*₆) δ 8.70 - 8.61 (m, 1H), 8.18 - 7.80 (m, 6H), 7.52 - 7.21 (m, 5H), 6.26 - 6.13 (m, 1H), 5.11 - 4.98 (m, 1H), 4.66 - 4.43 (m, 3H), 4.11 - 3.90 (m, 1H), 1.12 - 1.04 (m, 3H); MS (ESI): m/z 447.6 (M+H)⁺.

### Example 210:

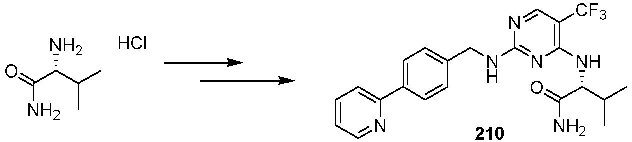

Compound **210** was obtained by referring to the synthesis of compound **24** starting from (R)-2-amino-3-methylbutanamide hydrochloride. ¹H NMR (500 MHz, DMSO-*d*₆) δ 8.68 - 8.61 (m, 1H), 8.18 - 7.96 (m, 4H), 7.94 - 7.90 (m, 1H), 7.86 (td, *J* = 7.7, 1.9 Hz, 1H), 7.61 (s, 1H), 7.49 - 7.37 (m, 2H), 7.35 - 7.27 (m, 2H), 6.10 - 5.90 (m, 1H), 4.70 - 4.57 (m, 2H), 4.54 - 4.40 (m, 1H), 2.11 - 1.92 (m, 1H), 0.92 - 0.76 (m, 6H); MS (ESI): m/z 445.6 (M+H)⁺.

### Example 211:

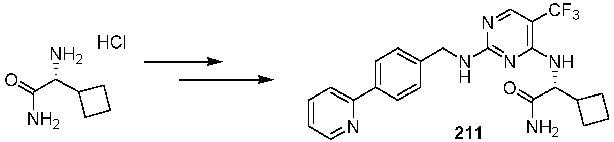

Compound **211** was obtained by referring to the synthesis of compound **24** starting from (R)-2-amino-2-cyclobutylacetamide hydrochloride. ¹H NMR (500 MHz, DMSO-*d*₆) δ 8.64 (dd, *J* = 5.0, 1.6 Hz, 1H), 8.14 - 7.74 (m, 6H), 7.55 (s, 1H), 7.50 - 7.36 (m, 2H), 7.36 - 7.29 (m, 1H), 7.27 - 7.17 (m, 1H), 6.14 - 6.02 (m, 1H), 4.70 - 4.42 (m, 3H), 2.67 - 2.58 (m, 1H), 1.89 - 1.50 (m, 6H); MS (ESI): m/z 457.6 (M+H)⁺.

### Example 212:

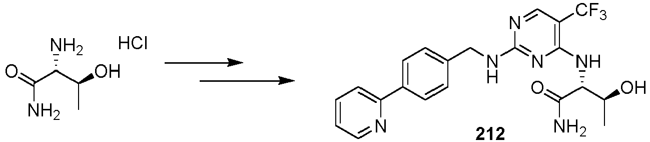

Starting from (2R,3S)-2-amino-3-hydroxybutanamide hydrochloride, compound **212** was obtained by referring to the synthesis of compound **24.** ¹H NMR (500 MHz, DMSO-*d*₆) δ 8.64 (d, *J* = 4.7 Hz, 1H), 8.22 - 7.98 (m, 4H), 7.94 - 7.89 (m, 1H), 7.89 - 7.83 (m, 1H), 7.49 - 7.29 (m, 4H), 7.19 (s, 1H), 6.10 - 6.01 (m, 1H), 5.22 - 5.16 (m, 1H), 4.57 - 4.43 (m, 3H), 4.26 - 4.12 (m, 1H), 1.09 - 0.98 (m, 3H); MS (ESI): m/z 447.6 (M+H)⁺.

### Example 213:

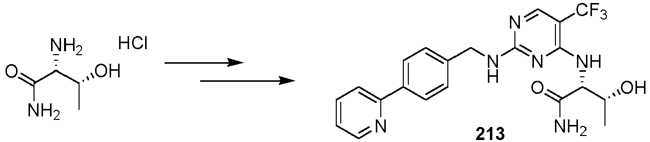

Compound **213** was obtained by referring to the synthesis of compound **24** starting from (2R,3R)-2-amino-3-hydroxybutanamide hydrochloride. ¹H NMR (500 MHz, DMSO-*d*₆) δ 8.68 - 8.60 (m, 1H), 8.26 - 7.98 (m, 4H), 7.95 - 7.89 (m, 1H), 7.89 - 7.79 (m, 1H), 7.51 - 7.30 (m, 4H), 7.25 - 7.13 (m, 1H), 6.11 - 5.99 (m, 1H), 5.20 (s, 1H), 4.52 - 4.43 (m, 3H), 4.26 - 4.13 (m, 1H), 1.09 - 0.96 (m, 3H); MS (ESI): m/z 447.6 (M+H)⁺.

### Example 214:

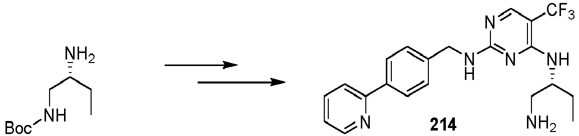

Starting from tert-butyl (R)-(2-aminobutyl)carbamate, compound **214** was obtained by referring to the synthesis of compound **101.** ¹H NMR (500 MHz, DMSO-*d*₆) δ 8.67 - 8.59 (m, 1H), 8.11 - 7.94 (m, 4H), 7.93 - 7.89 (m, 1H), 7.88 - 7.83 (m, 1H), 7.45 - 7.37 (m, 2H), 7.35 - 7.30 (m, 1H), 6.32 - 6.20 (m, 1H), 4.61 - 4.41 (m, 2H), 4.19 - 4.09 (m, 1H), 2.89 - 2.63 (m, 2H), 1.60 - 1.40 (m, 2H), 0.87 - 0.65 (m, 3H); MS (ESI): m/z 417.6 (M+H)⁺.

### Test example

### Biological activity detection of HGC27 (human gastric cancer cell) cell growth inhibition

This detection method is used to evaluate the cellular level biological activity of the compounds of the present invention.

Harvest HGC27 cells (purchased from Chinese Academy of Sciences), resuspend in complete culture medium and adjust the cell density to 0.2 x 10⁶ cells per ml. Add 100 µL of cell suspension to each well into a 96-well plate and incubate overnight in a 37°C, 5% CO₂ incubator. Prepare the compound to be tested and add it to the cell plate so that the maximum concentration of the compound is 10 µM. Set 8 concentration points according to 3-fold dilution. At the same time, set up a 100% inhibition control well, that is, a well with no cells added and only an equal volume of complete culture medium; and a 0% inhibition control well, that is, a cell well with 0.1% DMSO added. The above cell plate was cultured at 37°C and 5% CO₂ for 24 hours. Take out the cell culture plate, add 25 µl CellTiter-Glo^{®} Luminescent Cell Viability reagent (promega cat#G7573) to each well, incubate in the dark for 10 minutes, transfer 100 µL to a white plate, and use Molecular Devices SpectraMax i3 to detect chemiluminescence. Data processing uses the following formula to calculate the inhibition rate: compound inhibition rate = (0% inhibition of control well signal - compound treatment well signal) / (0% inhibition of control well signal - 100% inhibition of control well signal) * 100%. The calculated data were fitted using graphpad prism software with four parameters and the corresponding IC₅₀ was calculated.

According to the above detection method, the biological activity of the compound of the present invention was evaluated at the cellular level. The activity results are shown in the table below. Wherein, CR8 is a literature compound, which was purchased from Shanghai Bide Pharmaceutical. The specific structure is as follows:

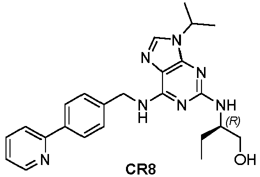

| Compound number | HGC27 CTG IC₅₀ (nM) | Compound number | HGC27 CTG IC₅₀ (nM) |
|---|---|---|---|
| **1** | 385 | **161** | 193 |
| **13** | 307 | **163** | 393 |
| **18** | 281 | **164** | 386 |
| **21** | 155 | **165** | 89 |
| **25** | 158 | **166** | 24 |
| **35** | 379 | **167** | 49 |
| **40** | 341 | **168** | 60 |
| **43** | 266 | **169** | 53 |
| **64** | 251 | **170** | 48 |
| **69** | 185 | **171** | 25 |
| **70** | 160 | **174** | 345 |
| **85** | 405 | **175** | 406 |
| **91** | 486 | **176** | 171 |
| **104** | 275 | **177** | 193 |
| **105** | 210 | **179** | 108 |
| **114** | 251 | **181** | 63 |
| **119** | 479 | **182** | 68 |
| **125** | 191 | **183** | 423 |
| **126** | 170 | **188** | 258 |
| **127** | 446 | **192** | 25 |
| **129** | 77 | **193** | 39 |
| **131** | 100 | **194** | 278 |
| **134** | 370 | **195** | 25 |
| **135** | 427 | **198** | 439 |
| **138** | 254 | **199** | 205 |
| **146** | 281 | **202** | 367 |
| **147** | 410 | **206** | 65 |
| **149** | 238 | **207** | 103 |
| **151** | 197 | **210** | 34 |
| **152** | 202 | **211** | 110 |
| **156** | 169 | **212** | 187 |
| **158** | 49 | **213** | 123 |
| **159** | 45 | **214** | 292 |
| **160** | 48 | **CR8** | 429 |

From the above results, it can be seen that the compound of the present invention has good tumor cell growth inhibitory activity.

### Western blot detection of Cyclin K (CCNK) degradation

Harvest HEK293 cells (ATCC, cat #CRL-1573) and adjust the cell density to 1 × 10⁶ cells per ml. Pour 1 ml of cell suspension per well into a 6-well plate and **culture overnight.** Take the compound stock solution and dilute it to the appropriate concentration with DMSO. Add the diluted compound into the cell wells using culture medium at a ratio of 1:1000 to ensure that the DMSO concentration in each well is 0.1%. At the same time, negative control wells were set, that is, complete culture medium containing 0.1% DMSO. After the compounds were treated and fixed at the concentrations shown in the figure and for different times, total cell protein was extracted with RIPA cell lysis buffer (Beyotime, cat#P0013B) added with PMSF. After protein quantification using the BCA protein quantification kit (Thermo Fisher, cat#A53225), each sample was subjected to subsequent SDS-PAGE and western blot experiments at a loading volume of 40 µg. The specific conditions are: after running the gel at 120V constant pressure for 90 minutes, transfer the film at a constant current of 320 mA for 60 minutes. Carry out antibody incubation according to the recommended dilution ratio and incubation time of the antibody. The information of the antibodies used in the experiment is as follows: Anti-GAPDH antibody (abcam, cat#ab9485), Anti-Cyclin K antibody (abcam, cat#ab85854), Goat anti-Rabbit IgG (H+L) Cross-Adsorbed Secondary Antibody, HRP (invitrogen, cat #G-21234). ECL luminescence solution (Thermo Fisher, cat#32209) was used to develop color and luminescence. A gel imaging system was used to analyze the final results.

According to the above detection method, some compounds of the present invention were evaluated.

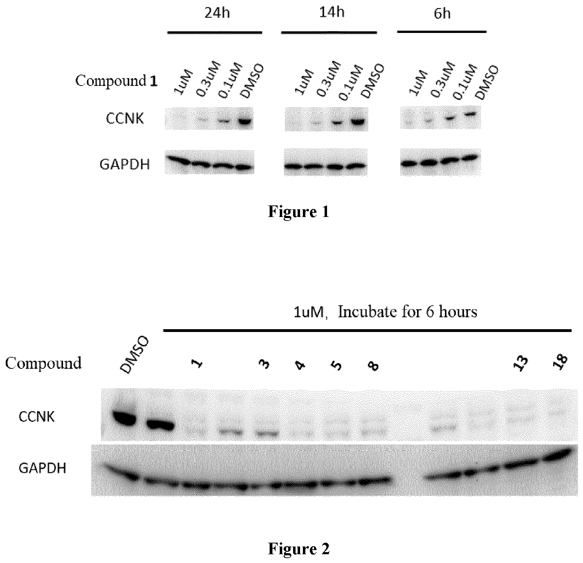

Figure 1 shows that compound 1 can significantly induce the degradation of Cyclin K in a strong time and dose dependence. The details are as follows. After incubation of compound 1 with HEK293 cells for 6 h, 14 h and 24 h, the degradation of Cyclin K was demonstrated compared with the control. Wherein, high concentration (1 µM) had the best effect, followed by medium concentration (0.3 µM), and finally low concentration (0.1 µM). At the same time, the degradation effect of compounds of the same concentration becomes better as the incubation time is extended. For example, the degradation effect of Cyclin K after treatment with low concentration (0.1 µM) compound 1 for 24 h was better than the degradation effect of Cyclin K after treatment with the same concentration for 14 h and 6 h.

Figure 2 shows that other compounds of the present invention, such as compounds 3, 4, 5, 8, 13, and 18, can significantly induce the degradation of Cyclin K compared with the control after treating HEK293 cells at 1 µM for 6 hours.

It can be seen from the above results that the compounds of the present invention can effectively degrade Cyclin K.

## Claims

1. Compounds with the structure of formula (I) or pharmaceutically acceptable salts, prodrugs, isotope derivatives, isomers, solvates or metabolites thereof: 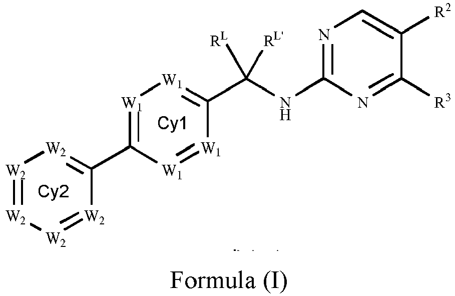
wherein, Cy1 and Cy2 each independently represent a 6-membered aromatic ring or a 6-membered heteroaromatic ring;
wherein, R^{L} and R^{L'} each independently represent hydrogen, C₁-C₆ alkyl or C₃-C₆ cycloalkyl, and R^{L} and R^{L'} can form a 3-6 membered ring together with the carbon atom connected to them;
wherein, Wi each independently represents CR⁰ or N;
wherein, R⁰ each independently represents hydrogen, halogen, nitro, cyano, -R^{a}, -OR^{a}, -SR^{a}, -NR^{a}R^{b}, -C(O)R^{a}, -C(O)OR^{a}, -C(O)NR^{a}R^{b}, -S(O)₂R^{a}, -NR^{a} C(O)R^{b}, -S(O)2NR^{a}, -S(O)R^{a} or -P(O)R^{a}R^{b};
wherein, W₂ each independently represents CR¹ or N;
wherein, R¹ each independently represents hydrogen, halogen, nitro, cyano, -R^{a}, -OR^{a}, -SR^{a}, -NR^{a}R^{b}, -C(O)R^{a}, -C(O)OR^{a}, -C(O)NR^{a}R^{b}, - NR^{a}C(O)R^{b}, -S(O)₂R^{a}, -S(O)R^{a}, -S(O)₂NR^{a}R^{b}, -P(O)R^{a}R^{b}, and C₁-C₆ alkyl, (C₂-C₆) alkenyl, (C₂-C₆) alkynyl optionally substituted by 0, 1, 2, or 3 substituents selected from OR^{a}, SR^{a}, NR^{a}R^{b}, NR^{a} C(O)R^{b}, C(O)R^{a}, C(O)OR^{a}, C(O)NR^{a}R^{b}, S(O)₂R^{a}, S(O)R^{a}, S(O)₂NR^{a}R^{b}, P(O)R^{a}R^{b};
wherein, R² represents halogen, -R^{a}, -OR^{a}, -SR^{a}, nitro, cyano, -NR^{a}R^{b}, -NR^{a} C(O)R^{b}, -C(O)R^{a}, -C(O)OR^{a}, -C(O )NR^{a}R^{b}, -S(O)₂R^{a}, -S(O)R^{a}, -S(O)₂NR^{a}R^{b}, -P(O)R^{a}R^{b}, (C₂-C₆) alkenyl, or (C₂-C₆) alkynyl;
wherein, R3 represents C₁-C₆ alkyl, C₁-C₆ alkenyl, C₁-C₆ alkynyl, C₃-C₁₀ cycloalkyl, 3-10 membered heterocycloalkyl, C₆-C₁₀ membered aryl, 5-10 membered heteroaryl, -NR^{M}R^{N}, -NHR^{M}, -OR^{M};
when R³ represents C₁-C₆ alkyl, C₁-C₆ alkenyl, C₁-C₆ alkynyl, C₃-C₁₀ cycloalkyl, 3-10 membered heterocycloalkyl, it may optionally be substituted by 0, 1, 2, 3 of the following substituents: nitro, halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, -(C₀-C₆ alkylene)P(O)R^{a}R^{b};
when R³ represents a C₆-C₁₀-membered aryl or a 5-10-membered heteroaryl, it may optionally be substituted by 0, 1, 2, or 3 of the following substituents: nitro, halogen, cyano, - R^{a}, - (C₀-C₆ alkylene)OR^{a}, -(C₀-C₆ alkylene)SRa, -(C₀-C₆ alkylene)NR^{a}R^{b}, - (C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, -(C₀-C₆ alkylene)P(O)R^{a}R^{b};
when R³ represents -NR^{M}R^{N}, -NHR^{M}, -OR^{M}, R^{M} and R^{N} each independently represent C₁-C₆ alkyl, -(C₀-C₆ alkylene)(C₃-C₁₀ cycloalkyl), -(C₀-C₆ alkylene) (3-10 membered heterocycloalkyl), -(C₀-C₆ alkylene) (C₆-C₁₀ membered aryl), -(C₀-C₆ alkylene) (5-10 membered heteroaryl);
wherein, R^{M} and R^{N} can optionally be substituted by 0, 1, 2, or 3 of the following substituents: oxo, nitro, halogen, cyano, -R^{a}, -(C₀-C₆ alkylene)OR^{a}, -(C₀-C₆ alkylene)SR^{a}, -(C₀-C₆ alkylene)NR^{a}R^{b}, - (C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, -(C₀-C₆ alkylene)P(O)R^{a}R^{b};
wherein, R^{a} and R^{b} each independently represent hydrogen, C₁-C₆ alkyl or C₃-C₈ cycloalkyl, which may optionally be substituted by 0, 1, 2 or 3 halogen atoms;
provided that the compound of formula (I) does not include

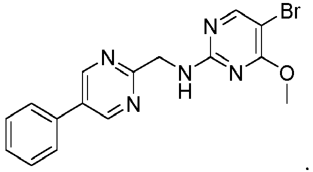

2. The compound according to claim 1 or a pharmaceutically acceptable salt, prodrug, isotope derivative, isomer, solvate or metabolite thereof, wherein R² represents halogen, trifluoromethyl or cyano.

3. The compound according to any one of the preceding claims or a pharmaceutically acceptable salt, prodrug, isotope derivative, isomer, solvate or metabolite thereof, wherein Cy1 is selected from 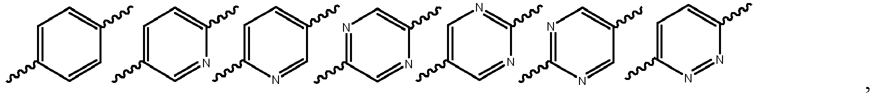 preferably Cy1 is selected from 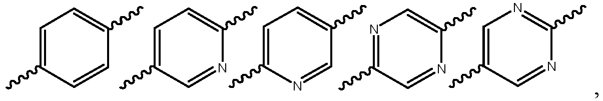 wherein the wavy line indicates the site of Cy1 which is connected to formula (I); wherein, the Cy1 is optionally substituted by 0, 1, 2 or 3 R⁰; preferably the Cy1 is substituted by 0 R⁰.

4. The compound according to any one of the preceding claims or a pharmaceutically acceptable salt, prodrug, isotope derivative, isomer, solvate or metabolite thereof, wherein Cy2 is selected from 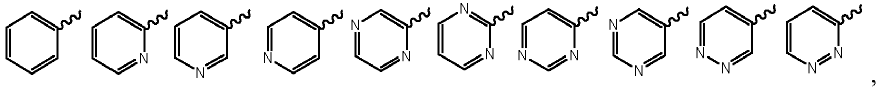 preferably Cy2 is selected from 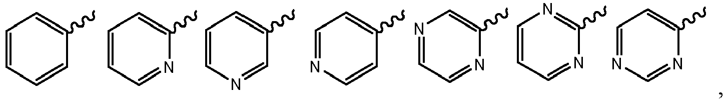 wherein the wavy line indicates that the site of Cy2 which is connected to formula (I); wherein, the Cy2 is optionally substituted by 0, 1, 2 or 3 R¹; preferably, the Cy2 is substituted by 0 R¹; preferably, Cy2 is substituted by 1 or 2 R¹.

5. The compound according to any one of the preceding claims, or a pharmaceutically acceptable salt, prodrug, isotope derivative, isomer, solvate or metabolite thereof, wherein R⁰ each independently represents hydrogen, halogen, -R^{a}, -OR^{a} or -SR^{a}.

6. The compound according to any one of the preceding claims, or a pharmaceutically acceptable salt, prodrug, isotope derivative, isomer, solvate or metabolite thereof, wherein R¹ each independently represents hydrogen, halogen, -R^{a}, -OR^{a}, -NR^{a}R^{b}, -C(O)R^{a}, -C(O)OR^{a}, -C(O)NR^{a}R^{b}, -NR^{a} C(O)R^{b}, -S(O)₂R^{a}, -S(O)R^{a}, -P(O)R^{a}R^{b}, -(C₂-C₆)alkenyl, -(C₂-C₆)alkynyl, -(C₁-C₆ alkylene)OR^{a}, -(C₁-C₆ alkylene)NR^{a}R^{b}, - (C₁-C₆ alkylene)NR^{a}C(O)R^{b}, -(C₁-C₆ alkylene)C(O)R^{a}, -(C₁-C₆ alkylene)C(O)OR^{a}, -(C₁-C₆ alkylene)C(O)NR^{a}R^{b}, -(C₁-C₆ alkylene)S(O)₂R^{a}, -(C₁-C₆ alkylene)S(O)R^{a} or -(C₁-C₆ alkylene)P(O)R^{a}R^{b}.

7. The compound according to any one of the preceding claims, or a pharmaceutically acceptable salt, prodrug, isotope derivative, isomer, solvate or metabolite thereof, wherein R³ represents C₁-C₆ alkyl, C₁-C₆ alkenyl, C₁-C₆ alkynyl, C₃-C₁₀ cycloalkyl or 3-10 membered heterocycloalkyl, which may be optionally substituted by 0, 1, 2 or 3 of the following substituents: nitro, halogen , cyano, -R^{a}, -(C₀-C₆ alkylene)OR^{a}, -(C₀-C₆ alkylene)SR^{a}, -(C₀-C₆ alkylene)NR^{a}R^{b}, -NR^{a}C(O)R^{b}, -C(O)R^{a}, -C(O)OR^{a}, -C(O)NR^{a}R^{b}, -S(O)₂Ra, -S(O)R^{a}, -S(O)₂NR^{a}R^{b}, -P(O)R^{a}R^{b}.

8. The compound according to claim 7 or a pharmaceutically acceptable salt, prodrug, isotope derivative, isomer, solvate or metabolite thereof, wherein R³ represents C₁-C₆ alkyl, C₁-C₆ alkenyl, C₁-C₆ alkynyl, C₃-C₁₀ cycloalkyl or 3-10 membered heterocycloalkyl, which may optionally be substituted by 0, 1, 2, 3 of the following substituents: halogen, -R^{a}, -(C₀-C₆ alkylene)OR^{a} or -(C₀-C₆ alkylene)SR^{a}, -(C₀-C₆ alkylene)NR^{a}R^{b}.

9. The compound according to any one of the preceding claims, or a pharmaceutically acceptable salt, prodrug, isotope derivative, isomer, solvate or metabolite thereof, wherein R³ represents a C₆-C₁₀ aryl or 5-10 membered heteroaryl, which may be optionally substituted by 0, 1, 2, or 3 of the following substituents: nitro, halogen, cyano, - R^{a}, - (C₀-C₆ alkylene)OR^{a}, - (C₀-C₆ alkylene)SR^{a}, -(C₀-C₆ alkylene)NR^{a}R^{b}, - NR^{a}C(O)R^{b}, -C(O)R^{a}, -C(O)OR^{a}, -C(O)NR^{a}R^{b}, -S(O)₂Ra, -S(O)R^{a}, -S(O)₂NR^{a}R^{b} or -P(O)R^{a}R^{b}.

10. The compound according to claim 9 or a pharmaceutically acceptable salt, prodrug, isotope derivative, isomer, solvate or metabolite thereof, wherein R³ is optionally substituted by 0, 1 , 2 or 3 of the following substituents: halogen, -R^{a}, -(C₀-C₆ alkylene)OR^{a}, -(C₀-C₆ alkylene)SR^{a}, -(C₀-C₆ alkylene)NR^{a}R^{b}, -NR^{a}C(O)R^{b}, -C(O)R^{a}, -C(O)OR^{a}, -C(O)NR^{a}R^{b}, -S(O)₂Ra, -S(O)R^{a}, -S(O)₂NR^{a}R^{b} or -P(O)R^{a}R^{b}.

11. The compound according to any one of the preceding claims, or a pharmaceutically acceptable salt, prodrug, isotope derivative, isomer, solvate or metabolite thereof, wherein R³ represents -NR^{M}R^{N}, -NHR^{M}, - OR^{M}, wherein R^{M} and R^{N} each independently represent C₁-C₆ alkyl, -(C₀-C₆ alkylene) (C₃-C₁₀ cycloalkyl), -(C₀-C₆ alkylene) (3-10 membered heterocycloalkyl), -(C₀-C₆ alkylene) (C₆-C₁₀-membered aryl) or -(C₀-C₆ alkylene) (5-10-membered heteroaryl);
wherein, R^{M} and R^{N} can optionally be substituted by 0, 1, 2, or 3 of the following substituents: oxo, nitro, halogen, cyano, -R^{a}, -OR^{a}, -SR^{a}, -NR^{a}R^{b}, -NR^{a}C(O)R^{b}, -C(O)R^{a}, -C(O)OR^{a}, -C(O)NR^{a}R^{b}, -S(O)₂Ra, -S(O)R^{a}, -S(O)₂NR^{a}R^{b}, -P(O)R^{a}R^{b}.

12. The compound according to claim 11 or a pharmaceutically acceptable salt, prodrug, isotope derivative, isomer, solvate or metabolite thereof, wherein R^{M} and R^{N} are optionally substituted by 0, 1 , 2 or 3 of the following substituents: oxo, -R^{a}, -OR^{a}, -SR^{a}, -NR^{a}R^{b}, -NR^{a}C(O)R^{b}, -C(O)R^{a}, -C(O)OR^{a}, -C(O )NR^{a}R^{b}, -S(O)₂Ra, -S(O)R^{a}, -S(O)2NR^{a}R^{b} or -P(O)R^{a}R^{b}.

13. The compound according to any one of the preceding claims, or a pharmaceutically acceptable salt, prodrug, isotope derivative, isomer, solvate or metabolite thereof, wherein R^{a} and R^{b} each independently represent hydrogen, C₁-C₃ alkyl or C₃-C₆ cycloalkyl, which may be optionally substituted by 0, 1, 2, or 3 halogen atoms.

14. The compound according to any one of the preceding claims, or a pharmaceutically acceptable salt, prodrug, isotope derivative, isomer, solvate or metabolite thereof, wherein R^{a} and R^{b} each independently represent hydrogen, C₁-C₃ alkyl, which may be optionally substituted by 0, 1, 2, or 3 halogen atoms.

15. The compound according to any one of the preceding claims, which is selected from:
| | | | |
|---|---|---|---|
| **1** | 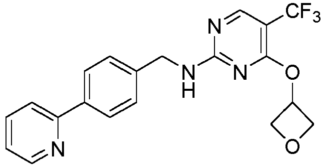 | **2** | 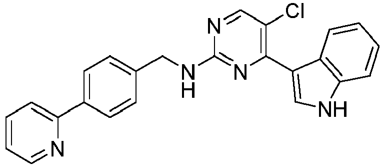 |
| 3 | 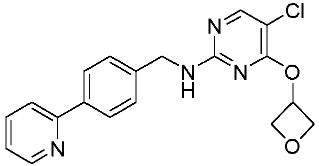 | **4** | 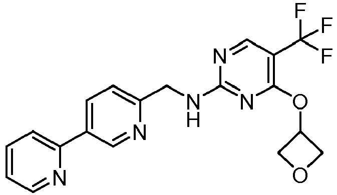 |
| 5 | 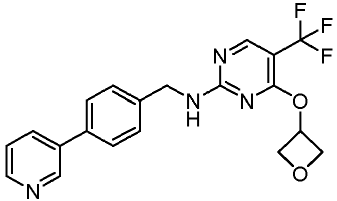 | **6** | 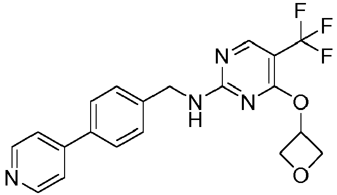 |
| 7 | 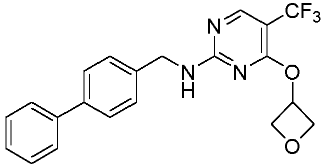 | **8** | 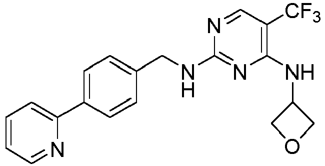 |
| 9 | 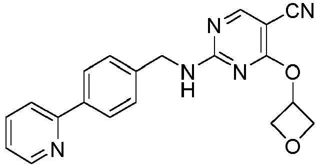 | **10** | 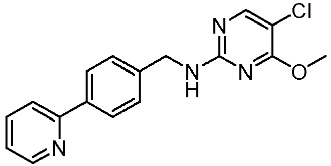 |
| **11** | 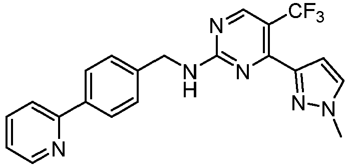 | **12** | 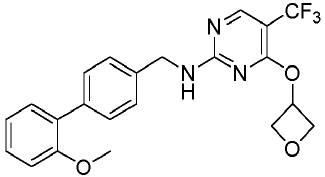 |
| **13** | 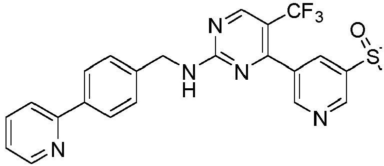 | **14** | 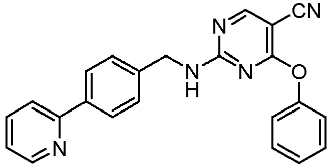 |
| **15** | 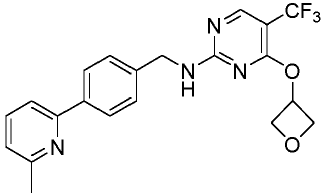 | **16** | 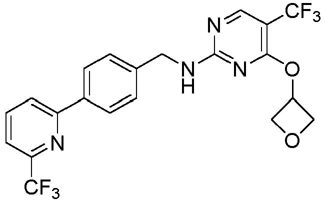 |
| **17** | 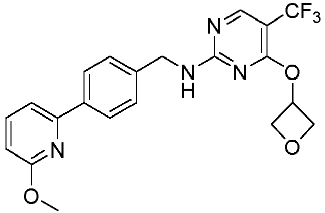 | **18** | 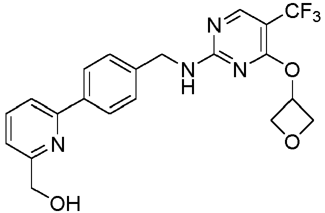 |
| **19** | 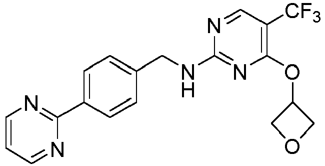 | **20** | 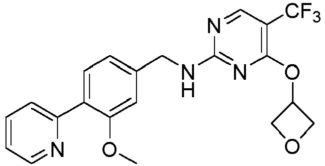 |
| **21** | 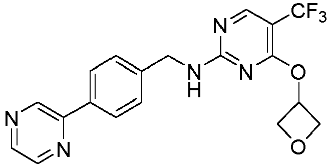 | **22** | 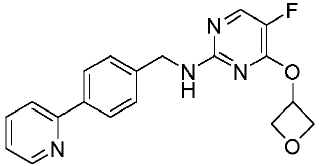 |
| **23** | 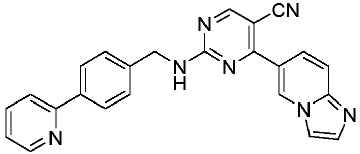 | **24** | 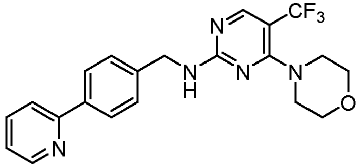 |
| **25** | 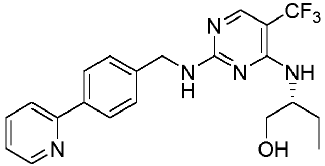 | **26** | 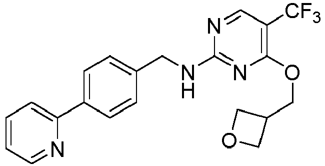 |
| **27** | 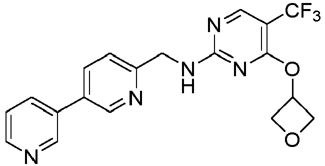 | **28** | 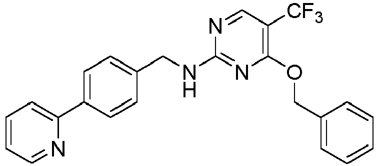 |
| **29** | 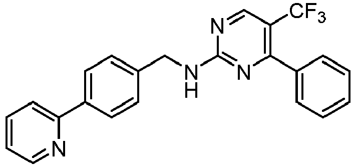 | **30** | 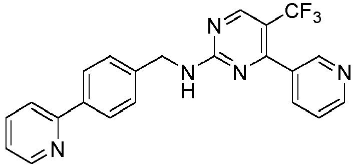 |
| **31** | 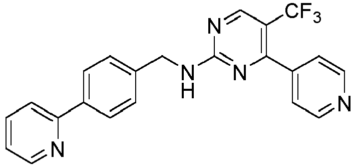 | **32** | 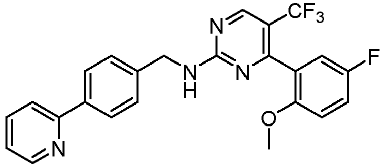 |
| **33** | 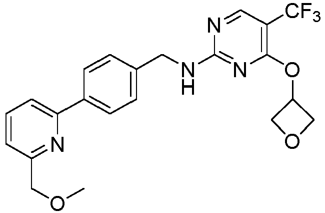 | **34** | 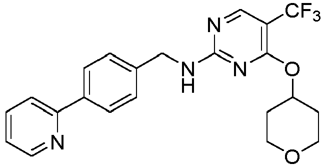 |
| **35** | 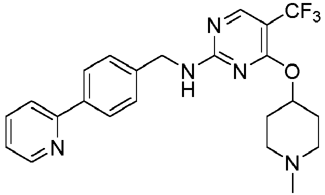 | **36** | 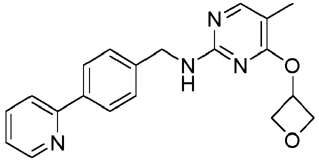 |
| **37** | 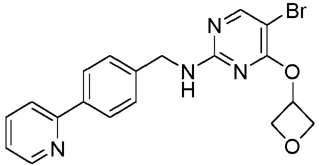 | **38** | 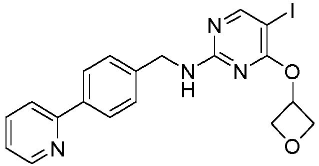 |
| **39** | 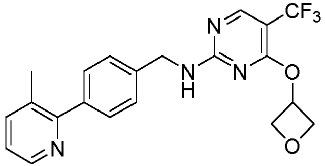 | **40** | 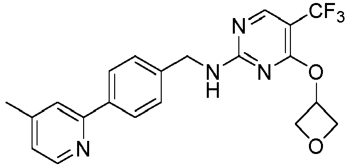 |
| **41** | 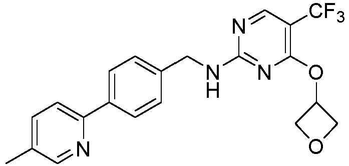 | **42** | 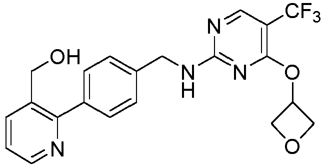 |
| **43** | 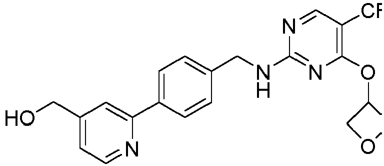 | **44** | 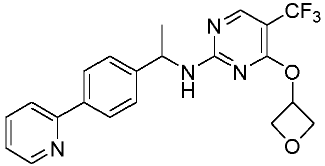 |
| **45** | 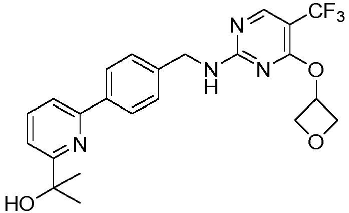 | **46** | 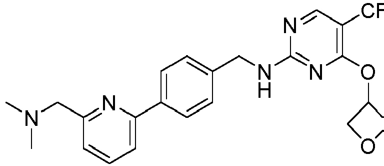 |
| **47** | 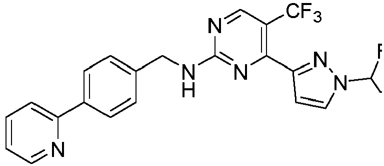 | **48** | 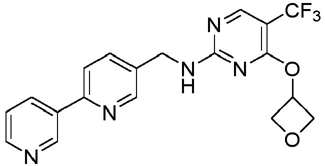 |
| **49** | 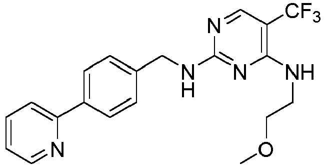 | **50** | 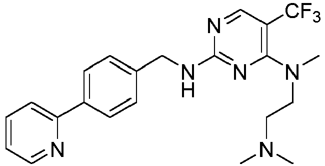 |
| **51** | 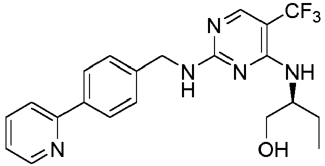 | **52** | 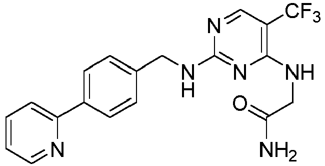 |
| **53** | 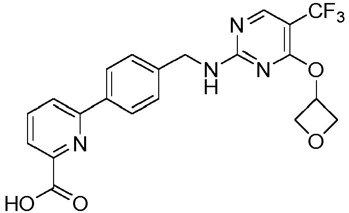 | **54** | 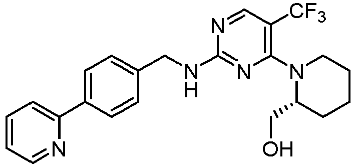 |
| **55** | 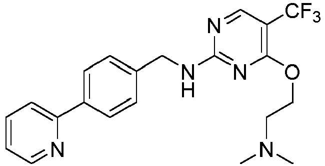 | **56** | 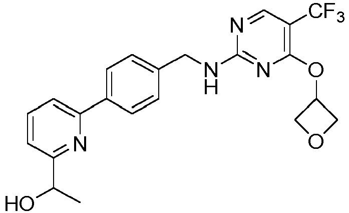 |
| **57** | 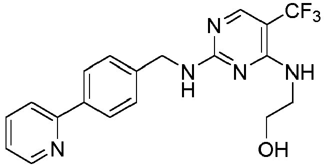 | **58** | 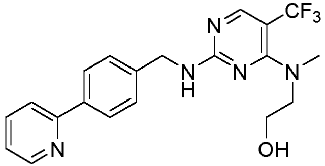 |
| **59** | 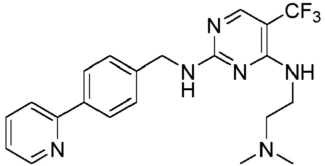 | **60** | 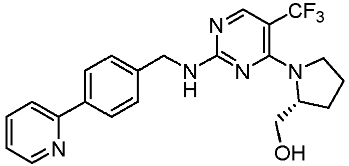 |
| **61** | 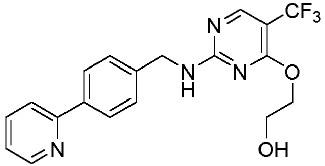 | **62** | 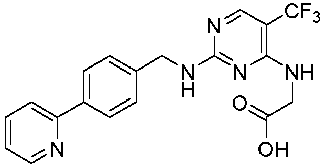 |
| **63** | 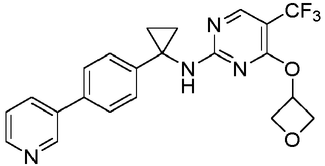 | **64** | 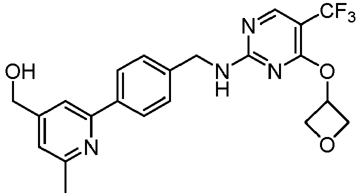 |
| **65** | 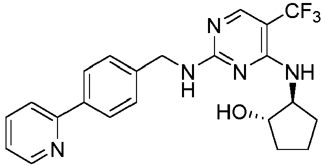 | **66** | 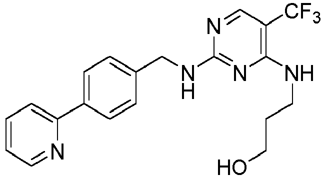 |
| **67** | 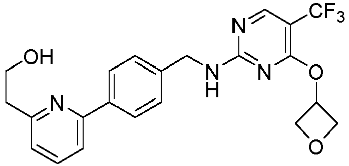 | **68** | 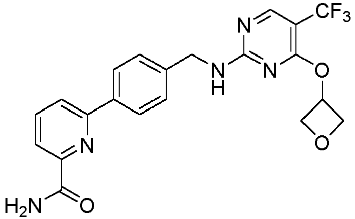 |
| **69** | 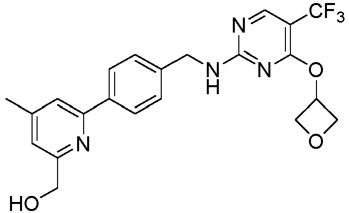 | **70** | 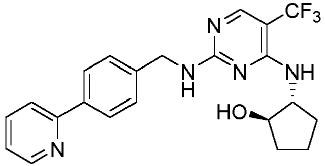 |
| **71** | 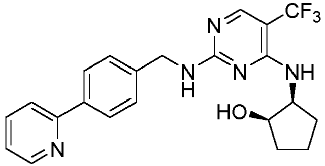 | **72** | 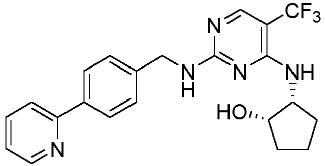 |
| **73** | 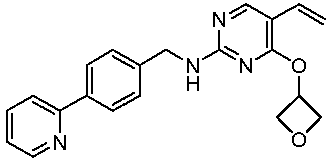 | **74** | 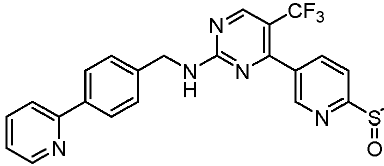 |
| **75** | 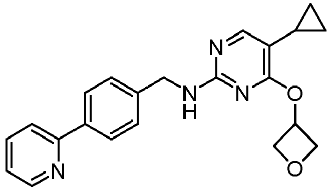 | **76** | 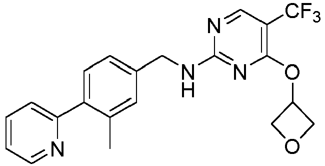 |
| **77** | 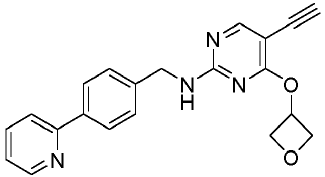 | **78** | 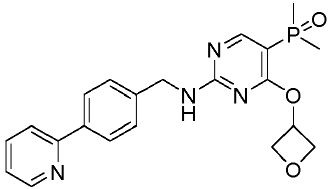 |
| **79** | 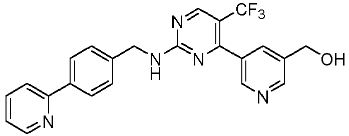 | **80** | 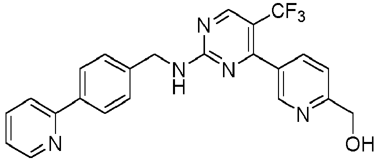 |
| **81** | 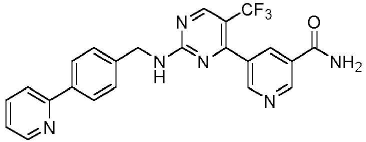 | **82** | 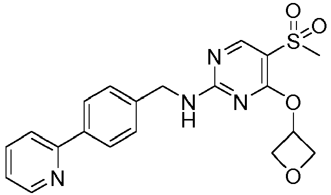 |
| **83** | 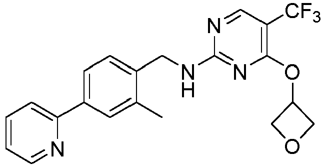 | **84** | 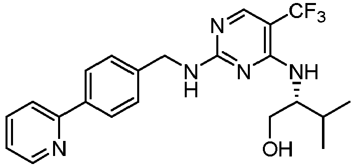 |
| **85** | 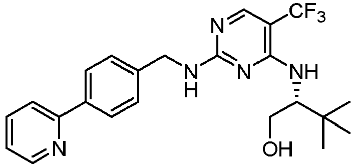 | **86** | 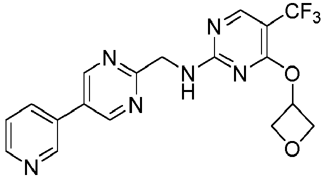 |
| **87** | 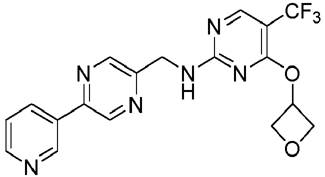 | **88** | 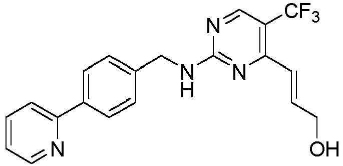 |
| **89** | 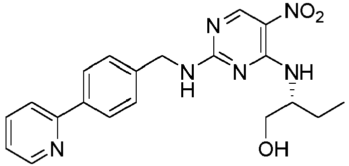 | **90** | 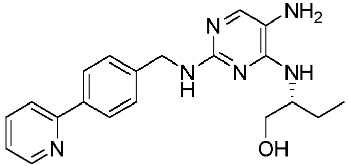 |
| **91** | 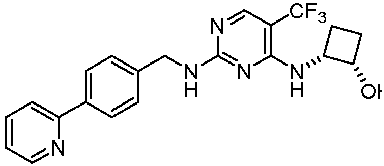 | **92** | 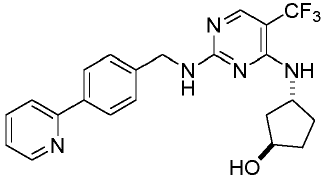 |
| **93** | 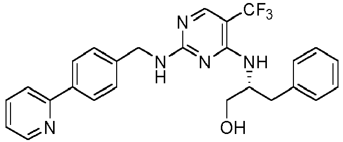 | **94** | 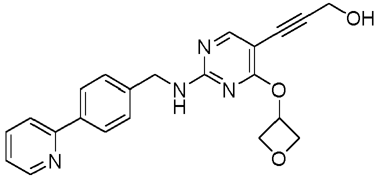 |
| **95** | 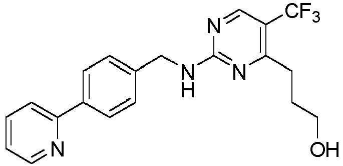 | **96** | 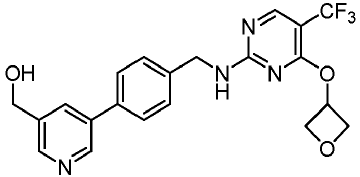 |
| **97** | 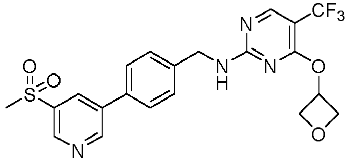 | **98** | 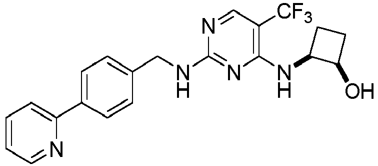 |
| **99** | 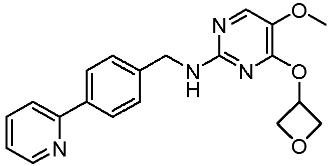 | **10 0** | 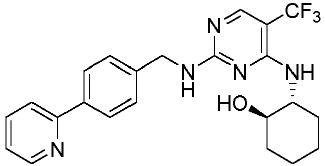 |
| **10 1** | 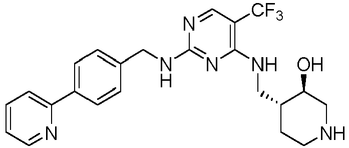 | **10 2** | 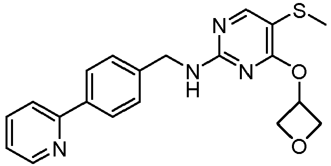 |
| **10 3** | 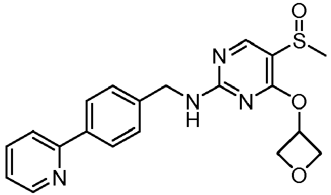 | **10 4** | 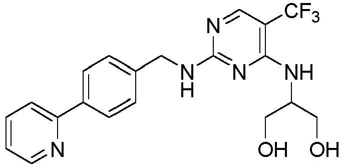 |
| **10 5** | 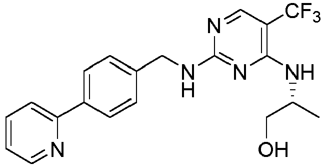 | **10 6** | 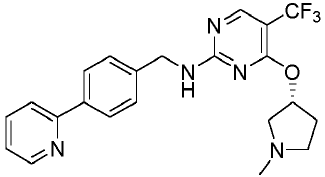 |
| **10 7** | 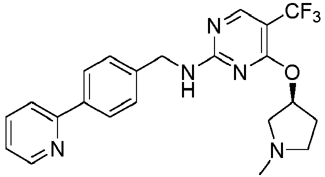 | **10 8** | 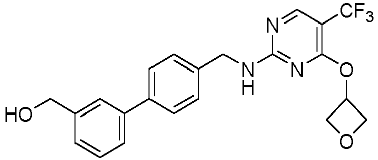 |
| **10 9** | 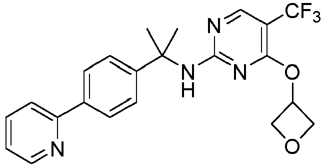 | **11 0** | 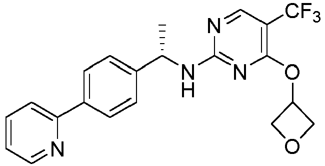 |
| **11 1** | 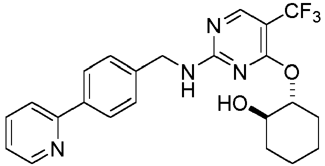 | **11 2** | 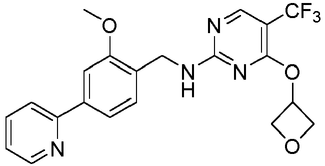 |
| **11 3** | 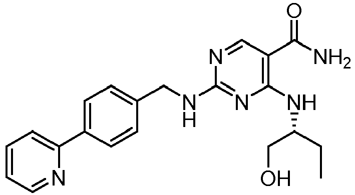 | **11 4** | 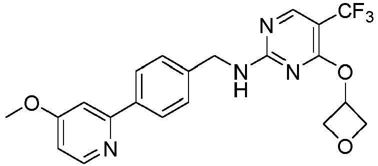 |
| **11 5** | 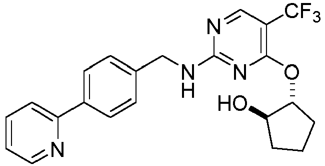 | **11 6** | 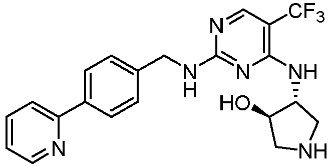 |
| **11 7** | 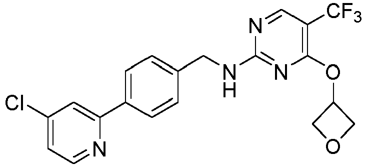 | **11 8** | 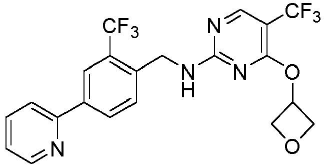 |
| **11 9** | 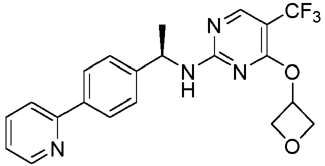 | **12 0** | 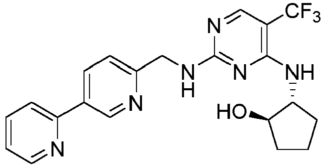 |
| **12 1** | 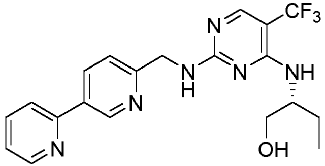 | **12 2** | 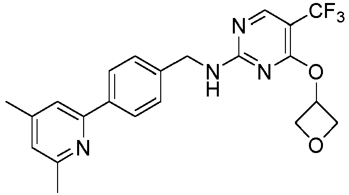 |
| **12 3** | 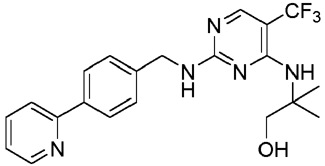 | **12 4** | 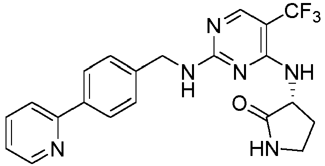 |
| **12 5** | 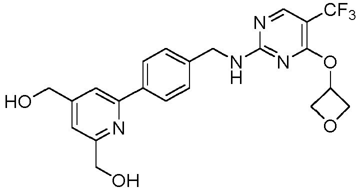 | **12 6** | 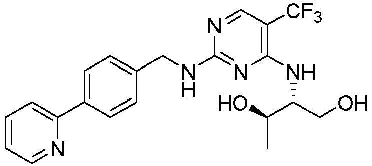 |
| **12 7** | 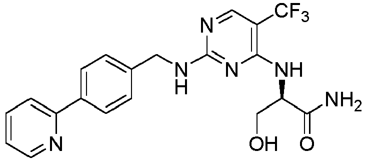 | **12 8** | 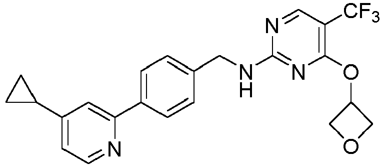 |
| **12 9** | 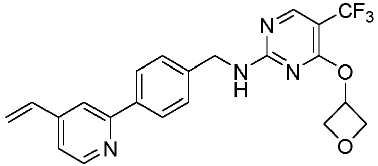 | **13 0** | 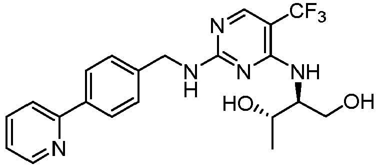 |
| **13 1** | 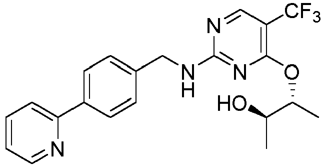 | **13 2** | 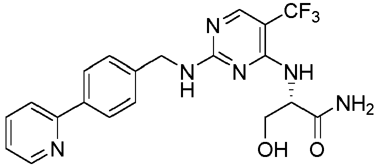 |
| **13 3** | 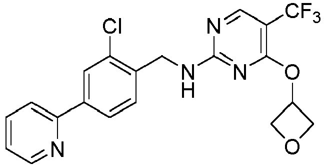 | **13 4** | 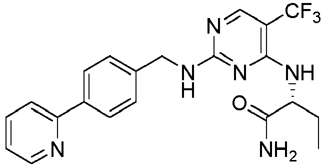 |
| **13 5** | 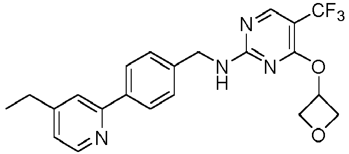 | **13 6** | 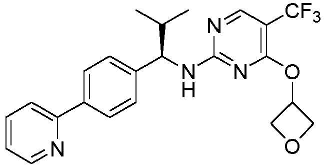 |
| **13 7** | 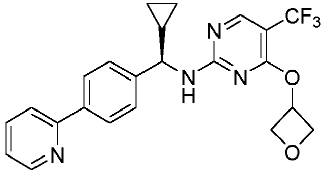 | **13 8** | 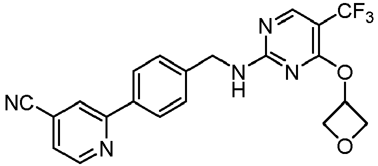 |
| **13 9** | 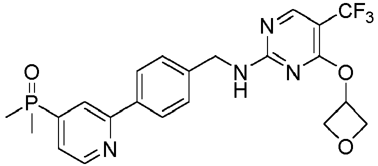 | **14 0** | 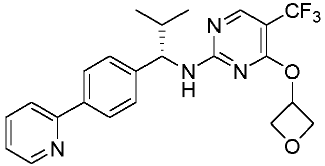 |
| **14 1** | 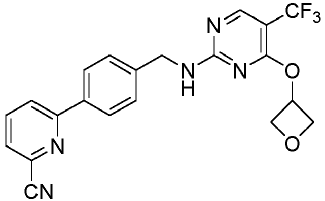 | **14 2** | 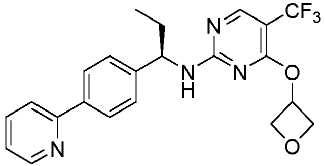 |
| **14 3** | 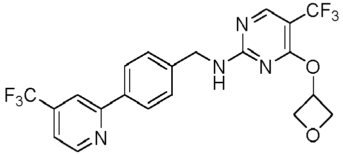 | **14 4** | 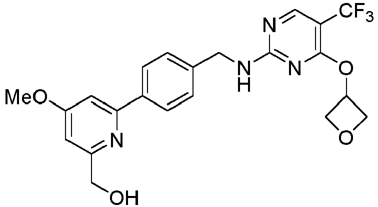 |
| **14 5** | 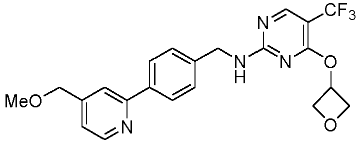 | **14 6** | 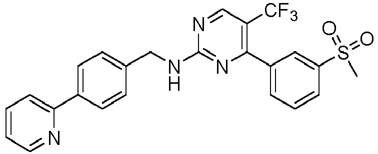 |
| **14 7** | 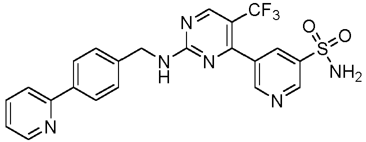 | **14 8** | 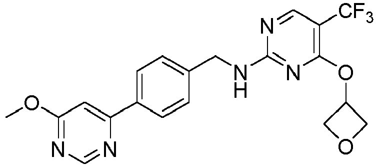 |
| **14 9** | 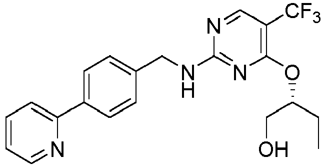 | **15 0** | 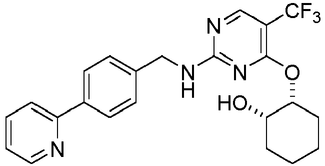 |
| **15 1** | 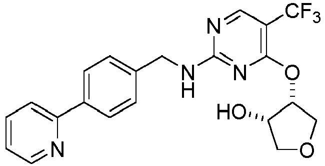 | **15 2** | 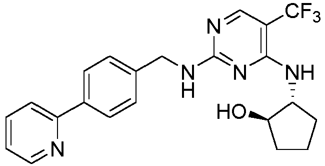 |
| **15 3** | 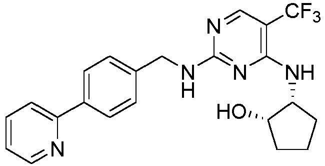 | **15 4** | 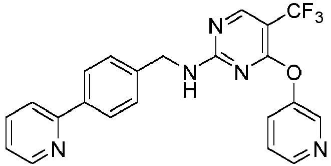 |
| **15 5** | 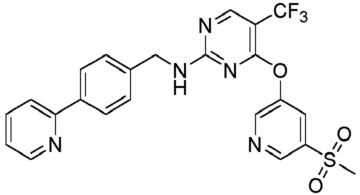 | **15 6** | 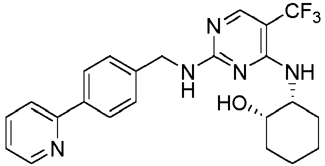 |
| **15 7** | 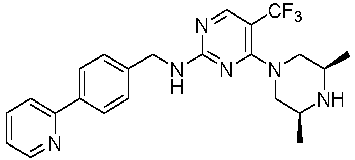 | **15 8** | 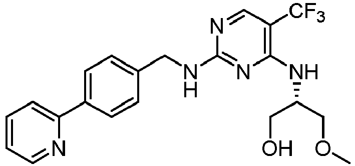 |
| **15 9** | 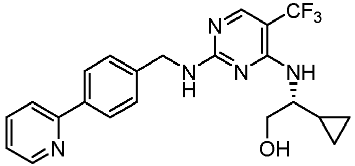 | **16 0** | 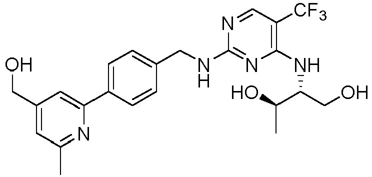 |
| **16 1** | 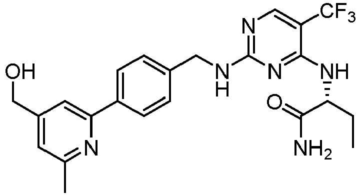 | **16 2** | 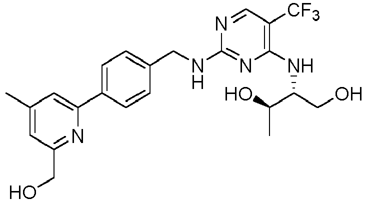 |
| **16 3** | 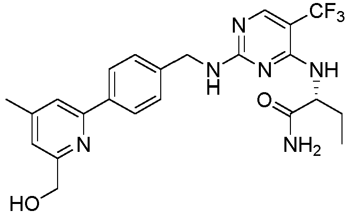 | **16 4** | 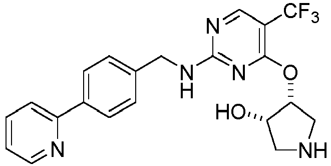 |
| **16 5** | 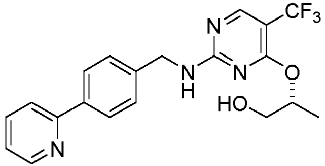 | **16 6** | 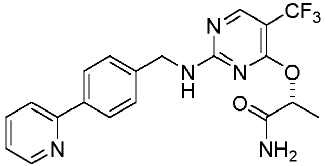 |
| **16 7** | 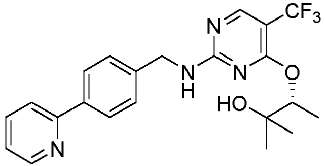 | **16 8** | 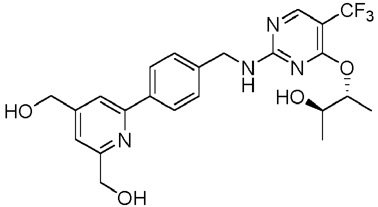 |
| **16 9** | 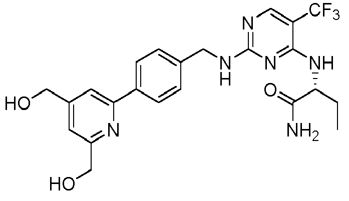 | **17 0** | 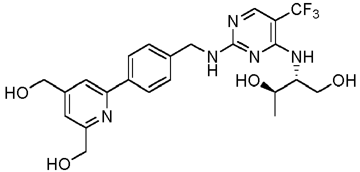 |
| **17 1** | 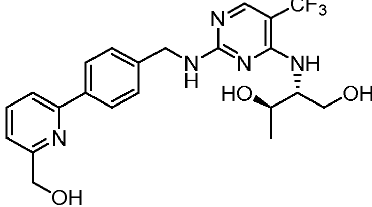 | **17 2** | 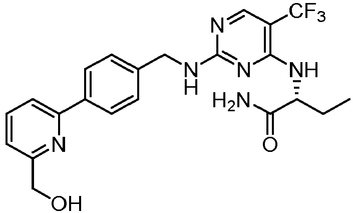 |
| **17 3** | 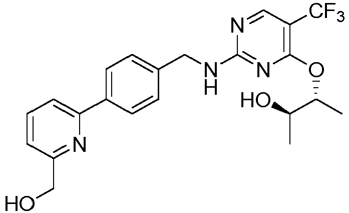 | **17 4** | 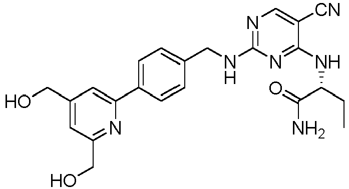 |
| **17 5** | 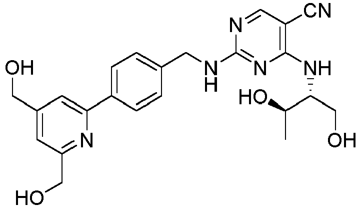 | **17 6** | 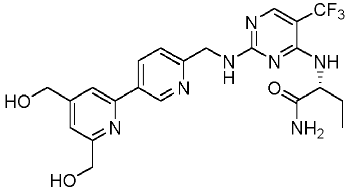 |
| **17 7** | 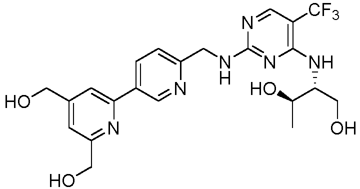 | **17 8** | 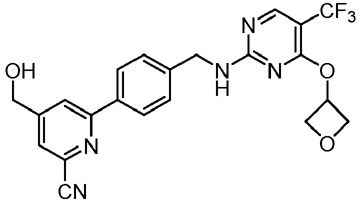 |
| **17 9** | 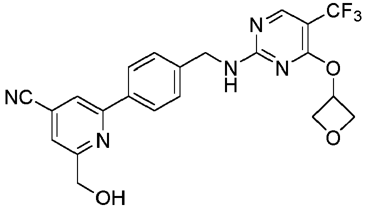 | **18 0** | 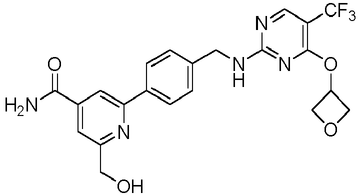 |
| **18 1** | 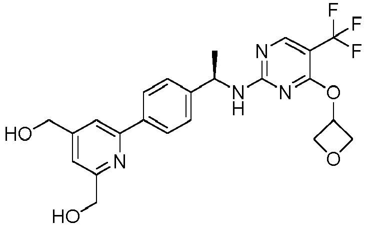 | **18 2** | 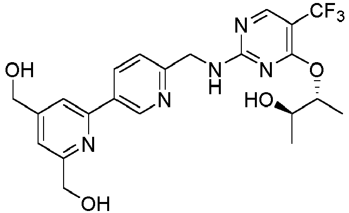 |
| **18 3** | 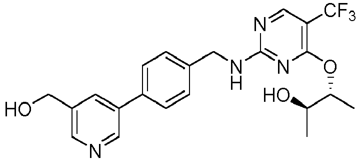 | **18 4** | 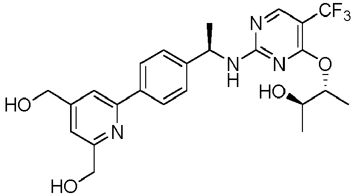 |
| **18 5** | 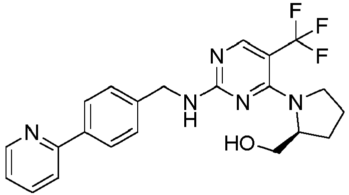 | **18 6** | 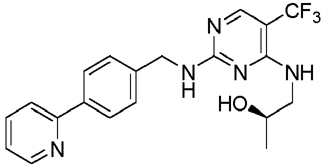 |
| **18 7** | 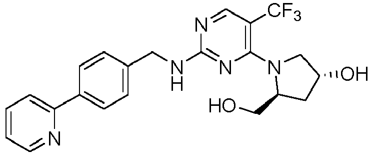 | **18 8** | 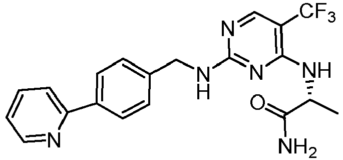 |
| **18 9** | 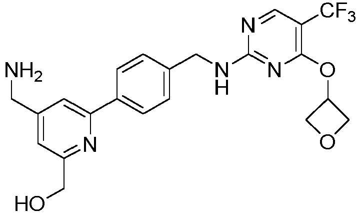 | **19 0** | 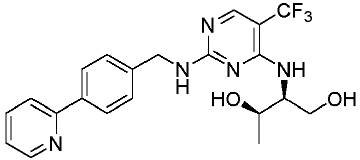 |
| **19 1** | 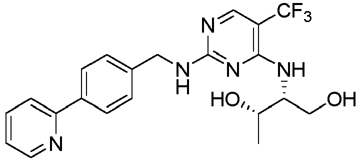 | **19 2** | 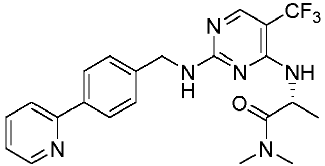 |
| **19 3** | 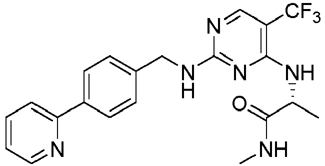 | **19 4** | 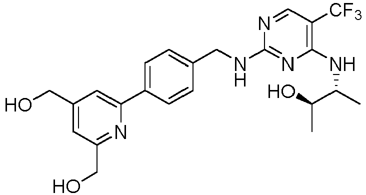 |
| **19 5** | 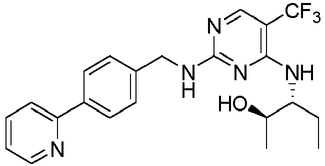 | **19 6** | 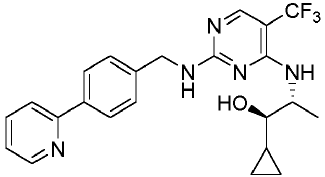 |
| **19 7** | 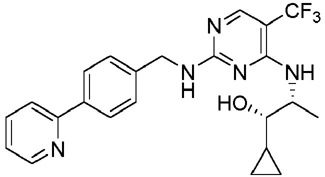 | **19 8** | 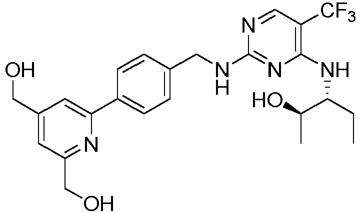 |
| **19 9** | 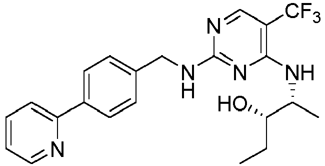 | **20 0** | 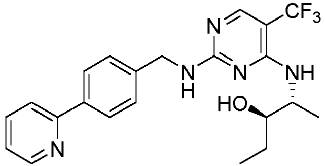 |
| **20 1** | 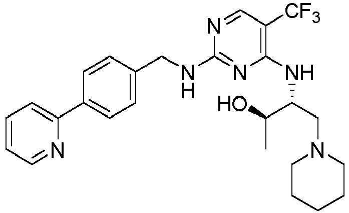 | **20 2** | 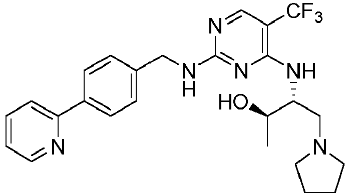 |
| **20 3** | 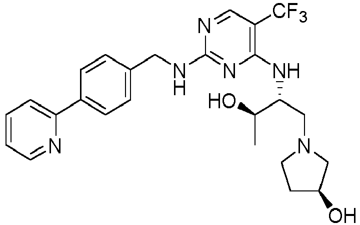 | **20 4** | 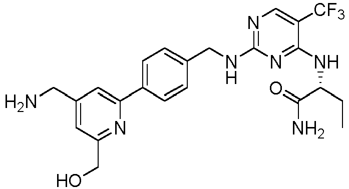 |
| **20 5** | 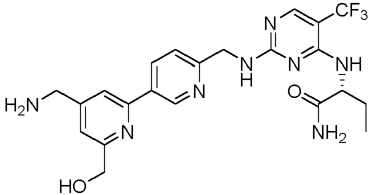 | **20 6** | 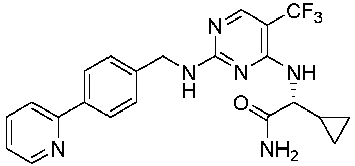 |
| **20 7** | 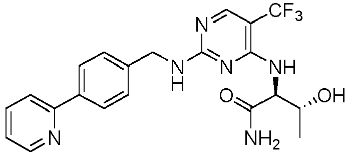 | **20 8** | 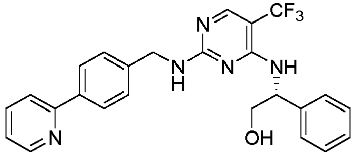 |
| **20 9** | 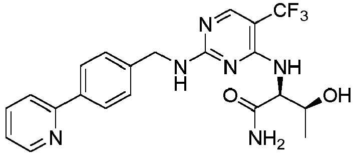 | **21 0** | 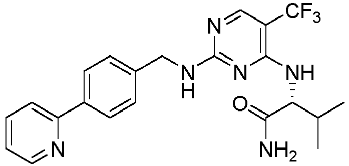 |
| **21 1** | 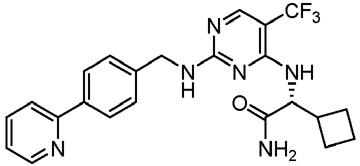 | **21 2** | 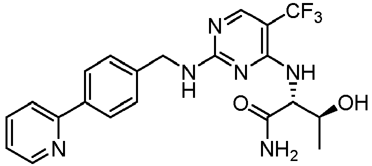 |
| **21 3** | 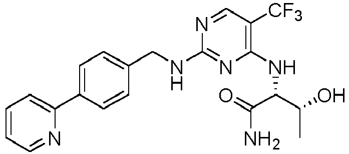 | **21 4** | 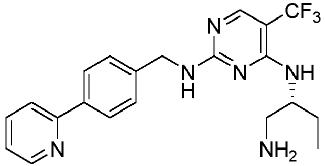 |
| **21 5** | 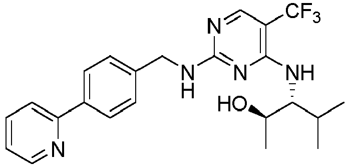 | **21 6** | 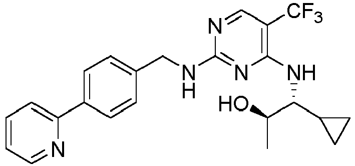 |
| **21 7** | 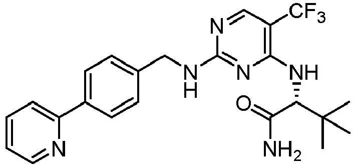 | **21 8** | 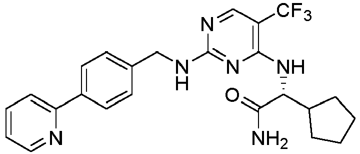 |
| **21 9** | 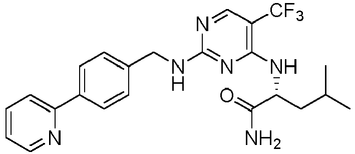 | **22 0** | 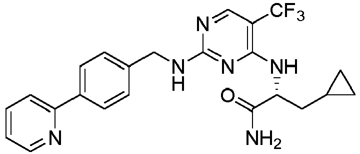 |
| **22 1** | 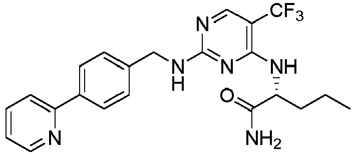 | **22 2** | 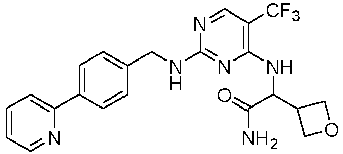 |
| **22 3** | 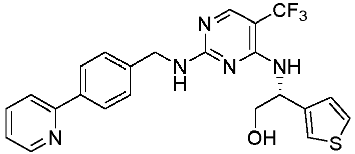 | **22 4** | 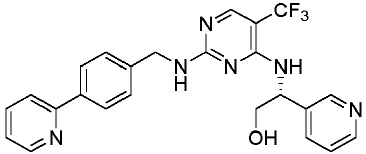 |

16. A pharmaceutical composition comprising a compound according to any one of the preceding claims or a pharmaceutically acceptable salt, prodrug, isotopic derivative, isomer, solvate or metabolite thereof, and optionally a pharmaceutically acceptable carrier.

17. Use of the compound according to any one of claims 1-15 or a pharmaceutically acceptable salt, prodrug, isotope derivative, isomer, solvate or metabolite thereof, or the pharmaceutical composition according to claim 16 in the preparation of medicaments for preventing or treating diseases or conditions related to Cyclin K protein.

18. The use according to claim 17, wherein the disease or disorder is selected from the group consisting of tumors, cancer, viral infections, inflammation-related diseases and autoimmune diseases.

19. A method for treating diseases or conditions related to Cyclin K protein, which comprises administering the compound according to any one of claims 1 to 15 or a pharmaceutically acceptable salt, prodrug, isotope derivative, isomer, solvate or metabolite thereof, or the pharmaceutical composition of claim 16 to a mammal in need thereof.
